# EUROPÄISCHE PATENTSCHRIFT

(11) **EP 4 232 443 B1**
(45) Veröffentlichungstag und Bekanntmachung des Hinweises auf die Patenterteilung: **18.12.2024**
(21) Anmeldenummer: 21790929.0
(22) Anmeldetag: 19.10.2021
(51) Int. Cl.: C07D 401/14, A01N 43/653, C07D 405/14

(54) **1-(PYRIDYL)-5-AZINYLPYRAZOL DERIVATE UND DEREN VERWENDUNG ZUR BEKÄMPFUNG UNERWÜNSCHTEN PFLANZENWACHSTUMS**
1-(PYRIDYL)-5-AZINYLPYRAZOL DERIVATIVES AND USE OF SAME FOR COMBATING UNDESIRED PLANT GROWTH
DÉRIVÉS DE 1-(PYRIDYL)-5-AZINYLPYRAZOLE ET LEUR UTILISATION POUR LUTTER CONTRE LA CROISSANCE VÉGÉTALE INDÉSIRABLE

(30) Priorität: 23.10.2020 EP 20203487
(43) Veröffentlichungstag der Anmeldung: 30.08.2023
(73) Patentinhaber: Bayer Aktiengesellschaft, 51373 Leverkusen (DE)
(72) Erfinder: BUSCATO, Estella, 08174 Sant Cugat del Valles (ES); HOFFMANN, Michael Gerhard, 78467 Konstanz (DE); JAKOBI, Harald, 60598 Frankfurt (DE); MUELLER, Thomas, 60323 Frankfurt (DE); BOLLENBACH-WAHL, Birgit, 55413 Weiler/Bingen (DE); DITTGEN, Jan, 60316 Frankfurt (DE); GATZWEILER, Elmar, 61231 Bad Nauheim (DE); REINGRUBER, Anna Maria, 64646 Heppenheim (DE)
(74) Vertreter: BIP Patents
(86) Internationale Anmeldenummer: PCT/EP2021/078878
(87) Internationale Veröffentlichungsnummer: WO 2022/084278

(56) Entgegenhaltungen:
- EP-A1- 0 007 019
- EP-A1- 0 333 131
- EP-A1- 1 101 764
- WO-A2-2008/083233
- CN-A- 101 284 815
- SALANOUVE ELISE ET AL: "Few unexpected results from a Suzuki-Miyaura reaction", TETRAHEDRON, vol. 68, no. 9, 16 January 2012 (2012-01-16), AMSTERDAM, NL, pages 2135 - 2140, XP055784669, ISSN: 0040-4020, DOI: 10.1016/j.tet.2012.01.019

## Beschreibung

Die Erfindung betrifft das technische Gebiet der Pflanzenschutzmittel, insbesondere das der Herbizide zur selektiven Bekämpfung von Unkräutern und Ungräsern in Nutzpflanzenkulturen sowie im Ziergartenbereich und zur generellen Bekämpfung von Unkräutern und Ungräsern in Umweltbreich, in denen Pflanzenwuchs störend ist.

Die Erfindung betrifft substituierte 1-Pyridyl-5-azinylpyrazolyl-3-oxyalkylsäuren, 1-Pyridyl-5-azinylpyrazolyl-3-sulfanylalkylsäuren, 1-Pyridyl-5-azinylpyrazolyl-3-sulfinylalkylsäuren und 1-Pyridyl-5-azinylpyrazolyl-3-sulfonylalkylsäuren sowie deren Derivate, Verfahren zu deren Herstellung und deren Verwendung zur Bekämpfung von Schadpflanzen.

Die Derivate der 1-Pyridyl-5-azinylpyrazolyl-3-oxyalkylsäuren, 1-Pyridyl-5-azinylpyrazolyl-3-sulfanylalkylsäuren, 1-Pyridyl-5-azinylpyrazolyl-3-sulfinylalkylsäuren und 1-Pyridyl-5-azinylpyrazolyl-3-sulfonylalkylsäuren umfassen insbesondere deren Ester, Salze und/oder Amide.

Die erfindungsgemäßen 1-Pyridyl-5-azinylpyrazole zeichnen sich dadurch aus, dass sie einen weiteren Substituenten in der 4-Position des Pyrazolrings aufweisen.

Die erfindungsgemäßen 1-Pyridyl-5-azinylpyrazolyl-3-oxyalkylsäuren sowie deren Derivate unterscheiden sich von den bereits bekannten 1,5-Diphenyl-pyrazolyl-3-oxoessigsäuren durch einen Pyridylrest (Q1-Q3) in 1-Position und einem variablen Azinylrest (A1-A15) in 5-Position des Pyrazolrings.

Aus dem Stand der Technik sind biologische Wirkungen von substituierten 1,5-Diphenyl-pyrazolyl-3-oxoessigsäuren sowie Verfahren zur Herstellung dieser Verbindungen bekannt. In DE 2828529 A1 werden die Herstellung und die lipidsenkende Wirkung von 1,5-Diphenyl-pyrazolyl-3-oxoessigsäuren beschrieben.

Als bakterizid wirksame Agrochemikalien werden 1,5-Diphenyl-pyrazolyl-3-oxoessigsäuren in CN 101284815 offenbart. In Journal of Heterocyclic Chemistry (2012), 49(6), 1370-1375 werden weitere Synthesen und die fungizide Wirkung von 1,5-Diphenyl-pyrazolyl-3-oxoessigsäuren beschrieben.

In WO 2008083233 A2 werden in der 4 Position des Pyrazols substituierte 1,5-Diphenyl-pyrazolyl-3-oxyalkylsäuren sowie deren Derivate als Substanzen, die zum Aufbrechen von Zellaggregaten geeignet sind, beschrieben. Spezifisch offenbart wird Ethyl-(4-chlor-1,5-diphenyl-1H-pyrazol-3-yl)oxy]acetat.

Darüber hinaus ist die Synthese einiger 4-Chlor-1,5-diphenylpyrazolyl-3-oxyessigsäuren sowie deren Ethylester in European Journal of Organic Chemistry (2011), 2011 (27), 5323-5330 beschrieben.

EP 0 333 131 A1 werden pflanzenschützende Mittel auf Basis von Pyrazolcarbonsäurederivaten offenbart, welche sich zum Schutz vor den phototoxischen Effekten von Herbiziden eigenen.

EP 1 101 764 A1 offenbart 2-Aryloxy-4-methyl-6-pyrazol-1-yl-Pyridine, welche wirksam als Herbizide sind.

Dagegen sind 1-Pyridyl-5-azinylpyrazolyl-3-oxyessigsäuren, 1-Pyridyl-5-azinylpyrazolyl-3-oxyalkylsäuren, 1-Pyridyl-5-azinylpyrazolyl-3-sulfanylalkylsäuren, 1-Pyridyl-5-azinylpyrazolyl-3-sulfinylalkylsäuren und 1-Pyridyl-5-azinylpyrazolyl-3-sulfonylalkylsäuren, sowie deren Derivate, bislang unbekannt.

Die Aufgabe der vorliegenden Erfindung besteht in der Bereitstellung neuer Pyrazol-Derivate, nämlich von 1-Pyridyl-5-azinylpyrazolyl-3-oxyalkylsäuren, 1-Pyridyl-5-azinylpyrazolyl-3-sulfanylalkylsäuren, 1-Pyridyl-5 -azinylpyrazolyl-3 -sulfinylalkylsäuren und 1-Pyridyl-5 -azinylpyrazolyl-3 -sulfonylalkylsäuren und deren Derviaten, welche als Herbizide oder Pflanzenwachstumsregulatoren, mit einer zufriedenstellenden herbiziden Wirkung und einem breiten Wirkspektrum gegenüber Schadpflanzen und/oder mit einer hohen Selektivität in Nutzpflanzenkulturen, eingesetzt werden können.

Gelöst wird die Aufgabe durch substituierte 1-Pyridyl-5-azinylpyrazolyl-3-oxoalkylsäuren, 1-Pyridyl-5-azinylpyrazolyl-3-sulfanylalkylsäuren, 1 -Pyridyl-5 -azinylpyrazolyl-3 -sulfinylalkylsäuren und 1-Pyridyl-5-azinylpyrazolyl-3-sulfonylalkylsäuren, die sich durch einen Pyridylrest in der 1-Position, einen Azinylrest in der 5-Position und einen weiteren Substituenten in der 4-Position des Pyrazolrings auszeichnen und eine sehr gute herbizide Wirkung und darüber hinaus auch sehr gute Selektivitäten aufweisen.

Überraschenderweise sind diese Verbindungen gegen eine große Bandbreite wirtschaftlich wichtiger Ungräser und Unkräuter hochwirksam. Die Verbindungen zeigen zugleich eine gute Verträglichkeit gegenüber Kulturpflanzen. Somit können diese bei guter Wirksamkeit gegen Schadpflanzen selektiv in Kulturpflanzen eingesetzt werden.

Gegenstand der vorliegenden Erfindung ist daher die Bereitstellung von substituierten 1-Pyridyl-5-azinylpyrazolyl-3-oxyalkylsäuren, 1-Pyridyl-5-azinylpyrazolyl-3-sulfanylalkylsäuren, 1-Pyridyl-5-azinylpyrazolyl-3-sulfinylalkylsäuren und 1-Pyridyl-5-azinylpyrazolyl-3-sulfonylalkylsäuren der allgemeinen Formel (I) 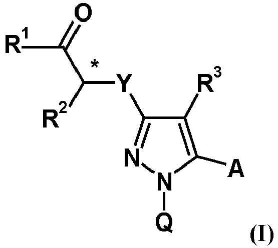 und deren agrochemisch verträglichen Salze, wobei
- A: ausgewählt ist aus der Gruppe, bestehend aus A1-A15 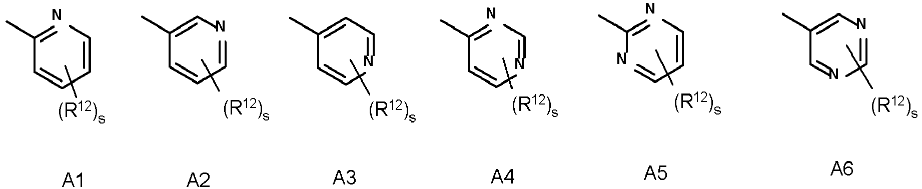 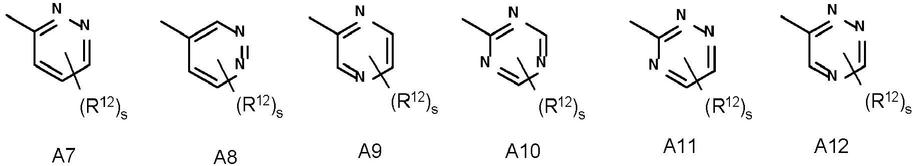 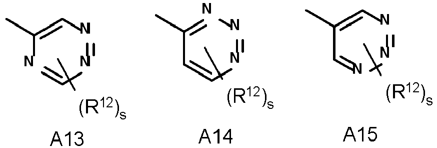
- Q: ausgewählt ist aus der Gruppe, bestehend aus Q1-Q3 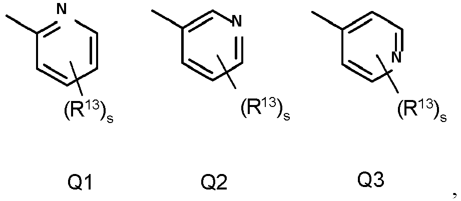
- R¹: OR^{1a}, NR⁹R¹⁰ oder O(CH₂)ₓO(CH₂)_{y}O(CH₂)_{z} H bedeutet,
- R^{1a}: Wasserstoff bedeutet oder (C₁-C₆)-Alkyl, (C₃-C₆)-Cycloalkyl bedeutet, welches jeweils unsubstituiert oder substituiert ist durch einen oder mehrere Substituenten ausgewählt aus der Gruppe bestehend aus Halogen, (C₁-C₆)-Alkyl, (C₁-C₆)-Halogenalkyl, (C₃-C₆)-Cycloalkyl, (C₁-C₆)-Alkoxy, -N=R⁵, Cyano und Nitro oder (C₂-C₄)-Alkenyl, (C₂-C₄)-Alkinyl bedeutet oder (C₁-C₆)-Alkyl-SO-(C₁-C₆)-Alkyl-, (C₁-C₆)-Alkyl-SO₂-(C₁-C₆)-Alkyl- bedeutet oder Heterocyclyl, Heteroaryl, Aryl bedeutet oder Heterocyclyl-(C₁₋C₄)-Alkyl-, Heteroaryl-(C₁₋C₄)-Alkyl-, Aryl-(C₁₋C₄)-Alkyl- bedeutet, welches jeweils unsubstituiert oder substituiert ist durch einen oder mehrere Substituenten ausgewählt aus der Gruppe bestehend aus Halogen, (C₁-C₆)-Alkyl, (C₁-C₆)-Haloalkyl;
- R⁹: Wasserstoff, (C₁-C₁₂)-Alkyl bedeutet;
- R¹⁰: Wasserstoff, Aryl, Heteroaryl, Heterocyclyl, (C₁-C₁₂)-Alkyl, (C₃-C₈)-Cycloalkyl, (C₃-C₈)-Cycloalkyl-(C₁-C₇)-Alkyl-, (C₂-C₁₂)-Alkenyl, (C₅-C₇)-Cycloalkenyl, (C₂-C₁₂)-Alkinyl, S(O)ₙR⁵, Cyano, Nitro, OR⁵, SO₂NR⁶R⁷, CO₂R⁸, COR⁸, NR⁶R⁸, NR⁶COR⁸, NR⁶CO₂R⁸, NR⁶SO₂R⁸ bedeutet, wobei die oben genannten Alkyl, Cycloalkyl, Alkenyl, Cycloalkenyl und Alkinyl Reste unsubstituiert sind oder jeweils unabhängig voneinander substituiert sind durch "m" Reste ausgewählt aus der Gruppe bestehend aus Wasserstoff, gegebenenfalls einfach oder mehrfach substituiertes Aryl, Halogen, Cyano, Nitro, OR⁵, S(O)ₙR⁵, SO₂NR⁶R⁷, CO₂R⁸, CONR⁶R⁸, COR⁶, NR⁶R⁸, NR⁶COR⁸, NR⁶CONR⁸R⁸, NR⁶CO2R⁸, NR⁶SO₂R⁸, NR⁶SO₂NR⁶R⁸, C(R⁶)=NOR⁸;
oder
- R⁹ und R¹⁰: bilden mit dem Stickstoffatom, an das sie gebunden sind, einen gegebenenfalls ein- bis sechsfach durch Reste aus der Gruppe bestehend aus Halogen, (C₁-C₆)-Alkyl, (C₁-C₆)-Halogenalkyl, OR⁵, S(O)ₙR⁵, CO₂R⁸, CONR⁶R⁸, COR⁶ und C(R⁶)=NOR⁸ substituierten, gesättigten, teilweise oder vollständig ungesättigten fünf-, sechs- oder siebengliedrigen Ring, der neben diesem Stickstoffatom "r" Kohlenstoffatome, "o" Sauerstoffatome, "p" Schwefelatome und "q" Elemente aus der Gruppe bestehend aus NR⁷, CO und NCOR⁷ als Ringatome enthält;
- R⁵: (C₁-C₆)-Alkyl, (C₃-C₆)-Cycloalkyl, (C₁-C₆)-Halogenalkyl, Aryl bedeutet;
- R⁶: Wasserstoff, (C₁-C₆)-Alkyl, (C₃-C₆)-Cycloalkyl, (C₁-C₆)-Halogenalkyl, Aryl bedeutet;
- R⁷: Wasserstoff, (C₁-C₆)-Alkyl, (C₃-C₆)-Cycloalkyl, (C₃-C₄)-Alkenyl, (C₃-C₄)-Alkinyl bedeutet;
- R⁸: Wasserstoff, (C₁-C₆)-Alkyl, (C₃-C₆)-Cycloalkyl, (C₃-C₄)-Alkenyl, (C₁-C₆)-Alkyl-COO(C₁-C₂)-Alkyl oder (C₃-C₄)-Alkinyl bedeutet;
- R²: Wasserstoff, Cyano, (C₁-C₆)-Alkyl, (C₁-C₆)-Alkoxy-(C₁-C₆)-Alkyl-, (C₁-C₆)-Halogenalkyl, (C₁-C₆)-Alkoxy, (C₂-C₆)-Alkenyl, (C₂-C₆)-Halogenalkenyl, (C₂-C₆)-Alkinyl, (C₂-C₆)-Halogenalkinyl, (C₃- C₆)-Cycloalkyl bedeutet;
- R³: Halogen, Cyano, Isocyano, NO₂, (C₃-C₆)-Cycloalkyl, (C₁-C₆)-Alkyl, (C₁-C₆)-Halogenalkyl, (C₁-C₆)-Alkylcarbonyl-, (C₁-C₆)-Halogenalkylcarbonyl-, (C₁-C₆)-Alkyloxycarbonyl-, (C₂- C₃)-Alkenyl, (C₂- C₃)-Halogenalkenyl, (C₂-C₃)-Alkinyl, (C₂- C₃)-Halogenalkinyl, (C₁-C₆)-Alkyl-S(O)ₙ und (C₁-C₆)-Halogenalkyl-S(O)ₙ, CHO, NH₂ bedeutet;
- R¹²: Halogen, Cyano, Isocyano, NO₂, (C₁-C₆)-Alkyl, (C₁-C₆)-Halogenalkyl, (C₁-C₆)-Alkylcarbonyl, (C₁-C₆)-Halogenalkylcarbonyl, (C₁-C₆)-Alkyloxycarbonyl, (C₁-C₆)-Alkoxy, (C₁-C₆)-Halogenalkoxy, (C₁-C₆)-Alkyl-, S(O)ₙ, (C₂- C₃)-Alkenyl, (C₂- C₃)-Halogenalkenyl, (C₂-C₃)-Alkinyl, (C₂- C₃)-Halogenalkinyl, NH₂ bedeutet;
- R¹³: Halogen, Cyano, Isocyano, NO₂, (C₁-C₆)-Alkyl, (C₁-C₆)-Halogenalkyl, (C₁-C₆)-Alkylcarbonyl, (C₁-C₆)-Halogenalkylcarbonyl, (C₁-C₆)-Alkyloxycarbonyl, (C₁-C₆)-Alkoxy, (C₁-C₆)-Halogenalkoxy, (C₁-C₆)-Alkyl- S(O)ₙ, (C₂- C₃)-Alkenyl, (C₂- C₃)-Halogenalkenyl; (C₂-C₃)-Alkinyl, (C₂- C₃)-Halogenalkinyl, NH₂ bedeutet;
- Y: Sauerstoff, S(O)ₙ bedeutet;
- m: 0, 1 oder 2 bedeutet;
- n: 0, 1 oder 2 bedeutet;
- o: 0, 1 oder 2 bedeutet;
- p: 0 oder 1 bedeutet;
- q: 0 oder 1 bedeutet;
- r: 3, 4, 5 oder 6 bedeutet;
- s: 0, 1, 2, 3 oder 4 bedeutet;
- x: 1,2,3,4 oder 5 bedeutet:
- y: 1,2,3,4 oder 5 bedeutet;
- z: 1,2,3,4 oder 5 bedeutet.

Im Folgenden werden, jeweils für die einzelnen Substituenten, bevorzugte, besonders bevorzugte und ganz besonders bevorzugte Bedeutungen beschrieben. Die übrigen Substituenten der allgemeinen Formel (I), welche nachfolgend nicht genannt werden, weisen die oben genannte Bedeutung auf.

Somit ergeben sich verschiedene Ausführungsformen für die Verbindung der allgemeinen Formel (I).

Bevorzugt sind Verbindungen der allgemeinen Formel (I), in denen
- A: ausgewählt ist aus der Gruppe, bestehend aus A1- A4, A6-A9, und A12-A15 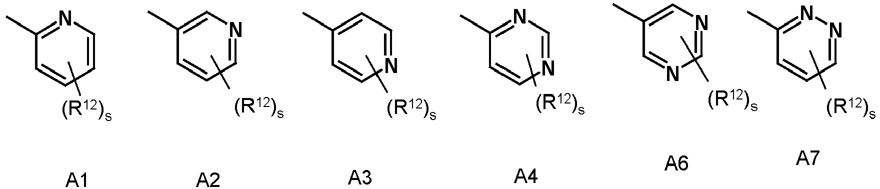 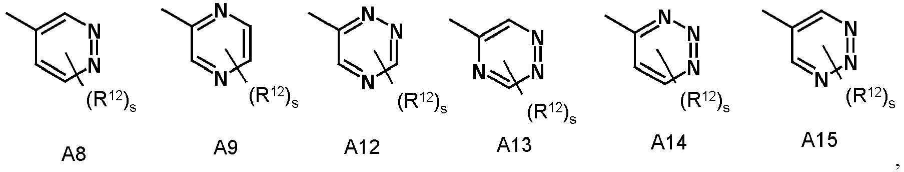
- Q: ausgewählt ist aus der Gruppe, bestehend aus Q1-Q3 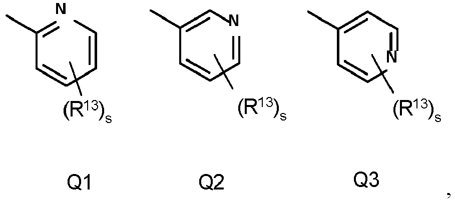
- R¹: OR^{1a}, NR⁹R¹⁰ oder O(CH₂)ₓO(CH₂)_{y}O(CH₂)_{z} H bedeutet,
- R^{1a}: Wasserstoff bedeutet oder (C₁-C₆)-Alkyl, (C₃-C₆)-Cycloalkyl bedeutet welches jeweils unsubstituiert oder substituiert ist und durch einen oder mehrere Substituenten ausgewählt aus der Gruppe bestehend aus Halogen, (C₁-C₆)-Alkyl, (C₁-C₆)-Halogenalkyl, (C₃-C₆)-Cycloalkyl, (C₁-C₆)-Alkoxy, Cyano und Nitro oder (C₂-C₄)-Alkenyl, (C₂-C₄)- Alkinyl bedeutet oder (C₁-C₆)-Alkyl-SO-(C₁-C₆)-Alkyl-, (C ₁-C₆)-Alkyl-SO₂-(C₁-C₆)-Alkyl-, Aryl-(C₁₋C₄)-Alkyl-bedeutet, welches jeweils unsubstituiert oder substituiert ist durch einen oder mehrere Substituenten ausgewählt aus der Gruppe bestehend aus Halogen, (C₁-C₆)-Alkyl, (C₁-C₆)-Halogenalkyl;
- R⁹: Wasserstoff, (C₁-C₆)-Alkyl bedeutet;
- R¹⁰: Wasserstoff, Phenyl, (C₁-C₆)-Alkyl, (C₃-C₆)-Cycloalkyl, (C₃-C₆)-Cycloalkyl-(C₁-C₄)-Alkyl-, (C₂-C₄)-Alkenyl, (C₅-C₇)-Cycloalkenyl, (C₂-C₄)-Alkinyl, S(O)ₙR⁵, Cyano, Nitro, OR⁵, SO₂NR⁶R⁷, CO₂R⁸, COR⁸, NR⁶R⁸ bedeutet, wobei die oben genannten Alkyl, Cycloalkyl, Alkenyl, Cycloalkenyl und Alkinyl Reste unsubstituiert sind oder jeweils unabhängig voneinander substituiert sind durch "m" Reste ausgewählt aus der Gruppe bestehend aus gegebenenfalls einfach oder mehrfach substituiertes Phenyl, Halogen, Cyano, Nitro, OR⁵, S(O)ₙR⁵, SO₂NR⁶R⁷, CO₂R⁸, CONR⁶R⁸, COR⁶, NR⁶R⁸, NR⁶COR⁸, NR⁶CONR⁸R⁸, NR⁶CO₂R⁸ oder
- R⁹ und R¹⁰: bilden mit dem Stickstoffatom, an das sie gebunden sind, einen gegebenenfalls ein- bis sechsfach durch Reste aus der Gruppe bestehend aus Halogen, (C₁-C₆)-Alkyl, (C₁-C₆)-Halogenalkyl, OR⁵, S(O)ₙR⁵, CO₂R⁸, CONR⁶R⁸, COR⁶ und C(R⁶)=NOR⁸ substituierten, gesättigten, teilweise oder vollständig ungesättigten fünf-, sechs- oder siebengliedrigen Ring, der neben diesem Stickstoffatom "r" Kohlenstoffatome, "o" Sauerstoffatome, "p" Schwefelatome und "q" Elemente aus der Gruppe bestehend aus NR⁷, CO und NCOR⁷ als Ringatome enthält;

- R⁵: (C₁-C₆)-Alkyl, (C₃-C₆)-Cycloalkyl, (C₁-C₆)-Halogenalkyl oder Phenyl bedeutet;
- R⁶: Wasserstoff, (C₁-C₆)-Alkyl, (C₃-C₆)-Cycloalkyl, (C₁-C₆)-Halogenalkyl oder Phenyl bedeutet;
- R⁷: Wasserstoff, (C₁-C₆)-Alkyl, (C₃-C₆)-Cycloalkyl, (C₃-C₄)-Alkenyl oder (C₃-C₄)-Alkinyl bedeutet;
- R⁸: Wasserstoff, (C₁-C₆)-Alkyl, (C₃-C₆)-Cycloalkyl, (C₃-C₄)-Alkenyl oder (C₃-C₄)-Alkinyl bedeutet;

- R²: Wasserstoff, Cyano, (C₁-C₆)-Alkyl, (C₁-C₆)-Alkoxy-(C₁-C₆)-Alkyl, (C₁-C₆)-Halogenalkyl, (C₁-C₆)-Alkoxy, (C₂-C₄)-Alkenyl, (C₂-C₄)-Halogenalkenyl; (C₂-C₄)-Alkinyl, (C₂-C₄)-Halogenalkinyl; (C₃-C₆)-Cycloalkyl bedeutet;

- R³: Halogen, Cyano, Isocyano, NO₂, (C₃-C₆)-Cycloalkyl, (C₁-C₆)-Alkyl, (C₁-C₆)-Halogenalkyl, (C₁-C₆)-Alkylcarbonyl, (C₁-C₆)-Halogenalkylcarbonyl, (C₁-C₆)-Alkyloxycarbonyl, (C₂-C₃)-Alkenyl, (C₂-C₃)-Halogenalkenyl, (C₂-C₃)-Alkinyl, (C₂- C₃)-Halogenalkinyl, (C₁-C₆)-Alkyl-S(O)ₙ-, (C₁-C₆)-Halogenalkyl-S(O)ₙ₋, CHO, NH₂ bedeutet;

- R¹²: Halogen, Cyano, Isocyano, NO₂, (C₁-C₆)-Alkyl, (C₁-C₆)-Halogenalkyl, (C₁-C₆)-Alkylcarbonyl, (C₁-C₆)-Halogenalkylcarbonyl, (C₁-C₆)-Alkyloxycarbonyl, (C₁-C₆)-Alkoxy, (C₁-C₆)-Halogenalkoxy, (C₁-C₆)-Alkyl- S(O)ₙ, (C₂- C₃)-Alkenyl, (C₂- C₃)-Halogenalkenyl, (C₂-C₃)-Alkinyl, (C₂- C₃)-Halogenalkinyl, NH₂ bedeutet;

- R¹³: Halogen, Cyano, Isocyano, NO₂, (C₁-C₆)-Alkyl, (C₁-C₆)-Halogenalkyl, (C₁-C₆)-Alkylcarbonyl, (C₁-C₆)-Halogenalkylcarbonyl, (C₁-C₆)-Alkyloxycarbonyl, (C₁-C₆)-Alkoxy, (C₁-C₆)-Halogenalkoxy, (C₁-C₆)-Alkyl- S(O)ₙ, (C₂- C₃)-Alkenyl, (C₂- C₃)-Halogenalkenyl, (C₂-C₃)-Alkinyl, (C₂- C₃)-Halogenalkinyl, NH₂ bedeutet;

- Y: Sauerstoff, S(O)ₙ bedeutet;

- m: 0, 1, 2 bedeutet;
- n: 0, 1, 2 bedeutet;
- o: 0, 1, 2 bedeutet;
- p: 0 oder 1 bedeutet;
- q: 0 oder 1 bedeutet;
- r: 3, 4, 5 oder 6 bedeutet;
- s: 0, 1, 2, 4 bedeutet;
- x: 1,2,3,4 bedeutet;
- y: 1,2,3,4 bedeutet;
- z: 1,2,3,4 bedeutet.

### Besonders bevorzugt sind Verbindungen der allgemeinen Formel (I), in denen

### A ausgewählt ist aus der Gruppe, bestehend aus A1-A4, A6 und A8

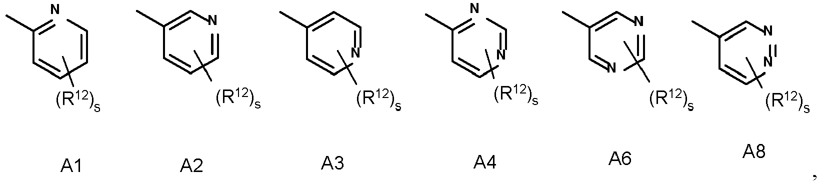

- Q: ausgewählt ist aus der Gruppe, bestehend aus Q1-Q3 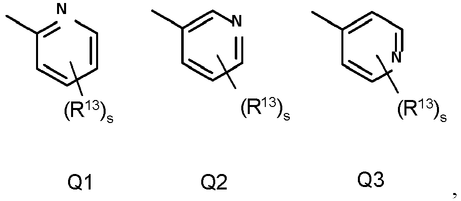
- R¹: OR^{1a}, NR⁹R¹⁰ oder O(CH₂)ₓO(CH₂)_{y}O(CH₂)_{z} H bedeutet,
- R^{1a}: Wasserstoff bedeutet oder (C₁-C₄)-Alkyl, (C₃-C₆)-Cycloalkyl bedeutet welches jeweils unsubstituiert oder substituiert ist durch einen oder mehrere Substituenten ausgewählt aus der Gruppe bestehend aus Halogen, (C₁-C₄)-Alkyl, (C₁-C₄)-Halogenalkyl oder Aryl-(C₁₋C₄)-Alkyl- bedeutet, welches jeweils unsubstituiert oder substituiert ist durch einen oder mehrere Substituenten ausgewählt aus der Gruppe bestehend aus Halogen, (C₁-C₄)-Alkyl, (C₁-C₄)-Haloalkyl;
- R⁹: Wasserstoff;
- R¹⁰: (C₁-C₆)-Alkyl, S(O)ₙR⁵, SO₂NR⁶R⁷, CO₂R⁸ bedeutet, wobei die oben genannten Reste unsubstituiert sind oder jeweils unabhängig voneinander substituiert sind durch "m" Reste ausgewählt aus der Gruppe bestehend aus Aryl, S(O)ₙR⁵, SO₂NR⁶R⁷, CO₂R⁸, NR⁶CO₂R⁸;
- R⁵: Ethyl, Methyl, CF₃ oder CH₂CF₃ bedeutet;
- R⁶: Wasserstoff bedeutet;
- R⁷: Wasserstoff, Methyl oder Ethyl bedeutet;
- R⁸: Methyl oder Ethyl bedeutet;
- R²: Wasserstoff, (C₁-C₆)-Alkyl, (C₁-C₆)-Halogenalkyl, (C₁-C₆)-Alkoxy bedeutet;
- R³: Halogen, Cyano, NO₂, (C₃-C₆)-Cycloalkyl, (C₁-C₆)-Alkyl, (C₁-C₆)-Halogenalkyl, (C₁-C₆)-Halogenalkoxy, (C₁-C₆)-Alkylthio bedeutet;
- R¹²: Fluor, Chlor, Brom, Cyano, Methyl, Ethyl, CF₃, OCF₃ bedeutet;
- R¹³: Fluor, Chlor, Brom, Cyano, Methyl, Ethyl, CF₃, OCF₃ bedeutet;
- Y: Sauerstoff und S(O)ₙ bedeutet;
- m: 0, 1 2 bedeutet;
- n: 0, 1, 2 bedeutet;
- s: 0, 1, 2 bedeutet;
- x: 1,2 bedeutet;
- y: 1,2 bedeutet;
- z: 1,2 bedeutet.

### Ganz besonders bevorzugt sind Verbindungen der allgemeinen Formel (I), in denen

### A ausgewählt ist aus der Gruppe, bestehend aus A2, A3 und A6

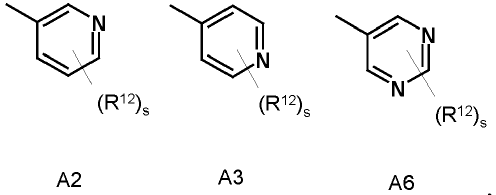

- Q: ausgewählt ist aus der Gruppe, bestehend aus Q1-Q3 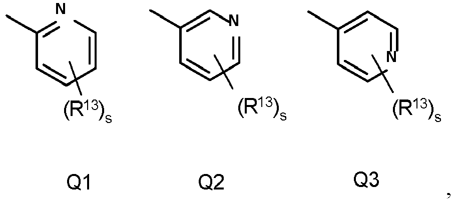
- R¹: OR^{1a};
- R^{1a}: Wasserstoff, Ethyl, Methyl, CF₃CH₂SO₂NH-, Me₂NSO₂NH-, MeO₂CCH₂NH-
- R²: Wasserstoff, Methyl oder Ethyl bedeutet;
- R³: Fluor, Brom, Chlor, Cyano, NO₂, Methyl, Cyclopropyl, CF₃ und OCF bedeutet;
- R¹²: Fluor, Chlor, Brom, Cyano, CF₃ bedeutet;
- R¹³: Fluor, Chlor, Brom, Cyano, CF₃ bedeutet;
- Y: Sauerstoff und S(O)ₙ bedeutet;
- n: 0, 1, 2 bedeutet;
- s: 0, 1, 2 bedeutet.

Ein weiterer Gegenstand der vorliegenden Erfindung sind Verbindungen der Formel (**Z**) 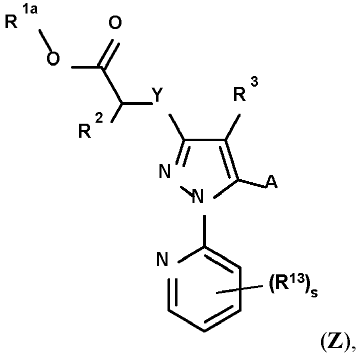 wobei die oben beschriebenen Definitionen gelten einschließlich aller bevorzugten, besonders bevorzugten und ganz besonders bevorzugten Definitionen.

Ein weiterer Gegenstand der vorliegenden Erfindung sind Verbindungen der Formel (**Y**) 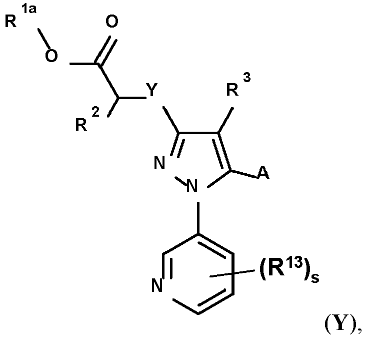 wobei die oben beschriebenen Definitionen gelten einschließlich aller bevorzugten, besonders bevorzugten und ganz besonders bevorzugten Definitionen.

Ein weiterer Gegenstand der vorliegenden Erfindung sind Verbindungen der Formel (**W**) 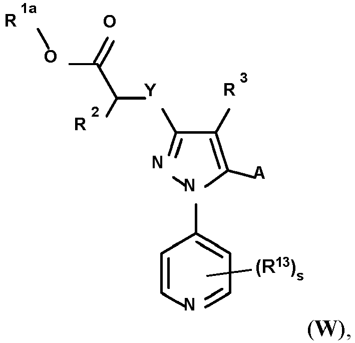 wobei die oben beschriebenen Definitionen gelten einschließlich aller bevorzugten, besonders bevorzugten und ganz besonders bevorzugten Definitionen.

Ein weiterer Gegenstand der vorliegenden Erfindung sind Säuren der Formel (**Ia**) 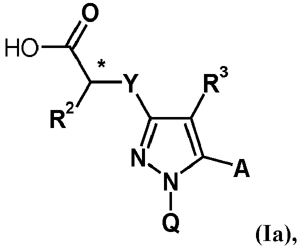 wobei die oben beschriebenen Definitionen gelten einschließlich aller bevorzugten, besonders bevorzugten und ganz besonders bevorzugten Definitionen.

Ein weiterer Gegenstand der vorliegenden Erfindung sind Verbindungen der Formel (**V**) 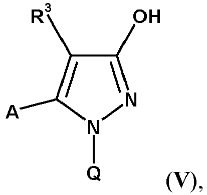 wobei die oben beschriebenen Definitionen gelten einschließlich aller bevorzugten, besonders bevorzugten und ganz besonders bevorzugten Definitionen.

Ein weiterer Gegenstand der vorliegenden Erfindung sind Verbindungen der Formel (**VI**) 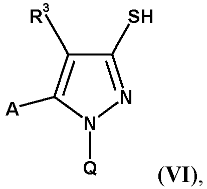 wobei die oben beschriebenen Definitionen gelten einschließlich aller bevorzugten, besonders bevorzugten und ganz besonders bevorzugten Definitionen.

In allen nachfolgend genannten Formeln haben die Substituenten und Symbole, sofern nicht anders definiert, dieselbe Bedeutung wie unter Formel (**I**) beschrieben.

Nicht umfasst sind Kombinationen, die gegen Naturgesetze widersprechen und welche der Fachmann daher aufgrund seines Wissens ausschließen würde.

Alkyl bedeutet gesättigte, geradkettige oder verzweigte Kohlenwasserstoffreste mit der jeweils angegebenen Anzahl von Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl,1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methyl-propyl.

Durch Halogen substitiertes Alkyl bedeutet geradkettige oder verzweigte Alkylgruppen, wobei in diesen Gruppen teilweise oder vollständig die Wasserstoffatome durch Halogenatome ersetzt sein können, z.B. C₁-C₂-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor,2-difluorethyl, 2,2-Dichlor-2-fluorethyl, 2,2,2-Trichlorethyl, Pentafluorethyl und 1,1,1-Trifluorprop-2-yl.

Alkenyl bedeutet ungesättigte, geradkettige oder verzweigte Kohlenwasserstoffreste mit der jeweils angegebenen Anzahl von Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl.

Alkinyl bedeutet geradkettige oder verzweigte Kohlenwasserstoffreste mit der jeweils angegebenen Anzahl von Kohlenstoffatomen und einer Dreifachbindung in einer beliebigen Position, z.B. C₂-C₆-Alkinyl wie Ethinyl, 1-Propinyl, 2-Propinyl (oder Propargyl), 1-Butinyl, 2-Butinyl, 3-Butinyl, 1-Methyl-2-propinyl, 1-Pentinyl, 2-Pentinyl, 3-Pentinyl, 4-Pentinyl, 3-Methyl-1-butinyl, 1-Methyl-2-butinyl, 1-Methyl-3-butinyl, 2-Methyl-3-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 3-Methyl-1-pentinyl, 4-Methyl-1-pentinyl, 1-Methyl-2-pentinyl, 4-Methyl-2-pentinyl, 1-Methyl-3-pentinyl, 2-Methyl-3-pentinyl, 1-Methyl-4-pentinyl, 2-Methyl-4-pentinyl, 3-Methyl-4-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl.

Cycloalkyl bedeutet ein carbocyclisches, gesättigtes Ringsystem mit vorzugsweise 3 bis 6 Ring-C-Atomen, z.B. Cyclopropyl, Cyclobutyl, Cyclopentyl oder Cyclohexyl. Im Falle von gegebenenfalls substituiertem Cycloalkyl werden cyclische Systeme mit Substituenten umfasst, wobei auch Substituenten mit einer Doppelbindung am Cycloalkylrest, z. B. eine Alkylidengruppe wie Methyliden, umfasst sind. Im Falle von gegebenenfalls substituiertem Cycloalkyl werden auch mehrcyclische aliphatische Systeme umfaßt, wie beispielsweise Bicyclo[1.1.0]butan-1-yl, Bicyclo[1.1.0]butan-2-yl, Bicyclo[2.1.0]pentan-1-yl, Bicyclo[2.1.0]pentan-2-yl, Bicyclo[2.1.0]pentan-5-yl, Bicyclo[2.2.1]hept-2-yl (Norbornyl), Adamantan-1-yl und Adamantan-2-yl.

Im Falle von substituiertem Cycloalkyl werden auch spirocyclische aliphatische Systeme umfaßt, wie beispielsweise Spiro[2.2]pent-1-yl, Spiro[2.3]hex-1-yl, Spiro[2.3]hex-4-yl, 3-Spiro[2.3]hex-5-yl.

Cycloalkenyl bedeutet ein carbocyclisches, nicht aromatisches, partiell ungesättigtes Ringsystem mit vorzugsweise 4-8 C-Atomen, z.B. 1-Cyclobutenyl, 2-Cyclobutenyl, 1-Cyclopentenyl, 2-Cyclopentenyl, 3-Cyclopentenyl, oder 1-Cyclohexenyl, 2-Cyclohexenyl, 3-Cyclohexenyl, 1,3-Cyclohexadienyl oder 1,4-Cyclohexadienyl, wobei auch Substituenten mit einer Doppelbindung am Cycloalkenylrest, z. B. eine Alkylidengruppe wie Methyliden, umfasst sind. Im Falle von gegebenenfalls substituiertem Cycloalkenyl gelten die Erläuterungen für substituiertes Cycloalkyl entsprechend.

Alkoxy bedeutet gesättigte, geradkettige oder verzweigte Alkoxyreste mit der jeweils angegebenen Anzahl von Kohlenstoffatomen, z.B. C₁-C₆-Alkoxy wie Methoxy, Ethoxy, Propoxy, 1-Methylethoxy, Butoxy, 1-Methyl-propoxy, 2-Methylpropoxy, 1,1-Dimethylethoxy, Pentoxy, 1-Methylbutoxy, 2-Methylbutoxy, 3-Methylbutoxy, 2,2-Di-methylpropoxy, 1-Ethylpropoxy, Hexoxy, 1,1-Dimethylpropoxy, 1,2-Dimethylpropoxy,1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy. Durch Halogen substitiertes Alkoxy bedeutet geradkettige oder verzweigte Alkoxyreste mit der jeweils angegebenen Anzahl von Kohlenstoffatomen, wobei in diesen Gruppen teilweise oder vollständig die Wasserstoffatome durch Halogenatome wie vorstehend genannt ersetzt sein können, z.B. C₁-C₂-Halogenalkoxy wie Chlormethoxy, Brommethoxy, Dichlormethoxy, Trichlormethoxy, Fluormethoxy, Difluormethoxy, Trifluormethoxy, Chlorfluormethoxy, Dichlor-fluormethoxy, Chlordifluormethoxy, 1-Chlorethoxy, 1-Bromethoxy, 1-Fluorethoxy, 2-Fluorethoxy, 2,2-Difluorethoxy, 2,2,2-Trifluorethoxy, 2-Chlor-2-fluorethoxy, 2-Chlor-1,2-difluorethoxy, 2,2-Dichlor-2-fluorethoxy, 2,2,2-Trichlorethoxy, Pentafluor-ethoxy und 1,1,1-Trifluorprop-2-oxy.

Heterocyclyl, bedeutet gesättigte oder partiell ungesättigte mono-, bi- oder tricyclische Ringsystemgruppe, aus C-Atomen und mindestens einem Heteroatom, vorzugsweise ausgewählt aus N, O und/oder S.

Heteroaryl, soweit nicht an anderer Stelle anders definiert: mono-, bi- oder tricyclische heterocyclische Gruppe aus C-Atomen und mindestens einem Hetero¬atom, wobei mindestens ein Zyklus aromatisch ist. In einer Ausführungsform ist mindestens ein Heteroatom N, O oder S. In einer Ausführungsform sind alle Heteroatome ausgewählt aus N, O oder S. In einer Ausführungsform ist das Ringsystem ein 5 bis 10- bzw. ein 5 bis 6-gliedriges Ringsystem. In einer Ausführungsform ist Heteroaryl ein aromatisches monocyclisches Ringsystem aus 5 oder 6 Ringatomen. In einer weiteren Ausführungsform ist Heteroaryl ein aromatisches monocyclisches Ringsystem, enthaltend 1 bis 4 Hetero¬atome aus der Gruppe N, O oder S. Weiterhin kann Heteroaryl ein bicyclisches Ringsystem darstellen, das aus 8 bis 14 Ringatomen besteht oder ein tricyclisches Ringsystem, das aus 13 bis 14 Ringatomen besteht. Beispiele: Furyl, Thienyl, Pyrazolyl, Imidazolyl, Triazolyl, Thiazolyl, Indolyl, Benzimidazolyl, Indazolyl, Benzofuranyl, Benzothiophenyl, Benzothiazolyl, Benzoxazolyl, Chinolinyl, Isochinolinyl.

Der Begriff "Aryl" bedeutet ein gegebenenfalls substituiertes mono-, bi- oder polycyclisches aromatisches System mit vorzugsweise 6 bis 14, insbesondere 6 bis 10 Ring-C-Atomen, beispielsweise Phenyl, Naphthyl, Anthryl, Phenanthrenyl, und ähnliches, vorzugsweise Phenyl.

Vom Begriff "gegebenenfalls substituiertes Aryl" sind auch mehrcyclische Systeme, wie Tetrahydronaphtyl, Indenyl, Indanyl, Fluorenyl, Biphenylyl, umfasst, wobei die Bindungsstelle am aromatischen System ist. Von der Systematik her ist "Aryl" in der Regel auch von dem Begriff "gegebenenfalls substituiertes Phenyl" umfasst.

Die oben aufgeführten Aryle sind bevorzugt unabhängig voneinander ein- bis fünffach beispielsweise durch Wasserstoff, Halogen, Alkyl, Haloalkyl, Hydroxy, Alkoxy, Cycloalkoxy, Aryloxy, Alkoxyalkyl, Alkoxyalkoxy, Cycloalkyl, Halocycloalkyl, Aryl, Arylalkyl, Heteroaryl, Heterocyclyl, Alkenyl, Alkylcarbonyl, Cycloalkylcarbonyl, Arylcarbonyl, Heteroarylcarbonyl, Alkoxycarbonyl, Hydroxycarbonyl, Cycloalkoxycarbonyl, Cycloalkylalkoxycarbonyl, Alkoxycarbonylalkyl, Arylalkoxycarbonyl, Arylalkoxycarbonylalkyl, Alkinyl, Alkinylalkyl, Alkylalkinyl, Trisalkylsilylalkinyl, Nitro, Amino, Cyano, Haloalkoxy, Haloalkylthio, Alkylthio, Hydrothio, Hydroxyalkyl, Heteroarylalkoxy, Arylalkoxy, Heterocyclylalkoxy, Heterocyclylalkylthio, Heterocyclyloxy, Heterocyclylthio, Heteroaryloxy, Bis-alkylamino, Alkylamino, Cycloalkylamino, Hydroxycarbonylalkylamino, Alkoxycarbonylalkylamino, Arylalkoxycarbonylalkylamino, Alkoxycarbonylalkyl(alkyl)amino, Aminocarbonyl, Alkylaminocarbonyl, Bis-alkylaminocarbonyl, Cycloalkylaminocarbonyl, Hydroxycarbonylalkylaminocarbonyl, Alkoxycarbonylalkylaminocarbonyl, Arylalkoxycarbonylalkylaminocarbonyl substituiert.

Wenn ein Grundkörper "durch einen oder mehrere Reste" aus einer Aufzählung von Resten (= Gruppe) oder einer generisch definierten Gruppe von Resten substituiert ist, so schließt dies jeweils die gleichzeitige Substitution durch mehrere gleiche und/oder strukturell unterschiedliche Reste ein.

Die Bezeichnung "Halogen" bedeutet Fluor, Chlor, Brom oder Iod. Wird die Bezeichnung für einen Rest verwendet, dann bedeutet "Halogen" ein Fluor-, Chlor-, Brom- oder Iodatom.

Je nach Art der oben definierten Substituenten weisen die Verbindungen der Formel (I) saure Eigenschaften auf und können mit anorganischen oder organischen Basen oder mit Metallionen Salze, gegebenenfalls auch innere Salze oder Addukte bilden. Tragen die Verbindungen der Formel (I) Hydroxy, Carboxy oder andere, saure Eigenschaften induzierende Gruppen, so können diese Verbindungen mit Basen zu Salzen umgesetzt werden.Geeignete Basen sind beispielsweise Hydroxide, Carbonate, Hydrogencarbonate der Alkali- und Erdalkalimetalle, insbesondere die von Natrium, Kalium, Magnesium und Calcium, weiterhin Ammoniak, primäre, sekundäre und teritäre Amine mit (C₁-C₄-)-Alkyl-Gruppen, Mono-, Di- und Trialkanolamine von (C₁-C₄)-Alkanolen, Cholin sowie Chlorcholin, sowie organische Amine, wie Trialkylamine, Morpholin, Piperidin oder Pyridin. Diese Salze sind Verbindungen, in denen der acide Wasserstoff durch ein für die Landwirtschaft geeignetes Kation ersetzt wird, beispielsweise Metallsalze, insbesondere Alkalimetallsalze oder Erdalkalimetallsalze, insbesondere Natrium- und Kaliumsalze, oder auch Ammoniumsalze, Salze mit organischen Aminen oder quartäre (quaternäre) Ammoniumsalze, zum Beispiel mit Kationen der Formel [NRR'R"R‴]⁺, worin R bis R‴ jeweils unabhängig voneinander einen organischen Rest, insbesondere Alkyl, Aryl, Aralkyl oder Alkylaryl darstellen. Infrage kommen auch Alkylsulfonium- und Alkylsulfoxoniumsalze, wie (C₁-C₄)-Trialkylsulfonium- und (C₁-C₄)-Trialkylsulfoxoniumsalze.

Die Verbindungen der Formel (I) können durch Anlagerung einer geeigneten anorganischen oder organischen Säure, wie beispielsweise Mineralsäuren, wie beispielsweise HCl, HBr, H₂SO₄, H₃PO₄ oder HNO₃, oder organische Säuren, z. B. Carbonsäuren, wie Ameisensäure, Essigsäure, Propionsäure, Oxalsäure, Milchsäure oder Salicylsäure oder Sulfonsäuren, wie zum Beispiel p-Toluolsulfonsäure, an eine basische Gruppe, wie z.B. Amino, Alkylamino, Dialkylamino, Piperidino, Morpholino oder Pyridino, Salze bilden. Diese Salze enthalten dann die konjugierte Base der Säure als Anion.

Geeignete Substituenten, die in deprotonierter Form, wie z.B. Sulfonsäuren oder Carbonsäuren, vorliegen, können innere Salze mit ihrerseits protonierbaren Gruppen, wie Aminogruppen bilden.

Ist eine Gruppe mehrfach durch Reste substituiert, so bedeutet dies, dass diese Gruppe durch einen oder mehrere gleiche oder verschiedene der genannten Reste substituiert ist.

In allen nachfolgend genannten Formeln haben die Substituenten und Symbole, sofern nicht anders definiert, dieselbe Bedeutung wie unter Formel (I) beschrieben. Pfeile in einer chemischen Formel bedeuten die Verknüpfungsorte zum restlichen Molekül.

Im Folgenden werden, jeweils für die einzelnen Substituenten, bevorzugte, besonders bevorzugte und ganz besonders bevorzugte Bedeutungen beschrieben. Die übrigen Substituenten der allgemeinen Formel (I), welche nachfolgend nicht genannt werden, weisen die oben genannte Bedeutung auf.

Die vorliegenden Verbindungen der allgemeinen Formel (I) weisen am zweiten Kohlenstoff der Alkylsäurestruktur ein chirales Kohlenstoffatom auf, welches in der unten dargestellten Struktur durch die Kennzeichnung (*) verdeutlicht ist:

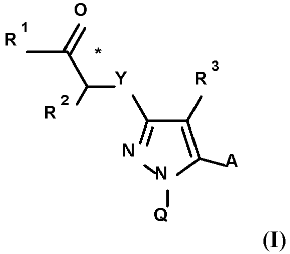

Gemäß den Regeln nach Cahn, Ingold und Prelog (CIP-Regeln) kann dieses Kohlenstoffatom sowohl eine (R)- als auch eine (S)-Konfiguration aufweisen.

Von der vorliegenden Erfindung werden Verbindungen der allgemeinen Formel (I) sowohl mit (S)- als auch mit (R)-Konfiguration erfasst, d.h., dass die vorliegende Erfindung die Verbindungen der allgemeinen Formel (I) erfasst, in welchen das betreffende Kohlenstoffatom
(1) eine (R)-Konfiguration; oder
(2) eine (S)-Konfiguration
   aufweist.
   Darüber hinaus werden im Rahmen der vorliegenden Erfindung auch
(3) beliebige Mischungen von Verbindungen der allgemeinen Formel (I), welche eine (R)-Konfiguration (Verbindungen der allgemeinen Formel (I-(R)) aufweisen, mit Verbindungen der allgemeinen Formel (I), welche eine (S)-Konfiguration (Verbindungen der allgemeinen Formel (I-S)) aufweisen, erfasst, wobei eine racemische Mischung der Verbindungen der allgemeinen Formel (I) mit (R)- und (S)-Konfiguration von der vorliegenden Erfindung ebenfalls umfasst ist.

Allerdings sind im Rahmen der vorliegenden Erfindung insbesondere Verbindungen der allgemeinen Formel (I) mit (R)-Konfiguration mit einer Selektivität von 60 bis 100%, vorzugsweise 80 bis 100%, insbesondere 90 bis 100%, ganz besonders 95 bis 100%, bevorzugt, wobei die jeweilige (R)-Verbindung mit einer Enantioselektivität von jeweils mehr als 50% ee, vorzugsweise 60 bis 100% ee, insbesondere 80 bis 100% ee, ganz besonders 90 bis 100% ee, meist bevorzugt 95 bis 100% ee, bezogen auf den Gesamtgehalt an betreffender (R)-Verbindung vorliegt.

Daher betrifft die vorliegende Erfindung insbesondere Verbindungen der allgemeinen Formel (I*), in welchen die stereochemische Konfiguration an dem mit (*) gekennzeichnet Kohlenstoffatom mit einer stereochemischen Reinheit von 60 bis 100 % (R), vorzugsweise 80 bis 100 % (R), insbesondere 90 bis 100 % (R), ganz besonders 95 bis 100 % (R), vorliegt.

Darüber hinaus können, je nach Wahl der jeweiligen Reste, weitere Stereoelemente in den erfindungsgemäßen Verbindungen der allgemeinen Formel (I) vorliegen.

Bevorzugt sind die in den folgenden Tabellen genannten Verbindungen. Die Verbindungen der allgemeinen Formel (I) mit (R)-Konfiguration sind in der Spalte, in welcher der Rest R² genannt ist, entsprechend gekennzeichnet. Falls zum Beispiel gilt, dass R² = Alkyl, so ist die bevorzugte stereochemische Konfiguration an dem mit (*) gekennzeichnet Kohlenstoffatom der allgemeinen Formel (I) die (R)-Konfiguration.

Falls dagegen zum Beispiel gilt, dass R² = Alkoxy, so ist die bevorzugte stereochemische Konfiguration an dem mit (*) gekennzeichnet Kohlenstoffatom der allgemeinen Formel (I) die (S)-Konfiguration.

**Tabelle I: 2-Pyridyl**

| 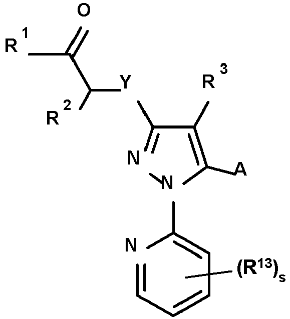 | | | | | | |
|---|---|---|---|---|---|---|
| Beispielnummer | R¹ | R² | R³ | A | Y | (R¹³)ₛ |
| I-01 | OEt | H | Br | (2-fluoropyridin-4-yl) | O | H |
| I-02 | OEt | H | Br | (6-fluoropyridin-3-yl) | O | H |
| I-03 | OMe | (R)-Me | Br | pyridin-3-yl | O | 3-F |
| I-04 | OMe | (R)-Me | CN | pyridin-3-yl | O | 3-F |
| I-05 | OMe | (R)-Me | Br | (6-fluoropyridin-3-yl) | O | 3-F |
| I-06 | OMe | (R)-Me | CN | (6-fluoropyridin-3-yl) | O | 3-F |
| I-07 | OMe | H | Br | (6-fluoropyridin-3-yl) | O | 3-F |
| I-08 | OH | H | Br | (6-fluoropyridin-3-yl) | O | 3-F |
| I-09 | OMe | H | CN | (6-fluoropyridin-3-yl) | O | 3-F |
| I-10 | OH | (R)-Me | Br | (6-fluoropyridin-3-yl) | O | 3-F |
| I-11 | OMe | (R)-Me | Br | (6-fluoropyridin-3-yl) | O | 5-F |
| I-12 | OMe | H | Br | (6-fluoropyridin-3-yl) | O | 5-F |
| I-13 | OH | (R)-Me | Br | (5 -fluoropyridin-3 -yl) | O | 5-F |
| I-14 | OH | H | Br | (5 -fluoropyridin-3 -yl) | O | 5-F |
| I-15 | OMe | (R)-Me | Br | (5 -fluoropyridin-3 -yl) | O | 3-F |
| I-16 | OMe | H | Br | (5 -fluoropyridin-3 -yl) | O | 3-F |
| I-17 | OH | H | Br | (6-fluoropyridin-3-yl) | O | 3-F |
| I-18 | OH | (R)-Me | Br | (6-fluoropyridin-3-yl) | O | 3-F |
| I-19 | OMe | (R)-Me | Cl | (2-fluoropyridin-4-yl) | O | H |
| I-20 | OH | (R)-Me | Cl | pyrimidin-5-yl | O | H |
| I-21 | OH | (R)-Me | Cl | (6-fluoropyridin-3-yl) | O | H |
| I-22 | OH | (R)-Me | Cl | (6-fluoropyridin-3-yl) | O | H |
| I-23 | OEt | H | Br | (6-fluoropyridin-3-yl) | O | 3-Cl |
| I-24 | OMe | (R)-Me | Br | (6-fluoropyridin-3-yl) | O | 3-Cl |
| I-25 | OH | (R)-Me | Br | (6-fluoropyridin-3-yl) | O | 3-Cl |
| I-26 | OH | H | Br | (6-fluoropyridin-3-yl) | O | 3-Cl |
| I-27 | OEt | (R)-Me | Br | (6-fluoropyridin-3-yl) | O | 3-Cl |
| I-28 | OMe | H | Br | (6-fluoropyridin-3-yl) | O | 3-Cl |
| I-29 | -OCH₂CH₂OMe | H | Br | (6-fluoropyridin-3-yl) | O | 3-F |
| I-30 | -OCH₂CN | H | Br | (6-fluoropyridin-3-yl) | O | 3-F |
| I-31 | -NH-allyl | H | Br | (6-fluoropyridin-3-yl) | O | 3-F |
| I-32 | OEt | (R)-Me | Cl | (6-fluoropyridin-3-yl) | O | 3-F |
| I-33 | OEt | (R)-Me | Br | (6-fluoropyridin-3-yl) | O | 3-F |
| I-34 | -OCH₂CH₂O-CH₂CH₂OMe | (R)-Me | Br | (6-fluoropyridin-3-yl) | O | 3-F |
| I-35 | -OCH₂CH₂OMe | (R)-Me | Br | (6-fluoropyridin-3-yl) | O | 3-F |
| I-36 | -NHSO₂N(Me)₂ | H | Br | (6-fluoropyridin-3-yl) | O | 3-F |
| I-37 | -OCH₂CH₂O-CH₂CH₂OMe | H | Br | (6-fluoropyridin-3-yl) | O | 3-F |
| I-38 | OEt | H | Cl | (6-fluoropyridin-3-yl) | O | 3-F |
| I-39 | OEt | H | Br | (6-fluoropyridin-3-yl) | O | 3-F |
| I-40 | OEt | H | cyclopropyl | (6-fluoropyridin-3-yl) | O | 3-F |
| I-41 | OMe | (R)-Me | Cl | (6-fluoropyridin-3 -yl) | O | 3-F |
| I-42 | OMe | H | cyclopropyl | (6-fluoropyridin-3-yl) | O | 3-F |
| I-43 | OH | H | cyclopropyl | (6-fluoropyridin-3-yl) | O | 3-F |
| I-44 | -NHSO₂Me | H | Br | (6-fluoropyridin-3-yl) | O | 3-F |
| I-45 | -O-N=CH(CH₃)₂ | H | Br | (6-fluoropyridin-3-yl) | O | 3-F |
| I-46 | -CH2-pyridin-2-yl | H | Br | (6-fluoropyridin-3-yl) | O | 3-F |
| I-47 | 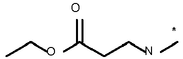 | H | Br | (6-OMepyridin-3-yl) | O | 3-F |
| I-48 | 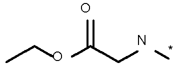 | H | Br | (6-OMepyridin-3-yl) | O | 3-F |
| I-49 | OMe | H | Br | (5 -fluoropyridin-3 -yl) | O | 3-F |
| I-50 | OH | (R)-Me | Br | (6-fluoropyridin-3-yl) | O | 3-F |
| I-51 | OEt | H | Br | (6-fluoropyridin-3-yl) | O | 3-F |
| I-52 | -CH2-pyridin-2-yl | (R)-Me | Br | (6-fluoropyridin-3-yl) | O | 3-F |
| I-53 | 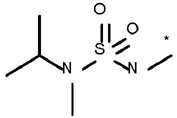 | H | Br | (6-fluoropyridin-3-yl) | O | 3-F |
| I-54 | 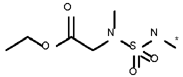 | H | Br | (6-fluoropyridin-3-yl) | O | 3-F |
| I-55 | OEt | H | I | (6-fluoropyridin-3-yl) | O | 3-F |
| I-56 | OEt | H | CF3 | (6-fluoropyridin-3-yl) | O | 3-F |
| I-57 | OEt | H | nitro | (6-fluoropyridin-3-yl) | O | 3-F |
| I-58 | OEt | Me | Br | (6-fluoropyridin-3-yl) | O | H |
| I-59 | 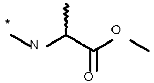 | H | Br | (2-fluoropyridin-4-yl) | O | H |
| I-60 | 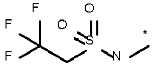 | H | Br | (6-fluoropyridin-3-yl) | O | H |
| I-61 | 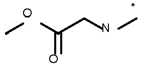 | H | Br | (6-fluoropyridin-3-yl) | O | H |
| I-62 | 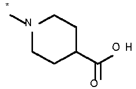 | H | Br | (6-fluoropyridin-3-yl) | O | H |
| I-63 | 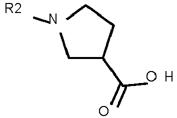 | H | Br | (5 -fluoropyridin-3 -yl) | O | H |
| I-64 | 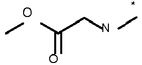 | H | Cl | (6-fluoropyridin-3-yl) | O | H |
| I-65 | 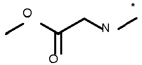 | H | Br | pyrimidin-5-yl | S | H |
| I-66 | OH | H | Br | pyrimidin-5-yl | O | H |
| I-67 | prop-2-ynoxy | H | Cl | (5 -fluoropyridin-3 -yl) | O | H |
| I-68 | OEt | H | Br | (6-fluoropyridin-3-yl) | O | H |
| I-69 | OH | H | Br | (6-fluoropyridin-3-yl) | S | H |
| I-70 | 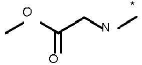 | H | cyclopropyl | (6-fluoropyridin-3-yl) | O | H |
| I-71 | -OCH2CH2COOMe | H | cyclopropyl | (5 -fluoropyridin-3 -yl) | O | H |
| I-72 | OH | H | cyclopropyl | (5 -fluoropyridin-3 -yl) | O | H |
| I-73 | -OCH2CH2COOMe | (R)-Me | Br | (5 -fluoropyridin-3 -yl) | O | H |
| I-74 | 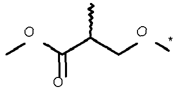 | H | Cl | (5 -fluoropyridin-3 -yl) | O | H |
| I-75 | -OCH2CH2COOMe | H | Cl | (5 -fluoropyridin-3 -yl) | O | H |
| I-76 | 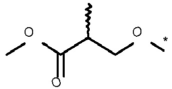 | H | Br | (6-fluoropyridin-3-yl) | O | H |
| I-77 | -OCH2CH2COOMe | H | Br | (5 -fluoropyridin-3 -yl) | O | H |
| I-78 | OEt | H | cyclopropyl | (6-fluoropyridin-3-yl) | O | H |
| I-79 | OH | (R)-Me | Br | (5 -fluoropyridin-3 -yl) | O | H |
| I-80 | 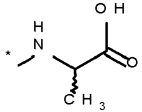 | H | Br | (5 -fluoropyridin-3 -yl) | O | H |
| I-81 | OH | H | Cl | (5 -fluoropyridin-3 -yl) | O | H |
| I-82 | 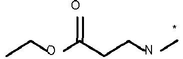 | H | Br | (5 -fluoropyridin-3 -yl) | S | H |
| I-83 | OMe | (R)-Me | Br | (5 -fluoropyridin-3 -yl) | O | H |
| I-84 | OH | H | Br | (5 -fluoropyridin-3 -yl) | O | H |
| I-85 | OEt | H | Cl | (6-fluoropyridin-3-yl) | O | H |
| I-86 | OEt | H | Br | (6-fluoropyridin-3-yl) | O | H |
| I-87 | 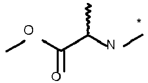 | H | Cl | (6-fluoropyridin-3-yl) | O | H |
| I-88 | -OCH2CH2COOMe | H | Cl | (6-fluoropyridin-3-yl) | O | H |
| I-89 | 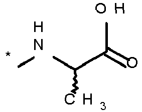 | H | Br | (6-fluoropyridin-3-yl) | O | H |
| I-90 | 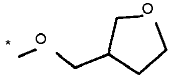 | H | Br | (6-fluoropyridin-3-yl) | O | H |
| I-91 | OEt | (R)-Me | Br | (6-fluoropyridin-3-yl) | O | H |
| I-92 | 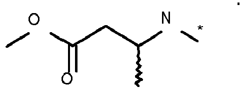 | H | Br | (6-fluoropyridin-3-yl) | O | H |
| I-93 | 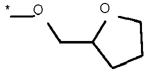 | H | Br | (6-fluoropyridin-3-yl) | O | H |
| I-94 | OH | Me | CF3 | (6-fluoropyridin-3-yl) | O | H |
| I-95 | -OCH₂CH₂SMe | H | Br | (6-fluoropyridin-3-yl) | O | H |
| I-96 | -OCH₂CH₂OMe | H | Br | (6-fluoropyridin-3-yl) | O | H |
| I-97 | 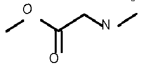 | H | Br | (6-fluoropyridin-3 -yl) | O | H |
| I-98 | OH | H | Cl | (6-fluoropyridin-3-yl) | O | H |
| I-99 | OH | H | CF3 | (6-fluoropyridin-3-yl) | O | H |
| I-100 | OEt | H | Cl | (6-fluoropyridin-3-yl) | O | H |
| I-101 | OEt | Me | I | (6-fluoropyridin-3-yl) | O | H |
| I-102 | OEt | Me | Br | (6-fluoropyridin-3-yl) | O | H |
| I-103 | OH | H | I | (6-fluoropyridin-3-yl) | O | H |
| I-104 | OEt | H | Br | (5 -fluoropyridin-3 -yl) | SO 2 | H |
| I-105 | OEt | H | CF3 | (6-fluoropyridin-3-yl) | O | H |
| I-106 | OEt | H | Br | (5 -fluoropyridin-3 -yl) | SO 2 | H |
| I-107 | OEt | Me | CF3 | (6-fluoropyridin-3-yl) | O | H |
| I-108 | 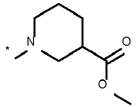 | H | Br | (6-fluoropyridin-3-yl) | O | H |
| I-109 | 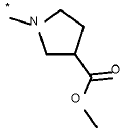 | H | Br | (6-fluoropyridin-3-yl) | O | H |
| I-110 | 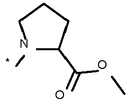 | H | Br | (6-fluoropyridin-3-yl) | O | H |
| I-111 | 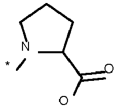 | H | Br | (6-fluoropyridin-3-yl) | O | H |
| I-112 | 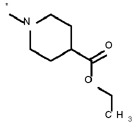 | H | Br | (6-fluoropyridin-3-yl) | O | H |
| I-113 | OEt | H | Br | (6-Clpyridin-3-yl) | S | H |
| I-114 | 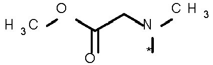 | H | Br | (6-fluoropyridin-3-yl) | O | H |
| I-115 | OEt | H | Br | pyrimidin-5-yl | S | H |
| I-116 | -OCH2CH2COOMe | H | Br | (6-fluoropyridin-3-yl) | O | H |
| I-117 | -NHCH2CH2COOMe | H | Br | (6-fluoropyridin-3-yl) | O | H |
| I-118 | 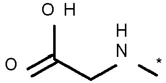 | H | Cl | (6-fluoropyridin-3-yl) | O | H |
| I-119 | 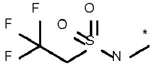 | H | Cl | (6-fluoropyridin-3-yl) | O | H |
| I-120 | 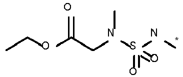 | H | Br | (6-fluoropyridin-3-yl) | O | H |
| I-121 | OEt | H | I | (6-fluoropyridin-3-yl) | O | H |
| I-122 | OH | H | Br | (2-fluoropyridin-4-yl) | O | H |
| I-123 | OEt | H | Br | (5-chloropyridin-3-yl) | S | H |
| I-124 | OEt | H | Br | (5-chloropyridin-3-yl) | S | H |
| I-125 | OEt | H | Br | (5-fluoropyridin-3-yl) | S | H |
| I-126 | OH | H | Br | (5-chloropyridin-3-yl) | S | H |

**Tabelle II: 3-Pyridyl**

| 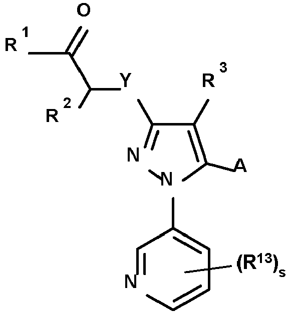 | | | | | | |
|---|---|---|---|---|---|---|
| Beispielnummer | R¹ | R² | R³ | A | (R¹²)ₛ | Y |
| II-01 | OEt | H | Br | (6-fluoropyridin-3-yl) | 2-F | O |
| II-03 | OH | H | Br | (6-fluoropyridin-3-yl) | 2-F | O |
| II-04 | OH | R-(Me) | Br | (6-fluoropyridin-3-yl) | 2-F | O |
| II-05 | OEt | R(-Me) | Br | (6-fluoropyridin-3-yl) | 2-F | O |
| II-06 | CF₃CH₂SO₂NH- | H | Br | (6-fluoropyridin-3-yl) | 2-F | O |
| II-07 | Me₂NSO₂NH- | H | Br | (6-fluoropyridin-3-yl) | 2-F | O |
| II-08 | MeO₂CCH₂NH- | H | Br | (6-fluoropyridin-3-yl) | 2-F | O |
| II-09 | OEt | H | Br | (6-fluoropyridin-3-yl) | H | O |
| II-10 | OH | H | Br | (6-fluoropyridin-3-yl) | H | O |
| II-11 | OH | R-(Me) | Br | (6-fluoropyridin-3-yl) | H | O |
| II-12 | OEt | R(-Me) | Br | (6-fluoropyridin-3-yl) | H | O |
| II-13 | CF₃CH₂SO₂NH- | H | Br | (6-fluoropyridin-3-yl) | H | O |
| II-14 | Me₂NSO₂NH- | H | Br | (6-fluoropyridin-3-yl) | H | O |
| II-15 | MeO₂CCH₂NH- | H | Br | (6-fluoropyridin-3-yl) | 2-F | O |
| II-16 | OEt | H | Br | (pyrimidin-5-yl) | 2-F | O |
| II-17 | OH | H | Br | (pyrimidin-5-yl) | 2-F | O |
| II-18 | OH | R-(Me) | Br | (pyrimidin-5-yl) | 2-F | O |
| II-19 | OEt | R(-Me) | Br | (pyrimdin-5-yl) | 2-F | O |
| II-20 | CF₃CH₂SO₂NH- | H | Br | (pyrimidin-5-yl) | 2-F | O |
| II-21 | Me₂NSO₂NH- | H | Br | (pyrimidin-5-yl) | 2-F | O |
| II-22 | MeO₂CCH₂NH- | H | Br | (pyrimidin-5-yl) | 2-F | O |
| II-23 | OEt | H | Br | (pyrimidin-5-yl) | H | O |
| II-24 | OH | H | Br | (pyrimidin-5-yl) | H | O |
| II-25 | OH | R-(Me) | Br | (pyrimidin-5-yl) | H | O |
| II-26 | OEt | R(-Me) | Br | (pyrimidin-5-yl) | H | O |
| II-27 | CF₃CH₂SO₂NH- | H | Br | (pyrimidin-5-yl) | H | O |
| II-28 | Me₂NSO₂NH- | H | Br | (pyrimidin-5-yl) | H | O |
| II-29 | MeO₂CCH₂NH- | H | Br | (pyridimin-5-yl) | H | O |
| II-30 | OH | R-(Me) | CF3 | (6-fluoropyridin-3-yl) | 2-F | O |
| II-31 | OEt | R-(Me) | CF3 | (6-fluoropyridin-3-yl) | 2-F | O |
| II-32 | CF₃CH₂SO₂NH- | H | CF3 | (6-fluoropyridin-3-yl) | 2-F | O |
| II-33 | Me₂NSO₂NH- | H | CF3 | (6-fluoropyridin-3-yl) | 2-F | O |
| II-34 | MeO₂CCH₂NH- | H | CF3 | (6-fluoropyridin-3-yl) | 2-F | O |
| II-35 | OEt | H | Br | (6-fluoropyridin-3-yl) | H | S |
| II-36 | OMe | (R)-Me | Br | (6-fluoropyridin-3-yl) | 2-F | O |

**Tabelle III: 4-Pyridyl**

| 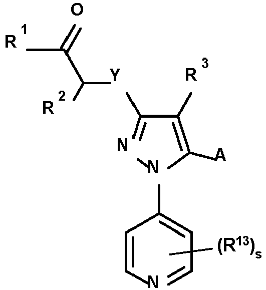 | | | | | | |
|---|---|---|---|---|---|---|
| Beispielnummer | R¹ | R² | R³ | R⁴ | (R¹²)ₛ | Y |
| III-01 | OEt | H | Br | (6-fluoropyridin-3-yl) | 2-F | O |
| III-02 | OH | H | Br | (6-fluoropyridin-3-yl) | 2-F | O |
| III-03 | OH | R-(Me) | Br | (6-fluoropyridin-3-yl) | 2-F | O |
| III-04 | OEt | R(-Me) | Br | (6-fluoropyridin-3-yl) | 2-F | O |
| III-05 | CF₃CH₂SO₂NH- | H | Br | (6-fluoropyridin-3-yl) | 2-F | O |
| III-06 | Me₂NSO₂NH- | H | Br | (6-fluoropyridin-3-yl) | 2-F | O |
| III-07 | MeO₂CCH₂NH- | H | Br | (6-fluoropyridin-3-yl) | 2-F | O |
| III-08 | OEt | H | Br | (6-fluoropyridin-3-yl) | H | O |

| Beispielnummer | R¹ | R² | R³ | R⁴ | (R¹²)ₛ | Y |
|---|---|---|---|---|---|---|
| III-09 | OH | H | Br | (6-fluoropyridin-3-yl) | H | O |
| III-10 | OH | R-(Me) | Br | (6-fluoropyridin-3-yl) | H | O |
| III-11 | OEt | R-(Me) | Br | (6-fluoropyridin-3-yl) | H | O |
| III-12 | CF₃CH₂SO₂NH- | H | Br | (6-fluoropyridin-3-yl) | H | O |
| III-13 | Me₂NSO₂NH- | H | Br | (6-fluoropyridin-3-yl) | H | O |
| III-14 | MeO₂CCH₂NH- | H | Br | (6-fluoropyridin-3-yl) | 2-H | O |
| III-15 | OEt | H | Br | (pyrimidin-5-yl) | 2-F | O |
| III-15 | OEt | H | Br | (pyrimidin-5-yl) | 2-F | S |
| III-16 | OH | H | Br | (pyrimidin-5-yl) | 2-F | S |
| III-17 | 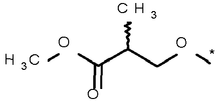 | H | Br | (6-fluoropyridin-3-yl) | 2-Cl | O |
| III-18 | 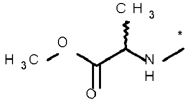 | H | Br | (6-fluoropyridin-3-yl) | 2-Cl | O |
| III-19 | -OCH₂CH₂COOMe | H | Br | (6-fluoropyridin-3-yl) | 2-Cl | O |
| III-20 | CF₃CH₂SO₂NH- | H | Br | (6-fluoropyridin-3-yl) | 2-Cl | O |
| III-21 | 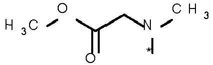 | H | Br | (6-fluoropyridin-3-yl) | 2-Cl | O |
| III-22 | OMe | (R)-Me | Br | (6-fluoropyridin-3-yl) | 2-Cl | O |
| III-23 | OH | H | Br | (6-fluoropyridin-3-yl) | 2-Cl | O |
| III-24 | OEt | H | Br | (6-fluoropyridin-3-yl) | 2-Cl | O |
| III-25 | 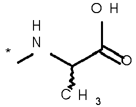 | H | Br | (6-fluoropyridin-3-yl) | 2-F | O |
| III-26 | 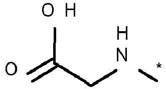 | H | Br | (6-fluoropyridin-3-yl) | 2-F | O |
| III-27 | -OCH₂CH₂COOMe | H | Br | (6-fluoropyridin-3-yl) | 2-F | O |
| III-28 | 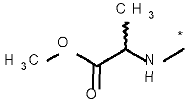 | H | Br | (6-fluoropyridin-3-yl) | 2-F | O |

Ein weiterer Aspekt der Erfindung betrifft die Herstellung der erfindungsgemäßen Verbindungen der allgemeinen Formel (I). Die erfindungsgemäßen Verbindungen können auf unterschiedliche Weise hergestellt werden.

Erfindungsgemäße Verbindungen können beispielsweise nach den im nachfolgenden Schema 1 aufgeführten Syntheseverfahren aus substituierten 1-Phenyl-5-azinyl-1H-pyrazol-3-ols (II) hergestellt werden.

Die Synthese der erfindungsgemäßen Verbindungen der allgemeinen Formel (**Ib**) erfolgt über eine Amidkupplung von einer Säure der allgemeinen Formel (**Ia**) mit einem Amin der allgemeinen Formel (**II**) in Gegenwart eines Amidkupplungsreagenzes wie zum Beispiel T3P, Dicyclohexylcarbodiimid, *N*-(3-Dimethylaminopropyl)-*N'*-ethylcarbodiimid, *N*,*N*'-Cabonyldiimidazol, 2-Chlor-1,3-dimethyl-imidazolium chlorid oder 2-Chlor-1-methylpyridinium iodid (siehe Chemistry of Peptide Synthsis, Ed. N. Leo Benoiton, Taylor & Francis, 2006, ISBN-10: 1-57444-454-9). Polymergebundene Reagenzien wie zum Beispiel polymergebundenes Dicyclohexylcarbodiimid sind auch für diese Kupplungs-reaktion geeignet. Die Reaktion findet bevorzugt in dem Temperaturbereich zwischen 0 °C und 80 °C, in einem adäquaten Lösungsmittel wie zum Beispiel Dichlormethan, Acetonitril, *N*,*N*-Dimethyl-formamid oder Ethylacetat und in Gegenwart eine Base wie zum Beispiel Triethylamin, *N*,*N*-Diisopropylethylamin oder 1,8-Diazabicyclo[5.4.0]undec-7-cen statt (siehe Schema 1). Für die T3P Peptidkupplungsbedingungen siehe Organic Process Research & Development 2009, 13, 900-906.

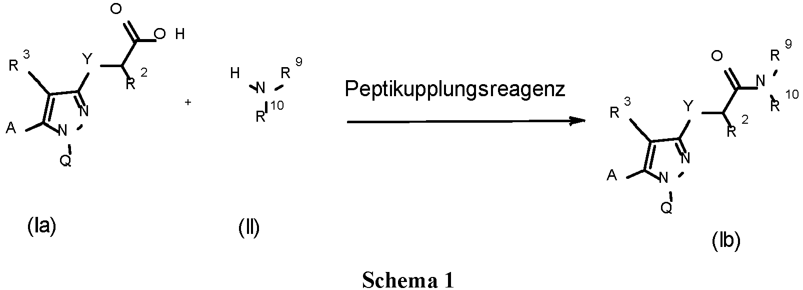

Die Synthese der Säure der allgemeinen Formel (Ia) lässt sich durch Verseifung der Verbindung der allgemeinen Formel (Ic) nach oder lassen sich analog dem Fachmann bekannten Methoden herstellen (Schema 2). Die Verseifung lässt sich in Gegenwart einer Base oder einer Lewis-Säure durchführen. Die Base kann ein Hydroxid-Salz von einem Alkali-Metall (wie zum Beispiel Lithium, Natrium oder Kalium) sein, und die Verseifungsreaktion findet bevorzugt in dem Temperaturbereich zwischen Raumtemperatur und 120 °C statt.

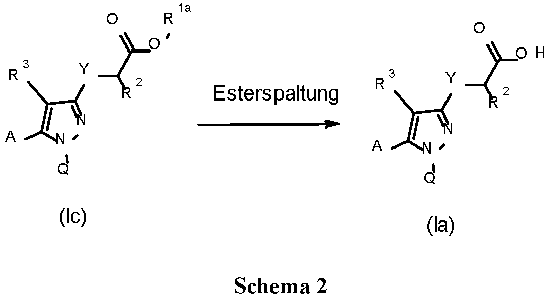

Die Synthese der Verbindung der allgemeinen Formel (Ic) lässt sich durch Alkylierung der Verbindung der allgemeinen Formel (III) mit einem Halogenid der allgemeinen Formel (IV) in Gegenwart einer Base nach oder analog dem Fachmann bekannten Methoden herstellen (siehe Schema 3). Die Base kann ein Carbonat-Salz von einem Alkali-Metall sein. Bevorzugt ist die Base Carbonat-Salz von einem Alkali-Metall ausgewählt aus der Gruppe bestehend aus Lithium, Natrium, Kalium und Cäsium.
(wie zum Beispiel Lithium, Natrium, Kalium oder Cäsium) sein, und die Reaktion findet bevorzugt in dem Temperaturbereich zwischen Raumtemperatur und 150 °C in einem adäquaten Lösungsmittel wie zum Beispiel Dichlormethan, Acetonitril, *N*,*N*-Dimethylformamid oder Ethylacetat statt. Siehe J. Med. Chem. 2011, 54(16), 5820-5835 und WO2010/010154. Der Rest "X" steht beispielsweise für Chlor, Brom oder lod.

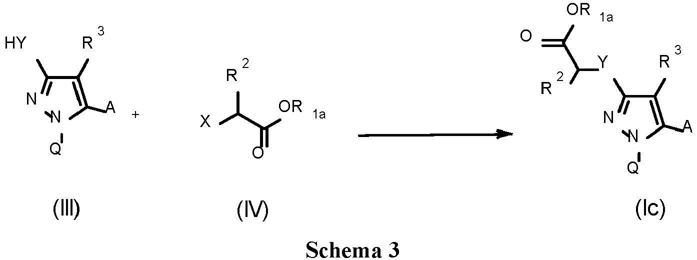

In wird die Synthese der Verbindung der allgemeinen Formel (VI) durch Reaktion eines Pyrazolones der allgemeinen Formel (V) in Gegenwart eines Schwefelungsreagenzes wie zum Beispiel Phosphorpentasulfid oder Lawesson-Reagenz in einem adäquaten Lösungsmittel wie zum Beispiel Toluol (Schema 4).

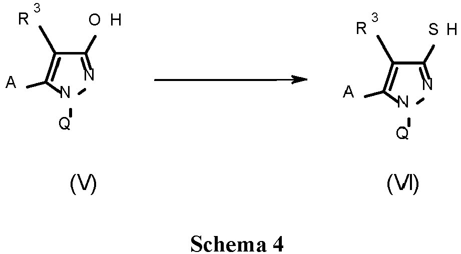

In Schema 5 wird die Synthese der Verbindung der allgemeinen Formel (V, R³ =Cl, Br, I) durch Reaktion eines Pyrazoles der allgemeinen Formel (VII) mit einem elektrophilen Halogenierungsreagenz der allgemeinen Formel (VIII) wie zum Beispiel N-Chlorsuccinimid (VIII, X= Cl), N-Bromosuccinimid (VIII, X =Br), oder N-Iodsuccinimid (VIII, R³ = I), beschrieben. In analoger Weise können auch andere elektrophile Reagenzien, beispielsweise elektrophile Nitrierungsreagenzien wie Nitriersäure, Nitroniumtetrafluoroborat oder Ammoniumnitrat/Trifluoressigsäure (für R³ = NO₂) oder elektrophile Fluorierungsreagenzien, wie DAST, Selectfluor oder *N*-Fluorbenzolsulfonimid (für R³ = F) zum Einsatz kommen. Die Reaktion findet bevorzugt in dem Temperaturbereich zwischen 0°C und 120 °C in einem adäquaten Lösungsmittel wie zum Beispiel *N*,*N*-Dimethylformamid, 1,2-Dichlorethan oder Acetonitril statt.

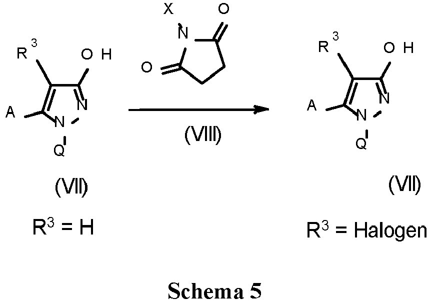

In Schema 6 wird die Synthese der Verbindung der allgemeinen Formel (Ic) durch Reaktion eines Pyrazoles der allgemeinen Formel (Ic) mit einem Halogensuccinimid der allgemeinen Formel (VIII) in einem adäquaten Lösungsmittel wie zum Beispiel N,N-Dimethylformamid beschrieben.

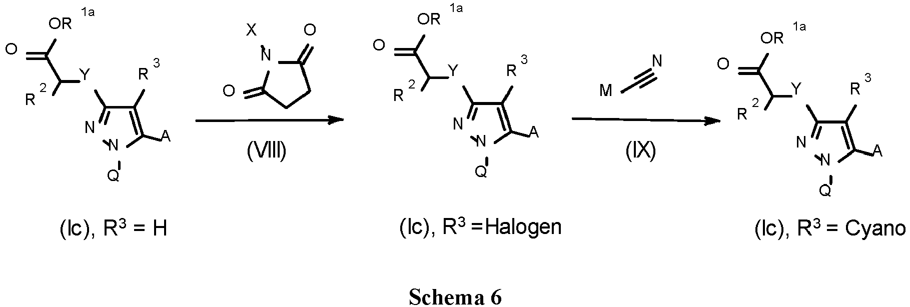

Eine Verbindung der allgemeinen Formel (Ic) lässt sich beispielsweise durch Reaktion von einer Verbindung der Formel (Ic) in einem geeigneten Lösungsmittel mit einem Metallcynid M-CN (IX) unter Zusatz einer adäquaten Menge eines Übergangsmetallkatalysators, insbesondere Palladium-Katalysatoren wie Palladium(0)tetrakis(triphenylphosphin) oder Palladiumdiacetat oder Bis(triphenylphosphin)-palladium(ll)dichlorid oder um Nickelkatalysatoren wie Nickel(ll)acetylacetonat oder Bis(triphenylphosphin)nickel(ll)chlorid vorzugsweise bei erhöhter Temperatur in einem organischen Lösungsmittel wie zum Beispiel 1,2-Dimethoxyethan oder N,N-Dimethylformamid darstellen (Schema 6). Der Rest "M" steht beispielsweise für Magnesium, Zink, Lithium oder Natrium. Allgemein eignen sich Methoden von Kreuzkupplungen, die in R. D. Larsen, Organometallics in Process Chemistry 2004 Springer Verlag, die in I. Tsuji, Palladium Reagents and Catalysts 2004 Wiley, die in M. Belier, C. Bolm, Transition Metals for Organic Synthesis 2004 VCH-Wiley beschrieben werden. Weitere geeignete Synthesemethoden sind in Chem. Rev. 2006, 106, 2651; Platinum Metals Review, 2009, 53, 183; Platinum Metals Review 2008, 52, 172 und Acc. Chem. Res. 2008, 41, 1486 beschrieben.

Die 3-Hydroxypyrazole (V) können analog literaturbekannter Methoden aus substituierten 3-Azinylpropinsäurederivaten und Phenyhydrazinen (Schema 7); z. B.: Adv. Synth. Catal. 2014, 356, 3135-3147) oder aus substituierten Azinylacrylsäurederivaten und Phenylhydrazinen (Schema 3; z. B.: J. Heterocyclic Chem., 49, 130 (2012)) hergestellt werden.

Die Synthese der Verbindungen der allgemeinen Formel (XII) erfolgt über eine Amidkupplung von einer Säure der allgemeinen Formel (X) mit einem Arylhydrazin oder Hetarylhydrazin der allgemeinen Formel (XI) in Gegenwart eines Amidkupplungsreagenzes wie zum Beispiel T3P, Dicyclohexylcarbodiimid, *N-*(3-Dimethylaminopropyl)-N'-ethylcarbodiimid, *N*,*N*'-Cabonyldiimidazol, 2-Chlor-1,3-dimethyl-imidazolium chlorid oder 2-Chlor-1-methylpyridinium iodid (siehe Chemistry of Peptide Synthesis, Ed. N. Leo Benoiton, Taylor & Francis, 2006, ISBN-10: 1-57444-454-9). Polymergebundene Reagenzien wie zum Beispiel polymergebundenes Dicyclohexylcarbodiimid sind auch für diese Kupplungsreaktion geeignet. Die Reaktion findet bevorzugt in dem Temperaturbereich zwischen 0 °C und 80 °C, in einem adäquaten Lösungsmittel wie zum Beispiel Dichlormethan, Tetrahydrofuran, Acetonitril, *N,N-*Dimethylformamid oder Ethylacetat und in Gegenwart eine Base wie zum Beispiel Triethylamin, *N,N-*Diisopropylethylamin oder 1,8-Diazabicyclo[5.4.0]undec-7-cen statt (siehe Schema 7). Für die T3P Peptidkupplungsbedingungen siehe Organic Process Research & Development 2009, 13, 900-906.

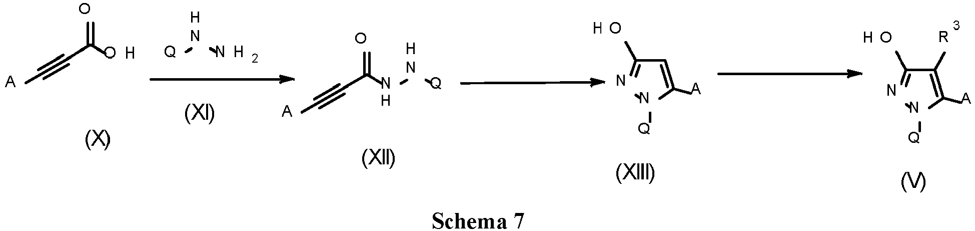

In Schema 7 wird die Synthese der Verbindung der allgemeinen Formel (V) durch Reaktion eines Pyrazoles der allgemeinen Formel (XIII) mit einem Elektrophil wie zum Beispiel N-Bromsuccinimid beschrieben. Die Reaktion findet bevorzugt in dem Temperaturbereich zwischen 0°C und 120 °C in einem adäquaten Lösungsmittel wie zum Beispiel N,N-Dimethylformamid, 1,2-Dichlorethan oder Acetonitril statt.

Die Synthese der 3-Hydroxypyrazole der allgemeinen Formel (XIII) erfolgt durch Reaktion der Verbindungen der allgemeinen Formel (XII) in Gegenwart eines Kupferhalogenides wie zum Beispiel Kupfer(I)iodid, Kupfer(I)bromid oder einer Base wie Natriummethylat oder einer Säure wie Methansulfonsäure zu 3-Hydroxypyrazolen der allgemeinen Formel (XIII) zyklysiert. Die Reaktion findet bevorzugt in dem Temperaturbereich zwischen 0 °C und 120 °C, in einem adäquaten Lösungsmittel wie zum Beispiel 1,2-Dichlorethan, Acetonitril, *N*,*N*-Dimethylformamid, n-Propanol oder Ethylacetat statt. Die Reaktion findet bevorzugt in *N*,*N*-Dimethylformamid statt.

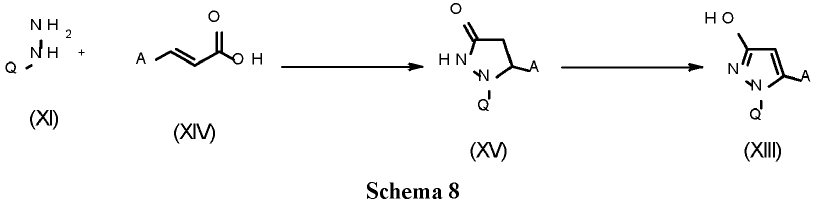

Verbindungen der allgemeinen Formel (XV) lassen sich durch eine eine Amidkupplung von einer substituiertem Propinsäure der allgemeinen Formel (XIV) mit einem Arylhydrazin oder Hetarylhydrazin der allgemeinen Formel (XI) in Gegenwart eines Amidkupplungsreagenzes wie zum Beispiel T3P, Dicyclohexylcarbodiimid, *N-*(3-Dimethylaminopropyl)-*N*'-ethylcarbodiimid, *N*,*N*'-Cabonyldiimidazol, 2-Chlor-1,3-dimethyl-imidazolium chlorid oder 2-Chlor-1-methylpyridinium iodid. Die Reaktion findet bevorzugt in dem Temperaturbereich zwischen 0 °C und 80 °C, in einem adäquaten Lösungsmittel wie zum Beispiel Dichlormethan, Acetonitril, *N,N-*Dimethylformamid oder Ethylacetat und in Gegenwart eine Base wie zum Beispiel Triethylamin, *N*,*N*-Diisopropylethylamin oder 1,8-Diazabicyclo[5.4.0]undec-7-cen statt (siehe Schema 8).

Die Synthese der 3-Hydroxypyrazole der allgemeinen Formel (XIII) erfolgt durch Reaktion der Verbindungen der allgemeinen Formel (XV) in Gegenwart eines Eisenhalogenides wie zum Beispiel Eisen(III)chlorid. Die Reaktion findet bevorzugt in dem Temperaturbereich zwischen 0 °C und 120 °C, in einem adäquaten Lösungsmittel wie zum Beispiel 1,2-Dichlorethan, Acetonitril, *N*,*N*-Dimethylformamid oder Ethylacetat statt.

Verbindungen der allgemeinen Formel (XVI) lassen sich durch eine N-Arylierung von einer 3-Hydroxypyrazole der allgemeinen Formel (XIV) mit einem Arylhalogenid in Gegegenwart eines Kupferhalogenides wie zum Beispiel Kupfer(I)iodid. Die Reaktion findet bevorzugt in dem Temperaturbereich zwischen 0 °C und 120 °C, in einem adäquaten Lösungsmittel wie zum Beispiel Acetonitril oder *N*,*N*-Dimethylformamid und in Gegenwart eine Base wie zum Beispiel Triethylamin, Cäsium Carbonat (siehe Schema 9). Die Verbindungen der allgemeinen Formeln (XIV) können nach analog dem Fachmann bekannten Methoden hergestellt werden (Chem. Med. Chem. 2015, 10, 1184-1199). Der Rest "X"s teht beispielsweise für Chlor, Brom oder lod.

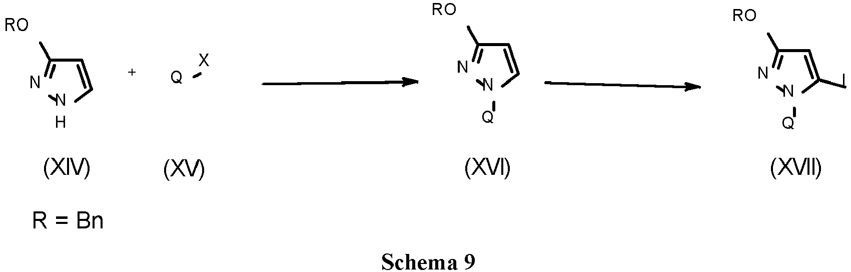

Die Synthese der 5-Iodpyrazole der allgemeinen Formel (XIV) erfolgt durch Reaktion der Verbindungen der allgemeinen Formel (XIII) in Gegenwart einer Base wie zum Beispiel Lithiumdiisopropylamid und Iod. Die Reaktion (Schema 7) findet bevorzugt in dem Temperaturbereich zwischen -78 °C und -60°C, in einem adäquaten Lösungsmittel wie zum Beispiel Diethylether und Tetrahydrofuran.

Eine Verbindung der Formel (XIII) lasst sich beispielsweise durch Reaktion von einer Verbindung der Formel (XVI) in einem geeigneten Lösungsmittel mit einem A-M unter Zusatz einer adäquaten Menge eines Ubergangsmetallkatalysators, insbesondere Palladium-katalysatoren wie Palladiumdiacetat oder Bis(triphenylphosphin)palladium(ll)dichlorid oder um Nickelkatalysatoren wie Nickel(ll)acetylacetonat oder Bis(triphenylphosphin)nickel(ll) chlorid vorzugsweise bei erhöhter Temperatur in einem organischen Lösungsmittel wie 1,2-Dimethoxyethan darstellen. Der Rest "M" steht beispielsweise für B(OR^{b})(OR^{c}), wobei die Reste R^{b} und R^{c} unabhängig voneinander beispielsweise Wasserstoff, (C₁-C₄)-Alkyl, oder, wenn die Reste R^{b} und R^{c} miteinander verbunden sind, gemeinsam Ethylen oder Propylen bedeuten (Schema 10).

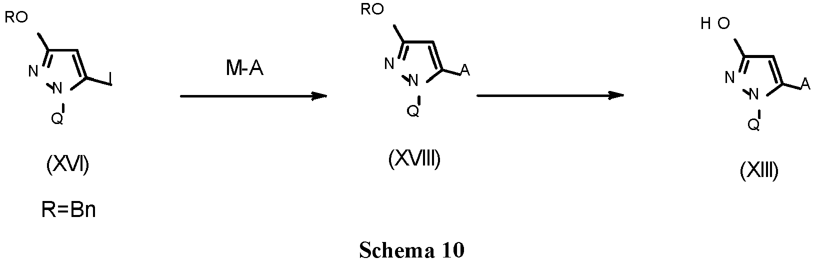

Die Synthese der Verbindung der allgemeinen Formel (XX) lässt sich durch Alkylierung der Verbindung der allgemeinen Formel (XIX) mit einem Halogenid der allgemeinen Formel (IV) in Gegenwart einer Base nach oder analog dem Fachmann bekannten Methoden herstellen (siehe Schema 11). Die Base kann ein Carbonat-Salz von einem Alkali-Metall (wie zum Beispiel Lithium, Natrium, Kalium oder Cäsium) sein, und die Reaktion findet bevorzugt in dem Temperaturbereich zwischen Raumtemperatur und 150 °C in einem adäquaten Lösungsmittel wie zum Beispiel Dichlormethan, Acetonitril, *N,N-*Dimethylformamid oder Ethylacetat statt.

Eine Verbindung der Formel (Ic) lasst sich beispielsweise durch Reaktion von einer Verbindung der Formel (XXI) in einem geeigneten Lösungsmittel mit einem A-M unter Zusatz einer adäquaten Menge eines Ubergangsmetallkatalysators, insbesondere Palladium-katalysatoren wie Palladiumdiacetat oder Bis(triphenylphosphin)palladium(ll)dichlorid oder um Nickelkatalysatoren wie Nickel(ll)acetylacetonat oder Bis(triphenylphosphin)nickel(ll) chlorid vorzugsweise bei erhöhter Temperatur in einem organischen Lösungsmittel wie 1,2-Dimethoxyethan darstellen. Der Rest "M" steht beispielsweise für Mg-Hal, Zn-Hal, Sn((C₁-C₄)Alkyl)₃, Lithium, Kupfer oder B(OR^{b})(OR^{c}), wobei die Reste R^{b} und R^{c} unabhängig voneinander beispielsweise Wasserstoff, (C₁-C₄)-Alkyl, oder, wenn die Reste R^{b} und R^{c} miteinander verbunden sind, gemeinsam Ethylen oder Propylen bedeuten.

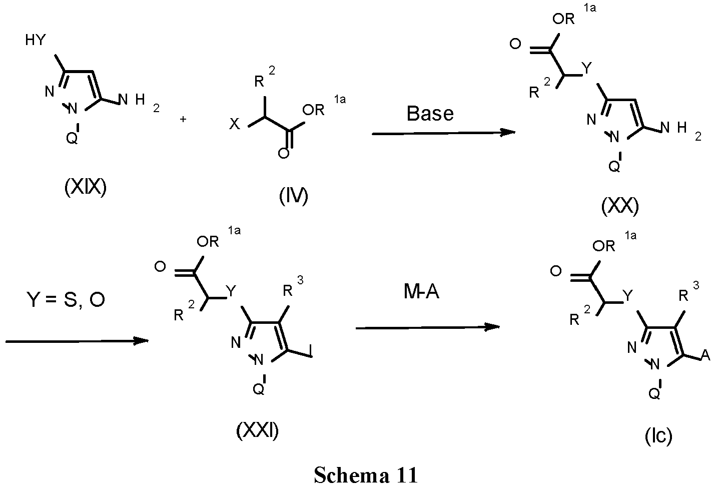

Verbindungen der allgemeinen Formel (XIX) lassen sich durch eine Diazotierung oder Sandmeyer Reaktion mit der Verbindung der allgemeinen Formel (XVIII) mit den üblichen organischen und anorganischen Nitrite wie beispielsweise 1,1-Dimethylethylnitrit, tert-Butylnitrit oder Isoamylnitrit in Gegenwart verwendbare Reagenzien wie beispielsweise Gemische aus Kupfer(I)- und Kupfer(II)bromide/chlorid oder Iod (Schema 11). Die Reaktion findet bevorzugt in dem Temperaturbereich zwischen Raumtemperatur und 0 und 120°C in einem adäquaten Lösungsmittel wie zum Beispiel Dichlormethan, Acetonitril, *N*,*N*-Dimethylformamid oder Diiodmethan statt. Der Rest "X" steht beispielsweise für Chlor, Brom oder lod.

In Schema 12 wird die Synthese der Verbindung der allgemeinen Formel (XXIII) durch Reaktion eines Pyrazolones der allgemeinen Formel (XXII) in Gegenwart eines Schwefelungsreagenzes wie zum Beispiel Phosphorpentasulfid oder Lawesson-Reagenz in einem adäquaten Lösungsmittel wie zum Beispiel Toluol. Die Verbindungen der allgemeinen Formeln (XXII) sind kommerziell erhältlich oder analog dem Fachmann bekannten Methoden herstellen.

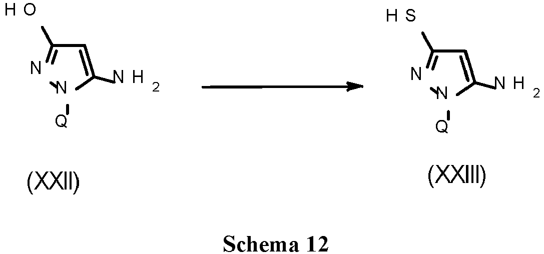

Die Synthese der Verbindung der allgemeinen Formel (Id) lässt sich durch Reaktion eines 3-Aminopyrazoles der allgemeinen Formel (XXIV) mit einem Disulfid der allgemeinen Formel (XXV) in Gegenwart eines organischen Nitrites wie beispielsweise 1,1-Dimethylethylnitrit, tert-Butylnitrit oder Isoamylnitrit in Gegenwart von einem Metall wie zum Beispiel Kupfer statt (siehe Schema 13 ). Die Reaktion findet bevorzugt in dem Temperaturbereich zwischen Raumtemperatur und 120°C in einem adäquaten Lösungsmittel wie zum Beispiel Dichlormethan, Acetonitril, *N,N-*Dimethylformamid oder 1,2-Dichlorethan statt. 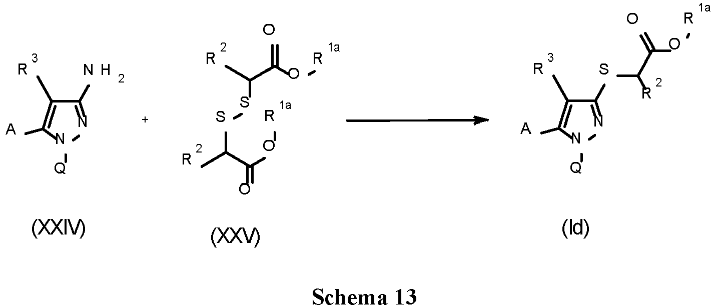 Mit R^{1a} = (C₁-C₄)-Alkyl.

Die Synthese der Säure der allgemeinen Formel (XXIV) lässt sich durch Reaktion der Verbindung der allgemeinen Formel (XXVI) in Gegenwart einer Lewis-Säure wie zum Beispiel Trifluoressigsäure nach oder lassen sich analog dem Fachmann bekannten Methoden herstellen (Schema 14). Die Reaktion findet bevorzugt in dem Temperaturbereich zwischen Raumtemperatur und 140 °C statt. 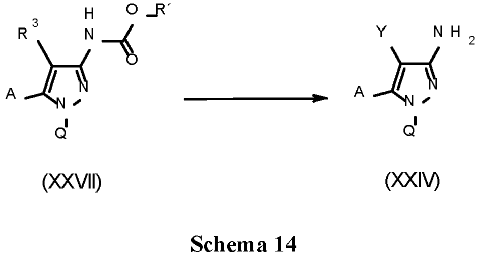 Mit R' = (C₁-C₄)-Alkyl

Die Synthese der Verbindungen der allgemeinen Formel (XXVI) erfolgt über eine Curtius-Reaktion von einer Säure der allgemeinen Formel (XXVII) mit einem Azid der allgemeinen Formel (XXVIII). Die Reaktion findet bevorzugt in dem Temperaturbereich zwischen 0 °C und 100 °C, in einem adäquaten Lösungsmittel wie zum Beispiel tert.-Butanol und in Gegenwart eine Base wie zum Beispiel Triethylamin, *N*,*N*-Diisopropylethylamin oder 1,8-Diazabicyclo[5.4.0]undec-7-cen statt (siehe Schema 15).

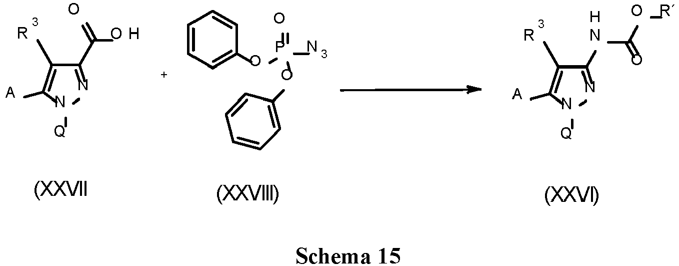

Die Synthese der Säure der allgemeinen Formel (XXIX) lässt sich durch Verseifung der Verbindung der allgemeinen Formel (XXVII) nach oder lassen sich analog dem Fachmann bekannten Methoden herstellen (Schema 16). Die Verseifung lässt sich in Gegenwart einer Base oder einer Lewis-Säure durchführen. Die Base kann ein Hydroxid-Salz von einem Alkali-Metall (wie zum Beispiel Lithium, Natrium oder Kalium) sein, und die Verseifungsreaktion findet bevorzugt in dem Temperaturbereich zwischen Raumtemperatur und 120 °C statt. 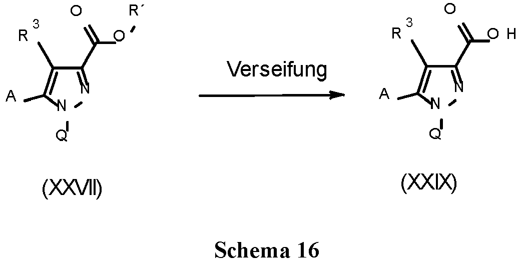 Mit R' = (C₁-C₄)-Alkyl.

In Schema 17 wird die Synthese der Verbindung der allgemeinen Formel (XXX) durch Reaktion eines Pyrazoles der allgemeinen Formel (XXVII) mit einem Halogensuccinimid der allgemeinen Formel (VIII) in einem adäquaten Lösungsmittel wie zum Beispiel DMF beschrieben. 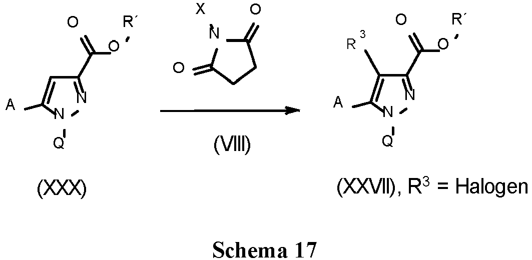 Mit X = Halogen.

Die Synthese der Verbindungen der allgemeinen Formel (XXIX) erfolgt über eine Kondensation von eines Diketoesters der allgemeinen Formel (XXXI) mit einem Arylhydrazin oder Hetarylhydrazin der allgemeinen Formel (XI) in Gegenwart einer Broensted-Säure wie zum Beispiel Essigsäure oder Chlorwasserstoff in einem adäquaten Lösungsmittel wie zum Beispiel Methanol, Ethanol, Isopropanol, n-Butanol, Tetrahydrofuran, Dioxan, Toluol oder Chlorbenzol (Schema 18). Die Reaktion findet bevorzugt in dem Temperaturbereich zwischen 0 °C und 150 °C statt. Die Verbindungen der allgemeinen Formeln (XI) und (XXXI) sind kommerziell erhältlich oder lassen sich analog dem Fachmann bekannten Methoden herstellen. 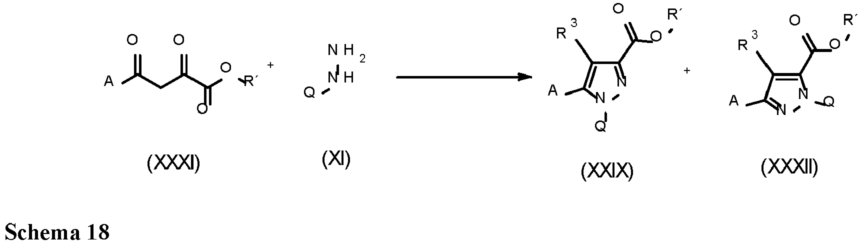 Mit R' = (C₁-C₄)-Alkyl.

Verbindungen der allgemeinen Formeln (XXXIV) und (XXXV) lassen sich durch Reaktion von einer Verbindung der Formel (XXXIII) in Gegenwart eines Oxidationsmittels wie zum Beispiel mCPBA (3-Chlorperbenzoesäure) in einem adäquaten Lösungsmittel wie zum Beispiel Dichlormethan oder 1,2-Dichlorethan herstellen (Schema 19). Die Reaktion findet bevorzugt in dem Temperaturbereich zwischen -30 °C und 100 °C statt.

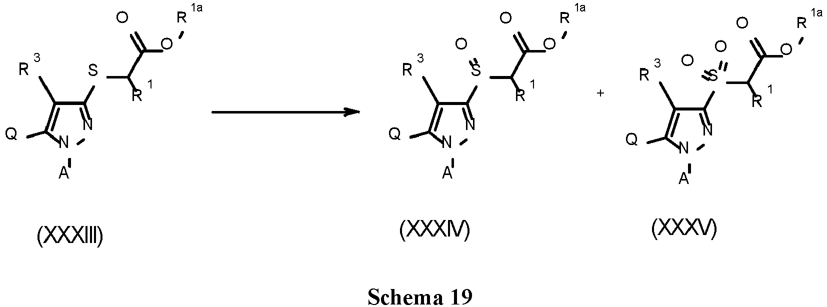

Die erfindungsgemäßen Verbindungen der Formel (I) (und/oder deren Salze), im folgenden zusammen als "erfindungsgemäße Verbindungen" bezeichnet, weisen eine ausgezeichnete herbizide Wirksamkeit gegen ein breites Spektrum wirtschaftlich wichtiger mono- und dikotyler annueller Schadpflanzen auf. Gegenstand der vorliegenden Erfindung ist daher auch ein Verfahren zur Bekämpfung von unerwünschten Pflanzen oder zur Wachstumsregulierung von Pflanzen, vorzugsweise in Pflanzenkulturen, worin eine oder mehrere erfindungsgemäße Verbindung(en) auf die Pflanzen (z.B. Schadpflanzen wie mono- oder dikotyle Unkräuter oder unerwünschte Kulturpflanzen), das Saatgut (z.B. Körner, Samen oder vegetative Vermehrungsorgane wie Knollen oder Sprossteile mit Knospen) oder die Fläche, auf der die Pflanzen wachsen (z.B. die Anbaufläche), ausgebracht werden. Dabei können die erfindungsgemäßen Verbindungen z.B. im Vorsaat- (ggf. auch durch Einarbeitung in den Boden), Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne dass durch die Nennung eine Beschränkung auf bestimmte Arten erfolgen soll.

Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Dikotyle Unkräuter der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Die erfindungsgemäßen Verbindungen können in Nutzkulturen Selektivitäten aufweisen und können auch als nichtselektive Herbizide eingesetzt werden.

Aufgrund ihrer herbiziden und pflanzenwachstumsregulatorischen Eigenschaften können die Wirkstoffe auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten in der Agrarindustrie verwendeten Wirkstoff, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z.B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt. Weitere besondere Eigenschaften liegen in einer Toleranz oder Resistenz gegen abiotische Stressoren z.B. Hitze, Kälte, Trockenheit, Salz und ultraviolette Strahlung.

Bevorzugt ist die Anwendung der erfindungsgemäßen Verbindungen der Formel (I) oder deren Salze in wirtschaftlich bedeutenden transgenen Kulturen von Nutz-und Zierpflanzen,

Die Verbindungen der Formel (I) können als Herbizide in Nutzpflanzenkulturen eingesetzt werden, welche gegenüber den phytotoxischen Wirkungen der Herbizide resistent sind bzw. gentechnisch resistent gemacht wurden.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z.B. EP 0221044, EP 0131624). Beschrieben wurden beispielsweise in mehreren Fällen gentechnische Veränderungen von Kulturpflanzen zwecks Modifikation der in den Pflanzen synthetisierten Stärke (z.B. WO 92/011376 A, WO 92/014827 A, WO 91/019806 A), transgene Kulturpflanzen, welche gegen bestimmte Herbizide vom Typ Glufosinate (vgl. z.B. EP 0242236 A, EP 0242246 A) oder Glyphosate (WO 92/000377 A) oder der Sulfonylharnstoffe (EP 0257993 A, US 5,013,659) oder gegen Kombinationen oder Mischungen dieser Herbizide durch "gene stacking" resistent sind, wie transgenen Kulturpflanzen z. B. Mais oder Soja mit dem Handelsnamen oder der Bezeichnung Optimum^{™} GAT^{™} (Glyphosate ALS Tolerant).
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP 0142924 A, EP 0193259 A).
- transgene Kulturpflanzen mit modifizierter Fettsäurezusammensetzung (WO 91/013972 A).
- gentechnisch veränderte Kulturpflanzen mit neuen Inhalts- oder Sekundärstoffen z.B. neuen Phytoalexinen, die eine erhöhte Krankheitsresistenz verursachen (EP 0309862 A, EP 0464461 A)
- gentechnisch veränderte Pflanzen mit reduzierter Photorespiration, die höhere Erträge und höhere Stresstoleranz aufweisen (EP 0305398 A)
- transgene Kulturpflanzen, die pharmazeutisch oder diagnostisch wichtige Proteine produzieren ("molecular pharming")
- transgene Kulturpflanzen, die sich durch höhere Erträge oder bessere Qualität auszeichnen
- transgene Kulturpflanzen die sich durch eine Kombinationen z.B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt; siehe z.B. I. Potrykus und G. Spangenberg (eds.) Gene Transfer to Plants, Springer Lab Manual (1995), Springer Verlag Berlin, Heidelberg. oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z.B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z.B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet. Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z.B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen. So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen. Vorzugsweise können die erfindungsgemäßen Verbindungen (I) in transgenen Kulturen eingesetzt werden, welche gegen Wuchsstoffe, wie z.B. 2,4-D, Dicamba oder gegen Herbizide, die essentielle Pflanzenenzyme, z.B. Acetolactatsynthasen (ALS), EPSP Synthasen, Glutaminsynthasen (GS) oder Hydoxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, oder gegen beliebige Kombinationen dieser Wirkstoffe, resistent sind.

Besonders bevorzugt können die erfindungsgemäßen Verbindungen in transgenen Kulturpflanzen eingesetzt werden, die gegen eine Kombination von Glyphosaten und Glufosinaten, Glyphosaten und Sulfonylharnstoffen oder Imidazolinonen resistent sind. Ganz besonders bevorzugt können die erfindungsgemäßen Verbindungen in transgenen Kulturpflanzen wie z. B. Mais oder Soja mit dem Handelsnamen oder der Bezeichnung OptimumTM GATTM (Glyphosate ALS Tolerant) eingesetzt werden.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen der Formel (I) als Herbizide zur Bekämpfung von Schadpflanzen in transgenen Kulturpflanzen.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, welche die erfindungsgemäßen Verbindungen enthalten.

Die erfindungsgemäßen Verbindungen können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse. Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986, Wade van Valkenburg, "Pesticide Formulations", Marcel Dekker, N.Y., 1973, K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry", 2nd Ed., J. Wiley & Sons, N.Y., C. Marsden, "Solvents Guide", 2nd Ed., Interscience, N.Y. 1963, McCutcheon's "Detergents and Emulsifiers Annual", MC Publ. Corp., Ridgewood N.J., Sisley and Wood, "Encyclopedia of Surface Active Agents", Chem. Publ. Co. Inc., N.Y. 1964, Schönfeldt, "Grenzflächenaktive Äthylenoxid-addukte", Wiss. Verlagsgesell., Stuttgart 1976, Winnacker-Küchler, "Chemische Technologie", Band 7, C. Hanser Verlag München, 4. Aufl. 1986. Auf der Basis dieser Formulierungen lassen sich auch Kombinationen mit anderen Wirkstoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Als Kombinationspartner für die erfindungsgemäßen Verbindungen in Mischungsformulierungen oder im Tank-Mix sind beispielsweise bekannte Wirkstoffe, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II oder Protoporphyrinogen-Oxidase beruhen, einsetzbar, wie sie z.B. aus Weed Research 26 (1986) 441-445 oder "The Pesticide Manual", 16th edition, The British Crop Protection Council und the Royal Soc. of Chemistry, 2006 und dort zitierter Literatur beschrieben sind. Nachfolgend werden beispielhaft bekannte Herbizide oder Pflanzenwachstumsregulatoren genannt, die mit den erfindungsgemäßen Verbindungen kombiniert werden können, wobei diese Wirkstoffe entweder mit ihrem "common name" in der englischsprachigen Variante gemäß International Organization for Standardization (ISO) oder mit dem chemischen Namen bzw. mit der Codenummer bezeichnet sind. Dabei sind stets sämtliche Anwendungsformen wie beispielsweise Säuren, Salze, Ester sowie auch alle isomeren Formen wie Stereoisomere und optische Isomere umfaßt, auch wenn diese nicht explizit erwähnt sind. Beispiele für solche herbiziden Mischungspartner sind:
Acetochlor, acifluorfen, acifluorfen-sodium, aclonifen, alachlor, allidochlor, alloxydim, alloxydimsodium, ametryn, amicarbazone, amidochlor, amidosulfuron, 4-amino-3-chloro-5-fluoro-6-(7-fluoro-1H-indol-6-yl)pyridine-2-carboxylic acid, aminocyclopyrachlor, aminocyclopyrachlor-potassium, aminocyclopyrachlor-methyl, aminopyralid, amitrole, ammoniumsulfamate, anilofos, asulam, atrazine, azafenidin, azimsulfuron, beflubutamid, benazolin, benazolin-ethyl, benfluralin, benfuresate, bensulfuron, bensulfuron-methyl, bensulide, bentazone, benzobicyclon, benzofenap, bicyclopyron, bifenox, bilanafos, bilanafos-sodium, bispyribac, bispyribac-sodium, bixlozone, bromacil, bromobutide, bromofenoxim, bromoxynil, bromoxynil-butyrate, -potassium, -heptanoate und -octanoate, busoxinone, butachlor, butafenacil, butamifos, butenachlor, butralin, butroxydim, butylate, cafenstrole, carbetamide, carfentrazone, carfentrazone-ethyl, chloramben, chlorbromuron, 1-{2-Chlor-3-[(3-cyclopropyl-5-hydroxy-1-methyl-1H-pyrazol-4-yl)carbonyl]-6-(trifluormethyl)phenyl}piperidin-2-on, 4-{2-Chlor-3-[(3,5-dimethyl-1H-pyrazol-1-yl)methyl]-4-(methylsulfonyl)benzoyl}-1,3-dimethyl-1H-pyrazol-5-yl-1,3-dimethyl-1H-pyrazol-4-carboxylat, chlorfenac, chlorfenac-sodium, chlorfenprop, chlorflurenol, chlorflurenol-methyl, chloridazon, chlorimuron, chlorimuron-ethyl, 2-[2-Chlor-4-(methylsulfonyl)-3-(morpholin-4-ylmethyl)benzoyl]-3-hydroxycyclohex-2-en-1-on, 4-{2-Chlor-4-(methylsulfonyl)-3-[(2,2,2-trifluorethoxy)methyl]benzoyl}-1-ethyl-1H-pyrazol-5-yl-1,3-dimethyl-1H-pyrazol-4-carboxylat, chlorophthalim, chlorotoluron, chlorthal-dimethyl, chlorsulfuron, 3-[5-Chlor-4-(trifluormethyl)pyridin-2-yl]-4-hydroxy-1-methylimidazolidin-2-on, cinidon, cinidon-ethyl, cinmethylin, cinosulfuron, clacyfos, clethodim, clodinafop, clodinafop-propargyl, clomazone, clomeprop, clopyralid, cloransulam, cloransulam-methyl, cumyluron, cyanamide, cyanazine, cycloate, cyclopyranil, cyclopyrimorate, cyclosulfamuron, cycloxydim, cyhalofop, cyhalofop-butyl, cyprazine, 2,4-D, 2,4-D-butotyl, -butyl, - dimethylammonium, -diolamin, -ethyl, 2-ethylhexyl, -isobutyl, -isooctyl, -isopropylammonium, - potassium, -triisopropanolammonium und -trolamine, 2,4-DB, 2,4-DB-butyl, -dimethylammonium, isooctyl, -potassium und -sodium, daimuron (dymron), dalapon, dazomet, n-decanol, desmedipham, detosyl-pyrazolate (DTP), dicamba, dichlobenil, dichlorprop, dichlorprop-P, diclofop, diclofop-methyl, diclofop-P-methyl, diclosulam, difenzoquat, diflufenican, diflufenzopyr, diflufenzopyr-sodium, dimefuron, dimepiperate, dimethachlor, dimethametryn, dimethenamid, dimethenamid-P, 3-(2,6-Dimethylphenyl)-6-[(2-hydroxy-6-oxocyclohex-1-en-1-yl)carbonyl]-1-methylchinazolin-2,4(1H,3H)-dion, 1,3-Dimethyl-4-[2-(methylsulfonyl)-4-(trifluormethyl)benzoyl]-1H-pyrazol-5-yl-1,3-dimethyl-1H-pyrazol-4-carboxylat, dimetrasulfuron, dinitramine, dinoterb, diphenamid, diquat, diquat-dibromid, dithiopyr, diuron, DMPA, DNOC, endothal, EPTC, esprocarb, ethalfluralin, ethametsulfuron, ethametsulfuron-methyl, ethiozin, ethofumesate, ethoxyfen, ethoxyfen-ethyl, ethoxysulfuron, etobenzanid, Ethyl-[(3-{2-chlor-4-fluor-5-[3-methyl-2,6-dioxo-4-(trifluormethyl)-3,6-dihydropyrimidin-1 (2H)-yl]phenoxy}pyridin-2-yl)oxy]acetat, F-9960, F-5231, i.e. N-[2-Chlor-4-fluor-5-[4-(3-fluorpropyl)-4,5-dihydro-5-oxo-1H-tetrazol-1-yl]-phenyl]-ethansulfonamid, F-7967, i.e. 3-[7-Chlor-5-fluor-2-(trifluormethyl)-1H-benzimidazol-4-yl]-1-methyl-6-(trifluormethyl)pyrimidin-2,4(1H,3H)-dion, fenoxaprop, fenoxaprop-P, fenoxaprop-ethyl, fenoxaprop-P-ethyl, fenoxasulfone, fenquinotrione, fentrazamide, flamprop, flamprop-M-isopropyl, flamprop-M-methyl, flazasulfuron, florasulam, florpyrauxifen, florpyrauxifen-benzyl, fluazifop, fluazifop-P, fluazifop-butyl, fluazifop-P-butyl, flucarbazone, flucarbazone-sodium, flucetosulfuron, fluchloralin, flufenacet, flufenpyr, flufenpyr-ethyl, flumetsulam, flumiclorac, flumiclorac-pentyl, flumioxazin, fluometuron, flurenol, flurenol-butyl, - dimethylammonium und -methyl, fluoroglycofen, fluoroglycofen-ethyl, flupropanate, flupyrsulfuron, flupyrsulfuron-methyl-sodium, fluridone, flurochloridone, fluroxypyr, fluroxypyr-meptyl, flurtamone, fluthiacet, fluthiacet-methyl, fomesafen, fomesafen-sodium, foramsulfuron, fosamine, glufosinate, glufosinate-ammonium, glufosinate-P-sodium, glufosinate-P-ammonium, glufosinate-P-sodium, glyphosate, glyphosate-ammonium, -isopropylammonium, -diammonium, -dimethylammonium, - potassium, -sodium und -trimesium, H-9201, i.e. O-(2,4-Dimethyl-6-nitrophenyl)-O-ethyl-isopropylphosphoramidothioat, halauxifen, halauxifen-methyl, halosafen, halosulfuron, halosulfuronmethyl, haloxyfop, haloxyfop-P, haloxyfop-ethoxyethyl, haloxyfop-P-ethoxyethyl, haloxyfop-methyl, haloxyfop-P-methyl, hexazinone, HW-02, i.e. 1-(Dimethoxyphosphoryl)-ethyl-(2,4-dichlorphenoxy)acetat, 4-Hydroxy-1-methoxy-5-methyl-3-[4-(trifluormethyl)pyridin-2-yl]imidazolidin-2-on, 4-Hydroxy-1-methyl-3-[4-(trifluormethyl)pyridin-2-yl]imidazolidin-2-on, (5-Hydroxy-1-methyl-1H-pyrazol-4-yl)(3,3,4-trimethyl-1,1-dioxido-2,3-dihydro-1-benzothiophen-5-yl)methanon, 6-[(2-Hydroxy-6-oxocyclohex-1-en-1-yl)carbonyl]-1,5-dimethyl-3-(2-methylphenyl)chinazolin-2,4(1H,3H)-dion, imazamethabenz, Imazamethabenz-methyl, imazamox, imazamox-ammonium, imazapic, imazapicammonium, imazapyr, imazapyr-isopropylammonium, imazaquin, imazaquin-ammonium, imazethapyr, imazethapyr-immonium, imazosulfuron, indanofan, indaziflam, iodosulfuron, iodosulfuron-methyl-sodium, ioxynil, ioxynil-octanoate, -potassium und sodium, ipfencarbazone, isoproturon, isouron, isoxaben, isoxaflutole, karbutilate, KUH-043, i.e. 3-({[5-(Difluormethyl)-1-methyl-3-(trifluormethyl)-1H-pyrazol-4-yl]methyl}sulfonyl)-5,5-dimethyl-4,5-dihydro-1,2-oxazol, ketospiradox, lactofen, lenacil, linuron, MCPA, MCPA-butotyl, -dimethylammonium, -2-ethylhexyl, -isopropylammonium, -potassium und -sodium, MCPB, MCPB-methyl, -ethyl und -sodium, mecoprop, mecoprop-sodium, und -butotyl, mecoprop-P, mecoprop-P-butotyl, -dimethylammonium, -2-ethylhexyl und -potassium, mefenacet, mefluidide, mesosulfuron, mesosulfuron-methyl, mesotrione, methabenzthiazuron, metam, metamifop, metamitron, metazachlor, metazosulfuron, methabenzthiazuron, methiopyrsulfuron, methiozolin, 2-({2-[(2-Methoxyethoxy)methyl] -6-(trifluormethyl)pyridin-3 -yl} carbonyl)cyclohexan-1,3 -dion, methyl isothiocyanate, 1-Methyl-4-[(3,3,4-trimethyl-1,1-dioxido-2,3-dihydro-1-benzothiophen-5-yl)carbonyl]-1H-pyrazol-5-ylpropan-1-sulfonat, metobromuron, metolachlor, S-metolachlor, metosulam, metoxuron, metribuzin, metsulfuron, metsulfuron-methyl, molinat, monolinuron, monosulfuron, monosulfuron-ester, MT-5950, i.e. N-[3-chlor-4-(1-methylethyl)-phenyl]-2-methylpentanamid, NGGC-011, napropamide, NC-310, i.e. 4-(2,4-Dichlorbenzoyl)-1-methyl-5-benzyloxypyrazol, neburon, nicosulfuron, nonanoic acid (Pelargonsäure), norflurazon, oleic acid (fatty acids), orbencarb, orthosulfamuron, oryzalin, oxadiargyl, oxadiazon, oxasulfuron, oxaziclomefon, oxotrione (lancotrione), oxyfluorfen, paraquat, paraquat dichloride, pebulate, pendimethalin, penoxsulam, pentachlorphenol, pentoxazone, pethoxamid, petroleum oils, phenmedipham, picloram, picolinafen, pinoxaden, piperophos, pretilachlor, primisulfuron, primisulfuron-methyl, prodiamine, profoxydim, prometon, prometryn, propachlor, propanil, propaquizafop, propazine, propham, propisochlor, propoxycarbazone, propoxycarbazone-sodium, propyrisulfuron, propyzamide, prosulfocarb, prosulfuron, pyraclonil, pyraflufen, pyraflufen-ethyl, pyrasulfotole, pyrazolynate (pyrazolate), pyrazosulfuron, pyrazosulfuron-ethyl, pyrazoxyfen, pyribambenz, pyribambenz-isopropyl, pyribambenz-propyl, pyribenzoxim, pyributicarb, pyridafol, pyridate, pyriftalid, pyriminobac, pyriminobac-methyl, pyrimisulfan, pyrithiobac, pyrithiobac-sodium, pyroxasulfone, pyroxsulam, quinclorac, quinmerac, quinoclamine, quizalofop, quizalofop-ethyl, quizalofop-P, quizalofop-P-ethyl, quizalofop-P-tefuryl, QYM-201, QYR-301, rimsulfuron, saflufenacil, sethoxydim, siduron, simazine, simetryn, sulcotrion, sulfentrazone, sulfometuron, sulfometuron-methyl, sulfosulfuron, , SYN-523, SYP-249, i.e. 1-Ethoxy-3-methyl-1-oxobut-3-en-2-yl-5-[2-chlor-4-(trifluormethyl)phenoxy]-2-nitrobenzoat, SYP-300, i.e. 1-[7-Fluor-3-oxo-4-(prop-2-in-1-yl)-3,4-dihydro-2H-1,4-benzoxazin-6-yl]-3-propyl-2-thioxoimidazolidin-4,5-dion, 2,3,6-TBA, TCA (Trifluoressigsäure), TCA-sodium, tebuthiuron, tefuryltrione, tembotrione, tepraloxydim, terbacil, terbucarb, terbumeton, terbuthylazin, terbutryn, tetflupyrolimet, thenylchlor, thiazopyr, thiencarbazone, thiencarbazone-methyl, thifensulfuron, thifensulfuron-methyl, thiobencarb, tiafenacil, tolpyralate, topramezone, tralkoxydim, triafamone, tri-allate, triasulfuron, triaziflam, tribenuron, tribenuron-methyl, triclopyr, trietazine, trifloxysulfuron, trifloxysulfuron-sodium, trifludimoxazin, trifluralin, triflusulfuron, triflusulfuronmethyl, tritosulfuron, urea sulfate, vernolate, ZJ-0862, i.e. 3,4-Dichlor-N-{2-[(4,6-dimethoxypyrimidin-2-yl)oxy]benzyl} anilin.

Beispiele für Pflanzenwachstumsregulatoren als mögliche Mischungspartner sind:
Acibenzolar, acibenzolar-S-methyl, 5-Aminolävulinsäure, ancymidol, 6-benzylaminopurine, Brassinolid, Catechin, chlormequat chloride, cloprop, cyclanilide, 3-(Cycloprop-1-enyl)propionsäure, daminozide, dazomet, n-decanol, dikegulac, dikegulac-sodium, endothal, endothal-dipotassium, -disodium, und mono(N,N-dimethylalkylammonium), ethephon, flumetralin, flurenol, flurenol-butyl, flurprimidol, forchlorfenuron, gibberellic acid, inabenfide, indol-3-acetic acid (IAA), 4-indol-3-ylbutyric acid, isoprothiolane, probenazole, Jasmonsäure, Jasmonsäuremethylester, maleic hydrazide, mepiquat chloride, 1-methylcyclopropene, 2-(1-naphthyl)acetamide, 1-naphthylacetic acid, 2- naphthyloxyacetic acid, nitrophenolate-mixture, 4-Oxo-4[(2-phenylethyl)amino]buttersäure, paclobutrazol, N-phenylphthalamic acid, prohexadione, prohexadione-calcium, prohydrojasmone, Salicylsäure, Strigolacton, tecnazene, thidiazuron, triacontanol, trinexapac, trinexapac-ethyl, tsitodef, uniconazole, uniconazole-P.

Safener, die in Kombination mit den erfindungsgemäßen Verbindungen der Formel (I) und ggf. in Kombinationen mit weiteren Wirkstoffen wie z.B. Insektiziden, Akariziden, Herbiziden, Fungiziden wie oben aufgelistet, eingesetzt werden können, sind vorzugsweise ausgewählt aus der Gruppe bestehend aus:
S1) Verbindungen der Formel (S1), 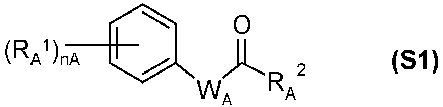 wobei die Symbole und Indizes folgende Bedeutungen haben:
   n_{A} ist eine natürliche Zahl von 0 bis 5, vorzugsweise 0 bis 3;
   R_{A}¹ ist Halogen, (C₁-C₄)Alkyl, (C₁-C₄)Alkoxy, Nitro oder (C₁-C₄)Haloalkyl;
   W_{A} ist ein unsubstituierter oder substituierter divalenter heterocyclischer Rest aus der Gruppe der teilungesättigten oder aromatischen Fünfring-Heterocyclen mit 1 bis 3 Heteroringatomen aus der Gruppe N und O, wobei mindestens ein N-Atom und höchstens ein O-Atom im Ring enthalten ist, vorzugsweise ein Rest aus der Gruppe (W_{A}¹) bis (W_{A}⁴), 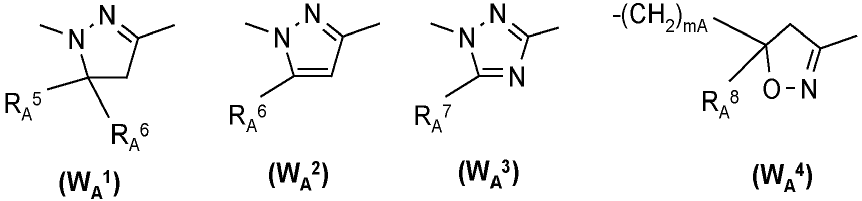
   m_{A} ist 0 oder 1;
   R_{A}² ist OR_{A}³, SR_{A}³ oder NR_{A}³R_{A}⁴ oder ein gesättigter oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S1) verbunden ist und unsubstituiert oder durch Reste aus der Gruppe (C₁-C₄)Alkyl, (C₁-C₄)Alkoxy oder gegebenenfalls substituiertes Phenyl substituiert ist, vorzugsweise ein Rest der Formel OR_{A}³, NHR_{A}⁴ oder N(CH₃)₂, insbesondere der Formel OR_{A}³;
   R_{A}³ ist Wasserstoff oder ein unsubstituierter oder substituierter aliphatischer Kohlenwasserstoffrest, vorzugsweise mit insgesamt 1 bis 18 C-Atomen;
   R_{A}⁴ ist Wasserstoff, (C₁-C₆)Alkyl, (C₁-C₆)Alkoxy oder substituiertes oder unsubstituiertes Phenyl;
   R_{A}⁵ ist H, (C₁-C₈)Alkyl, (C₁-C₈)Haloalkyl, (C₁-C₄)Alkoxy(C₁-C₈)Alkyl, Cyano oder COOR_{A}⁹, worin R_{A}⁹ Wasserstoff, (C₁-C₈)Alkyl, (C₁-C₈)Haloalkyl, (C₁-C₄)Alkoxy-(C₁-C₄)alkyl, (C₁-C₆)Hydroxyalkyl, (C₃-C₁₂)Cycloalkyl oder Tri-(C₁-C₄)-alkyl-silyl ist;
   R_{A}⁶, R_{A}⁷, R_{A}⁸ sind gleich oder verschieden Wasserstoff, (C₁-C₈)Alkyl, (C₁-C₈)Haloalkyl, (C₃-C₁₂)Cycloalkyl oder substituiertes oder unsubstituiertes Phenyl;
   vorzugsweise:
      a) Verbindungen vom Typ der Dichlorphenylpyrazolin-3-carbonsäure (S1^{a}), vorzugsweise Verbindungen wie 1-(2,4-Dichlorphenyl)-5-(ethoxycarbonyl)-5-methyl-2-pyrazolin-3-carbonsäure, 1-(2,4-Dichlorphenyl)-5-(ethoxycarbonyl)-5-methyl-2-pyrazolin-3-carbonsäureethylester (S1-1) ("Mefenpyr-diethyl"), und verwandte Verbindungen, wie sie in der WO-A-91/07874 beschrieben sind;
      b) Derivate der Dichlorphenylpyrazolcarbonsäure (S1^{b}), vorzugsweise Verbindungen wie 1-(2,4-Dichlorphenyl)-5-methyl-pyrazol-3-carbonsäureethylester (S1-2), 1-(2,4-Dichlorphenyl)-5-isopropyl-pyrazol-3-carbonsäureethylester (S1-3), 1-(2,4-Dichlorphenyl)-5-(1,1-dimethyl-ethyl)pyrazol-3-carbonsäureethyl-ester (S1-4) und verwandte Verbindungen, wie sie in EP-A-333 131 und EP-A-269 806 beschrieben sind;
      c) Derivate der 1,5-Diphenylpyrazol-3-carbonsäure (S1^{c}), vorzugsweise Verbindungen wie 1-(2,4-Dichlorphenyl)-5-phenylpyrazol-3-carbonsäureethylester (S1-5), 1-(2-Chlorphenyl)-5-phenylpyrazol-3-carbonsäuremethylester (S1-6) und verwandte Verbindungen wie sie beispielsweise in der EP-A-268554 beschrieben sind;
      d) Verbindungen vom Typ der Triazolcarbonsäuren (S1^{d}), vorzugsweise Verbindungen wie Fenchlorazol(-ethylester), d.h. 1-(2,4-Dichlorphenyl)-5-trichlormethyl-(1H)-1,2,4-triazol-3-carbonsäure-ethylester (S1-7), und verwandte Verbindungen wie sie in EP-A-174 562 und EP-A-346 620 beschrieben sind;
      e) Verbindungen vom Typ der 5-Benzyl- oder 5-Phenyl-2-isoxazolin-3- carbonsäure oder der 5,5-Diphenyl-2-isoxazolin-3-carbonsäure (S1^{e}), vorzugsweise Verbindungen wie 5-(2,4-Dichlorbenzyl)-2-isoxazolin-3-carbonsäureethylester (S1-8) oder 5-Phenyl-2-isoxazolin-3-carbonsäureethylester (S1-9) und verwandte Verbindungen, wie sie in WO-A-91/08202 beschrieben sind, bzw. 5,5-Diphenyl-2-isoxazolin-3-carbonsäure (S1-10) oder 5,5-Diphenyl-2-isoxazolin-3-carbonsäureethylester (S1-11) ("Isoxadifen-ethyl") oder -n-propylester (S1-12) oder der 5-(4-Fluorphenyl)-5-phenyl-2-isoxazolin-3-carbonsäureethylester (S1-13), wie sie in der Patentanmeldung WO-A-95/07897 beschrieben sind.
S2) Chinolinderivate der Formel (S2), 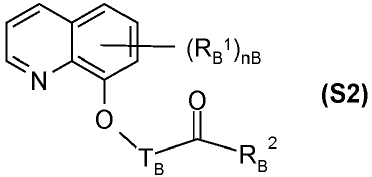 wobei die Symbole und Indizes folgende Bedeutungen haben:
   R_{B}¹ ist Halogen, (C₁-C₄)Alkyl, (C₁-C₄)Alkoxy, Nitro oder (C₁-C₄)Haloalkyl;
   n_{B} ist eine natürliche Zahl von 0 bis 5, vorzugsweise 0 bis 3;
   R_{B}² ist OR_{B}³, SR_{B}³ oder NR_{B}³R_{B}⁴ oder ein gesättigter
   oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S2) verbunden ist und unsubstituiert oder durch Reste aus der Gruppe (C₁-C₄)Alkyl, (C₁-C₄)Alkoxy oder gegebenenfalls substituiertes Phenyl substituiert ist, vorzugsweise ein Rest der Formel OR_{B}³, NHR_{B}⁴ oder N(CH₃)₂, insbesondere der Formel OR_{B}³;
   R_{B}³ ist Wasserstoff oder ein unsubstituierter oder substituierter aliphatischer Kohlenwasserstoffrest, vorzugsweise mit insgesamt 1 bis 18 C-Atomen;
   R_{B}⁴ ist Wasserstoff, (C₁-C₆)Alkyl, (C₁-C₆)Alkoxy oder substituiertes oder unsubstituiertes Phenyl;
   T_{B} ist eine (C₁ oder C₂)-Alkandiylkette, die unsubstituiert oder mit einem oder zwei (C₁-C₄)Alkylresten oder mit [(C₁-C₃)-Alkoxy]-carbonyl substituiert ist;
   vorzugsweise:
      a) Verbindungen vom Typ der 8-Chinolinoxyessigsäure (S2^{a}), vorzugsweise (5-Chlor-8-chinolinoxy)essigsäure-(1-methylhexyl)ester ("Cloquintocet-mexyl") (S2-1), (5-Chlor-8-chinolinoxy)essigsäure-(1,3-dimethyl-but-1-yl)ester (S2-2), (5-Chlor-8-chinolinoxy)essigsäure-4-allyloxy-butylester (S2-3), (5-Chlor-8-chinolinoxy)essigsäure-1-allyloxy-prop-2-ylester (S2-4), (5-Chlor-8-chinolinoxy)essigsäureethylester (S2-5), (5-Chlor-8-chinolinoxy)essigsäuremethylester (S2-6), (5-Chlor-8-chinolinoxy)essigsäureallylester (S2-7), (5-Chlor-8-chinolinoxy)essigsäure-2-(2-propyliden-iminoxy)-1-ethylester (S2-8), (5-Chlor-8-chinolinoxy)essigsäure-2-oxo-prop-1-ylester (S2-9) und verwandte Verbindungen, wie sie in EP-A-86 750, EP-A-94 349 und EP-A-191 736 oder EP-A-0 492 366 beschrieben sind, sowie (5-Chlor-8-chinolinoxy)essigsäure (S2-10), deren Hydrate und Salze, beispielsweise deren Lithium-, Natrium-Kalium-, Kalzium-, Magnesium-, Aluminium-, Eisen-, Ammonium-, quartäre Ammonium-, Sulfonium-, oder Phosphoniumsalze wie sie in der WO-A-2002/34048 beschrieben sind;
      b) Verbindungen vom Typ der (5-Chlor-8-chinolinoxy)malonsäure (S2^{b}), vorzugsweise Verbindungen wie (5-Chlor-8-chinolinoxy)malonsäurediethylester, (5-Chlor-8-chinolinoxy)malonsäurediallylester, (5-Chlor-8-chinolinoxy)malonsäure-methyl-ethylester und verwandte Verbindungen, wie sie in EP-A-0 582 198 beschrieben sind.
S3) Verbindungen der Formel (S3) 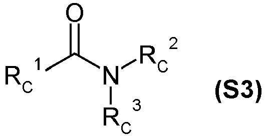 wobei die Symbole und Indizes folgende Bedeutungen haben:
   R_{C}¹ ist (C₁-C₄)Alkyl, (C₁-C₄)Haloalkyl, (C₂-C₄)Alkenyl, (C₂-C₄)Haloalkenyl, (C₃-C₇)Cycloalkyl, vorzugsweise Dichlormethyl;
   R_{C}², R_{C}³ sind gleich oder verschieden Wasserstoff, (C₁-C₄)Alkyl, (C₂-C₄)Alkenyl, (C₂-C₄)Alkinyl, (C₁-C₄)Haloalkyl, (C₂-C₄)Haloalkenyl, (C₁-C₄)Alkylcarbamoyl-(C₁-C₄)alkyl, (C₂-C₄)Alkenylcarbamoyl-(C₁-C₄)alkyl, (C₁-C₄)Alkoxy-(C₁-C₄)alkyl, Dioxolanyl-(C₁-C₄)alkyl, Thiazolyl, Furyl, Furylalkyl, Thienyl, Piperidyl, substituiertes oder unsubstituiertes Phenyl, oder R_{C}² und R_{C}³ bilden zusammen einen substituierten oder unsubstituierten heterocyclischen Ring, vorzugsweise einen Oxazolidin-, Thiazolidin-, Piperidin-, Morpholin-, Hexahydropyrimidin- oder Benzoxazinring;
   vorzugsweise:
      Wirkstoffe vom Typ der Dichloracetamide, die häufig als Vorauflaufsafener (bodenwirksame Safener) angewendet werden, wie z. B. "Dichlormid" (N,N-Diallyl-2,2-dichloracetamid) (S3-1), "R-29148" (3-Dichloracetyl-2,2,5-trimethyl-1,3-oxazolidin) der Firma Stauffer (S3-2), "R-28725" (3-Dichloracetyl-2,2,-dimethyl-1,3-oxazolidin) der Firma Stauffer (S3-3), "Benoxacor" (4-Dichloracetyl-3,4-dihydro-3-methyl-2H-1,4-benzoxazin) (S3-4), "PPG-1292" (N-Allyl-N-[(1,3-dioxolan-2-yl)-methyl]-dichloracetamid) der Firma PPG Industries (S3-5), "DKA-24" (N-Allyl-N-[(allylaminocarbonyl)methyl]-dichloracetamid) der Firma Sagro-Chem (S3-6), "AD-67" oder "MON 4660" (3-Dichloracetyl-1-oxa-3-aza-spiro[4,5]decan) der Firma Nitrokemia bzw. Monsanto (S3-7), "TI-35" (1-Dichloracetyl-azepan) der Firma TRI-Chemical RT (S3-8), "Diclonon" (Dicyclonon) oder "BAS145138" oder "LAB145138" (S3-9) ((RS)-1-Dichloracetyl-3,3,8a-trimethylperhydropyrrolo[1,2-a]pyrimidin-6-on) der Firma BASF, "Furilazol" oder "MON 13900" ((RS)-3-Dichloracetyl-5-(2-furyl)-2,2-dimethyloxazolidin) (S3-10); sowie dessen (R)-Isomer (S3-11).
S4) N-Acylsulfonamide der Formel (S4) und ihre Salze, 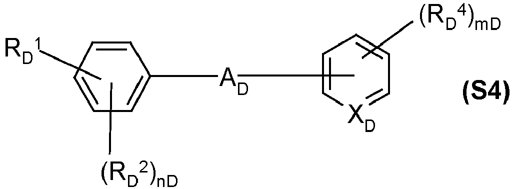 worin die Symbole und Indizes folgende Bedeutungen haben:
   A_{D} ist SO₂-NR_{D}³-CO oder CO-NR_{D}³-SO₂
   X_{D} ist CH oder N;
   R_{D}¹ ist CO-NR_{D}⁵R_{D}⁶ oder NHCO-R_{D}⁷;
   R_{D}² ist Halogen, (C₁-C₄)Haloalkyl, (C₁-C₄)Haloalkoxy, Nitro, (C₁-C₄)Alkyl, (C₁-C₄)Alkoxy, (C₁-C₄)Alkylsulfonyl, (C₁-C₄)Alkoxycarbonyl oder (C₁-C₄)Alkylcarbonyl;
   R_{D}³ ist Wasserstoff, (C₁-C₄)Alkyl, (C₂-C₄)Alkenyl oder (C₂-C₄)Alkinyl,
   R_{D}⁴ ist Halogen, Nitro, (C₁-C₄)Alkyl, (C₁-C₄)Haloalkyl, (C₁-C₄)Haloalkoxy, (C₃-C₆)Cycloalkyl, Phenyl, (C₁-C₄)Alkoxy, Cyano, (C₁-C₄)Alkylthio, (C₁-C₄)Alkylsulfinyl, (C₁-C₄)Alkylsulfonyl, (C₁-C₄)Alkoxycarbonyl oder (C₁-C₄)Alkylcarbonyl;
   R_{D}⁵ ist Wasserstoff, (C₁-C₆)Alkyl, (C₃-C₆)Cycloalkyl, (C₂-C₆)Alkenyl, (C₂-C₆)Alkinyl, (C₅-C₆)Cycloalkenyl, Phenyl oder 3- bis 6-gliedriges Heterocyclyl enthaltend v_{D} Heteroatome aus der Gruppe Stickstoff, Sauerstoff und Schwefel, wobei die sieben letztgenannten Reste durch v_{D} Substituenten aus der Gruppe Halogen, (C₁-C₆)Alkoxy, (C₁-C₆)Haloalkoxy, (C₁-C₂)Alkylsulfinyl, (C₁-C₂)Alkylsulfonyl, (C₃-C₆)Cycloalkyl, (C₁-C₄)Alkoxycarbonyl, (C₁-C₄)Alkylcarbonyl und Phenyl und im Falle cyclischer Reste auch (C₁-C₄) Alkyl und (C₁-C₄)Haloalkyl substituiert sind;
   R_{D}⁶ ist Wasserstoff, (C₁-C₆)Alkyl, (C₂-C₆)Alkenyl oder (C₂-C₆)Alkinyl, wobei die drei letztgenannten Reste durch v_{D} Reste aus der Gruppe Halogen, Hydroxy, (C₁-C₄)Alkyl, (C₁-C₄)Alkoxy und (C₁-C₄)Alkylthio substituiert sind, oder
   R_{D}⁵ und R_{D}⁶ gemeinsam mit dem dem sie tragenden Stickstoffatom einen Pyrrolidinyl- oder Piperidinyl-Rest bilden;
   R_{D}⁷ ist Wasserstoff, (C₁-C₄)Alkylamino, Di-(C₁-C₄)alkylamino, (C₁-C₆)Alkyl, (C₃-C₆)Cycloalkyl, wobei die 2 letztgenannten Reste durch v_{D} Substituenten aus der Gruppe Halogen, (C₁-C₄)Alkoxy, (C₁-C₆)Haloalkoxy und (C₁-C₄)Alkylthio und im Falle cyclischer Reste auch (C₁-C₄)Alkyl und (C₁-C₄)Haloalkyl substituiert sind;
   n_{D} ist 0, 1 oder 2;
   m_{D} ist 1 oder 2;
   v_{D} ist 0, 1, 2 oder 3;
   davon bevorzugt sind Verbindungen vom Typ der N-Acylsulfonamide, z.B. der nachfolgenden Formel (S4^{a}), die z. B. bekannt sind aus WO-A-97/45016 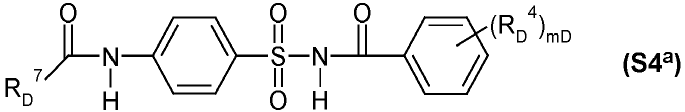
   worin
   R_{D}⁷ (C₁-C₆)Alkyl, (C₃-C₆)Cycloalkyl, wobei die 2 letztgenannten Reste durch v_{D} Substituenten aus der Gruppe Halogen, (C₁-C₄)Alkoxy, (C₁-C₆)Haloalkoxy und (C₁-C₄)Alkylthio und im Falle cyclischer Reste auch (C₁-C₄)Alkyl und (C₁-C₄)Haloalkyl substituiert sind;
   R_{D}⁴ Halogen, (C₁-C₄)Alkyl, (C₁-C₄)Alkoxy, CF₃,
   m_{D} 1 oder 2;
   v_{D} ist 0, 1, 2 oder 3 bedeutet;
   sowie
   Acylsulfamoylbenzoesäureamide, z.B. der nachfolgenden Formel (S4^{b}), die z.B. bekannt sind aus WO-A-99/16744, 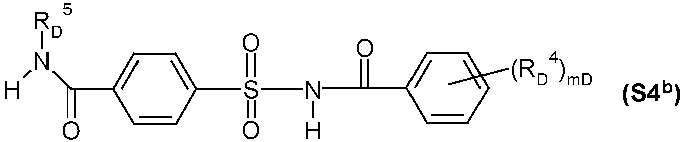
   z.B. solche worin
   R_{D}⁵ = Cyclopropyl und (R_{D}⁴) = 2-OMe ist ("Cyprosulfamide", S4-1),
   R_{D}⁵ = Cyclopropyl und (R_{D}⁴) = 5-Cl-2-OMe ist (S4-2),
   R_{D}⁵ = Ethyl und (R_{D}⁴) = 2-OMe ist (S4-3),
   R_{D}⁵ = Isopropyl und (R_{D}⁴) = 5-Cl-2-OMe ist (S4-4) und
   R_{D}⁵ = Isopropyl und (R_{D}⁴) = 2-OMe ist (S4-5).
   sowie
   Verbindungen vom Typ der N-Acylsulfamoylphenylharnstoffe der Formel (S4^{c}), die z.B. bekannt sind aus der EP-A-365484, 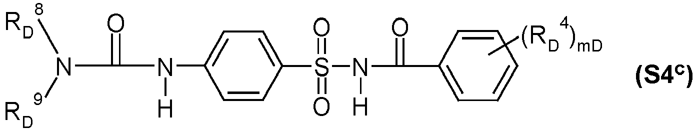
   worin
   R_{D}⁸ und R_{D}⁹ unabhängig voneinander Wasserstoff, (C₁-C₈)Alkyl, (C₃-C₈)Cycloalkyl, (C₃-C₆)Alkenyl, (C₃-C₆)Alkinyl,
   R_{D}⁴ Halogen, (C₁-C₄)Alkyl, (C₁-C₄)Alkoxy, CF₃
   m_{D} 1 oder 2 bedeutet;
   beispielsweise
   1-[4-(N-2-Methoxybenzoylsulfamoyl)phenyl]-3-methylharnstoff,
   1-[4-(N-2-Methoxybenzoylsulfamoyl)phenyl]-3,3-dimethylharnstoff,
   1-[4-(N-4,5-Dimethylbenzoylsulfamoyl)phenyl]-3-methylharnstoff,
   sowie
   N-Phenylsulfonylterephthalamide der Formel (S4^{d}), die z.B. bekannt sind aus CN 101838227, 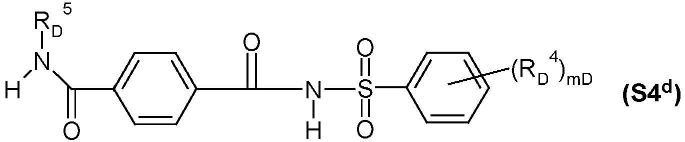
   z.B. solche worin
   R_{D}⁴ Halogen, (C₁-C₄)Alkyl, (C₁-C₄)Alkoxy, CF₃,
   m_{D} 1 oder 2;
   R_{D}⁵ Wasserstoff, (C₁-C₆)Alkyl, (C₃-C₆)Cycloalkyl, (C₂-C₆)Alkenyl, (C₂-C₆)Alkinyl, (C₅-C₆)Cycloalkenyl bedeutet.
S5) Wirkstoffe aus der Klasse der Hydroxyaromaten und der aromatisch-aliphatischen Carbonsäurederivate (S5), z.B.
   3,4,5-Triacetoxybenzoesäureethylester, 3,5-Dimethoxy-4-hydroxybenzoesäure, 3,5-Dihydroxybenzoesäure, 4-Hydroxysalicylsäure, 4-Fluorsalicyclsäure, 2-Hydroxyzimtsäure, 2,4-Dichlorzimtsäure, wie sie in der WO-A-2004/084631, WO-A-2005/015994, WO-A-2005/016001 beschrieben sind.
S6) Wirkstoffe aus der Klasse der 1,2-Dihydrochinoxalin-2-one (S6), z.B. 1-Methyl-3-(2-thienyl)-1,2-dihydrochinoxalin-2-on, 1-Methyl-3-(2-thienyl)-1,2-dihydrochinoxalin-2-thion, 1-(2-Aminoethyl)-3-(2-thienyl)-1,2-dihydro-chinoxalin-2-on-hydrochlorid, 1-(2-Methylsulfonylaminoethyl)-3-(2-thienyl)-1,2-dihydro-chinoxalin-2-on, wie sie in der WO-A-2005/112630 beschrieben sind.
S7) Verbindungen der Formel (S7),wie sie in der WO-A-1998/38856 beschrieben sind 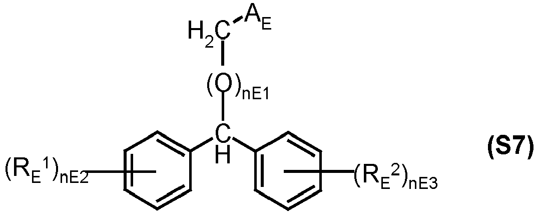 worin die Symbole und Indizes folgende Bedeutungen haben:
   R_{E}¹, R_{E}² sind unabhängig voneinander Halogen, (C₁-C₄)Alkyl, (C₁-C₄)Alkoxy, (C₁-C₄)Haloalkyl, (C₁-C₄)Alkylamino, Di-(C₁-C₄)Alkylamino, Nitro;
   A_{E} ist COOR_{E}³ oder COSR_{E}⁴
   R_{E}³, R_{E}⁴ sind unabhängig voneinander Wasserstoff, (C₁-C₄)Alkyl, (C₂-C₆)Alkenyl, (C₂-C₄)Alkinyl, Cyanoalkyl, (C₁-C₄)Haloalkyl, Phenyl, Nitrophenyl, Benzyl, Halobenzyl, Pyridinylalkyl und Alkylammonium,
   n_{E}¹ ist 0 oder 1
   n_{E}², n_{E}³ sind unabhängig voneinander 0, 1 oder 2,
   vorzugsweise:
      Diphenylmethoxyessigsäure,
      Diphenylmethoxyessigsäureethylester,
      Diphenylmethoxyessigsäuremethylester (CAS-Reg.Nr. 41858-19-9) (S7-1).
S8) Verbindungen der Formel (S8),wie sie in der WO-A-98/27049 beschrieben sind 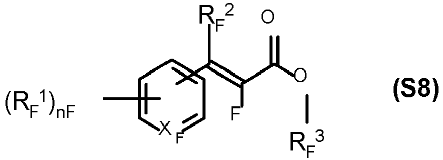 worin
   X_{F} CH oder N,
   n_{F} für den Fall, dass X_{F}=N ist, eine ganze Zahl von 0 bis 4 und
      für den Fall, dass X_{F}=CH ist, eine ganze Zahl von 0 bis 5 ,
   R_{F}¹ Halogen, (C₁-C₄)Alkyl, (C₁-C₄)Haloalkyl, (C₁-C₄)Alkoxy, (C₁-C₄)Haloalkoxy, Nitro, (C₁-C₄)Alkylthio, (C₁-C₄)-Alkylsulfonyl, (C₁-C₄)Alkoxycarbonyl, ggf. substituiertes. Phenyl, ggf. substituiertes Phenoxy,
   R_{F}² Wasserstoff oder (C₁-C₄)Alkyl
   R_{F}³ Wasserstoff, (C₁-C₈)Alkyl, (C₂-C₄)Alkenyl, (C₂-C₄)Alkinyl, oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist; bedeuten, oder deren Salze,
   vorzugsweise Verbindungen worin
   X_{F} CH,
   n_{F} eine ganze Zahl von 0 bis 2 ,
   R_{F}¹ Halogen, (C₁-C₄)Alkyl, (C₁-C₄)Haloalkyl, (C₁-C₄)Alkoxy, (C₁-C₄)Haloalkoxy,
   R_{F}² Wasserstoff oder (C₁-C₄)Alkyl,
   R_{F}³ Wasserstoff, (C₁-C₈)Alkyl, (C₂-C₄)Alkenyl, (C₂-C₄)Alkinyl, oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist, bedeuten,
   oder deren Salze.
S9) Wirkstoffe aus der Klasse der 3-(5-Tetrazolylcarbonyl)-2-chinolone (S9), z.B. 1,2-Dihydro-4-hydroxy-1-ethyl-3-(5-tetrazolylcarbonyl)-2-chinolon (CAS-Reg.Nr. 219479-18-2), 1,2-Dihydro-4-hydroxy-1-methyl-3-(5-tetrazolyl-carbonyl)-2-chinolon (CAS-Reg.Nr. 95855-00-8), wie sie in der WO-A-1999/000020 beschrieben sind.
S10) Verbindungen der Formeln (S10^{a}) oder (S10^{b})
   wie sie in der WO-A-2007/023719 und WO-A-2007/023764 beschrieben sind 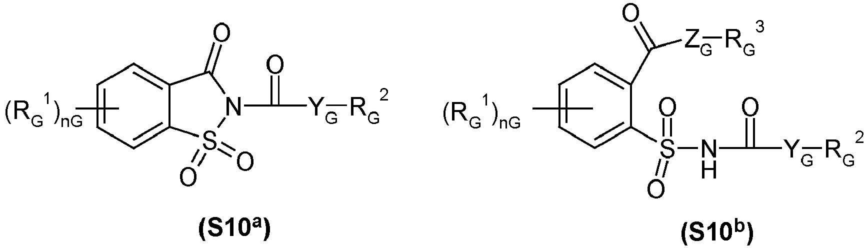 worin
   R_{G}¹ Halogen, (C₁-C₄)Alkyl, Methoxy, Nitro, Cyano, CF₃, OCF₃
   Y_{G}, Z_{G} unabhängig voneinander O oder S,
   n_{G} eine ganze Zahl von 0 bis 4,
   R_{G}² (C₁-C₁₆)Alkyl, (C₂-C₆)Alkenyl, (C₃-C₆)Cycloalkyl, Aryl; Benzyl, Halogenbenzyl,
   R_{G}³ Wasserstoff oder (C₁-C₆)Alkyl bedeutet.
S11) Wirkstoffe vom Typ der Oxyimino-Verbindungen (S11), die als Saatbeizmittel bekannt sind, wie z. B.
   "Oxabetrinil" ((Z)-1,3-Dioxolan-2-ylmethoxyimino(phenyl)acetonitril) (S11-1), das als Saatbeiz-Safener für Hirse gegen Schäden von Metolachlor bekannt ist,
   "Fluxofenim" (1-(4-Chlorphenyl)-2,2,2-trifluor-1-ethanon-O-(1,3-dioxolan-2-ylmethyl)-oxim) (S11-2), das als Saatbeiz-Safener für Hirse gegen Schäden von Metolachlor bekannt ist, und
   "Cyometrinil" oder "CGA-43089" ((Z)-Cyanomethoxyimino(phenyl)acetonitril) (S11-3), das als Saatbeiz-Safener für Hirse gegen Schäden von Metolachlor bekannt ist.
S12) Wirkstoffe aus der Klasse der Isothiochromanone (S12), wie z.B. Methyl-[(3-oxo-1H-2-benzothiopyran-4(3H)-yliden)methoxy]acetat (CAS-Reg.Nr. 205121-04-6) (S12-1) und verwandte Verbindungen aus WO-A-1998/13361.
S13) Eine oder mehrere Verbindungen aus Gruppe (S13):
   "Naphthalic anhydrid" (1,8-Naphthalindicarbonsäureanhydrid) (S13-1), das als Saatbeiz-Safener für Mais gegen Schäden von Thiocarbamatherbiziden bekannt ist,
   "Fenclorim" (4,6-Dichlor-2-phenylpyrimidin) (S13-2), das als Safener für Pretilachlor in gesätem Reis bekannt ist,
   "Flurazole" (Benzyl-2-chlor-4-trifluormethyl-1,3-thiazol-5-carboxylat) (S13-3), das als Saatbeiz-Safener für Hirse gegen Schäden von Alachlor und Metolachlor bekannt ist,
   "CL 304415" (CAS-Reg.Nr. 31541-57-8)
   (4-Carboxy-3,4-dihydro-2H-1-benzopyran-4-essigsäure) (S13-4) der Firma American Cyanamid, das als Safener für Mais gegen Schäden von Imidazolinonen bekannt ist,
   "MG 191" (CAS-Reg.Nr. 96420-72-3) (2-Dichlormethyl-2-methyl-1,3-dioxolan) (S13-5) der Firma Nitrokemia, das als Safener für Mais bekannt ist,
   "MG 838" (CAS-Reg.Nr. 133993-74-5)
   (2-propenyl 1-oxa-4-azaspiro[4.5]decan-4-carbodithioat) (S13-6) der Firma Nitrokemia,
   "Disulfoton" (O,O-Diethyl S-2-ethylthioethyl phosphordithioat) (S13-7),
   "Dietholate" (O,O-Diethyl-O-phenylphosphorothioat) (S13-8),
   "Mephenate" (4-Chlorphenyl-methylcarbamat) (S13-9).
   S14) Wirkstoffe, die neben einer herbiziden Wirkung gegen Schadpflanzen auch Safenerwirkung an Kulturpflanzen wie Reis aufweisen, wie z. B.
   "Dimepiperate" oder "MY 93" (S-1-Methyl-1-phenylethyl-piperidin-1-carbothioat), das als Safener für Reis gegen Schäden des Herbizids Molinate bekannt ist,
   "Daimuron" oder "SK 23" (1-(1-Methyl-1-phenylethyl)-3-p-tolyl-harnstoff), das als Safener für Reis gegen Schäden des Herbizids Imazosulfuron bekannt ist,
   "Cumyluron" = "JC 940" (3-(2-Chlorphenylmethyl)-1-(1-methyl-1-phenyl-ethyl)harnstoff, siehe JP-A-60087254), das als Safener für Reis gegen Schäden einiger Herbizide bekannt ist,
   "Methoxyphenon" oder "NK 049" (3,3'-Dimethyl-4-methoxy-benzophenon), das als Safener für Reis gegen Schäden einiger Herbizide bekannt ist,
   "CSB" (1-Brom-4-(chlormethylsulfonyl)benzol) von Kumiai, (CAS-Reg.Nr. 54091-06-4), das als Safener gegen Schäden einiger Herbizide in Reis bekannt ist.
S15) Verbindungen der Formel (S15) oder deren Tautomere 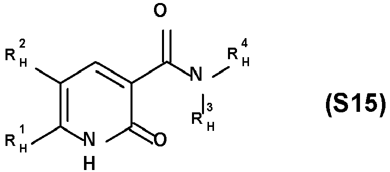
   wie sie in der WO-A-2008/131861 und WO-A-2008/131860 beschrieben sind, worin
   R_{H}¹ einen (C₁-C₆)Haloalkylrest bedeutet und
   R_{H}² Wasserstoff oder Halogen bedeutet und
   R_{H}³, R_{H}⁴ unabhängig voneinander Wasserstoff, (C₁-C₁₆)Alkyl, (C₂-C₁₆)Alkenyl oder (C₂-C₁₆)Alkinyl,
   wobei jeder der letztgenannten 3 Reste unsubstituiert oder durch einen oder mehrere Reste aus der Gruppe Halogen, Hydroxy, Cyano, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-C₄)Alkoxy]-carbonyl, [(C₁-C₄)Haloalkoxy]-carbonyl, (C₃-C₆)Cycloalkyl, das unsubstituiert oder substituiert ist, Phenyl, das unsubstituiert oder substituiert ist, und Heterocyclyl, das unsubstituiert oder substituiert ist, substituiert ist,
   oder (C₃-C₆)Cycloalkyl, (C₄-C₆)Cycloalkenyl, (C₃-C₆)Cycloalkyl, das an einer Seite des Rings mit einem 4 bis 6-gliedrigen gesättigten oder ungesättigten carbocyclischen Ring kondensiert ist, oder (C₄-C₆)Cycloalkenyl, das an einer Seite des Rings mit einem 4 bis 6-gliedrigen gesättigten oder ungesättigten carbocyclischen Ring kondensiert ist,
   wobei jeder der letztgenannten 4 Reste unsubstituiert oder durch einen oder mehrere Reste aus der Gruppe Halogen, Hydroxy, Cyano, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-c₄)Alkoxy]-carbonyl, [(C₁-C₄)Haloalkoxy]-carbonyl, (C₃-C₆)Cycloalkyl, das unsubstituiert oder substituiert ist, Phenyl, das unsubstituiert oder substituiert ist, und Heterocyclyl, das unsubstituiert oder substituiert ist, substituiert ist,
   bedeutet oder
   R_{H}³ (C₁-C₄)-Alkoxy, (C₂-C₄)Alkenyloxy, (C₂-C₆)Alkinyloxy oder (C₂-C₄)Haloalkoxy bedeutet und
   R_{H}⁴ Wasserstoff oder (C₁-C₄)-Alkyl bedeutet oder
   R_{H}³ und R_{H}⁴ zusammen mit dem direkt gebundenen N-Atom einen vier- bis achtgliedrigen heterocyclischen Ring, der neben dem N-Atom auch weitere Heteroringatome, vorzugsweise bis zu zwei weitere Heteroringatome aus der Gruppe N, O und S enthalten kann und der unsubstituiert oder durch einen oder mehrere Reste aus der Gruppe Halogen, Cyano, Nitro, (C₁-C₄)Alkyl, (C₁-C₄)Haloalkyl, (C₁-C₄)Alkoxy, (C₁-C₄)Haloalkoxy und (C₁-C₄)Alkylthio substituiert ist, bedeutet.
S16) Wirkstoffe, die vorrangig als Herbizide eingesetzt werden, jedoch auch Safenerwirkung auf Kulturpflanzen aufweisen, z.B.
   (2,4-Dichlorphenoxy)essigsäure (2,4-D), (4-Chlorphenoxy)essigsäure,
   (R,S)-2-(4-Chlor-o-tolyloxy)propionsäure (Mecoprop), 4-(2,4-Dichlorphenoxy)buttersäure (2,4-DB), (4-Chlor-o-tolyloxy)essigsäure (MCPA), 4-(4-Chlor-o-tolyloxy)buttersäure,
   4-(4-Chlorphenoxy)buttersäure,
   3,6-Dichlor-2-methoxybenzoesäure (Dicamba), 1-(Ethoxycarbonyl)ethyl-3,6-dichlor-2-methoxybenzoat (Lactidichlor-ethyl).

Besonders bevorzugte Safener sind Mefenpyr-diethyl, Cyprosulfamid, Isoxadifen-ethyl, Cloquintocetmexyl, Dichlormid und Metcamifen.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolether-sulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepoly-glykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfett-säureester oder Polyoxethylensorbitanester wie z.B. Polyoxyethylensorbitan-fettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London, J.E. Browning, "Agglomeration", Chemical and Engineering 1967, Seiten 147 ff, "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0.1 bis 99 Gew.-%, insbesondere 0.1 bis 95 Gew.-%, erfindungsgemäße Verbindungen. In Spritzpulvern beträgt die Wirkstoff-konzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0.05 bis 80, vorzugsweise 2 bis 50 Gew.-% Wirkstoff. Bei wasser-dispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%. Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix. Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I) und deren Salze. Sie kann innerhalb weiter Grenzen schwanken, z.B. zwischen 0,001 und 10,0 kg/ha oder mehr Aktivsubstanz, vorzugsweise liegt sie jedoch zwischen 0,005 bis 5 kg/ha, weiter bevorzugt im Bereich von 0,01 bis 1,5 kg/ha, insbesondere bevorzugt im Bereich von 0,05 bis 1 kg/ha g/ha. Dies gilt sowohl für die Anwendung im Vorauflauf oder im Nachauflauf.

Trägerstoff bedeutet eine natürliche oder synthetische, organische oder anorganische Substanz, mit welchen die Wirkstoffe zur besseren Anwendbarkeit, v.a. zum Aufbringen auf Pflanzen oder Pflanzenteile oder Saatgut, gemischt oder verbunden sind. Der Trägerstoff, welcher fest oder flüssig sein kann, ist im Allgemeinen inert und sollte in der Landwirtschaft verwendbar sein.

Als feste oder flüssige Trägerstoffe kommen infrage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und natürliche oder synthetische Silikate, Harze, Wachse, feste Düngemittel, Wasser, Alkohole, besonders Butanol, organische Solventien, Mineral- und Pflanzenöle sowie Derivate hiervon. Mischungen solcher Trägerstoffe können ebenfalls verwendet werden. Als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstängel.

Als verflüssigte gasförmige Streckmittel oder Trägerstoffe kommen solche Flüssigkeiten infrage, welche bei normaler Temperatur und unter Normaldruck gasförmig sind, z.B. Aerosol-Treibgase, wie Halogenkohlenwasserstoffe, sowie Butan, Propan, Stickstoff und Kohlendioxid.

Es können in den Formulierungen Haftmittel wie Carboxymethylcellulose, natürliche und synthetische pulverige, körnige oder latexförmige Polymere verwendet werden, wie Gummiarabikum, Polyvinylalkohol, Polyvinylacetat, sowie natürliche Phospholipide, wie Kephaline und Lecithine, und synthetische Phospholipide. Weitere Additive können mineralische und vegetabile Öle sein.

Im Falle der Benutzung von Wasser als Streckmittel können z.B. auch organische Lösungsmittel als Hilfslösungsmittel verwendet werden. Als flüssige Lösungsmittel kommen im Wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Dichlormethan, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone, wie Aceton, Methylethylketon, Methylisobutylketon oder Cyclohexanon, stark polare Lösungsmittel wie Dimethylformamid und Dimethylsulfoxid, sowie Wasser.

Die erfindungsgemäßen Mittel können zusätzlich weitere Bestandteile enthalten, wie z.B. oberflächenaktive Stoffe. Als oberflächenaktive Stoffe kommen Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe infrage. Beispiele hierfür sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, substituierten Phenolen (vorzugsweise Alkylphenole oder Arylphenole), Salze von Sulphobernsteinsäureestern, Taurinderivate (vorzugsweise Alkyltaurate), Phosphorsäureester von polyethoxylierten Alkoholen oder Phenole, Fettsäureester von Polyolen, und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist notwendig, wenn einer der Wirkstoff und/oder einer der inerten Trägerstoffe nicht in Wasser löslich ist und wenn die Anwendung in Wasser erfolgt. Der Anteil an oberflächenaktiven Stoffen liegt zwischen 5 und 40 Gewichtsprozent des erfindungsgemäßen Mittels. Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Gegebenenfalls können auch andere zusätzliche Komponenten enthalten sein, z.B. schützende Kolloide, Bindemittel, Klebstoffe, Verdicker, thixotrope Stoffe, Penetrationsförderer, Stabilisatoren, Sequestiermittel, Komplexbildner. Im Allgemeinen können die Wirkstoffe mit jedem festen oder flüssigen Additiv, welches für Formulierungszwecke gewöhnlich verwendet wird, kombiniert werden. Im Allgemeinen enthalten die erfindungsgemäßen Mittel und Formulierungen zwischen 0,05 und 99 Gew.- %, 0,01 und 98 Gew.-%, vorzugsweise zwischen 0,1 und 95 Gew.-%, besonders bevorzugt zwischen 0,5 und 90 % Wirkstoff, ganz besonders bevorzugt zwischen 10 und 70 Gewichtsprozent. Die erfindungsgemäßen Wirkstoffe bzw. Mittel können als solche oder in Abhängigkeit von ihren jeweiligen physikalischen und/oder chemischen Eigenschaften in Form ihrer Formulierungen oder den daraus bereiteten Anwendungsformen, wie Aerosole, Kapselsuspensionen, Kaltnebelkonzentrate, Heißnebelkonzentrate, verkapselte Granulate, Feingranulate, fließfähige Konzentrate für die Behandlung von Saatgut, gebrauchsfertige Lösungen, verstäubbare Pulver, emulgierbare Konzentrate, Öl-in-Wasser-Emulsionen, Wasser-in-Öl-Emulsionen, Makrogranulate, Mikrogranulate, Öl dispergierbare Pulver, Öl mischbare fließfähige Konzentrate, Öl mischbare Flüssigkeiten, Schäume, Pasten, Pestizid ummanteltes Saatgut, Suspensionskonzentrate, Suspensions-Emulsions-Konzentrate, lösliche Konzentrate, Suspensionen, Spritzpulver, lösliche Pulver, Stäubemittel und Granulate, wasserlösliche Granulate oder Tabletten, wasserlösliche Pulver für Saatgut-behandlung, benetzbare Pulver, Wirkstoff-imprägnierte Natur- und synthetische Stoffe sowie Feinstverkapselungen in polymeren Stoffen und in Hüllmassen für Saatgut, sowie ULV-Kalt- und Warmnebel-Formulierungen eingesetzt werden.

Die genannten Formulierungen können in an sich bekannter Weise hergestellt werden, z.B. durch Vermischen der Wirkstoffe mit mindestens einem üblichen Streckmittel, Lösungs- bzw. Verdünnungsmittel, Emulgator, Dispergier- und/oder Binde- oder Fixiermittels, Netzmittel, Wasser-Repellent, gegebenenfalls Sikkative und UV-Stabilisatoren und gegebenenfalls Farbstoffen und Pigmenten, Entschäumer, Konservierungsmittel, sekundäre Verdickungsmittel, Kleber, Gibberelline sowie weiteren Verarbeitungshilfsmitteln.

Die erfindungsgemäßen Mittel umfassen nicht nur Formulierungen, welche bereits anwendungsfertig sind und mit einer geeigneten Apparatur auf die Pflanze oder das Saatgut ausgebracht werden können, sondern auch kommerzielle Konzentrate, welche vor Gebrauch mit Wasser verdünnt werden müssen.

Die erfindungsgemäßen Wirkstoffe können als solche oder in ihren (handelsüblichen) Formulierungen sowie in den aus diesen Formulierungen bereiteten Anwendungsformen in Mischung mit anderen (bekannten) Wirkstoffen, wie Insektiziden, Lockstoffen, Sterilantien, Bakteriziden, Akariziden, Nematiziden, Fungiziden, Wachstumsregulatoren, Herbiziden, Düngemitteln, Safener bzw. Semiochemicals vorliegen.

Die erfindungsgemäße Behandlung der Pflanzen und Pflanzenteile mit den Wirkstoffen bzw. Mitteln erfolgt direkt oder durch Einwirkung auf deren Umgebung, Lebensraum oder Lagerraum nach den üblichen Behandlungsmethoden, z.B. durch Tauchen, (Ver-)Spritzen, (Ver-)Sprühen, Berieseln, Verdampfen, Zerstäuben, Vernebeln, (Ver-)Streuen, Verschäumen, Bestreichen, Verstreichen, Gießen (drenchen), Tröpfchenbewässerung und bei Vermehrungsmaterial, insbesondere bei Samen, weiterhin durch Trockenbeizen, Nassbeizen, Schlämmbeizen, Inkrustieren, ein- oder mehrschichtiges Umhüllen usw. Es ist ferner möglich, die Wirkstoffe nach dem Ultra-Low-Volume-Verfahren auszubringen oder die Wirkstoffzubereitung oder den Wirkstoff selbst in den Boden zu injizieren.

Wie auch weiter unten beschrieben, ist die Behandlung von transgenem Saatgut mit den erfindungsgemäßen Wirkstoffen bzw. Mitteln von besonderer Bedeutung. Dies betrifft das Saatgut von Pflanzen, die wenigstens ein heterologes Gen enthalten, das die Expression eines Polypeptids oder Proteins mit insektiziden Eigenschaften ermöglicht. Das heterologe Gen in transgenem Saatgut kann z.B. aus Mikroorganismen der Arten Bacillus, Rhizobium, Pseudomonas, Serratia, Trichoderma, Clavibacter, Glomus oder Gliocladium stammen. Bevorzugt stammt dieses heterologe Gen aus Bacillus sp., wobei das Genprodukt eine Wirkung gegen den Maiszünsler (European corn borer) und/oder Western Corn Rootworm besitzt. Besonders bevorzugt stammt das heterologe Gen aus Bacillus thuringiensis.

Im Rahmen der vorliegenden Erfindung wird das erfindungsgemäße Mittel alleine oder in einer geeigneten Formulierung auf das Saatgut aufgebracht. Vorzugsweise wird das Saatgut in einem Zustand behandelt, in dem so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde. So kann zum Beispiel Saatgut verwendet werden, das geerntet, gereinigt und bis zu einem Feuchtigkeitsgehalt von unter 15 Gew.-% getrocknet wurde. Alternativ kann auch Saatgut verwendet werden, das nach dem Trocknen z.B. mit Wasser behandelt und dann erneut getrocknet wurde.

Im Allgemeinen muss bei der Behandlung des Saatguts darauf geachtet werden, dass die Menge des auf das Saatgut aufgebrachten erfindungsgemäßen Mittels und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die erfindungsgemäßen Mittel können unmittelbar aufgebracht werden, also ohne weitere Komponenten zu enthalten und ohne verdünnt worden zu sein. In der Regel ist es vorzuziehen, die Mittel in Form einer geeigneten Formulierung auf das Saatgut aufzubringen. Geeignete Formulierungen und Verfahren für die Saatgutbehandlung sind dem Fachmann bekannt und werden z.B. in den folgenden Dokumenten beschrieben: US 4,272,417 A, US 4,245,432 A, US 4,808,430, US 5,876,739, US 2003/0176428 A1, WO 2002/080675 A1, WO 2002/028186 A2.

Die erfindungsgemäßen Wirkstoffe können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man die Wirkstoffe mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalin-Sulfonate, wie Diisopropyl- oder Diisobutyl-naphthalin-Sulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vorzugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Die erfindungsgemäß verwendbaren Beizmittel-Formulierungen können entweder direkt oder nach vorherigem Verdünnen mit Wasser zur Behandlung von Saatgut der verschiedensten Art, auch von Saatgut transgener Pflanzen, eingesetzt werden. Dabei können im Zusammenwirken mit den durch Expression gebildeten Substanzen auch zusätzliche synergistische Effekte auftreten.

Zur Behandlung von Saatgut mit den erfindungsgemäß verwendbaren Beizmittel-Formulierungen oder den daraus durch Zugabe von Wasser hergestellten Zubereitungen kommen alle üblicherweise für die Beizung einsetzbaren Mischgeräte in Betracht. Im einzelnen geht man bei der Beizung so vor, dass man das Saatgut in einen Mischer gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes. Sie können vorzugsweise als Pflanzenschutzmittel eingesetzt werden. Sie sind gegen normal sensible und resistente Arten sowie gegen alle oder einzelne Entwicklungsstadien wirksam.

Als Pflanzen, welche erfindungsgemäß behandelt werden können, seien folgende Hauptanbaupflanzen erwähnt: Mais, Sojabohne, Baumwolle, Brassica Ölsaaten wie Brassica napus (z.B. Canola), Brassica rapa, B. juncea (z.B. (Acker-)Senf) und Brassica carinata, Reis, Weizen Zuckerrübe, Zurckerrohr, Hafer, Roggen, Gerste, Hirse, Triticale, Flachs, Wein und verschiedene Früchte und Gemüse von verschiedenen botanischen Taxa wie z.B. Rosaceae sp. (beispielsweise Kernfrüchte wie Apfel und Birne, aber auch Steinfrüchte wie Aprikosen, Kirschen, Mandeln und Pfirsiche und Beerenfrüchte wie Erdbeeren), Ribesioidae sp., Juglandaceae sp., Betulaceae sp., Anacardiaceae sp., Fagaceae sp., Moraceae sp., Oleaceae sp., Actinidaceae sp., Lauraceae sp., Musaceae sp. (beispielsweise Bananenbäume und - plantagen), Rubiaceae sp. (beispielsweise Kaffee), Theaceae sp., Sterculiceae sp., Rutaceae sp. (beispielsweise Zitronen, Organen und Grapefruit); Solanaceae sp. (beispielsweise Tomaten, Kartoffeln, Pfeffer, Auberginen), Liliaceae sp., Compositae sp. (beispielsweise Salat, Artischocke and Chicoree - einschließlich Wurzelchicoree, Endivie oder gemeinen Chicoree), Umbelliferae sp. (beispielsweise Karrotte, Petersilie, Stangensellerie und Knollensellerie), Cucurbitaceae sp. (beispielsweise Gurke - einschließlich Gewürzgurke, Kürbis, Wassermelone, Flaschenkürbis und Melonen), Alliaceae sp. (beispielsweise Lauch und Zwiebel), Cruciferae sp. (beispielsweise Weißkohl, Rotkohl, Brokkoli, Blumenkohl, Rosenkohl, Pak Choi, Kohlrabi, Radieschen, Meerrettich, Kresse und Chinakohl), Leguminosae sp. (beispielsweise Erdnüsse, Erbsen, und Bohnen - wie z.B. Stangenbohne und Ackerbohne), Chenopodiaceae sp. (beispielsweise Mangold, Futterrübe, Spinat, Rote Rübe), Malvaceae (beispielsweise Okra), Asparagaceae (beispielsweise Spargel); Nutzpflanzen und Zierpflanzen in Garten und Wald; sowie jeweils genetisch modifizierte Arten dieser Pflanzen.

Wie oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert. Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

Das erfindungsgemäße Behandlungsverfahren kann für die Behandlung von genetisch modifizierten Organismen (GMOs), z. B. Pflanzen oder Samen, verwendet werden. Genetisch modifizierte Pflanzen (oder transgene Pflanzen) sind Pflanzen, bei denen ein heterologes Gen stabil in das Genom integriert worden ist. Der Begriff "heterologes Gen" bedeutet im wesentlichen ein Gen, das außerhalb der Pflanze bereitgestellt oder assembliert wird und das bei Einführung in das Zellkerngenom, das Chloroplastengenom oder das Mitochondriengenom der transformierten Pflanze dadurch neue oder verbesserte agronomische oder sonstige Eigenschaften verleiht, dass es ein interessierendes Protein oder Polypeptid exprimiert oder dass es ein anderes Gen, das in der Pflanze vorliegt bzw. andere Gene, die in der Pflanze vorliegen, herunterreguliert oder abschaltet (zum Beispiel mittels Antisense-Technologie, Cosuppressionstechnologie oder RNAi-Technologie [RNA Interference]). Ein heterologes Gen, das im Genom vorliegt, wird ebenfalls als Transgen bezeichnet. Ein Transgen, das durch sein spezifisches Vorliegen im Pflanzengenom definiert ist, wird als Transformations- bzw. transgenes Event bezeichnet.

In Abhängigkeit von den Pflanzenarten oder Pflanzensorten, ihrem Standort und ihren Wachstumsbedingungen (Böden, Klima, Vegetationsperiode, Ernährung) kann die erfindungsgemäße Behandlung auch zu überadditiven ("synergistischen") Effekten führen. So sind zum Beispiel die folgenden Effekte möglich, die über die eigentlich zu erwartenden Effekte hinausgehen: verringerte Aufwandmengen und/oder erweitertes Wirkungsspektrum und/oder erhöhte Wirksamkeit der Wirkstoffe und Zusammensetzungen, die erfindungsgemäß eingesetzt werden können, besseres Pflanzenwachstum, erhöhte Toleranz gegenüber hohen oder niedrigen Temperaturen, erhöhte Toleranz gegenüber Trockenheit oder Wasser- oder Bodensalzgehalt, erhöhte Blühleistung, Ernteerleichterung, Reifebeschleunigung, höhere Erträge, größere Früchte, größere Pflanzenhöhe, intensiver grüne Farbe des Blatts, frühere Blüte, höhere Qualität und/oder höherer Nährwert der Ernteprodukte, höhere Zuckerkonzentration in den Früchten, bessere Lagerfähigkeit und/oder Verarbeitbarkeit der Ernteprodukte.

Zu Pflanzen und Pflanzensorten, die vorzugsweise erfindungsgemäß behandelt werden, zählen alle Pflanzen, die über Erbgut verfügen, das diesen Pflanzen besonders vorteilhafte, nützliche Merkmale verleiht (egal, ob dies durch Züchtung und/oder Biotechnologie erzielt wurde).

Beispiele für Nematoden-resistente Pflanzen sind z.B. folgenden US Patentanmeldungen beschrieben: 11/765,491, 11/765,494, 10/926,819, 10/782,020, 12/032,479, 10/783,417, 10/782,096, 11/657,964, 12/192,904, 11/396,808, 12/166,253, 12/166,239, 12/166,124, 12/166,209, 11/762,886, 12/364,335, 11/763,947, 12/252,453, 12/209,354, 12/491,396 und 12/497,221.

Pflanzen, die erfindungsgemäß behandelt werden können, sind Hybridpflanzen, die bereits die Eigenschaften der Heterosis bzw. des Hybrideffekts exprimieren, was im Allgemeinen zu höherem Ertrag, höherer Wüchsigkeit, besserer Gesundheit und besserer Resistenz gegen biotische und abiotische Stressfaktoren führt. Solche Pflanzen werden typischerweise dadurch erzeugt, dass man eine ingezüchtete pollensterile Elternlinie (den weiblichen Kreuzungspartner) mit einer anderen ingezüchteten pollenfertilen Elternlinie (dem männlichen Kreuzungspartner) kreuzt. Das Hybridsaatgut wird typischerweise von den pollensterilen Pflanzen geerntet und an Vermehrer verkauft. Pollensterile Pflanzen können manchmal (z. B. beim Mais) durch Entfahnen (d.h. mechanischem Entfernen der männlichen Geschlechtsorgane bzw. der männlichen Blüten), produziert werden; es ist jedoch üblicher, dass die Pollensterilität auf genetischen Determinanten im Pflanzengenom beruht. In diesem Fall, insbesondere dann, wenn es sich bei dem gewünschten Produkt, da man von den Hybridpflanzen ernten will, um die Samen handelt, ist es üblicherweise günstig, sicherzustellen, dass die Pollenfertilität in Hybridpflanzen, die die für die Pollensterilität verantwortlichen genetischen Determinanten enthalten, völlig restoriert wird. Dies kann erreicht werden, indem sichergestellt wird, dass die männlichen Kreuzungspartner entsprechende Fertilitätsrestorergene besitzen, die in der Lage sind, die Pollenfertilität in Hybridpflanzen, die die genetischen Determinanten, die für die Pollensterilität verantwortlich sind, enthalten, zu restorieren. Genetische Determinanten für Pollensterilität können im Cytoplasma lokalisiert sein. Beispiele für cytoplasmatische Pollensterilität (CMS) wurden zum Beispiel für Brassica-Arten beschrieben. Genetische Determinanten für Pollensterilität können jedoch auch im Zellkerngenom lokalisiert sein. Pollensterile Pflanzen können auch mit Methoden der pflanzlichen Biotechnologie, wie Gentechnik, erhalten werden. Ein besonders günstiges Mittel zur Erzeugung von pollensterilen Pflanzen ist in WO 89/10396 beschrieben, wobei zum Beispiel eine Ribonuklease wie eine Barnase selektiv in den Tapetumzellen in den Staubblättern exprimiert wird. Die Fertilität kann dann durch Expression eines Ribonukleasehemmers wie Barstar in den Tapetumzellen restoriert werden.

Pflanzen oder Pflanzensorten (die mit Methoden der Pflanzenbiotechnologie, wie der Gentechnik, erhalten werden), die erfindungsgemäß behandelt werden können, sind herbizidtolerante Pflanzen, d. h. Pflanzen, die gegenüber einem oder mehreren vorgegebenen Herbiziden tolerant gemacht worden sind. Solche Pflanzen können entweder durch genetische Transformation oder durch Selektion von Pflanzen, die eine Mutation enthalten, die solch eine Herbizidtoleranz verleiht, erhalten werden.

Herbizidtolerante Pflanzen sind zum Beispiel glyphosatetolerante Pflanzen, d. h. Pflanzen, die gegenüber dem Herbizid Glyphosate oder dessen Salzen tolerant gemacht worden sind. Pflanzen können mit verschiedenen Methoden tolerant gegenüber Glyphosate gemacht werden. So können zum Beispiel glyphosatetolerante Pflanzen durch Transformation der Pflanze mit einem Gen, das für das Enzym 5-Enolpyruvylshikimat-3-phosphatsynthase (EPSPS) kodiert, erhalten werden. Beispiele für solche EPSPS-Gene sind das AroA-Gen (Mutante CT7) des Bakterium Salmonella typhimurium (Comai et al., 1983, Science 221, 370-371), das CP4-Gen des Bakteriums Agrobacterium sp. (Barry et al., 1992, Curr. Topics Plant Physiol. 7, 139-145), die Gene, die für eine EPSPS aus der Petunie (Shah et al., 1986, Science 233, 478-481), für eine EPSPS aus der Tomate (Gasser et al., 1988, J. Biol. Chem. 263, 4280-4289) oder für eine EPSPS aus Eleusine (WO 01/66704) kodieren. Es kann sich auch um eine mutierte EPSPS handeln. Glyphosate-tolerante Pflanzen können auch dadurch erhalten werden, dass man ein Gen exprimiert, das für ein Glyphosate-Oxidoreduktase-Enzym kodiert. Glyphosate-tolerante Pflanzen können auch dadurch erhalten werden, dass man ein Gen exprimiert, das für ein Glyphosate-acetyltransferase-Enzym kodiert. Glyphosatetolerante Pflanzen können auch dadurch erhalten werden, dass man Pflanzen, die natürlich vorkommende Mutationen der oben erwähnten Gene enthalten, selektiert. Pflanzen, die EPSPS Gene, welche Glyphosate-Toleranz verleihen, exprimieren, sind beschrieben. Pflanzen, welche andere Gene, die Glyphosate-Toleranz verleihen, z.B. Decarboxylase-Gene, sind beschrieben.

Sonstige herbizidresistente Pflanzen sind zum Beispiel Pflanzen, die gegenüber Herbiziden, die das Enzym Glutaminsynthase hemmen, wie Bialaphos, Phosphinotricin oder Glufosinate, tolerant gemacht worden sind. Solche Pflanzen können dadurch erhalten werden, dass man ein Enzym exprimiert, das das Herbizid oder eine Mutante des Enzyms Glutaminsynthase, das gegenüber Hemmung resistent ist, entgiftet. Solch ein wirksames entgiftendes Enzym ist zum Beispiel ein Enzym, das für ein Phosphinotricin-acetyltransferase kodiert (wie zum Beispiel das bar- oder pat-Protein aus Streptomyces-Arten). Pflanzen, die eine exogene Phosphinotricin-acetyltransferase exprimieren, sind beschrieben. Weitere herbizidtolerante Pflanzen sind auch Pflanzen, die gegenüber den Herbiziden, die das Enzym Hydroxyphenylpyruvatdioxygenase (HPPD) hemmen, tolerant gemacht worden sind. Bei den Hydroxyphenylpyruvatdioxygenasen handelt es sich um Enzyme, die die Reaktion, in der para-Hydroxyphenylpyruvat (HPP) zu Homogentisat umgesetzt wird, katalysieren. Pflanzen, die gegenüber HPPD-Hemmern tolerant sind, können mit einem Gen, das für ein natürlich vorkommendes resistentes HPPD-Enzym kodiert, oder einem Gen, das für ein mutiertes oder chimäres HPPD-Enzym kodiert, transformiert werden, wie in WO 96/38567, WO 99/24585, WO 99/24586, WO 2009/144079, WO 2002/046387 oder US 6,768,044 beschrieben. Eine Toleranz gegenüber HPPD-Hemmern kann auch dadurch erzielt werden, dass man Pflanzen mit Genen transformiert, die für gewisse Enzyme kodieren, die die Bildung von Homogentisat trotz Hemmung des nativen HPPD-Enzyms durch den HPPD-Hemmer ermöglichen. Solche Pflanzen sind in WO 99/34008 und WO 02/36787 beschrieben. Die Toleranz von Pflanzen gegenüber HPPD-Hemmern kann auch dadurch verbessert werden, dass man Pflanzen zusätzlich zu einem Gen, das für ein HPPD-tolerantes Enzym kodiert, mit einem Gen transformiert, das für ein Prephenatdehydrogenase-Enzym kodiert, wie in WO 2004/024928 beschrieben ist. Außerdem können Pflanzen noch toleranter gegen HPPD-Hemmern gemacht werden, indem man ein Gen in ihr Genom einfügt, welches für ein Enzym kodiert, das HPPD-Hemmer metabolisiert oder abbaut, wie z.B. CYP450 Enzyme (siehe WO 2007/103567 und WO 2008/150473).

Weitere herbizidresistente Pflanzen sind Pflanzen, die gegenüber Acetolactatsynthase (ALS)-Hemmern tolerant gemacht worden sind. Zu bekannten ALS-Hemmern zählen zum Beispiel Sulfonylharnstoff, Imidazolinon, Triazolopyrimidine, Pyrimidinyloxy(thio)benzoate und/oder Sulfonylaminocarbonyltriazolinon-Herbizide. Es ist bekannt, dass verschiedene Mutationen im Enzym ALS (auch als Acetohydroxysäure-Synthase, AHAS, bekannt) eine Toleranz gegenüber unterschiedlichen Herbiziden bzw. Gruppen von Herbiziden verleihen wie z.B. in Tranel und Wright (Weed Science 2002, 50, 700-712) beschrieben ist. Die Herstellung von sulfonylharnstofftoleranten Pflanzen und imidazolinontoleranten Pflanzen ist beschrieben. Weitere sulfonylharnstoff- und imidazolinontolerante Pflanzen sind auch beschrieben.

Weitere Pflanzen, die gegenüber Imidazolinonen und/oder Sulfonylharnstoffen tolerant sind, können durch induzierte Mutagenese, Selektion in Zellkulturen in Gegenwart des Herbizids oder durch Mutationszüchtung erhalten werden (vgl. z.B. für Sojabohne US 5,084,082, für Reis WO 97/41218, für Zuckerrübe US 5,773,702 und WO 99/057965, für Salat US 5,198,599 oder für Sonnenblume WO 01/065922).

Pflanzen oder Pflanzensorten (die nach Methoden der pflanzlichen Biotechnologie, wie der Gentechnik, erhalten wurden), die ebenfalls erfindungsgemäß behandelt werden können, sind gegenüber abiotischen Stressfaktoren tolerant. Solche Pflanzen können durch genetische Transformation oder durch Selektion von Pflanzen, die eine Mutation enthalten, die solch eine Stressresistenz verleiht, erhalten werden. Zu besonders nützlichen Pflanzen mit Stresstoleranz zählen folgende:
a. Pflanzen, die ein Transgen enthalten, das die Expression und/oder Aktivität des Gens für die Poly(ADP-ribose)polymerase (PARP) in den Pflanzenzellen oder Pflanzen zu reduzieren vermag.
b. Pflanzen, die ein stresstoleranzförderndes Transgen enthalten, das die Expression und/oder Aktivität der für PARG kodierenden Gene der Pflanzen oder Pflanzenzellen zu reduzieren vermag;
c. Pflanzen, die ein stresstoleranzfördemdes Transgen enthalten, das für ein in Pflanzen funktionelles Enzym des Nicotinamidadenindinukleotid-Salvage-Biosynthesewegs kodiert, darunter Nicotinamidase, Nicotinatphosphoribosyltransferase, Nicotinsäuremononukleotidadenyltransferase, Nicotinamidadenindinukleotidsynthetase oder Nicotinamidphosphoribosyltransferase.

Pflanzen oder Pflanzensorten (die nach Methoden der pflanzlichen Biotechnologie, wie der Gentechnik, erhalten wurden), die ebenfalls erfindungsgemäß behandelt werden können, weisen eine veränderte Menge, Qualität und/oder Lagerfähigkeit des Ernteprodukts und/oder veränderte Eigenschaften von bestimmten Bestandteilen des Ernteprodukts auf, wie zum Beispiel:
1) Transgene Pflanzen, die eine modifizierte Stärke synthetisieren, die bezüglich ihrer chemisch-physikalischen Eigenschaften, insbesondere des Amylosegehalts oder des Amylose/Amylopektin-Verhältnisses, des Verzweigungsgrads, der durchschnittlichen Kettenlänge, der Verteilung der Seitenketten, des Viskositätsverhaltens, der Gelfestigkeit, der Stärkekomgröße und/oder Stärkekommorphologie im Vergleich mit der synthetisierten Stärke in Wildtyppflanzenzellen oder -pflanzen verändert ist, so dass sich diese modifizierte Stärke besser für bestimmte Anwendungen eignet.
2) Transgene Pflanzen, die Nichtstärkekohlenhydratpolymere synthetisieren, oder Nichtstärkekohlenhydratpolymere, deren Eigenschaften im Vergleich zu Wildtyppflanzen ohne genetische Modifikation verändert sind. Beispiele sind Pflanzen, die Polyfructose, insbesondere des Inulin- und Levantyps, produzieren, Pflanzen, die alpha-1,4-Glucane produzieren, Pflanzen, die alpha-1,6-verzweigte alpha-1,4-Glucane produzieren und Pflanzen, die Alternan produzieren.
3) Transgene Pflanzen, die Hyaluronan produzieren.
4) Transgene Pflanzen oder Hybridpflanzen wie Zwiebeln mit bestimmten Eigenschaften wie "hohem Anteil an löslichen Feststoffen" (,high soluble solids content'), geringe Schärfe (,low pungency', LP) und/oder lange Lagerfähigkeit (,long storage', LS).

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Baumwollpflanzen mit veränderten Fasereigenschaften. Solche Pflanzen können durch genetische Transformation oder durch Selektion von Pflanzen, die eine Mutation enthalten, die solche veränderten Fasereigenschaften verleiht, erhalten werden; dazu zählen:
a) Pflanzen wie Baumwollpflanzen, die eine veränderte Form von Cellulosesynthasegenen enthalten,
b) Pflanzen wie Baumwollpflanzen, die eine veränderte Form von rsw2- oder rsw3-homologen Nukleinsäuren enthalten, wie Baumwollpflanzen mit einer erhöhten Expression der Saccharosephosphatsynthase;
c) Pflanzen wie Baumwollpflanzen mit einer erhöhten Expression der Saccharosesynthase;
d) Pflanzen wie Baumwollpflanzen bei denen der Zeitpunkt der Durchlaßsteuerung der Plasmodesmen an der Basis der Faserzelle verändert ist, z. B. durch Herunterregulieren der faserselektiven β-1,3-Glucanase;
e) Pflanzen wie Baumwollpflanzen mit Fasern mit veränderter Reaktivität, z. B. durch Expression des N-Acetylglucosamintransferasegens, darunter auch nodC, und von Chitinsynthasegenen.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Raps oder verwandte Brassica-Pflanzen mit veränderten Eigenschaften der Ölzusammensetzung. Solche Pflanzen können durch genetische Transformation oder durch Selektion von Pflanzen, die eine Mutation enthalten, die solche veränderten Öleigenschaften verleiht, erhalten werden; dazu zählen:
a) Pflanzen wie Rapspflanzen, die Öl mit einem hohen Ölsäuregehalt produziere;
b) Pflanzen wie Rapspflanzen, die Öl mit einem niedrigen Linolensäuregehalt produzieren.
c) Pflanzen wie Rapspflanzen, die Öl mit einem niedrigen gesättigten Fettsäuregehalt produzieren.

Pflanzen oder Pflanzensorten (die nach Methoden der pflanzlichen Biotechnologie, wie der Gentechnik, erhalten werden können), die ebenfalls erfindungsgemäß behandelt werden können, sind Pflanzen wie Kartoffeln, welche Virus-resistent sind z.B. gegen den Kartoffelvirus Y (Event SY230 und SY233 von Tecnoplant, Argentinien), oder welche resistent gegen Krankheiten wie die Kraut- und Knollenfäule (potato late blight) (z.B. RB Gen), oder welche eine verminderte kälteinduzierte Süße zeigen (welche die Gene Nt-Inh, II-INV tragen) oder welche den Zwerg-Phänotyp zeigen (Gen A-20 Oxidase).

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Raps oder verwandte Brassica-Pflanzen mit veränderten Eigenschaften im Samenausfall (seed shattering). Solche Pflanzen können durch genetische Transformation oder durch Selektion von Pflanzen, die eine Mutation enthalten, die solche veränderten Eigenschaften verleihen, und umfassen Pflanzen wie Raps mit verzögertem oder vermindertem Samenausfall.

Besonders nützliche transgene Pflanzen, die erfindungsgemäß behandelt werden können, sind Pflanzen mit Transformationsevents oder Kombinationen von Transformationsevent, welche in den USA beim Animal and Plant Health Inspection Service (APHIS) of the United States Department of Agriculture (USDA) Gegenstand von erteilten oder anhängigen Petitionen für den nicht-regulierten Status sind. Die Information hierzu ist jederzeit beim APHIS (4700 River Road Riverdale, MD 20737, USA) erhältlich, z.B. über die Internetseite http://www.aphis.usda.gov/brs/not_reg.html. Am Anmeldetag dieser Anmeldung waren beim APHIS die Petitionen mit folgenden Informationen entweder erteilt oder anhängig:
- Petition: Identifikationsnummer der Petition. Die Technische Beschreibung des Transformationsevents kann im einzelnen Petitionsdokument erhältlich von APHIS auf der Website über die Petitionsnummer gefunden werden. Diese Beschreibungen sind hiermit per Referenz offenbart.
- Erweiterung einer Petition: Referenz zu einer frühere Petition, für die eine Erweiterung oder Verlängerung beantragt wird.
- Institution: Name der die Petition einreichenden Person.
- Regulierter Artikel: die betroffen Pflanzenspecies.
- Transgener Phänotyp: die Eigenschaft ("Trait"), die der Pflanze durch das Transformationsevent verliehen wird.
- Transformationevent oder -linie: der Name des oder der Events (manchmal auch als Linie(n) bezeichnet), für die der nicht-regulierte Status beantragt ist.
- APHIS Documente: verschiedene Dokumente, die von APHIS bzgl. der Petition veröffentlicht warden oder von APHIS auf Anfrage erhalten werden können.

Besonders nützliche transgene Pflanzen, die erfindungsgemäß behandelt werden können, sind Pflanzen mit einem oder mehreren Genen, die für ein oder mehrere Toxine kodieren, sind die transgenen Pflanzen, die unter den folgenden Handelsbezeichnungen angeboten werden: YIELD GARD^{®} (zum Beispiel Mais, Baumwolle, Sojabohnen), KnockOut^{®} (zum Beispiel Mais), BiteGard^{®} (zum Beispiel Mais), BT-Xtra^{®} (zum Beispiel Mais), StarLink^{®} (zum Beispiel Mais), Bollgard^{®} (Baumwolle), Nucotn^{®} (Baumwolle), Nucotn 33B^{®} (Baumwolle), NatureGard^{®} (zum Beispiel Mais), Protecta^{®} und NewLeaf^{®} (Kartoffel). Herbizidtolerante Pflanzen, die zu erwähnen sind, sind zum Beispiel Maissorten, Baumwollsorten und Sojabohnensorten, die unter den folgenden Handelsbezeichnungen angeboten werden: Roundup Ready^{®} (Glyphosatetoleranz, zum Beispiel Mais, Baumwolle, Sojabohne), Liberty Link^{®} (Phosphinotricintoleranz, zum Beispiel Raps), IMI^{®} (Imidazolinontoleranz) und SCS^{®} (Sylfonylhamstofftoleranz), zum Beispiel Mais. Zu den herbizidresistenten Pflanzen (traditionell auf Herbizidtoleranz gezüchtete Pflanzen), die zu erwähnen sind, zählen die unter der Bezeichnung Clearfield^{®} angebotenen Sorten (zum Beispiel Mais).

Die nachstehenden Beispiele erläutern die vorliegende Erfindung.

### BEISPIELE

Die vorliegende Erfindung wird anhand der nachfolgenden Beispiele näher erläutert, welche die Erfindung jedoch keinesfalls beschränken.

### A. Synthesebeispiele

### Methyl-(2R)-2-{[4-brom-1-(3-fluorpyridin-2-yl)-5-(6-fluorpyridin-3-yl)-1H-pyrazol-3-yl]oxy}propanoat (1-05)

N'-(3-Fluorpyridin-2-yl)-3-(6-methylpyridin-3-yl)prop-2-inhydrazid:
Zu einer Lösung von 1,27 g (6,92 mmol) 3-(6-Fluorpyridin-3-yl)prop-2-insäure, 1,32 g (10,38 mmol) 2-Fluor-6-hydrazinopyridin und 3,5 g (34,61 mmol) Triethylamin in72 ml THF gibt man tropfenweise 6,6 g (10,38 mmol) einer 50%igen Propanphosphonsäureanhydrid-lösung in THF und rührt diese Mischung eine Stunde bei Raumtemperatur. Zur Aufarbeitung wird mit H₂O versetzt, die organische Phase abgetrennt und die wässerige Phase mehrmals mit CH₂Cl₂ extrahiert. Die vereinigte organische Phase wird über Na₂SO₄ getrocknet und eingeengt. Man erhält 2,25 g (95%) Rohprodukt, das ohne weitere Reinigung für die nächste Reaktionsstufe eingesetzt wird.

1-(3-Fluorpyridin-2-yl)-5-(6-fluorpyridin-3-y1)-1H-pyrazol-3-ol:
Eine Lösung von 2,23 g (6,50 mmol) N'-(3-Fluorpyridin-2-yl)-3-(6-methylpyridin-3-yl)prop-2-inhydrazid in 10 ml CH₂Cl₂ und 3 ml DMF wird mit 99 mg (0,52 mmol) CuI versetzt und eine Stunde bei 80 °C gerührt. Danach wird abfiltriert, eingeengt und das Rohprodukt säulenchromatographisch über Kieselgel mit Heptan/Essigester (3:7) gereinigt. Auf diese Weise erhält man 0,95 g (53%) Produkt als Feststoff.

¹H-NMR (400MHz, DMSO-d₆): δ 6.25 (bs, 1H), 7.15 (d, 1H), 7.50 (bs, 1H), 7.78 (m, 1H), 7.95 (m, 1H), 8.10 (s, 1H), 8.20 (bs, 1H).

Methyl-(2R)-2-{[1-(3-fluorpyridin-2-yl)-5-(6-fluorpyridin-3-yl)-1H-pyrazol-3-yl]oxy}propanoat:
Eine Lösung von 0,34 g (1,25 mmol) 1-(3-Fluorpyridin-2-yl)-5-(6-fluorpyridin-3-yl)-1H-pyrazol-3-ol in 10 ml DMF wird nacheinander mit 0,61 g (1,87 mmol)C₂CO₃ und 0,18 (1,50 mmol) Methyl-(2S)-2-chlorpropanoat versetzt und anschließend 1 Stunde bei 80°C gerührt. Danach wird das Reaktionsgemisch bis zur Trockene eingengt, in H₂O aufgenommen und mehrmals mit CH₂Cl₂ extrahiert. Die organische Phase wird über Na₂SO₄ getrocknet und anschließend eingeengt. Säulenchromatographische Reinigung über Kieselgel mit Heptan/Essigester (8.2) ergibt 0,4 g (87%) Produkt als farbloses Öl.

¹H-NMR (400 MHz, CDCl₃): δ 1.65 (d, 3H), 3.79 (s, 3H), 5.25 (q, 1H), 6.14 (s, 1H), 6.89 (m, 1H), 7.35 (m, 1H), 7.50 (m, 1H), 7.68 (m, 1H), 8.10 (s, 1H), 8.25 (d, 1H).

Methyl-(2R)-2-{[4-brom-1-(3-fluorpyridin-2-yl)-5-(6-fluorpyridin-3-yl)-1H-pyrazol-3- yl]oxy}propanoat:
Eine Lösung von 0,38 g (1,05 mmol) Methyl-(2R)-2-{[1-(3-fluorpyridin-2-yl)-5-(6-fluorpyridin-3-yl)-1H-pyrazol-3-yl]oxy}propanoat in 4 ml DMF wird mit 0,22 g (1,26 mmol) N-Bromsuccinimid versetzt und 1 Stunde bei 65°C gerührt. Danach gibt man das Reaktionsgemisch auf H₂O und extrahiert mehrmals mit CH₂Cl₂. Die organische Phase wird über Na₂SO₄ getrocknet, eingeengt und das so erhaltene Rohprodukt säulenchromatographisch über Kieselgel mit Heptan/Essigester (8:2) gereinigt. Auf diese Weise erhält man 0,45 g (95%) Produkt als Feststoff.

¹H-NMR (400 MHz, CDCl₃): δ 1.70 (d, 3H), 3.78 (s, 3H), 5.70 (q, 1H), 6.95 (m, 1H), 7.30 (m, 1H), 7.50 (m, 1H), 7.84 (m, 1H), 8.12 S, 1H), 8.20 (d, 1H).

### Methyl-(2R)-2-{[4-cyan-1-(3-fluorpyridin-2-yl)-5-(6-fluorpyridin-3-yl)-1H-pyrazol-3-yl]oxy}propanoat (1-06)

Unter Argon wird ein Gemisch aus 0,17 g (0,40 mmol) Methyl-(2R)-2-{[4-brom-1-(3-fluorpyridin-2-yl)-5-(6-fluorpyridin-3-yl)-1H-pyrazol-3-yl]oxy}propanoat, 0,045 g (0,38 mmol) Zn(CN)₂ und 0,046 g (0,040 mmol) Tetrakis(triphenylphosphin)palladium in einem ausgeheizten MW-Vial vorgelegt und mit 5 ml N,N-Dimethlacetamid versetzt. Das Gefäß wird entgast, mit Argon überlagert und das Reaktionsgemisch in der Mikrowelle 40 Minuten bei 180°C gerührt. Anschließend wird das Reaktionsgemisch eingeengt, in H₂O aufgenommen und mehrmals mit CH₂Cl₂ extrahiert. Die organische Phase wird über Na₂SO₄ getrocknet, eingeengt und das so erhaltene Rohprodukt säulenchromatographisch über Kieselgel mit Heptan/ Essigester (8:2) gereinigt. Auf diese Weise erhält man 0,082 g (52%) Produkt als Feststoff..

¹H-NMR (400 MHz, CDCl₃): δ 1.70 (d, 3H), 3.79 (s, 3H), 5.77 (q, 1H), 7.00 (m, 1H), 7.45 (m, 1H), 7.68 (m, 1H), 7.92 (m, 1H), 8.12 (s, 1H), 8.29 (d, 1H).

### Methyl-(2R)-2-{[4-chlor-5-(2-fluorpyridin-4-yl)-1-(pyridin-2-yl)-1H-pyrazol-3-yl]oxy}propanoat (..) und (2R)-2-{[4-Chlor-5-(2-fluorpyridin-4-yl)-1-(pyridin-2-yl)-1H-pyrazol-3-yl]oxy}propansäure (..)

### 5-Amino-4-chlor-1-(pyridin-2-yl)-1H-pyrazol-3-ol:

Zu einer Lösung von 2 g (11,35 mmol) 5-Amino-1-(pyridin-2-yl)-1H-pyrazol-3-ol in 80 ml DMF gibt man portionsweise 1,10 g (12,48 mmol) N-Chlorsuccinimid. Die Reaktionsmischung wird 5 Stunden bei 50°C gerührt und anschließend 12 Stunden bei Raumtemperatur stehen gelassen. Nach Zugabe von 80 ml H₂O und 60 ml CH₂Cl₂ fällt das Produkt als Feststoff aus. Filtration und Trocknung unter Vakuum liefert 1,43 g (60%) Produkt als Feststoff.

¹H-NMR (400 MHz, DMSO-d₆): δ 7.10 (bs, 2H, NH2), 7.20 (dd, 1H), 7.55 (d, 1H), 7.90 (dd, 1H), 8.46 (d, 1H).

### Methyl-(2R)-2-{ [5-amino-4-chlor-1-(pyridin-2-yl)-1H-pyrazol-3-yl]oxy}propanoat:

Zu einer Lösung von 1,4 g (6,64 mmol) 5-Amino-4-chlor-1-(pyridin-2-yl)-1H-pyrazol-3-ol gibt man 3,25 g (9,98 mmol) C₂CO₃ und rührt diese Mischung 10 Minuten bei Raumtemperatur. Nach Zugabe von 1,20 g (7,97 mmol) Methyl-(2S)-2-chlorpropanoat wird das Reaktionsgemisch 12 Stunden bei Raumtemperatur gerührt. Nach Filtration wird die Reaktionslösung auf H₂O gegeben und mehrmals mit CH₂Cl₂ extrahiert. Die organische Phase wird über Na₂SO₄ getrocknet und eingengt. Man erhält 1,56 g (75%) Produkt als Feststoff.

¹H-NMR (400 MHz, CDCl₃): δ 1.65 (d, 3H), 3.75 (s, 3H), 5.18 (q, 1H), 6.00 (bs, 2H, NH2), 7.04 (dd, 1H), 7.63 (d, 1H), 7.72 (dd, 1H), 8.26 (d, 2H).

### Methyl-(2R)-2-{ [4-chlor-5-iod-1-(pyridin-2-yl)-1H-pyrazol-3-yl]oxy}propanoat

Eine Lösung von 0,30 g (1,01 mmol) Methyl-(2R)-2-{[5-amino-4-chlor-1-(pyridin-2-yl)-1H-pyrazol-3-yl]oxy}propanoat in 6 ml Acetonitril wird nacheineander mit 1,08 g (4,04 mmol) Diiodmethan und 0,23 g (2,02 mmol) Isopentylnitrit versetzt und anschließend 1 Stunde bei 50°C gerührt. Danach wird die Reaktionsmischung auf H₂O gegeben und mehrmals mit CH₂Cl₂ extrahiert. Die organische Phase wird über Na₂SO₄ getrocknet, eingeengt und das Rohprodukt säulenchromatographisch über Kieselgel mit Heptan/Essigester (1:1) gereinigt. Auf diese Weise erhält man 0,24 g (55%) Produkt als Öl.

¹H-NMR (400 MHz, CDCl₃): δ 1.68 (d, 3H), 3.75 (s, 3H), 5.20 (q, 1H), 7.21 (dd, 1H), 7.60 (d, 1H), 7.70 (dd, 1H), 8.45 (d, 1H).

### (2R)-2-{[4-Chlor-5-(2-fluorpyridin-4-yl)-1-(pyridin-2-yl)-1H-pyrazol-3-yl]oxy}propansäure (I-21):

Unter Argon wird eine Mischung aus 0,18 g (0,45 mmol) Methyl-(2R)-2-{[4-chlor-5-iod-1-(pyridin-2-yl)-1H-pyrazol-3-yl]oxy}propanoat, 0,16 g (1,13 mmol) (2-Fluorpyridin-4-yl)boronsäure, 0,055 g (0,067 mmol) PdChdppf, DCM complex und 0,44 g (1,35 mmol) C₂CO₃ in 3 ml 1,4 Dioxan und 0,9 ml H₂O für 3,5 Stunden bei 130°C in der Mikrowelle gerührt. Danach gibt man das Reaktionsgemisch auf eine gesättigte wässerige NH₄Cl-Lösung und extrahiert mehrmals mit CH₂Cl₂. Die organische Phase wird über Na₂SO₄ getrocknet, eingeengt und das so erhaltene Rohprodukt säulenchromatographisch über Kieselgel mit Heptan/Essigester (1:1) gereinigt. Man erhält auf diese Weise 38 mg (21%) des Methylesters. Einengen der Wasserphase und anschließende säulenchromatographische Reinigung über Kieselgel mit Heptan/Essigester (3:7) liefert 67 mg (33%) der Carbonsäure.

¹H-NMR (400 MHz, DMSO-d₆): δ 1.55 (d, 3H), 5.10 (q, 1H), 7.26 (m, 2H) 7.65 (d, 1H), 7.96 (m, 2H), 8.10 (d, 1H), 8.20 (s, 1H).

### Ethyl-{[4-brom-5-(2-fluorpyridin-4-yl)-1-(pyridin-2-yl)-1H-pyrazol-3-yl]oxy}acetat (I-01)

### 2-[3-(Benzyloxy)-1H-pyrazol-1-yl]pyridin:

Eine Mischung bestehend aus 5 g (28,70 mmol) 3-(Benzyloxy)-1H-pyrazol, 6,80 g (43,05 mmol) 2-Brompyridin, 0,77 g (4,02 mmol) CuI und 13,09 (40,18 mmol) Cs₂CO₃ in 40 ml DMF wird 5 Stunden unter Argon bei 100°C gerührt. Danach wird das Reaktionsgemisch über Kieselgur filtriert, auf H₂O gegeben und mehrmals mit CH₂Cl₂ extrahiert. Die organische Phase wird über Na₂SO₄ getrocknet, eingeengt und das Rohprodukt säulenchromatographisch über Kieselgel mit Heptan/Essigester (1:1) gereinigt. Auf diese Weise erhält man 6,65 g (92%) Produkt als Feststoff.

¹H-NMR (400 MHz, CDCl₃): δ 5.31 (s, 2H), 5.95 (s, 1H), 7.10 (dd, 1H), 7.35 (m, 3H), 7.50 (m, 2H), 7.75 (m, 1H), 7.80 (d, 1H), 8.35 (d, 1H), 8.40 (s, 1H).

### 2-[3-(Benzyloxy)-5-iod-1H-pyrazol-1-yl]pyridin:

Zu einer Lösung von 31,80 mmol LDA in 140 ml THF gibt man unter Argon bei -78°C tropfenweise eine Lösung von 4,70 g (18,70 mmol) 2-[3-(Benzyloxy)-1H-pyrazol-1-yl]pyridin in 130 ml THF und rührt diese Mischung weitere 1,5 Stunden bei -78°C. Danach versetzt man das Reaktionsgemisch tropfenweise mit einer Lösung von 7,6 g (30,00 mmol) Iod in 130 ml THF und rührt das Reakionsgemisch weitere 2 Stunden bei -78°C, bevor man es auf Raumtemperatur kommen lässt. Danach gibt man das Reaktionsgemisch auf H₂O und und extrahiert mehrmals mit CH₂Cl₂. Die organische Phase wird über Na₂SO₄ getrocknet, eingeengt und das Rohprodukt säulenchromatographisch über Kieselgel mit Heptan/Essigester (8:2) gereinigt. Auf diese Weise erhält man 3,83 g (54%) Produkt als Öl.

¹H-NMR (400 MHz, CDCl₃): δ 5.29 (s, 2H), 6.20 (s, 1H), 7.25 (m, 1H), 7.24 (m, 1H), 7.38 (m, 2H), 7.45 (m, 1H), 7,73 (d, 1H), 8.50 (s, 1H).

4-[3-(Benzyloxy)-1-(pyridin-2-yl)-1H-pyrazol-5-yl]-2-fluorpyridin:
Zu einer Lösung von 0,50 g (1,32 mmol) 2-[3-(Benzyloxy)-5-iod-1H-pyrazol-1-yl]pyridin in 8 ml 1,4 Dioxan gibt man unter Argon nacheinander 0,65 g (2,91 mmol) 2-Fluor-4-(4,4,5,5-tetramethyl-1,3,2-dioxaborolan-2-yl)pyridin, 0,86 g (2,65 mmol) Cs₂CO₃, 13 mg (0,066 mmol) CuI, 1ml H₂O und 77 mg (0,066 mmol) Tetrakis(triphenylphosphin)palladium(0), rührt diese Mischung 3,5 Stunden bei 70°C und lässt sie anschließend über Nacht stehen. Nach Filtration wird die Reaktionslösung eingeengt und das Rohgemisch säulenchromatographisch über Kieselgel mit Heptan/Essigester (7:3) gereinigt. Auf diese Weise erhält man 0,32 g (69%) produkt als Feststoff.

1-H-NMR (400 MHz, CDCl3) d 5.35 (s, 1H), 6.10 (s, 1H), 6.85 (s, 1H), 7.08 (d, 1H), 7.20 (m, 1H), 7.40 (m, 3H) 7.50 (m, 2H), 7.70 (d, 1H), 7.82, (m, 1H), 8.20 (m, 2H), 8.40 (d, 1H).

5-(2-Fluorpyridin-4-yl)-1-(pyridin-2-yl)-1H-pyrazol-3-ol:
Eine Lösung von 0,44 g (0,63 mmol) 5-(2-Fluorpyridin-4-yl)-1-(pyridin-2-yl)-1H-pyrazol-3-ol in 18 ml Toluol wird mit 37 g (324,5 mmol) Trifluoressigsäure (über Molsieb getrocknet) versetzt und anschließend 3,5 Stunden bei 50°C gerührt und über Nacht bei Raumtemperatur stehen gelassen. Danach gibt man nochmals 3 ml Trifluoressigsäure zu und rührt weitere 3 Stunden bei 50°C. Nach Filtration wird die Reaktionslösung eingeengt und das Rohprodukt säulenchromatographisch über Kieslgel mit Heptan/Essigester (1:1) gereinigt. Das so erhaltene Gemisch enthält 50% Produkt und wird ohne weitere Reinigung im nächsten Reaktionsschritt eingesetzt.

Ethyl-{ [5-(2-fluorpyridin-4-yl)-1-(pyridin-2-yl)-1H-pyrazol-3-yl]oxy} acetat:
Eine Lösung von 0,07 g (0,273 mmol) 5-(2-Fluorpyridin-4-yl)-1-(pyridin-2-yl)-1H-pyrazol-3-ol in 5,5 ml Acetonitril wird mit 0,23 g (1,64 mmol) K₂CO₃ versetzt und 10 Minuten bei Raumtemperatur gerührt. Nach Hinzufügen von 0,091 g (0,55 mmol) Bromessigsäureethylester wird die Reaktionsmischung 5 Stunden unter Rückfluss gerührt und anschließend über Nacht bei Raumtemperatur stehen gelassen. Zur Aufarbeitung wird bis zur Trockene eingeengt, der Rückstand in H₂O aufgenommen und mehrmals mit CH₂Cl₂ extrahiert. Die organische Phase wird über Na₂SO₄ getrocknet, eingeengt und das Rohprodukt säulenchromatographisch über Kieselgel mit Heptan/Essigester (7:3) gereinigt. Man erhält 0,036 g (38%) Produkt als Feststoff.

¹H-NMR (400 MHz, CDCl₃): δ 1.32 (t, 3h), 4.30 (q, 2H), 4.90 (s, 2H), 6.15 (s, 1H), 6.85 (s, 1H), 7.08 (s, 1H), 7.08 (m, 1H), 7.15 (dd, 1H),7.65 (d, 1H), 7.80 dd, 1H), 8.15 (m, 2H).

Ethyl-{ [4-brom-5-(2-fluorpyridin-4-yl)-1-(pyridin-2-yl)-1H-pyrazol-3-yl]oxy}acetat:
Eine Lösung von 0,036 g (0,105 mmol) Ethyl-{[5-(2-fluorpyridin-4-yl)-1-(pyridin-2-yl)-1H-pyrazol-3-yl]oxy}acetat in 0,6 ml CH₂Cl₂ wird mit 0,021 g (0,116 mmol) N-Bromsuccinimid versetzt und 12 Stunden bei Raumtemperatur gerührt. Danach wird bis zur Trockene eingeengt, der Rückstand mit Diethylether ausgerührt und abfiltriert. Die Etherphase wird eingeengt und das so erhaltene Rohprodukt über Kieselgel mit Heptan/Essigester (1.1) gereinigt. Auf diese Weise erhält man 0.021 g(47%) Produkt als Feststoff.

¹H-NMR (400 MHz, CDCl₃): δ 1.30 (t, 3H), 4.70 (q, 2H), 4.95 (s, 2H), 6.93 (6.93 (s, 1H), 7.12 (m, 2H), 7.62 (d, 1H), 7.69 (m, 1H), 8.08 (m, 1H), 8.25 (s, 1H).

### NMR-Daten ausgewählter Beispiele

### NMR-Peak-Listenverfahren

Die 1H-NMR-Daten ausgewählter Beispiele werden in Form von 1H-NMR-Peaklisten notiert. Zu jedem Signalpeak wird erst der δ-Wert in ppm und dann die Signalintensität in runden Klammern aufgeführt.

Die δ-Wert - Signalintensitäts- Zahlenpaare von verschiedenen Signalpeaks werden durch Semikolons voneinander getrennt aufgelistet.

Die Peakliste eines Beispieles hat daher die Form:
δ₁ (Intensität₁⁾; δ₂ (Intensität₂);........; δᵢ (Intensitätᵢ⁾;......; δₙ (Intensitätₙ)

Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden.

Zur Kalibrierung der chemischen Verschiebung von 1H-NMR-Spektren benutzen wir Tetramethylsilan und/oder die chemische Verschiebung des Lösungsmittels, besondern im Falle von Spektren, die in DMSO gemessen werden. Daher kann in NMR-Peaklisten der Tetramethylsilan-Peak vorkommen, muss es aber nicht.

Die Listen der 1H-NMR-Peaks sind ähnlich den klassischen 1H-NMR-Ausdrucken und enthalten somit gewöhnlich alle Peaks, die bei einer klassischen NMR-Interpretation aufgeführt werden.

Darüber hinaus können sie wie klassische 1H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen.

Bei der Angabe von Verbindungssignalen im Delta-Bereich von Lösungsmitteln und/oder Wasser sind in unseren Listen von 1H-NMR-Peaks die gewöhnlichen Lösungsmittelpeaks, zum Beispiel Peaks von DMSO in DMSO-D₆ und der Peak von Wasser, gezeigt, die gewöhnlich im Durchschnitt eine hohe Intensität aufweisen.

Die Peaks von Stereoisomeren der Targetverbindungen und/oder Peaks von Verunreinigungen haben gewöhnlich im Durchschnitt eine geringere Intensität als die Peaks der Zielverbindungen (zum Beispiel mit einer Reinheit von >90%).

Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen.

Einem Experten, der die Peaks der Zielverbindungen mit bekannten Verfahren (MestreC, ACD-Simulation, aber auch mit empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen 1H-NMR-Interpretation.

Weitere Details zu 1H-NMR-Peaklisten können der Research Disclosure Database Number 564025 entnommen werden.

| |
|---|
| |
| III-17: ¹H-NMR(400.6 MHz, CDCl3): |
| δ= 8.2900 (1.0); 8.2858 (0.8); 8.2838 (1.1); 8.2369 (1.5); 8.2229 (1.5); 7.7967 (0.5); 7.7905 (0.5); 7.7784 (0.6); 7.7756 (0.7); 7.7721 (0.6); 7.7693 (0.6); 7.7572 (0.6); 7.7509 (0.6); 7.2622 (23.5); 7.2472 (1.5); 7.2431 (1.5); 7.2425 (1.5); 7.1074 (0.7); 7.1059 (0.7); 7.0998 (0.7); 7.0984 (0.7); 7.0862 (0.6); 7.0847 (0.7); 7.0786 (0.7); 7.0771 (0.7); 6.8507 (1.2); 6.8457 (1.1); 6.8367 (1.2); 6.8318 (1.2); 5.3008 (2.4); 4.9451 (6.9); 4.4248 (0.5); 4.4071 (0.5); 4.3977 (1.4); 4.3800 (1.5); 4.3691 (1.4); 4.3554 (1.5); 4.3420 (0.6); 4.3283 (0.5); 3.7264 (5.8); 3.6838 (16.0); 2.8442 (0.7); 2.8304 (0.7); 1.5598 (1.0); 1.2166 (6.4); 1.1985 (8.8); 1.1801 (2.8); 0.0080 (0.5); -0.0002 (20.0); -0.0085 (0.6) |
| III-18: ¹H-NMR(400.6 MHz, CDCl3): |
| δ= 8.2943 (1.2); 8.2920 (0.9); 8.2880 (1.2); 8.2667 (1.9); 8.2527 (2.0); 7.7848 (0.6); 7.7785 (0.6); 7.7664 (0.7); 7.7636 (0.7); 7.7602 (0.7); 7.7574 (0.7); 7.7454 (0.6); 7.7391 (0.6); 7.2730 (2.0); 7.2718 (1.9); 7.2681 (2.3); 7.2669 (2.2); 7.2613 (66.1); 7.2571 (1.1); 7.2547 (0.6); 7.1186 (0.9); 7.1110 (0.9); 7.0974 (1.0); 7.0959 (0.9); 7.0898 (1.2); 6.8962 (1.4); 6.8913 (1.4); 6.8822 (1.4); 6.8773 (1.4); 5.3005 (5.6); 4.8910 (7.1); 4.7277 (0.8); 4.7097 (1.2); 4.6916 (0.8); 3.7897 (16.0); 2.0457 (0.9); 1.5610 (0.8); 1.5155 (6.4); 1.4976 (6.4); 1.2598 (0.8); 0.0080 (1.3); -0.0002 (43.5); -0.0085 (1.2) |
| III-19: ¹H-NMR(400.6 MHz, CDCl3): |
| δ= 8.2865 (1.7); 8.2806 (1.7); 8.2423 (2.2); 8.2284 (2.2); 7.7914 (0.6); 7.7851 (0.6); 7.7728 (0.9); 7.7702 (1.0); 7.7667 (0.9); 7.7642 (0.9); 7.7518 (0.7); 7.7456 (0.7); 7.2618 (22.0); 7.2373 (2.4); 7.2328 (2.5); 7.1055 (1.1); 7.0987 (1.1); 7.0844 (1.0); 7.0776 (1.0); 6.8606 (1.5); 6.8557 (1.5); 6.8466 (1.5); 6.8417 (1.4); 5.3007 (3.1); 4.9430 (8.9); 4.5290 (2.3); 4.5135 (4.5); 4.4980 (2.3); 3.6977 (16.0); 2.7244 (2.3); 2.7090 (4.5); 2.6934 (2.2); 1.5550 (4.1); 0.0077 (1.0); -0.0002 (18.4); -0.0085 (0.8) |
| III-20: ¹H-NMR(400.0 MHz, CDCl3): |
| δ= 8.8739 (2.0); 8.3010 (4.7); 8.2871 (7.3); 7.7875 (1.5); 7.7666 (2.3); 7.7485 (1.3); 7.5191 (0.9); 7.2606 (138.6); 7.2459 (5.6); 7.1329 (2.3); 7.1119 (2.3); 6.9969 (1.0); 6.8927 (2.9); 6.8787 (2.6); 5.3001 (6.6); 4.9916 (16.0); 4.3623 (1.9); 4.3410 (5.6); 4.3199 (5.6); 4.2984 (2.1); 3.7009 (1.1); 3.6783 (3.4); 2.3863 (1.1); 2.0480 (1.9); 1.2551 (5.1); 0.8813 (1.0); -0.0002 (86.1) |
| III-21: ¹H-NMR(400.6 MHz, CDCl3): |
| δ= 8.2761 (1.6); 8.2700 (1.7); 8.2213 (2.2); 8.2074 (2.2); 7.7718 (0.8); 7.7655 (0.8); 7.7533 (1.0); 7.7506 (1.0); 7.7470 (1.0); 7.7444 (1.0); 7.7322 (0.9); 7.7259 (0.9); 7.3382 (2.1); 7.3341 (2.2); 7.2618 (48.0); 7.0931 (1.1); 7.0869 (1.0); 7.0720 (1.1); 7.0657 (0.9); 6.8511 (1.6); 6.8462 (1.6); 6.8371 (1.6); 6.8322 (1.8); 5.3006 (10.2); 5.1282 (8.2); 5.0132 (1.4); 4.1923 (7.1); 4.1629 (1.4); 3.8196 (2.8); 3.7545 (16.0); 3.7263 (1.9); 3.6778 (1.2); 3.1780 (15.4); 3.0640 (2.4); 2.0455 (1.5); 1.5623 (2.7); 1.2775 (0.5); 1.2596 (1.2); 1.1981 (0.9); 1.1800 (0.9); 0.0079 (1.2); -0.0002 (41.9); -0.0085 (1.3) |
| III-22: ¹H-NMR(400.6 MHz, CDCl3): |
| δ= 8.2716 (1.1); 8.2695 (0.8); 8.2674 (0.9); 8.2653 (1.1); 8.2244 (1.7); 8.2233 (1.7); 8.2104 (1.8); 8.2093 (1.7); 7.7748 (0.6); 7.7685 (0.6); 7.7563 (0.7); 7.7536 (0.7); 7.7501 (0.7); 7.7473 (0.7); 7.7352 (0.6); 7.7289 (0.6); 7.2610 (18.5); 7.2044 (1.7); 7.2033 (1.8); 7.1995 (1.8); 7.1984 (1.7); 7.1034 (0.8); 7.1018 (0.8); 7.0957 (0.8); 7.0942 (0.8); 7.0822 (0.7); 7.0807 (0.8); 7.0746 (0.8); 7.0731 (0.7); 6.8187 (1.6); 6.8138 (1.5); 6.8047 (1.5); 6.7998 (1.5); 5.3003 (4.6); 5.2557 (1.5); 5.2383 (1.5); 3.8171 (16.0); 1.7238 (6.1); 1.7064 (6.0); 1.5446 (5.7); 0.0079 (0.8); -0.0002 (29.6); -0.0044 (0.7); -0.0085 (0.9) |
| III-23: ¹H-NMR(400.6 MHz, d₆-DMSO): |
| δ= 8.3828 (3.2); 8.3765 (3.4); 8.3304 (5.1); 8.3293 (5.1); 8.3165 (5.3); 8.3153 (5.3); 8.1194 (1.3); 8.1132 (1.3); 8.1000 (1.7); 8.0982 (1.8); 8.0938 (1.7); 8.0920 (1.8); 8.0789 (1.4); 8.0726 (1.4); 7.4231 (1.9); 7.4175 (2.0); 7.4017 (2.0); 7.3962 (1.8); 7.3949 (1.8); 7.3413 (5.0); 7.3402 (5.4); 7.3365 (5.5); 7.3353 (5.0); 7.0466 (4.6); 7.0418 (4.3); 7.0327 (4.5); 7.0278 (4.5); 4.9455 (13.8); 4.0555 (1.0); 4.0377 (3.3); 4.0200 (3.4); 4.0022 (1.1); 3.3197 (14.5); 2.6746 (0.7); 2.6700 (1.0); 2.6654 (0.7); 2.5238 (2.6); 2.5191 (3.6); 2.5104 (53.3); 2.5058 (117.6); 2.5012 (165.8); 2.4966 (113.7); 2.4920 (50.1); 2.3329 (0.7); 2.3282 (1.0); 2.3236 (0.7); 1.9886 (16.0); 1.9084 (6.4); 1.1922 (4.8); 1.1744 (9.8); 1.1566 (4.6); 0.0079 (3.5); 0.0063 (0.8); 0.0055 (0.8); 0.0047 (1.0); 0.0039 (1.5); 0.0030 (2.6); 0.0022 (4.6); -0.0002 (126.0); -0.0027 (5.4); -0.0035 (3.7); -0.0043 (2.2); -0.0052 (1.6); -0.0060 (1.4); -0.0068 (1.2); -0.0085 (3.7); -0.0098 (0.8); -0.0107 (0.6) |
| III-24: ¹H-NMR(400.6 MHz, CDCl3): |
| δ= 8.2774 (2.2); 8.2755 (1.7); 8.2730 (1.8); 8.2712 (2.3); 8.2274 (3.4); 8.2135 (3.5); 7.7848 (1.1); 7.7785 (1.1); 7.7664 (1.2); 7.7637 (1.4); 7.7601 (1.3); 7.7574 (1.3); 7.7453 (1.2); 7.7390 (1.2); 7.2659 (5.4); 7.2566 (3.4); 7.2559 (3.3); 7.2518 (3.7); 7.1090 (1.5); 7.1076 (1.5); 7.1014 (1.6); 7.1000 (1.5); 7.0878 (1.4); 7.0865 (1.4); 7.0802 (1.5); 7.0789 (1.4); 6.8223 (2.7); 6.8174 (2.6); 6.8083 (2.6); 6.8034 (2.6); 5.3015 (10.6); 4.9365 (15.5); 4.3343 (2.0); 4.3165 (6.4); 4.2987 (6.4); 4.2809 (2.1); 1.5976 (1.8); 1.3536 (7.8); 1.3358 (16.0); 1.3180 (7.5); -0.0002 (7.7) |
| II-05: ¹H-NMR(599.6 MHz, CDCl3): |
| δ= 8.1957 (8.6); 8.1880 (8.5); 8.0835 (12.3); 8.0800 (12.8); 7.8348 (4.0); 7.8321 (4.4); 7.8195 (8.3); 7.8069 (4.3); 7.8042 (4.4); 7.7761 (3.9); 7.7721 (4.2); 7.7621 (6.7); 7.7597 (6.7); 7.7497 (4.1); 7.7456 (4.0); 7.2948 (6.5); 7.2866 (6.9); 7.2828 (6.7); 7.2740 (6.0); 7.2628 (23.5); 6.9817 (7.2); 6.9773 (7.4); 6.9676 (6.9); 6.9631 (6.9); 5.1781 (3.4); 5.1665 (10.9); 5.1549 (10.9); 5.1433 (3.4); 4.3424 (0.4); 4.3306 (0.3); 4.2776 (0.5); 4.2656 (1.4); 4.2596 (4.0); 4.2557 (4.8); 4.2477 (11.6); 4.2439 (12.5); 4.2358 (11.9); 4.2321 (12.2); 4.2240 (4.5); 4.2203 (4.1); 4.2140 (1.3); 4.2021 (0.4); 4.1278 (0.4); 4.1158 (0.4); 2.0443 (1.4); 1.6935 (42.5); 1.6819 (42.0); 1.6011 (0.4); 1.5896 (0.6); 1.5719 (39.4); 1.3895 (0.4); 1.3777 (0.8); 1.3660 (0.5); 1.3002 (1.1); 1.2766 (25.3); 1.2647 (50.0); 1.2529 (25.0); 1.2157 (0.3); 1.2037 (0.4); 0.8933 (2.0); 0.8819 (4.4); 0.8699 (2.2); -0.0001 (32.1) |
| II-01: ¹H-NMR(400.0 MHz, CDCl3_5mm): |
| δ= 8.2145 (1.4); 8.2105 (1.9); 8.2070 (1.5); 8.2025 (1.5); 8.1988 (1.9); 8.1949 (1.4); 8.0914 (2.7); 8.0853 (2.7); 7.8801 (1.2); 7.8756 (1.1); 7.8608 (1.5); 7.8568 (2.0); 7.8529 (1.3); 7.8381 (1.2); 7.8335 (1.2); 7.7941 (1.1); 7.7878 (1.1); 7.7754 (1.4); 7.7728 (1.6); 7.7692 (1.5); 7.7666 (1.4); 7.7542 (1.2); 7.7480 (1.1); 7.3180 (1.5); 7.3154 (1.5); 7.3058 (1.5); 7.3032 (1.5); 7.2987 (1.5); 7.2961 (1.4); 7.2866 (1.4); 7.2840 (1.3); 7.2629 (9.8); 6.9946 (1.8); 6.9882 (1.8); 6.9743 (1.6); 6.9733 (1.7); 6.9670 (1.6); 4.8918 (16.0); 4.3018 (2.0); 4.2840 (6.3); 4.2661 (6.4); 4.2483 (2.1); 2.0454 (0.9); 1.5681 (12.7); 1.3140 (7.4); 1.2962 (14.9); 1.2783 (7.6); 1.2596 (1.2); 0.8818 (1.2); 0.0079 (0.6); -0.0002 (13.6); -0.0084 (0.5) |
| II-03: ¹H-NMR(599.6 MHz, CDCl3): |
| δ= 8.2192 (7.4); 8.2115 (7.3); 8.1073 (11.4); 8.1034 (11.3); 7.9010 (3.7); 7.8981 (3.7); 7.8856 (7.1); 7.8730 (3.9); 7.8702 (3.6); 7.7990 (3.6); 7.7949 (3.6); 7.7848 (5.7); 7.7825 (5.5); 7.7725 (3.8); 7.7684 (3.4); 7.3295 (5.8); 7.3212 (6.1); 7.3169 (5.9); 7.3087 (5.3); 7.2638 (14.2); 7.0023 (6.5); 6.9979 (6.5); 6.9881 (6.3); 6.9837 (6.1); 5.9493 (0.3); 5.2999 (12.4); 5.0641 (0.3); 4.9612 (50.0); 2.1801 (2.2); 1.3126 (0.7); 1.3015 (1.4); 1.2897 (1.7); 1.2798 (1.6); 1.2652 (3.6); 0.8932 (3.4); 0.8817 (7.7); 0.8697 (3.7); -0.0001 (17.0) |
| II-36: ¹H-NMR(400.0 MHz, CDCl3_5mm): |
| δ= 8.2050 (0.8); 8.2012 (1.1); 8.1975 (0.9); 8.1930 (0.9); 8.1894 (1.1); 8.1855 (0.8); 8.0862 (1.6); 8.0801 (1.6); 7.8409 (0.6); 7.8363 (0.6); 7.8215 (0.8); 7.8176 (1.2); 7.8137 (0.8); 7.7989 (0.7); 7.7943 (0.7); 7.7831 (0.7); 7.7769 (0.6); 7.7644 (0.8); 7.7619 (0.9); 7.7583 (0.9); 7.7557 (0.8); 7.7433 (0.7); 7.7371 (0.6); 7.3053 (0.8); 7.3027 (0.8); 7.2932 (0.9); 7.2906 (0.9); 7.2861 (0.9); 7.2835 (0.8); 7.2739 (0.8); 7.2713 (0.9); 7.2624 (6.5); 6.9894 (1.0); 6.9884 (1.0); 6.9821 (1.0); 6.9682 (1.0); 6.9672 (1.0); 6.9608 (1.0); 5.2009 (1.7); 5.1834 (1.7); 3.7815 (16.0); 2.0454 (1.1); 1.7016 (6.8); 1.6841 (6.7); 1.5632 (8.5); 1.2773 (0.6); 1.2596 (1.2); 0.8818 (1.0); -0.0002 (9.3) |
| III-01: ¹H-NMR(400.6 MHz, CDCl3): |
| δ= 8.2767 (2.0); 8.2745 (1.5); 8.2724 (1.6); 8.2704 (2.0); 8.2685 (1.3); 8.0783 (2.9); 8.0642 (3.0); 7.7967 (1.1); 7.7905 (1.1); 7.7784 (1.2); 7.7756 (1.3); 7.7721 (1.2); 7.7693 (1.3); 7.7572 (1.2); 7.7510 (1.2); 7.2610 (34.6); 7.1099 (1.4); 7.1083 (1.4); 7.1023 (1.4); 7.1007 (1.4); 7.0887 (1.4); 7.0872 (1.4); 7.0811 (1.4); 7.0795 (1.3); 6.8357 (1.1); 6.8328 (1.3); 6.8311 (1.5); 6.8283 (1.4); 6.8216 (1.0); 6.8187 (1.2); 6.8170 (1.6); 6.8143 (1.5); 6.7998 (2.1); 6.7966 (2.8); 6.7932 (1.6); 4.9354 (14.4); 4.3306 (1.8); 4.3128 (5.9); 4.2950 (6.0); 4.2772 (1.9); 2.0455 (0.9); 1.5450 (8.2); 1.3469 (7.6); 1.3291 (16.0); 1.3113 (7.5); 1.2641 (0.7); 1.2597 (1.0); 0.8820 (1.6); 0.8643 (0.6); 0.0080 (1.2); -0.0002 (46.5); -0.0085 (1.3) |
| III-25: ¹H-NMR(400.6 MHz, d₆-DMSO): |
| δ= 12.6699 (1.3); 8.5145 (2.9); 8.4960 (3.0); 8.3741 (4.4); 8.3678 (4.5); 8.1583 (6.5); 8.1441 (6.8); 8.1147 (1.8); 8.1085 (1.8); 8.0953 (2.3); 8.0936 (2.5); 8.0891 (2.2); 8.0873 (2.4); 8.0742 (1.9); 8.0679 (1.9); 7.4245 (2.7); 7.4190 (2.7); 7.4046 (2.4); 7.4032 (2.6); 7.3977 (2.6); 7.3964 (2.4); 7.0636 (4.8); 7.0622 (4.9); 7.0059 (2.4); 7.0027 (3.1); 6.9984 (2.1); 6.9918 (2.4); 6.9886 (3.0); 6.9843 (2.1); 6.8707 (0.7); 4.8530 (15.4); 4.3384 (0.5); 4.3203 (2.6); 4.3020 (4.1); 4.2836 (2.7); 4.2656 (0.5); 3.6216 (0.9); 3.6177 (5.2); 3.6155 (2.9); 3.6114 (2.7); 3.6093 (2.0); 3.6074 (3.6); 3.6011 (12.2); 3.5989 (4.2); 3.5949 (3.8); 3.5930 (2.0); 3.5908 (2.7); 3.5866 (2.7); 3.5845 (5.4); 3.5806 (0.9); 3.3208 (25.7); 3.2024 (1.4); 2.6749 (0.6); 2.6703 (0.9); 2.6657 (0.6); 2.5409 (1.9); 2.5241 (2.4); 2.5195 (3.2); 2.5107 (48.1); 2.5061 (105.8); 2.5015 (148.8); 2.4969 (102.1); 2.4923 (44.6); 2.3332 (0.6); 2.3286 (0.9); 2.3239 (0.6); 2.1829 (1.2); 1.9949 (1.1); 1.9086 (0.8); 1.7809 (0.8); 1.7763 (5.3); 1.7686 (4.2); 1.7642 (2.5); 1.7597 (15.2); 1.7549 (2.6); 1.7507 (4.1); 1.7431 (5.2); 1.7385 (0.8); 1.3552 (11.4); 1.3254 (16.0); 1.3072 (15.9); 1.2363 (0.8); 1.0897 (0.6); 1.0743 (0.6); 0.0080 (1.4); 0.0039 (0.7); 0.0022 (1.9); -0.0002 (46.9); -0.0027 (1.9); -0.0034 (1.2); -0.0043 (0.6); - 0.0085 (1.2) |
| III-26: ¹H-NMR(400.6 MHz, d₆-DMSO): |
| δ= 12.6543 (1.2); 8.4833 (1.4); 8.4686 (2.9); 8.4537 (1.4); 8.3683 (4.3); 8.3620 (4.4); 8.1613 (6.3); 8.1472 (6.6); 8.1062 (1.7); 8.0999 (1.6); 8.0850 (2.4); 8.0804 (2.2); 8.0788 (2.3); 8.0656 (1.8); 8.0594 (1.8); 7.4229 (2.7); 7.4172 (2.6); 7.4016 (2.6); 7.3959 (2.5); 7.0825 (5.1); 7.0259 (2.4); 7.0227 (3.1); 7.0186 (2.0); 7.0118 (2.3); 7.0086 (3.0); 7.0044 (2.0); 4.8783 (16.0); 3.8523 (8.0); 3.8376 (8.1); 3.6176 (0.8); 3.6072 (0.6); 3.6010 (1.9); 3.5948 (0.7); 3.5865 (0.5); 3.5844 (0.8); 3.3207 (28.0); 2.6747 (0.7); 2.6702 (1.0); 2.6657 (0.7); 2.5239 (2.7); 2.5193 (4.1); 2.5104 (54.6); 2.5059 (116.9); 2.5013 (161.6); 2.4968 (111.6); 2.4922 (49.2); 2.4737 (0.6); 2.4692 (0.7); 2.3331 (0.7); 2.3285 (1.0); 2.3238 (0.7); 1.7762 (0.8); 1.7686 (0.7); 1.7596 (2.4); 1.7506 (0.7); 1.7431 (0.8); 1.3550 (3.8); 0.0080 (1.2); -0.0002 (45.9); -0.0085 (1.3) |
| III-02: ¹H-NMR(400.6 MHz, d₆-DMSO): |
| δ= 13.1557 (0.6); 8.3798 (3.0); 8.3736 (3.1); 8.1842 (4.4); 8.1701 (4.5); 8.1164 (1.3); 8.1100 (1.2); 8.0969 (1.6); 8.0952 (1.8); 8.0908 (1.6); 8.0889 (1.7); 8.0758 (1.4); 8.0695 (1.4); 7.4184 (1.9); 7.4129 (1.9); 7.3971 (1.8); 7.3917 (1.8); 7.0277 (1.4); 7.0233 (2.1); 7.0201 (1.7); 7.0137 (1.4); 7.0093 (2.2); 7.0061 (1.8); 6.9802 (3.7); 4.9454 (12.8); 4.0554 (1.1); 4.0377 (3.4); 4.0199 (3.4); 4.0021 (1.1); 3.3185 (43.8); 2.6790 (0.6); 2.6744 (1.3); 2.6698 (1.8); 2.6652 (1.3); 2.6605 (0.6); 2.5342 (1.0); 2.5294 (1.5); 2.5236 (5.5); 2.5190 (7.4); 2.5102 (97.6); 2.5056 (219.0); 2.5010 (309.1); 2.4964 (217.1); 2.4918 (96.9); 2.4596 (0.7); 2.4561 (0.7); 2.3374 (0.6); 2.3327 (1.3); 2.3281 (1.8); 2.3234 (1.3); 2.3190 (0.6); 1.9885 (16.0); 1.9082 (6.6); 1.1922 (4.7); 1.1744 (9.7); 1.1566 (4.7); 0.1458 (1.0); 0.0294 (0.5); 0.0279 (0.8); 0.0174 (0.5); 0.0150 (0.6); 0.0143 (0.7); 0.0134 (0.7); 0.0127 (0.8); 0.0119 (0.9); 0.0111 (1.0); 0.0102 (1.4); 0.0080 (9.5); 0.0064 (2.4); 0.0055 (2.6); 0.0047 (3.2); 0.0038 (4.5); -0.0002 (322.9); -0.0050 (4.7); -0.0059 (3.4); -0.0067 (2.8); -0.0085 (9.1); -0.0107 (1.2); -0.0115 (1.1); -0.0122 (0.8); -0.0130 (0.6); -0.0394 (0.6); -0.1493 (1.0) |
| III-07: ¹H-NMR(400.6 MHz, CDCl3): |
| δ= 8.2904 (1.3); 8.2861 (1.0); 8.2841 (1.3); 8.1193 (1.8); 8.1052 (1.9); 7.7981 (0.7); 7.7918 (0.7); 7.7798 (0.7); 7.7769 (0.8); 7.7735 (0.8); 7.7706 (0.8); 7.7586 (0.7); 7.7523 (0.7); 7.2633 (17.0); 7.1206 (0.9); 7.1193 (0.9); 7.1130 (0.9); 7.1117 (0.9); 7.0995 (0.8); 7.0980 (0.9); 7.0919 (0.8); 7.0905 (0.8); 6.9198 (0.8); 6.9170 (0.9); 6.9153 (0.9); 6.9126 (0.8); 6.9058 (0.7); 6.9030 (0.9); 6.9012 (0.9); 6.8985 (0.8); 6.8176 (1.2); 6.8141 (1.9); 6.8106 (1.1); 5.3011 (4.1); 4.9294 (7.5); 4.1853 (3.5); 4.1720 (3.4); 3.7992 (16.0); 2.0456 (1.8); 1.5772 (7.7); 1.2776 (0.5); 1.2597 (1.1); 1.2419 (0.5); -0.0002 (11.7) |
| III-27: ¹H-NMR(400.6 MHz, CDCl3): |
| δ= 8.2873 (1.2); 8.2853 (0.9); 8.2831 (0.9); 8.2810 (1.2); 8.0864 (1.6); 8.0724 (1.6); 7.8080 (0.6); 7.8017 (0.6); 7.7896 (0.7); 7.7869 (0.8); 7.7834 (0.7); 7.7806 (0.7); 7.7685 (0.7); 7.7622 (0.7); 7.2627 (17.0); 7.1099 (0.8); 7.1084 (0.8); 7.1023 (0.8); 7.1008 (0.8); 7.0888 (0.8); 7.0872 (0.8); 7.0811 (0.8); 7.0796 (0.8); 6.8565 (0.6); 6.8537 (0.8); 6.8520 (0.9); 6.8492 (0.8); 6.8425 (0.6); 6.8396 (0.7); 6.8379 (0.9); 6.8351 (0.8); 6.8129 (1.2); 6.8096 (1.6); 6.8063 (0.9); 5.3009 (2.2); 4.9395 (8.0); 4.5278 (2.0); 4.5123 (3.8); 4.4969 (2.0); 3.6987 (16.0); 2.7208 (1.9); 2.7054 (3.7); 2.6899 (1.9); 1.5644 (10.0); -0.0002 (11.8) |
| III-28: ¹H-NMR(400.6 MHz, CDCl3): |
| δ= 8.2929 (1.1); 8.2908 (0.9); 8.2885 (0.9); 8.2867 (1.2); 8.1171 (1.5); 8.1031 (1.5); 7.8021 (0.6); 7.7958 (0.6); 7.7838 (0.7); 7.7809 (0.7); 7.7775 (0.7); 7.7747 (0.7); 7.7626 (0.6); 7.7563 (0.6); 7.2643 (11.6); 7.1225 (0.8); 7.1211 (0.8); 7.1149 (0.9); 7.1135 (0.8); 7.1013 (1.0); 7.0999 (1.0); 7.0938 (1.1); 7.0924 (1.0); 6.9154 (0.7); 6.9126 (0.8); 6.9108 (0.8); 6.9082 (0.7); 6.9013 (0.6); 6.8985 (0.8); 6.8968 (0.8); 6.8941 (0.7); 6.8166 (1.0); 6.8132 (1.6); 6.8097 (1.0); 5.3014 (4.0); 4.8908 (7.1); 4.7269 (0.8); 4.7088 (1.2); 4.6908 (0.8); 3.7883 (16.0); 2.0458 (1.6); 1.5928 (5.1); 1.5143 (6.4); 1.4964 (6.3); 1.2777 (0.5); 1.2599 (1.1); - 0.0002 (8.1) |
| I-23: ¹H-NMR(400.6 MHz, CDCl3): |
| δ= 8.3881 (2.3); 8.3841 (2.4); 8.3764 (2.4); 8.3724 (2.4); 8.1389 (2.2); 8.1327 (2.4); 7.8626 (1.1); 7.8564 (1.0); 7.8438 (1.2); 7.8414 (1.3); 7.8376 (1.2); 7.8352 (1.3); 7.8227 (1.2); 7.8164 (1.1); 7.8012 (2.4); 7.7972 (2.4); 7.7811 (2.6); 7.7771 (2.6); 7.3335 (2.5); 7.3219 (2.5); 7.3135 (2.4); 7.3018 (2.3); 7.2617 (11.3); 6.9351 (1.5); 6.9280 (1.6); 6.9138 (1.5); 6.9067 (1.5); 4.9187 (16.0); 4.2820 (1.9); 4.2642 (6.0); 4.2464 (6.1); 4.2286 (2.0); 2.0453 (1.3); 1.5616 (5.4); 1.3002 (7.2); 1.2824 (14.6); 1.2646 (7.6); 1.2599 (1.5); 0.8821 (1.3); 0.8644 (0.5); -0.0002 (16.8); -0.0085 (0.6) |
| I-24: ¹H-NMR(400.6 MHz, CDCl3): |
| δ= 8.3500 (1.3); 8.3460 (1.4); 8.3384 (1.4); 8.3343 (1.4); 8.1353 (1.0); 8.1332 (0.8); 8.1311 (0.9); 8.1291 (1.1); 7.8576 (0.6); 7.8513 (0.6); 7.8388 (0.7); 7.8364 (0.7); 7.8325 (0.7); 7.8301 (0.7); 7.8176 (0.6); 7.8114 (0.6); 7.8028 (1.4); 7.7988 (1.4); 7.7827 (1.5); 7.7787 (1.5); 7.3170 (1.5); 7.3053 (1.5); 7.2969 (1.4); 7.2852 (1.4); 7.2619 (7.1); 6.9300 (0.7); 6.9286 (0.8); 6.9226 (0.8); 6.9211 (0.8); 6.9088 (0.8); 6.9073 (0.8); 6.9013 (0.8); 6.8998 (0.7); 5.2593 (1.5); 5.2419 (1.5); 4.1309 (0.6); 4.1130 (0.6); 3.7563 (16.0); 2.0453 (2.8); 1.6961 (6.3); 1.6787 (6.3); 1.5609 (3.4); 1.2773 (1.0); 1.2645 (0.8); 1.2595 (2.1); 1.2416 (0.9); 0.8818 (1.5); 0.8641 (0.6); -0.0002 (11.2) |
| I-25: ¹H-NMR(400.6 MHz, CDCl3): |
| δ= 8.3707 (3.6); 8.3667 (3.8); 8.3590 (3.8); 8.3550 (3.8); 8.1350 (3.4); 8.1287 (3.5); 7.8620 (1.7); 7.8557 (1.6); 7.8433 (2.0); 7.8408 (2.1); 7.8371 (2.0); 7.8346 (1.9); 7.8221 (1.8); 7.8158 (1.8); 7.8111 (3.8); 7.8071 (3.8); 7.7910 (4.1); 7.7870 (4.0); 7.3331 (4.0); 7.3214 (4.0); 7.3130 (3.8); 7.3012 (3.6); 7.2606 (50.9); 6.9398 (2.3); 6.9325 (2.4); 6.9186 (2.2); 6.9112 (2.2); 5.3337 (1.2); 5.3162 (4.6); 5.2988 (4.7); 5.2814 (1.3); 1.7459 (16.0); 1.7284 (15.9); 1.2644 (2.1); 0.8990 (1.1); 0.8821 (3.8); 0.8644 (1.5); 0.0080 (2.4); -0.0002 (81.0); -0.0086 (2.8) |
| I-26: ¹H-NMR(400.6 MHz, CDCl3): |
| δ= 8.3888 (3.3); 8.3872 (3.5); 8.3849 (3.6); 8.3771 (3.5); 8.3756 (3.7); 8.3732 (3.6); 8.1350 (4.4); 8.1291 (4.6); 7.8596 (1.8); 7.8534 (1.8); 7.8387 (2.6); 7.8327 (2.5); 7.8197 (2.0); 7.8135 (2.0); 7.8030 (4.1); 7.7990 (4.3); 7.7830 (4.6); 7.7790 (4.6); 7.3362 (4.0); 7.3245 (4.0); 7.3161 (3.9); 7.3044 (3.9); 7.2668 (73.3); 6.9349 (3.2); 6.9275 (3.3); 6.9137 (3.2); 6.9063 (3.2); 5.3020 (1.2); 4.9259 (16.0); 4.9220 (14.5); 4.0916 (0.8); 4.0876 (0.8); 2.5953 (1.2); 2.0982 (1.2); 1.2637 (2.1); 0.8983 (1.0); 0.8819 (2.9); 0.8641 (1.3); 0.0079 (2.0); -0.0002 (84.1); -0.0084 (4.0); -0.0266 (0.8) |
| I-27: ¹H-NMR(400.6 MHz, CDCl3): |
| δ= 8.3542 (2.5); 8.3502 (2.7); 8.3425 (2.7); 8.3385 (2.8); 8.1323 (2.4); 8.1261 (2.6); 7.8582 (1.2); 7.8520 (1.2); 7.8394 (1.4); 7.8371 (1.5); 7.8332 (1.4); 7.8309 (1.5); 7.8183 (1.3); 7.8120 (1.3); 7.7986 (2.6); 7.7946 (2.7); 7.7786 (2.9); 7.7745 (2.9); 7.3154 (2.8); 7.3038 (2.8); 7.2954 (2.7); 7.2837 (2.6); 7.2613 (17.2); 6.9288 (1.8); 6.9215 (1.8); 6.9076 (1.7); 6.9003 (1.7); 5.3002 (1.7); 5.2577 (0.9); 5.2403 (3.3); 5.2230 (3.4); 5.2056 (1.0); 4.2408 (1.8); 4.2230 (5.9); 4.2052 (6.2); 4.1875 (2.1); 2.1730 (1.1); 1.6909 (13.0); 1.6735 (13.0); 1.2677 (7.8); 1.2499 (16.0); 1.2321 (7.8); 0.0079 (0.8); -0.0002 (26.7); -0.0084 (1.2) |
| I-28: ¹H-NMR(400.6 MHz, CDCl3): |
| δ= 8.3902 (1.3); 8.3862 (1.4); 8.3785 (1.4); 8.3745 (1.4); 8.1420 (1.3); 8.1358 (1.4); 7.8654 (0.6); 7.8591 (0.6); 7.8466 (0.7); 7.8442 (0.8); 7.8404 (0.8); 7.8380 (0.8); 7.8255 (0.7); 7.8193 (0.7); 7.8062 (1.4); 7.8022 (1.4); 7.7860 (1.6); 7.7821 (1.5); 7.3382 (1.5); 7.3265 (1.5); 7.3182 (1.4); 7.3064 (1.4); 7.2610 (13.6); 6.9387 (0.9); 6.9314 (1.0); 6.9175 (0.9); 6.9101 (0.9); 5.3003 (0.6); 4.9372 (9.2); 3.7929 (16.0); 0.0079 (0.6); -0.0002 (21.5); -0.0085 (0.9) |
| 1-08: ¹H-NMR(400.6 MHz, d₆-DMSO): |
| δ= 13.0716 (0.6); 8.2690 (2.0); 8.2666 (2.7); 8.2656 (2.6); 8.2635 (2.2); 8.2573 (2.3); 8.2551 (2.9); 8.2541 (2.6); 8.2518 (2.2); 8.2214 (3.2); 8.2151 (3.5); 7.9863 (3.0); 7.9826 (2.2); 7.9804 (1.8); 7.9653 (4.1); 7.9613 (4.9); 7.9589 (2.9); 7.9578 (2.8); 7.9461 (1.8); 7.9401 (3.4); 7.9367 (2.2); 7.6052 (2.0); 7.5958 (2.4); 7.5936 (2.1); 7.5843 (3.7); 7.5750 (1.8); 7.5727 (1.9); 7.5634 (1.7); 7.2910 (2.0); 7.2897 (2.1); 7.2841 (2.2); 7.2828 (2.1); 7.2697 (2.0); 7.2684 (2.1); 7.2628 (2.1); 7.2614 (2.0); 5.7569 (1.2); 4.8568 (16.0); 3.3362 (17.9); 2.6709 (0.6); 2.5247 (1.5); 2.5200 (2.0); 2.5112 (28.2); 2.5067 (62.8); 2.5021 (89.3); 2.4975 (63.5); 2.4929 (30.3); 2.4693 (1.1); 2.3291 (0.6); 1.2472 (1.0); 0.8749 (0.5); 0.8581 (2.1); 0.8404 (0.7); 0.0080 (1.6); 0.0056 (0.5); 0.0047 (0.6); -0.0002 (52.2); -0.0050 (1.0); -0.0058 (0.9); -0.0067 (0.8); -0.0085 (1.7) |
| I-08: ¹H-NMR(400.0 MHz, d₆-DMSO): |
| δ= 13.0690 (0.6); 8.2662 (2.8); 8.2549 (3.0); 8.2218 (3.5); 8.2155 (3.6); 7.9865 (2.8); 7.9825 (2.1); 7.9655 (3.8); 7.9614 (4.8); 7.9464 (1.6); 7.9401 (3.2); 7.9368 (1.9); 7.6053 (1.8); 7.5958 (2.2); 7.5939 (2.0); 7.5843 (3.2); 7.5749 (1.7); 7.5729 (1.8); 7.5634 (1.4); 7.2900 (2.2); 7.2838 (2.3); 7.2687 (2.2); 7.2623 (2.1); 5.7570 (1.2); 4.8573 (16.0); 3.1690 (1.0); 2.5247 (0.8); 2.5201 (1.2); 2.5112 (22.3); 2.5067 (49.8); 2.5021 (70.0); 2.4976 (49.8); 2.4930 (22.7); 1.2466 (1.1); 0.8747 (0.5); 0.8580 (1.9); 0.8403 (0.7); 0.0080 (1.0); -0.0002 (39.0); -0.0085 (1.2) |
| I-18: ¹H-NMR(400.6 MHz, CDCl3): |
| δ= 8.4613 (3.2); 8.4547 (3.3); 8.3036 (3.5); 8.2283 (2.4); 8.2263 (2.5); 8.2183 (2.2); 8.2166 (2.5); 8.2147 (2.4); 7.5540 (1.6); 7.5496 (1.7); 7.5471 (1.8); 7.5427 (1.6); 7.5319 (1.7); 7.5275 (1.9); 7.5250 (1.8); 7.5190 (2.2); 7.5142 (1.7); 7.4972 (2.2); 7.4939 (2.7); 7.4911 (1.9); 7.4740 (2.1); 7.4703 (2.0); 7.3554 (2.0); 7.3463 (2.3); 7.3438 (2.1); 7.3347 (3.5); 7.3257 (1.6); 7.3230 (1.7); 7.3141 (1.6); 7.2704 (1.0); 7.2697 (1.0); 7.2680 (1.1); 7.2672 (1.3); 7.2664 (1.6); 7.2606 (212.2); 7.2494 (0.5); 6.9970 (1.1); 5.3576 (1.2); 5.3401 (4.7); 5.3226 (4.8); 5.3052 (1.2); 5.3002 (0.9); 2.7754 (0.7); 1.7488 (16.0); 1.7313 (15.9); 1.7142 (0.6); 1.2425 (1.2); 1.2390 (1.2); 1.2222 (0.5); 0.0080 (3.7); 0.0056 (1.1); 0.0047 (1.5); -0.0002 (126.8); -0.0058 (1.4); -0.0066 (1.2); - 0.0084 (3.3); -0.0115 (0.5) |
| I-18: ¹H-NMR(599.6 MHz, d₆-DMSO): |
| δ= 8.6435 (2.3); 8.6390 (2.2); 8.3566 (2.4); 8.2335 (1.4); 8.2259 (1.4); 7.9829 (0.7); 7.9667 (1.2); 7.9523 (0.7); 7.8251 (0.8); 7.8220 (0.9); 7.8179 (0.6); 7.8093 (0.8); 7.8062 (0.9); 7.8021 (0.6); 7.5878 (0.6); 7.5812 (0.9); 7.5739 (1.1); 7.5673 (0.8); 7.5601 (0.5); 5.0737 (0.4); 5.0622 (1.6); 5.0506 (1.6); 5.0390 (0.4); 3.3186 (21.5); 2.6135 (0.3); 2.5221 (1.2); 2.5191 (1.4); 2.5159 (1.6); 2.5042 (39.7); 2.5014 (50.0); 2.4985 (36.2); 1.5768 (5.4); 1.5651 (5.3); -0.0001 (5.4) |
| I-17: ¹H-NMR(400.6 MHz, d₆-DMSO): |
| δ= 13.0891 (1.1); 8.6502 (5.7); 8.6432 (5.8); 8.3674 (3.0); 8.3633 (5.6); 8.3594 (2.8); 8.2467 (2.1); 8.2444 (2.7); 8.2434 (2.7); 8.2412 (2.2); 8.2351 (2.3); 8.2328 (2.9); 8.2296 (2.2); 7.9959 (1.8); 7.9924 (1.8); 7.9751 (2.1); 7.9711 (3.1); 7.9673 (2.0); 7.9500 (2.0); 7.9464 (1.9); 7.8428 (2.0); 7.8384 (2.0); 7.8359 (2.1); 7.8315 (1.9); 7.8191 (2.1); 7.8147 (2.3); 7.8122 (2.0); 7.8078 (1.9); 7.6008 (2.1); 7.5914 (2.4); 7.5892 (2.2); 7.5799 (3.7); 7.5706 (1.8); 7.5683 (1.9); 7.5590 (1.6); 5.7569 (4.0); 4.8613 (16.0); 3.6011 (0.5); 3.3203 (13.8); 2.6748 (0.7); 2.6702 (0.9); 2.6656 (0.7); 2.5239 (2.4); 2.5193 (3.3); 2.5105 (49.0); 2.5060 (107.5); 2.5014 (150.7); 2.4968 (103.1); 2.4922 (45.4); 2.3330 (0.6); 2.3284 (0.9); 2.3238 (0.6); 1.7595 (0.6); 1.3552 (1.4); 1.2355 (0.7); 0.0080 (1.6); 0.0040 (0.6); -0.0002 (56.7); -0.0050 (0.7); -0.0058 (0.6); -0.0085 (1.5) |
| I-17: ¹H-NMR(400.6 MHz, d₆-DMSO): |
| δ= 13.0844 (1.7); 8.6499 (5.8); 8.6430 (5.8); 8.3670 (3.0); 8.3628 (5.8); 8.3589 (2.9); 8.2464 (2.1); 8.2440 (2.8); 8.2430 (2.8); 8.2409 (2.4); 8.2348 (2.4); 8.2325 (3.0); 8.2294 (2.3); 7.9959 (1.8); 7.9924 (1.8); 7.9750 (2.1); 7.9710 (3.2); 7.9672 (2.0); 7.9498 (2.1); 7.9463 (2.0); 7.8424 (2.0); 7.8381 (2.1); 7.8355 (2.1); 7.8311 (1.9); 7.8188 (2.2); 7.8144 (2.3); 7.8119 (2.0); 7.8075 (1.9); 7.6006 (2.1); 7.5913 (2.4); 7.5890 (2.2); 7.5797 (3.8); 7.5704 (1.8); 7.5682 (2.0); 7.5588 (1.7); 5.7568 (4.2); 4.8604 (16.0); 3.3190 (34.6); 2.6745 (1.0); 2.6698 (1.5); 2.6652 (1.1); 2.6607 (0.5); 2.5405 (1.2); 2.5237 (3.1); 2.5190 (4.7); 2.5102 (79.1); 2.5056 (175.0); 2.5011 (247.3); 2.4965 (172.8); 2.4919 (78.5); 2.4742 (3.7); 2.4703 (3.4); 2.3328 (1.0); 2.3281 (1.5); 2.3235 (1.1); 0.1457 (0.7); 0.0079 (6.6); 0.0055 (1.5); -0.0002 (243.0); -0.0068 (2.5); -0.0085 (7.4); -0.0139 (0.9); -0.0155 (0.8); -0.0187 (0.6); -0.0211 (0.6); -0.0226 (0.7); -0.0258 (0.5); -0.0275 (0.9); -0.0313 (0.9); -0.0322 (0.8); -0.0344 (0.5); -0.1495 (0.7) |
| I-17: ¹H-NMR(599.6 MHz, d₆-DMSO): |
| δ= 8.6435 (2.3); 8.6390 (2.2); 8.3566 (2.4); 8.2335 (1.4); 8.2259 (1.4); 7.9829 (0.7); 7.9667 (1.2); 7.9523 (0.7); 7.8251 (0.8); 7.8220 (0.9); 7.8179 (0.6); 7.8093 (0.8); 7.8062 (0.9); 7.8021 (0.6); 7.5878 (0.6); 7.5812 (0.9); 7.5739 (1.1); 7.5673 (0.8); 7.5601 (0.5); 5.0737 (0.4); 5.0622 (1.6); 5.0506 (1.6); 5.0390 (0.4); 3.3186 (21.5); 2.6135 (0.3); 2.5221 (1.2); 2.5191 (1.4); 2.5159 (1.6); 2.5042 (39.7); 2.5014 (50.0); 2.4985 (36.2); 1.5768 (5.4); 1.5651 (5.3); -0.0001 (5.4) |
| I-15: ¹H-NMR(400.6 MHz, CDCl3): |
| δ= 8.4443 (1.3); 8.4376 (1.4); 8.2932 (1.5); 8.2037 (0.8); 8.2020 (0.9); 8.2002 (0.9); 8.1921 (0.9); 8.1904 (1.0); 8.1885 (1.0); 7.5266 (1.0); 7.5239 (0.9); 7.5221 (0.9); 7.5197 (1.0); 7.5152 (0.7); 7.5070 (1.0); 7.5040 (1.8); 7.5003 (1.2); 7.4973 (0.9); 7.4929 (0.7); 7.4838 (0.8); 7.4801 (0.8); 7.3419 (0.7); 7.3329 (0.8); 7.3303 (0.8); 7.3213 (1.3); 7.3123 (0.6); 7.3096 (0.7); 7.3007 (0.6); 7.2614 (30.4); 5.2864 (1.6); 5.2690 (1.6); 3.7710 (16.0); 2.0454 (0.6); 1.7015 (6.6); 1.6841 (6.6); 1.5664 (1.7); 1.2596 (0.6); 0.8820 (0.6); 0.0080 (0.6); -0.0002 (18.4); -0.0084 (0.7) |
| I-15: ¹H-NMR(400.0 MHz, CDCl3): |
| δ= 8.4434 (1.7); 8.4369 (1.6); 8.2926 (1.9); 8.2004 (1.3); 8.1903 (1.3); 7.5258 (1.3); 7.5027 (2.0); 7.4922 (0.8); 7.4826 (0.8); 7.3410 (0.7); 7.3304 (1.0); 7.3202 (1.3); 7.3101 (0.8); 7.2998 (0.7); 7.2600 (23.4); 5.3035 (0.5); 5.2860 (1.8); 5.2684 (1.7); 5.2511 (0.6); 3.7709 (16.0); 1.7012 (7.0); 1.6838 (6.8); 1.5459 (12.9); 0.0072 (1.4); -0.0002 (32.0); -0.0081 (1.2) |
| I-29: ¹H-NMR(400.6 MHz, CDCl3): |
| δ= 8.2558 (0.6); 8.2541 (0.7); 8.2522 (0.7); 8.2505 (0.6); 8.2442 (0.6); 8.2424 (0.7); 8.2406 (0.7); 8.2389 (0.7); 8.1338 (0.9); 8.1318 (0.7); 8.1296 (0.8); 8.1276 (1.0); 7.8457 (0.6); 7.8432 (0.6); 7.8395 (0.6); 7.8370 (0.6); 7.8245 (0.6); 7.8183 (0.5); 7.5051 (0.5); 7.4881 (0.7); 7.4845 (0.8); 7.4820 (0.6); 7.4650 (0.7); 7.4613 (0.7); 7.3557 (0.6); 7.3467 (0.7); 7.3441 (0.7); 7.3351 (1.1); 7.3260 (0.5); 7.3234 (0.6); 7.2612 (21.3); 6.9646 (0.6); 6.9631 (0.7); 6.9571 (0.7); 6.9556 (0.7); 6.9434 (0.6); 6.9418 (0.7); 6.9359 (0.6); 6.9344 (0.6); 4.9905 (6.9); 4.3640 (1.6); 4.3556 (0.9); 4.3523 (1.7); 4.3486 (0.9); 4.3404 (1.8); 3.6273 (2.0); 3.6190 (0.9); 3.6154 (1.8); 3.6121 (1.0); 3.6037 (1.9); 3.3659 (16.0); 1.5532 (10.3); 0.0079 (0.6); -0.0002 (24.7); -0.0085 (0.9) |
| I-29: ¹H-NMR(400.6 MHz, CDCl3): |
| δ= 8.2539 (1.0); 8.2523 (1.0); 8.2424 (1.0); 8.2407 (1.0); 8.1338 (1.3); 8.1277 (1.4); 7.8643 (0.6); 7.8581 (0.6); 7.8455 (0.7); 7.8432 (0.8); 7.8393 (0.7); 7.8371 (0.7); 7.8244 (0.6); 7.8182 (0.6); 7.5089 (0.6); 7.5052 (0.6); 7.4881 (0.9); 7.4848 (1.1); 7.4651 (0.8); 7.4615 (0.7); 7.3557 (0.7); 7.3465 (0.8); 7.3442 (0.8); 7.3350 (1.2); 7.3259 (0.6); 7.3236 (0.6); 7.3144 (0.5); 7.2615 (29.8); 6.9633 (0.9); 6.9560 (0.9); 6.9421 (0.9); 6.9347 (0.8); 4.9903 (8.3); 4.3638 (2.1); 4.3521 (2.3); 4.3485 (1.4); 4.3402 (2.2); 3.6271 (2.3); 3.6189 (1.4); 3.6152 (2.4); 3.6121 (1.4); 3.6035 (2.2); 3.3657 (16.0); 1.5595 (1.4); 0.0078 (0.7); -0.0002 (18.1); -0.0085 (0.7) |
| I-30: ¹H-NMR(400.6 MHz, CDCl₃): |
| δ= 8.2566 (2.4); 8.2467 (2.4); 8.1491 (3.1); 7.8691 (1.2); 7.8627 (1.1); 7.8482 (1.8); 7.8441 (1.7); 7.8292 (1.2); 7.8231 (1.1); 7.5227 (1.2); 7.5023 (2.4); 7.4823 (1.5); 7.3725 (1.2); 7.3630 (1.8); 7.3519 (2.0); 7.3425 (1.4); 7.3313 (0.9); 7.2610 (54.2); 6.9706 (2.0); 6.9643 (1.9); 6.9499 (1.9); 6.9430 (1.8); 5.0371 (14.5); 4.8405 (16.0); 4.3074 (0.7); 2.0454 (0.8); 1.5505 (2.2); 1.2596 (0.9); -0.0002 (32.7) |
| I-30: ¹H-NMR(400.6 MHz, CDCl3): |
| δ= 8.2583 (1.2); 8.2564 (1.3); 8.2466 (1.3); 8.2448 (1.3); 8.1494 (1.7); 8.1431 (1.8); 7.8692 (0.9); 7.8629 (0.9); 7.8505 (1.0); 7.8480 (1.1); 7.8443 (1.0); 7.8418 (1.1); 7.8293 (1.0); 7.8231 (1.0); 7.5260 (0.9); 7.5223 (0.9); 7.5190 (0.6); 7.5053 (1.2); 7.5019 (1.5); 7.4993 (1.0); 7.4823 (1.2); 7.4785 (1.2); 7.3724 (1.2); 7.3633 (1.3); 7.3608 (1.2); 7.3517 (2.0); 7.3426 (0.9); 7.3401 (0.9); 7.3310 (0.9); 7.2606 (84.6); 6.9705 (1.2); 6.9644 (1.2); 6.9492 (1.2); 6.9431 (1.2); 5.0371 (13.7); 4.8404 (16.0); 1.5427 (8.1); 0.0081 (2.8); -0.0002 (97.2); -0.0085 (3.3) |
| I-31: ¹H-NMR(400.6 MHz, CDCl3): |
| δ= 8.2951 (1.6); 8.2934 (1.9); 8.2915 (1.9); 8.2898 (1.8); 8.2835 (1.8); 8.2818 (2.0); 8.2799 (2.0); 8.2782 (1.8); 8.1602 (2.5); 8.1582 (1.9); 8.1559 (2.0); 8.1539 (2.7); 7.8498 (1.4); 7.8435 (1.4); 7.8312 (1.5); 7.8286 (1.7); 7.8249 (1.6); 7.8224 (1.6); 7.8100 (1.5); 7.8037 (1.5); 7.5386 (1.3); 7.5349 (1.4); 7.5179 (1.9); 7.5156 (1.9); 7.5143 (2.1); 7.5121 (1.6); 7.4950 (1.8); 7.4913 (1.8); 7.3980 (1.8); 7.3888 (2.0); 7.3864 (1.9); 7.3773 (3.2); 7.3681 (1.4); 7.3657 (1.5); 7.3566 (1.3); 7.2627 (20.5); 6.9737 (1.8); 6.9723 (1.8); 6.9663 (1.9); 6.9648 (1.8); 6.9526 (1.7); 6.9510 (1.8); 6.9451 (1.8); 6.9436 (1.8); 6.6525 (0.6); 5.9488 (0.6); 5.9350 (1.3); 5.9230 (0.8); 5.9214 (0.7); 5.9093 (1.4); 5.9060 (0.8); 5.8956 (0.7); 5.8922 (1.6); 5.8801 (0.9); 5.8786 (0.9); 5.8665 (1.6); 5.8528 (0.8); 5.2850 (0.8); 5.2808 (2.0); 5.2776 (2.2); 5.2734 (1.0); 5.2422 (0.7); 5.2379 (1.8); 5.2347 (1.9); 5.2305 (0.9); 5.1997 (0.9); 5.1961 (2.3); 5.1928 (2.3); 5.1893 (0.9); 5.1740 (0.8); 5.1704 (2.2); 5.1671 (2.2); 5.1636 (0.9); 4.8925 (16.0); 4.0378 (1.1); 4.0338 (2.1); 4.0299 (1.3); 4.0233 (2.2); 4.0196 (3.8); 4.0158 (2.3); 4.0093 (1.4); 4.0053 (2.2); 4.0013 (1.3); 2.0454 (0.7); 1.5810 (5.4); 1.2595 (0.7); 1.2558 (0.5); -0.0002 (19.4); -0.0085 (0.7) |
| I-32: ¹H-NMR(400.6 MHz, CDCl3): |
| δ= 8.2186 (2.6); 8.2085 (2.6); 8.1250 (3.4); 8.1191 (3.3); 7.8562 (1.3); 7.8501 (1.3); 7.8352 (1.9); 7.8312 (1.8); 7.8164 (1.4); 7.8101 (1.3); 7.5151 (1.4); 7.5115 (1.3); 7.4913 (2.7); 7.4712 (1.7); 7.4676 (1.6); 7.3406 (1.4); 7.3315 (1.9); 7.3200 (2.5); 7.3108 (1.6); 7.2995 (1.1); 7.2882 (0.7); 7.2607 (91.6); 6.9971 (0.5); 6.9611 (2.2); 6.9542 (2.2); 6.9398 (2.1); 6.9328 (2.0); 5.2846 (1.0); 5.2673 (3.4); 5.2499 (3.4); 5.2327 (1.1); 4.2557 (1.9); 4.2382 (5.8); 4.2205 (6.0); 4.2027 (2.1); 1.6950 (13.6); 1.6776 (13.5); 1.5614 (3.7); 1.2730 (7.9); 1.2553 (16.0); 1.2375 (7.6); -0.0002 (53.1); -0.0082 (2.8) |
| I-32: ¹H-NMR(400.0 MHz, CDCl3): |
| δ= 8.2276 (0.6); 8.2159 (0.7); 8.1274 (0.9); 8.1214 (0.9); 7.8382 (0.6); 7.8357 (0.6); 7.8319 (0.5); 7.8293 (0.5); 7.4966 (0.6); 7.4935 (0.8); 7.4734 (0.6); 7.4697 (0.6); 7.3439 (0.5); 7.3349 (0.6); 7.3323 (0.5); 7.3232 (0.9); 7.2606 (32.9); 6.9615 (0.6); 6.9553 (0.6); 6.9402 (0.6); 6.9326 (0.6); 5.2744 (1.0); 5.2569 (1.1); 4.2582 (0.6); 4.2403 (1.8); 4.2233 (1.8); 4.2053 (0.6); 1.6959 (4.9); 1.6785 (4.8); 1.5489 (16.0); 1.2744 (3.0); 1.2566 (6.3); 1.2388 (2.9); 0.0080 (0.9); -0.0002 (33.9); -0.0085 (1.0) |
| I-33: ¹H-NMR(400.6 MHz, CDCl3): |
| δ= 8.2173 (1.4); 8.2155 (1.6); 8.2137 (1.6); 8.2120 (1.4); 8.2057 (1.5); 8.2039 (1.6); 8.2020 (1.6); 8.2004 (1.4); 8.1339 (2.0); 8.1317 (1.6); 8.1295 (1.7); 8.1276 (2.2); 7.8630 (1.1); 7.8567 (1.1); 7.8442 (1.3); 7.8418 (1.3); 7.8380 (1.3); 7.8356 (1.3); 7.8230 (1.2); 7.8168 (1.2); 7.5079 (1.1); 7.5043 (1.1); 7.4873 (1.5); 7.4844 (1.8); 7.4838 (1.8); 7.4810 (1.3); 7.4641 (1.4); 7.4604 (1.4); 7.3362 (1.4); 7.3272 (1.6); 7.3246 (1.4); 7.3156 (2.5); 7.3066 (1.1); 7.3039 (1.2); 7.2950 (1.0); 7.2630 (25.4); 6.9575 (1.5); 6.9560 (1.5); 6.9501 (1.5); 6.9486 (1.4); 6.9363 (1.4); 6.9348 (1.4); 6.9289 (1.5); 6.9274 (1.4); 5.2827 (0.8); 5.2653 (3.1); 5.2479 (3.1); 5.2306 (0.8); 4.2547 (1.2); 4.2529 (1.2); 4.2369 (3.8); 4.2352 (3.7); 4.2190 (4.0); 4.2175 (3.8); 4.2011 (1.4); 4.1998 (1.3); 1.6937 (12.0); 1.6763 (12.0); 1.5810 (1.1); 1.2719 (7.7); 1.2592 (0.7); 1.2541 (16.0); 1.2363 (7.4); -0.0002 (15.3) |
| I-33: ¹H-NMR(400.0 MHz, CDCl3): |
| δ= 8.2157 (1.3); 8.2139 (1.3); 8.2040 (1.3); 8.2023 (1.3); 8.1334 (1.7); 8.1272 (1.8); 7.8631 (0.8); 7.8569 (0.8); 7.8443 (1.0); 7.8419 (1.0); 7.8381 (1.0); 7.8357 (1.0); 7.8231 (0.9); 7.8168 (0.8); 7.5072 (0.8); 7.5035 (0.8); 7.4865 (1.1); 7.4831 (1.4); 7.4803 (1.0); 7.4633 (1.0); 7.4596 (1.0); 7.3355 (1.0); 7.3265 (1.2); 7.3239 (1.1); 7.3149 (1.8); 7.3058 (0.9); 7.3032 (0.9); 7.2942 (0.8); 7.2615 (20.5); 6.9557 (1.2); 6.9495 (1.2); 6.9344 (1.1); 6.9283 (1.1); 5.2825 (0.6); 5.2651 (2.3); 5.2477 (2.3); 5.2303 (0.6); 4.2531 (1.0); 4.2369 (2.9); 4.2354 (3.0); 4.2190 (3.1); 4.2176 (3.0); 4.2011 (1.1); 2.0453 (0.6); 1.6936 (8.8); 1.6762 (8.7); 1.5569 (16.0); 1.2719 (5.6); 1.2594 (1.2); 1.2541 (11.3); 1.2363 (5.3); 0.8819 (1.1); 0.0079 (0.6); -0.0002 (20.9); -0.0085 (0.6) |
| I-34: ¹H-NMR(400.6 MHz, CDCl3): |
| δ= 8.2179 (0.6); 8.2162 (0.7); 8.2143 (0.7); 8.2126 (0.6); 8.2063 (0.6); 8.2045 (0.7); 8.2027 (0.7); 8.2010 (0.6); 8.1309 (0.9); 8.1246 (0.9); 7.8398 (0.5); 7.8373 (0.6); 7.8335 (0.5); 7.8311 (0.6); 7.4884 (0.6); 7.4850 (0.7); 7.4822 (0.5); 7.4652 (0.6); 7.4615 (0.6); 7.3394 (0.6); 7.3305 (0.6); 7.3279 (0.6); 7.3188 (1.0); 7.2613 (39.3); 6.9573 (0.6); 6.9558 (0.6); 6.9498 (0.6); 6.9483 (0.6); 6.9361 (0.6); 6.9346 (0.6); 6.9286 (0.6); 6.9271 (0.6); 5.3053 (1.2); 5.2879 (1.3); 4.3627 (0.6); 4.3512 (0.8); 4.3492 (0.6); 4.3376 (0.6); 4.3327 (0.6); 4.3215 (0.7); 4.3194 (0.8); 4.3084 (0.6); 3.7115 (0.8); 3.7080 (0.8); 3.7008 (1.0); 3.6974 (1.2); 3.6945 (1.1); 3.6868 (0.8); 3.6833 (0.8); 3.6121 (0.8); 3.6079 (0.8); 3.6015 (1.8); 3.5969 (1.0); 3.5940 (1.3); 3.5881 (1.3); 3.5859 (1.3); 3.5110 (2.1); 3.5055 (0.5); 3.5022 (1.0); 3.4984 (1.8); 3.4883 (1.0); 3.3607 (16.0); 1.7082 (4.7); 1.6908 (4.6); 0.0080 (0.7); -0.0002 (22.8); -0.0027 (1.2); -0.0034 (0.9); -0.0043 (0.6); -0.0084 (0.7) |
| I-34: ¹H-NMR(400.0 MHz, CDC13_5mm): |
| δ= 8.2157 (0.8); 8.2139 (0.8); 8.2058 (0.8); 8.2040 (0.8); 8.2022 (0.8); 8.1301 (1.1); 8.1239 (1.1); 7.8575 (0.5); 7.8512 (0.5); 7.8387 (0.6); 7.8363 (0.7); 7.8325 (0.6); 7.8300 (0.6); 7.8175 (0.6); 7.8112 (0.6); 7.5084 (0.6); 7.5047 (0.6); 7.4877 (0.8); 7.4844 (1.0); 7.4816 (0.7); 7.4645 (0.7); 7.4608 (0.7); 7.3389 (0.7); 7.3299 (0.8); 7.3273 (0.8); 7.3183 (1.2); 7.3092 (0.6); 7.3067 (0.6); 7.2976 (0.6); 7.2606 (37.3); 6.9553 (0.7); 6.9492 (0.8); 6.9341 (0.7); 6.9280 (0.7); 5.3047 (1.4); 5.2873 (1.5); 4.3622 (0.7); 4.3506 (1.0); 4.3370 (0.8); 4.3326 (0.7); 4.3194 (1.0); 4.3084 (0.8); 3.7111 (1.0); 3.7076 (1.0); 3.7004 (1.2); 3.6968 (1.6); 3.6865 (0.9); 3.6829 (0.9); 3.6119 (0.9); 3.6077 (0.9); 3.6013 (2.0); 3.5968 (1.2); 3.5939 (1.4); 3.5878 (1.5); 3.5858 (1.6); 3.5110 (2.4); 3.5055 (0.6); 3.5022 (1.2); 3.4984 (2.1); 3.4948 (0.6); 3.4882 (1.2); 3.3601 (16.0); 1.7080 (5.4); 1.6906 (5.4); 1.6265 (2.2); 0.0079 (1.9); -0.0002 (53.3); -0.0085 (1.8) |
| I-35: ¹H-NMR(400.6 MHz, CDCl3): |
| δ= 8.2149 (0.6); 8.2132 (0.7); 8.2113 (0.7); 8.2096 (0.6); 8.2033 (0.6); 8.2015 (0.7); 8.1997 (0.7); 8.1981 (0.6); 8.1294 (0.9); 8.1232 (1.0); 7.8593 (0.5); 7.8406 (0.6); 7.8381 (0.6); 7.8343 (0.6); 7.8319 (0.6); 7.8194 (0.5); 7.8131 (0.5); 7.5040 (0.5); 7.4870 (0.7); 7.4836 (0.8); 7.4808 (0.6); 7.4638 (0.6); 7.4601 (0.6); 7.3357 (0.6); 7.3268 (0.7); 7.3241 (0.6); 7.3151 (1.1); 7.3035 (0.5); 7.2609 (56.6); 6.9576 (0.6); 6.9561 (0.7); 6.9501 (0.7); 6.9486 (0.7); 6.9364 (0.6); 6.9349 (0.6); 6.9289 (0.6); 6.9274 (0.6); 5.3136 (1.3); 5.2962 (1.4); 4.3344 (0.8); 4.3281 (0.9); 4.3247 (0.9); 4.3214 (1.0); 4.3171 (1.0); 4.3138 (0.9); 4.3100 (1.0); 4.3045 (0.9); 3.6013 (1.0); 3.5974 (1.0); 3.5887 (1.7); 3.5861 (1.8); 3.5773 (0.9); 3.5734 (0.9); 3.3304 (16.0); 1.7121 (5.1); 1.6947 (5.1); 1.5523 (2.4); 0.0080 (1.0); -0.0002 (34.2); -0.0085 (0.9) |
| I-35: ¹H-NMR(400.6 MHz, CDCl3): |
| δ= 8.2149 (0.6); 8.2130 (0.7); 8.2112 (0.7); 8.2095 (0.7); 8.2032 (0.6); 8.2015 (0.7); 8.1996 (0.7); 8.1979 (0.7); 8.1293 (0.9); 8.1231 (1.0); 7.8593 (0.5); 7.8406 (0.6); 7.8381 (0.6); 7.8343 (0.6); 7.8319 (0.6); 7.8194 (0.5); 7.8131 (0.5); 7.5075 (0.5); 7.5038 (0.5); 7.4869 (0.7); 7.4834 (0.9); 7.4806 (0.6); 7.4636 (0.6); 7.4599 (0.6); 7.3355 (0.7); 7.3266 (0.7); 7.3239 (0.7); 7.3149 (1.2); 7.3059 (0.5); 7.3033 (0.5); 7.2607 (20.0); 6.9575 (0.6); 6.9560 (0.7); 6.9500 (0.7); 6.9486 (0.7); 6.9362 (0.6); 6.9347 (0.7); 6.9288 (0.6); 6.9273 (0.6); 5.3137 (1.4); 5.2963 (1.4); 4.3344 (0.8); 4.3281 (0.9); 4.3247 (0.9); 4.3213 (1.1); 4.3171 (1.1); 4.3138 (1.0); 4.3100 (1.0); 4.3045 (0.9); 3.6013 (1.0); 3.5973 (1.0); 3.5886 (1.7); 3.5860 (1.8); 3.5772 (0.9); 3.5733 (1.0); 3.3304 (16.0); 1.7120 (5.3); 1.6946 (5.2); 1.5480 (12.0); 0.0080 (0.8); -0.0002 (30.1); -0.0085 (0.9) |
| I-36: ¹H-NMR(599.6 MHz, CDCl3): |
| δ= 8.6302 (2.0); 8.2879 (2.2); 8.2811 (2.2); 8.1464 (3.1); 7.8591 (0.9); 7.8452 (1.7); 7.8326 (1.0); 7.5382 (1.0); 7.5230 (2.1); 7.5091 (1.2); 7.4030 (0.9); 7.3962 (1.5); 7.3894 (1.6); 7.3829 (1.2); 7.2602 (32.6); 6.9805 (1.7); 6.9665 (1.7); 4.9133 (10.7); 4.1279 (0.6); 4.1159 (0.6); 2.9962 (33.0); 2.0444 (2.6); 1.5479 (41.5); 1.2710 (1.1); 1.2592 (2.1); 1.2474 (0.9); 0.8933 (0.4); 0.8821 (0.9); 0.8702 (0.4); -0.0001 (50.0) |
| I-36: ¹H-NMR(400.6 MHz, CDCl3): |
| δ= 8.1481 (0.5); 8.1418 (0.5); 7.3910 (0.7); 7.2610 (11.3); 4.9143 (4.6); 2.9970 (16.0); 2.9898 (0.6); 2.0455 (1.0); 1.5538 (2.7); 1.2596 (0.7); -0.0002 (17.2) |
| I-37: ¹H-NMR(400.0 MHz, CDCl3_5mm): |
| δ= 8.2588 (0.8); 8.2572 (0.9); 8.2554 (0.9); 8.2472 (0.8); 8.2456 (0.9); 8.2438 (0.9); 8.1350 (1.2); 8.1289 (1.2); 7.8645 (0.6); 7.8582 (0.6); 7.8457 (0.7); 7.8433 (0.7); 7.8395 (0.7); 7.8371 (0.7); 7.8245 (0.6); 7.8183 (0.6); 7.5103 (0.6); 7.5066 (0.6); 7.4895 (0.8); 7.4862 (1.0); 7.4836 (0.7); 7.4665 (0.7); 7.4628 (0.7); 7.3588 (0.7); 7.3498 (0.8); 7.3472 (0.8); 7.3382 (1.2); 7.3291 (0.6); 7.3266 (0.6); 7.2622 (8.2); 6.9646 (0.8); 6.9633 (0.8); 6.9571 (0.8); 6.9433 (0.8); 6.9421 (0.8); 6.9359 (0.8); 4.9801 (7.5); 4.3790 (1.8); 4.3701 (1.3); 4.3670 (2.0); 4.3634 (1.2); 4.3548 (1.8); 3.7374 (2.1); 3.7289 (1.3); 3.7252 (2.1); 3.7220 (1.3); 3.7132 (1.8); 3.6404 (1.5); 3.6321 (1.6); 3.6295 (2.0); 3.6243 (1.6); 3.6174 (2.5); 3.5371 (2.6); 3.5302 (1.6); 3.5250 (1.9); 3.5224 (1.5); 3.5142 (1.4); 3.3724 (16.0); 1.5661 (7.2); 0.0079 (0.6); -0.0002 (12.7) |
| I-37: ¹H-NMR(400.0 MHz, CDCl3): |
| δ= 8.2569 (0.9); 8.2435 (1.0); 8.1350 (1.2); 8.1285 (1.2); 7.8644 (0.5); 7.8580 (0.5); 7.8432 (0.7); 7.8395 (0.7); 7.8244 (0.6); 7.8184 (0.6); 7.5196 (0.6); 7.5095 (0.5); 7.5059 (0.5); 7.4862 (1.0); 7.4657 (0.7); 7.4618 (0.7); 7.3581 (0.8); 7.3487 (0.9); 7.3373 (1.1); 7.3282 (0.6); 7.2606 (108.8); 6.9966 (0.6); 6.9641 (0.8); 6.9565 (0.9); 6.9427 (0.8); 6.9351 (0.8); 4.9798 (7.6); 4.3788 (1.7); 4.3668 (1.9); 4.3546 (1.9); 3.7371 (2.0); 3.7287 (1.2); 3.7250 (2.0); 3.7130 (1.8); 3.6403 (1.4); 3.6293 (1.9); 3.6241 (1.5); 3.6172 (2.6); 3.5369 (2.5); 3.5300 (1.4); 3.5249 (1.9); 3.5137 (1.4); 3.3724 (16.0); 1.5424 (12.2); 0.0080 (1.6); -0.0002 (66.1); - 0.0085 (2.1) |
| I-38: ¹H-NMR(400.0 MHz, CDCl3): |
| δ= 8.2633 (1.7); 8.2517 (1.7); 8.1287 (2.3); 8.1225 (2.3); 7.8610 (1.1); 7.8546 (1.1); 7.8422 (1.3); 7.8397 (1.3); 7.8359 (1.3); 7.8335 (1.2); 7.8210 (1.2); 7.8147 (1.1); 7.5198 (0.5); 7.5167 (1.1); 7.5130 (1.1); 7.4959 (1.5); 7.4925 (1.9); 7.4900 (1.2); 7.4728 (1.4); 7.4691 (1.3); 7.3620 (1.3); 7.3529 (1.6); 7.3504 (1.4); 7.3413 (2.3); 7.3322 (1.1); 7.3297 (1.1); 7.3206 (1.0); 7.2610 (70.8); 6.9676 (1.5); 6.9614 (1.5); 6.9476 (1.5); 6.9401 (1.4); 4.9380 (16.0); 4.2936 (1.9); 4.2757 (6.1); 4.2578 (6.2); 4.2400 (2.0); 1.5488 (5.2); 1.3054 (7.3); 1.2875 (15.0); 1.2697 (7.2); 0.0079 (1.2); -0.0002 (42.2); -0.0085 (1.2) |
| I-38: ¹H-NMR(400.0 MHz, CDCl3): |
| δ= 8.2620 (2.5); 8.2517 (2.5); 8.1287 (3.4); 7.8610 (1.2); 7.8550 (1.2); 7.8418 (1.9); 7.8358 (1.8); 7.8211 (1.2); 7.8150 (1.1); 7.5167 (1.3); 7.4933 (2.5); 7.4731 (1.6); 7.3624 (1.3); 7.3526 (1.9); 7.3417 (2.1); 7.3323 (1.5); 7.3212 (0.9); 7.2613 (42.9); 6.9688 (2.1); 6.9615 (2.0); 6.9477 (2.0); 6.9404 (1.8); 4.9383 (16.0); 4.2938 (2.1); 4.2758 (6.2); 4.2580 (6.2); 4.2401 (2.1); 1.5586 (2.2); 1.3054 (6.9); 1.2876 (13.5); 1.2697 (6.6); -0.0002 (26.0) |
| I-39: ¹H-NMR(400.0 MHz, CDCl3_5mm): |
| δ= 8.2605 (1.5); 8.2588 (1.8); 8.2570 (1.8); 8.2553 (1.6); 8.2489 (1.6); 8.2471 (1.8); 8.2453 (1.8); 8.2437 (1.5); 8.1378 (2.3); 8.1359 (1.9); 8.1336 (1.9); 8.1316 (2.4); 7.8678 (1.2); 7.8616 (1.1); 7.8490 (1.4); 7.8466 (1.5); 7.8428 (1.4); 7.8404 (1.4); 7.8278 (1.2); 7.8216 (1.1); 7.5103 (1.2); 7.5066 (1.2); 7.4896 (1.7); 7.4861 (2.0); 7.4836 (1.4); 7.4665 (1.5); 7.4628 (1.4); 7.3583 (1.5); 7.3492 (1.7); 7.3466 (1.6); 7.3376 (2.5); 7.3285 (1.2); 7.3260 (1.2); 7.3169 (1.0); 7.2641 (6.7); 6.9654 (1.6); 6.9640 (1.6); 6.9581 (1.6); 6.9566 (1.6); 6.9442 (1.5); 6.9428 (1.6); 6.9368 (1.5); 6.9353 (1.5); 5.3007 (3.1); 4.9373 (16.0); 4.2908 (2.0); 4.2729 (6.2); 4.2551 (6.3); 4.2372 (2.0); 1.5858 (5.0); 1.3037 (7.7); 1.2974 (0.6); 1.2858 (15.7); 1.2796 (0.7); 1.2679 (7.5); -0.0002 (10.6) |
| I-39: ¹H-NMR(400.0 MHz, CDCl3): |
| δ= 8.2585 (1.8); 8.2567 (1.7); 8.2468 (1.8); 8.2450 (1.7); 8.1373 (2.3); 8.1311 (2.3); 7.8676 (1.1); 7.8613 (1.1); 7.8488 (1.3); 7.8463 (1.4); 7.8426 (1.3); 7.8401 (1.3); 7.8276 (1.2); 7.8214 (1.1); 7.5198 (0.5); 7.5087 (1.1); 7.5049 (1.1); 7.4879 (1.6); 7.4845 (1.8); 7.4649 (1.4); 7.4611 (1.4); 7.3565 (1.4); 7.3474 (1.7); 7.3449 (1.4); 7.3358 (2.4); 7.3268 (1.2); 7.3242 (1.2); 7.3151 (1.0); 7.2609 (90.8); 6.9629 (1.6); 6.9567 (1.6); 6.9430 (1.5); 6.9356 (1.6); 4.9367 (16.0); 4.2909 (2.0); 4.2730 (6.1); 4.2552 (6.2); 4.2373 (2.0); 1.5461 (10.5); 1.3038 (7.5); 1.2859 (15.3); 1.2680 (7.3); 0.0079 (1.6); -0.0002 (53.6); -0.0085 (1.6) |
| I-40: ¹H-NMR(400.6 MHz, CDCl3): |
| δ= 8.2293 (1.2); 8.2274 (1.4); 8.2256 (1.4); 8.2239 (1.2); 8.2176 (1.2); 8.2157 (1.4); 8.2140 (1.4); 8.2122 (1.2); 8.1363 (1.9); 8.1342 (1.5); 8.1321 (1.5); 8.1301 (2.0); 7.7748 (1.0); 7.7686 (1.0); 7.7558 (1.2); 7.7537 (1.3); 7.7496 (1.2); 7.7475 (1.2); 7.7347 (1.1); 7.7285 (1.0); 7.4624 (1.0); 7.4586 (1.1); 7.4418 (1.4); 7.4385 (1.8); 7.4352 (1.2); 7.4184 (1.3); 7.4146 (1.3); 7.2867 (1.2); 7.2777 (1.4); 7.2751 (1.3); 7.2660 (2.7); 7.2614 (66.0); 7.2546 (1.2); 7.2455 (0.9); 6.9194 (1.3); 6.9179 (1.4); 6.9120 (1.4); 6.9105 (1.3); 6.8983 (1.3); 6.8968 (1.3); 6.8909 (1.3); 6.8894 (1.3); 4.8877 (14.6); 4.2747 (1.8); 4.2568 (5.8); 4.2390 (5.8); 4.2212 (1.9); 1.6015 (0.5); 1.5999 (0.6); 1.5951 (0.8); 1.5877 (0.6); 1.5808 (1.9); 1.5730 (0.6); 1.5675 (0.6); 1.5655 (0.7); 1.5611 (0.9); 1.5540 (7.6); 1.5465 (0.6); 1.3011 (7.6); 1.2833 (16.0); 1.2655 (7.6); 0.7979 (1.2); 0.7932 (3.7); 0.7902 (3.0); 0.7871 (3.2); 0.7843 (4.7); 0.7823 (3.2); 0.7794 (4.4); 0.7753 (2.3); 0.7679 (2.4); 0.7639 (3.4); 0.7609 (1.6); 0.7583 (1.5); 0.0080 (1.2); -0.0002 (41.0); -0.0085 (1.0) |
| I-41: ¹H-NMR(400.6 MHz, CDCl3): |
| δ= 8.2220 (0.7); 8.2202 (0.8); 8.2183 (0.8); 8.2166 (0.7); 8.2103 (0.8); 8.2085 (0.8); 8.2067 (0.8); 8.2051 (0.7); 8.1279 (1.1); 8.1258 (0.8); 8.1236 (0.9); 8.1218 (1.1); 7.8570 (0.6); 7.8507 (0.6); 7.8382 (0.7); 7.8357 (0.7); 7.8320 (0.7); 7.8295 (0.7); 7.8170 (0.6); 7.8108 (0.6); 7.5206 (0.7); 7.5169 (0.6); 7.5000 (0.8); 7.4970 (1.0); 7.4936 (0.7); 7.4767 (0.8); 7.4730 (0.7); 7.3441 (0.7); 7.3351 (0.8); 7.3325 (0.8); 7.3234 (1.3); 7.3145 (0.6); 7.3118 (0.6); 7.3028 (0.5); 7.2611 (44.6); 6.9634 (0.8); 6.9619 (0.8); 6.9559 (0.8); 6.9544 (0.8); 6.9421 (0.8); 6.9406 (0.8); 6.9347 (0.8); 6.9331 (0.7); 5.2926 (1.5); 5.2752 (1.5); 3.7708 (16.0); 1.7001 (6.4); 1.6828 (6.4); 1.5547 (1.0); 0.0080 (0.8); -0.0002 (27.8); -0.0085 (0.8) |
| I-42: ¹H-NMR(400.6 MHz, CDCl3): |
| δ= 8.2315 (0.7); 8.2296 (0.9); 8.2279 (0.9); 8.2261 (0.8); 8.2198 (0.8); 8.2179 (0.9); 8.2162 (0.9); 8.2144 (0.8); 8.1366 (1.2); 8.1303 (1.2); 7.7747 (0.6); 7.7685 (0.6); 7.7557 (0.7); 7.7536 (0.8); 7.7495 (0.7); 7.7474 (0.8); 7.7346 (0.7); 7.7284 (0.7); 7.5191 (0.6); 7.4641 (0.6); 7.4604 (0.7); 7.4436 (0.8); 7.4401 (1.2); 7.4370 (0.7); 7.4201 (0.8); 7.4164 (0.8); 7.2895 (0.7); 7.2805 (0.9); 7.2778 (0.9); 7.2689 (1.8); 7.2607 (110.2); 7.2509 (0.7); 7.2484 (1.0); 6.9970 (0.6); 6.9201 (0.8); 6.9186 (0.9); 6.9126 (0.9); 6.9111 (0.9); 6.8990 (0.8); 6.8975 (0.8); 6.8915 (0.8); 6.8900 (0.8); 4.9056 (8.5); 3.7858 (16.0); 1.5980 (0.8); 1.5811 (1.2); 1.5639 (1.2); 1.5473 (3.0); 0.7823 (10.1); 0.7652 (6.5); 0.0079 (1.9); -0.0002 (66.1); -0.0085 (2.0) |
| I-43: ¹H-NMR(400.6 MHz, CDCl3): |
| δ= 8.2723 (1.8); 8.2623 (1.8); 8.2607 (1.8); 8.1385 (2.7); 8.1324 (2.8); 7.7709 (1.1); 7.7647 (1.1); 7.7519 (1.4); 7.7499 (1.5); 7.7457 (1.4); 7.7437 (1.5); 7.7309 (1.2); 7.7247 (1.2); 7.4642 (1.1); 7.4607 (1.1); 7.4435 (1.6); 7.4404 (2.2); 7.4375 (1.4); 7.4203 (1.4); 7.4168 (1.4); 7.3098 (1.2); 7.3007 (1.5); 7.2984 (1.4); 7.2892 (2.1); 7.2801 (1.1); 7.2778 (1.2); 7.2686 (1.0); 7.2626 (34.1); 6.9256 (1.7); 6.9193 (1.7); 6.9044 (1.7); 6.8982 (1.6); 5.3001 (1.1); 4.9419 (16.0); 1.6072 (0.5); 1.5933 (1.0); 1.5867 (1.1); 1.5809 (0.7); 1.5731 (2.0); 1.5669 (0.8); 1.5590 (1.0); 1.5526 (1.1); 1.5389 (0.6); 1.4320 (1.2); 1.2644 (1.6); 0.8986 (0.9); 0.8817 (3.3); 0.8640 (1.2); 0.7961 (0.6); 0.7808 (1.8); 0.7762 (3.1); 0.7734 (2.1); 0.7697 (2.5); 0.7601 (2.0); 0.7574 (2.2); 0.7532 (4.2); 0.7495 (4.2); 0.7462 (3.1); 0.7386 (3.0); 0.7341 (3.6); 0.7312 (2.2); 0.7282 (2.1); 0.0079 (0.5); -0.0002 (20.9); -0.0085 (0.7) |
| I-07: ¹H-NMR(400.6 MHz, CDCl3): |
| δ= 8.2632 (0.8); 8.2614 (0.9); 8.2596 (0.9); 8.2578 (0.8); 8.2516 (0.9); 8.2498 (1.0); 8.2479 (0.9); 8.2463 (0.8); 8.1390 (1.2); 8.1369 (0.9); 8.1347 (1.0); 8.1328 (1.2); 8.1309 (0.8); 7.8678 (0.7); 7.8615 (0.6); 7.8490 (0.8); 7.8466 (0.8); 7.8428 (0.8); 7.8403 (0.8); 7.8279 (0.7); 7.8216 (0.7); 7.5122 (0.6); 7.5085 (0.7); 7.4915 (0.9); 7.4890 (1.0); 7.4880 (1.0); 7.4855 (0.7); 7.4685 (0.9); 7.4648 (0.8); 7.3610 (0.8); 7.3519 (1.0); 7.3494 (0.8); 7.3403 (1.4); 7.3312 (0.7); 7.3287 (0.7); 7.3197 (0.6); 7.2628 (6.7); 6.9662 (0.9); 6.9647 (0.9); 6.9588 (0.9); 6.9572 (0.8); 6.9451 (0.9); 6.9435 (0.8); 6.9376 (0.9); 6.9361 (0.8); 4.9572 (8.6); 3.8007 (16.0); 2.0453 (1.1); 1.5693 (1.3); 1.2595 (0.8); 0.8818 (0.5); -0.0002 (9.2) |
| I-07: ¹H-NMR(400.6 MHz, CDCl3): |
| δ= 8.2637 (0.8); 8.2618 (0.9); 8.2600 (0.9); 8.2583 (0.9); 8.2520 (0.9); 8.2502 (1.0); 8.2484 (1.0); 8.2467 (0.9); 8.1384 (1.2); 8.1364 (1.0); 8.1322 (1.3); 7.8677 (0.7); 7.8615 (0.6); 7.8490 (0.7); 7.8465 (0.8); 7.8428 (0.8); 7.8403 (0.8); 7.8278 (0.7); 7.8216 (0.7); 7.5116 (0.7); 7.5079 (0.7); 7.4909 (0.9); 7.4873 (1.1); 7.4848 (0.8); 7.4679 (0.9); 7.4641 (0.9); 7.3606 (0.8); 7.3516 (1.0); 7.3490 (0.9); 7.3399 (1.5); 7.3309 (0.7); 7.3283 (0.7); 7.3193 (0.6); 7.2612 (61.9); 7.2530 (0.7); 7.2522 (0.6); 6.9660 (0.9); 6.9645 (0.9); 6.9585 (0.9); 6.9570 (0.9); 6.9448 (0.8); 6.9433 (0.9); 6.9373 (0.9); 6.9358 (0.8); 4.9570 (8.7); 3.8011 (16.0); 1.5493 (7.5); 0.0079 (1.0); -0.0002 (37.0); -0.0051 (0.9); -0.0059 (0.7); -0.0068 (0.7); -0.0085 (1.3) |
| I-10: ¹H-NMR(400.0 MHz, CDCl3): |
| δ= 8.2644 (2.7); 8.2549 (2.8); 8.1326 (4.0); 8.1264 (4.0); 7.8656 (1.8); 7.8594 (1.7); 7.8468 (2.1); 7.8444 (2.2); 7.8406 (2.0); 7.8382 (1.9); 7.8257 (1.8); 7.8194 (1.7); 7.5058 (1.6); 7.5022 (1.7); 7.4851 (2.4); 7.4818 (3.0); 7.4620 (2.2); 7.4583 (2.1); 7.3557 (2.1); 7.3465 (2.4); 7.3441 (2.2); 7.3350 (3.6); 7.3259 (1.7); 7.3234 (1.6); 7.3142 (1.4); 7.2608 (37.3); 6.9669 (2.6); 6.9604 (2.5); 6.9454 (2.5); 6.9391 (2.4); 5.3739 (1.2); 5.3564 (4.4); 5.3389 (4.5); 5.3214 (1.2); 5.3000 (1.9); 1.7448 (16.0); 1.7273 (15.8); 1.7156 (0.9); 0.0079 (1.4); -0.0002 (48.8); -0.0085 (1.4) |
| 1-10: ¹H-NMR(400.0 MHz, CDCl3_5mm): |
| δ= 8.2667 (4.7); 8.2558 (3.8); 8.1301 (5.8); 7.8630 (2.7); 7.8568 (2.7); 7.8420 (3.4); 7.8231 (1.8); 7.8170 (1.5); 7.5001 (2.6); 7.4800 (4.0); 7.4596 (2.2); 7.3555 (3.1); 7.3446 (3.6); 7.3349 (3.6); 7.3257 (2.4); 7.3143 (1.6); 7.2620 (14.9); 6.9659 (4.0); 6.9591 (3.6); 6.9448 (3.2); 6.9382 (2.6); 5.3607 (2.1); 5.3438 (4.2); 5.3264 (3.8); 5.3088 (1.4); 5.3005 (1.4); 3.6410 (4.4); 1.7398 (16.0); 1.7224 (13.3); 1.2541 (2.3); -0.0002 (22.2) |
| 1-10: ¹H-NMR(400.6 MHz, CDCl3): |
| δ= 8.2713 (2.2); 8.2698 (2.6); 8.2677 (2.6); 8.2663 (2.5); 8.2597 (2.4); 8.2581 (2.7); 8.2561 (2.7); 8.2547 (2.5); 8.1313 (3.6); 8.1251 (3.9); 7.8663 (1.8); 7.8601 (1.7); 7.8476 (2.0); 7.8451 (2.2); 7.8414 (2.0); 7.8389 (2.1); 7.8264 (1.9); 7.8202 (1.8); 7.5034 (1.7); 7.4996 (1.8); 7.4826 (2.4); 7.4791 (2.9); 7.4766 (2.2); 7.4596 (2.3); 7.4559 (2.3); 7.3542 (2.3); 7.3452 (2.5); 7.3426 (2.4); 7.3335 (3.9); 7.3245 (1.8); 7.3219 (1.9); 7.3128 (1.6); 7.2615 (24.5); 6.9682 (2.3); 6.9669 (2.5); 6.9610 (2.4); 6.9596 (2.4); 6.9471 (2.2); 6.9457 (2.4); 6.9397 (2.3); 6.9383 (2.3); 5.3723 (1.2); 5.3549 (4.8); 5.3374 (4.9); 5.3199 (1.2); 2.1740 (1.2); 1.7423 (16.0); 1.7248 (15.9); 1.7111 (0.7); 0.8818 (0.6); 0.0080 (0.8); -0.0002 (31.7); -0.0085 (1.0) |
| 1-10: ¹H-NMR(400.6 MHz, CDCl3): |
| δ= 8.2697 (2.5); 8.2677 (2.6); 8.2595 (2.4); 8.2580 (2.7); 8.2560 (2.7); 8.1330 (3.5); 8.1268 (3.9); 7.8671 (1.6); 7.8608 (1.6); 7.8484 (1.9); 7.8458 (2.1); 7.8421 (2.0); 7.8397 (2.1); 7.8272 (1.8); 7.8210 (1.8); 7.5068 (1.6); 7.5031 (1.7); 7.4860 (2.3); 7.4827 (2.9); 7.4801 (2.1); 7.4630 (2.2); 7.4593 (2.2); 7.3572 (2.1); 7.3481 (2.5); 7.3455 (2.3); 7.3364 (3.7); 7.3274 (1.8); 7.3248 (1.8); 7.3158 (1.6); 7.2610 (26.2); 6.9684 (2.4); 6.9623 (2.4); 6.9611 (2.4); 6.9471 (2.4); 6.9410 (2.4); 6.9399 (2.3); 5.3734 (1.2); 5.3560 (4.6); 5.3385 (4.7); 5.3210 (1.3); 1.7447 (15.9); 1.7272 (16.0); 1.7147 (0.8); 0.8819 (0.7); 0.0079 (1.2); -0.0002 (40.7); - 0.0085 (1.6) |
| I-05: ¹H-NMR(400.6 MHz, CDCl₃): |
| δ= 8.2168 (0.7); 8.2151 (0.8); 8.2132 (0.8); 8.2115 (0.8); 8.2052 (0.7); 8.2034 (0.8); 8.2016 (0.8); 8.1999 (0.8); 8.1359 (1.1); 8.1340 (0.8); 8.1318 (0.8); 8.1297 (1.1); 7.8634 (0.6); 7.8572 (0.6); 7.8447 (0.7); 7.8423 (0.7); 7.8384 (0.7); 7.8360 (0.7); 7.8235 (0.6); 7.8172 (0.6); 7.5130 (0.6); 7.5093 (0.6); 7.4923 (0.8); 7.4888 (1.0); 7.4860 (0.7); 7.4690 (0.8); 7.4653 (0.8); 7.3390 (0.8); 7.3300 (0.8); 7.3274 (0.8); 7.3183 (1.3); 7.3094 (0.6); 7.3067 (0.6); 7.2977 (0.6); 7.2621 (21.6); 6.9586 (0.7); 6.9572 (0.8); 6.9512 (0.8); 6.9497 (0.8); 6.9374 (0.7); 6.9359 (0.8); 6.9300 (0.8); 6.9285 (0.8); 5.2887 (1.6); 5.2713 (1.6); 3.7688 (16.0); 2.0454 (0.5); 1.6986 (6.5); 1.6812 (6.5); 1.5647 (2.2); 1.2596 (0.5); 0.8818 (0.7); -0.0002 (13.4) |
| I-05: ¹H-NMR(400.6 MHz, CDCl3): |
| δ= 8.2164 (0.8); 8.2146 (0.9); 8.2128 (0.9); 8.2111 (0.7); 8.2048 (0.8); 8.2030 (0.9); 8.2011 (0.9); 8.1995 (0.7); 8.1362 (1.1); 8.1341 (0.9); 8.1319 (0.9); 8.1300 (1.2); 7.8635 (0.6); 7.8572 (0.6); 7.8447 (0.7); 7.8423 (0.7); 7.8384 (0.7); 7.8360 (0.7); 7.8236 (0.6); 7.8173 (0.6); 7.5130 (0.6); 7.5093 (0.6); 7.4923 (0.8); 7.4889 (1.0); 7.4861 (0.7); 7.4691 (0.8); 7.4654 (0.7); 7.3388 (0.8); 7.3298 (0.8); 7.3272 (0.8); 7.3182 (1.3); 7.3091 (0.6); 7.3066 (0.6); 7.2976 (0.6); 7.2625 (5.9); 6.9586 (0.8); 6.9571 (0.8); 6.9511 (0.9); 6.9497 (0.8); 6.9374 (0.8); 6.9359 (0.8); 6.9299 (0.8); 6.9285 (0.7); 5.2890 (1.6); 5.2716 (1.6); 3.7686 (16.0); 1.6986 (6.5); 1.6812 (6.4); 1.5702 (0.8); 0.8818 (0.6); -0.0002 (7.9) |
| I-03: ¹H-NMR(400.0 MHz, CDCl3): |
| δ= 8.5762 (1.6); 8.5671 (1.5); 8.5643 (1.6); 8.4971 (2.3); 8.4934 (2.3); 8.2124 (1.7); 8.2010 (1.7); 7.7525 (1.3); 7.7325 (1.4); 7.4880 (0.8); 7.4674 (1.6); 7.4444 (1.0); 7.3219 (1.8); 7.3110 (1.8); 7.3023 (2.4); 7.2903 (1.7); 7.2819 (0.7); 7.2620 (9.2); 5.3215 (0.6); 5.3040 (1.8); 5.2866 (1.8); 5.2691 (0.6); 3.7703 (16.0); 2.7737 (1.9); 1.7011 (7.5); 1.6837 (7.4); -0.0002 (10.4) |
| I-06: ¹H-NMR(400.6 MHz, CDCl3): |
| δ= 8.2844 (0.8); 8.2828 (0.8); 8.2808 (0.9); 8.2792 (0.8); 8.2729 (0.8); 8.2712 (0.9); 8.2692 (0.9); 8.2677 (0.8); 8.1353 (1.1); 8.1334 (0.8); 8.1309 (0.9); 8.1290 (1.2); 7.9558 (0.6); 7.9494 (0.6); 7.9376 (0.7); 7.9345 (0.8); 7.9312 (0.7); 7.9281 (0.7); 7.9163 (0.7); 7.9099 (0.6); 7.5892 (0.6); 7.5855 (0.6); 7.5684 (0.9); 7.5665 (0.8); 7.5648 (0.9); 7.5629 (0.7); 7.5457 (0.8); 7.5421 (0.8); 7.4541 (0.8); 7.4449 (0.9); 7.4425 (0.8); 7.4333 (1.3); 7.4241 (0.6); 7.4217 (0.6); 7.4125 (0.5); 7.2632 (5.3); 7.0251 (0.8); 7.0236 (0.8); 7.0176 (0.8); 7.0161 (0.8); 7.0038 (0.8); 7.0023 (0.8); 6.9962 (0.8); 6.9947 (0.8); 5.2648 (1.5); 5.2474 (1.6); 3.7689 (16.0); 2.0453 (0.6); 1.7006 (6.3); 1.6832 (6.2); 1.5683 (0.7); -0.0002 (7.0) |
| I-09: ¹H-NMR(400.6 MHz, CDCl3): |
| δ= 8.3199 (0.8); 8.3184 (0.9); 8.3164 (0.9); 8.3150 (0.9); 8.3083 (0.8); 8.3069 (1.0); 8.3048 (0.9); 8.1365 (1.2); 8.1302 (1.3); 7.9623 (0.7); 7.9559 (0.6); 7.9441 (0.7); 7.9410 (0.8); 7.9377 (0.7); 7.9346 (0.7); 7.9228 (0.7); 7.9164 (0.7); 7.5890 (0.6); 7.5854 (0.6); 7.5681 (0.9); 7.5667 (0.8); 7.5645 (1.0); 7.5457 (0.8); 7.5420 (0.8); 7.4696 (0.8); 7.4604 (1.0); 7.4581 (0.9); 7.4488 (1.4); 7.4395 (0.6); 7.4373 (0.6); 7.4280 (0.6); 7.2616 (11.6); 7.0326 (0.8); 7.0313 (0.9); 7.0251 (0.9); 7.0238 (0.9); 7.0113 (0.8); 7.0100 (0.9); 7.0038 (0.8); 7.0025 (0.8); 4.9528 (8.7); 4.1309 (0.5); 4.1131 (0.5); 3.8038 (16.0); 2.0454 (2.4); 1.5509 (1.2); 1.2775 (0.7); 1.2596 (1.4); 1.2418 (0.6); -0.0002 (15.2) |
| I-04: ¹H-NMR(400.0 MHz, CDCl3): |
| δ= 8.6474 (1.0); 8.6434 (1.1); 8.6352 (1.1); 8.6312 (1.1); 8.4585 (1.4); 8.4539 (1.4); 8.2784 (1.0); 8.2689 (1.0); 7.8547 (0.7); 7.8503 (0.9); 7.8448 (0.7); 7.8347 (0.8); 7.8301 (1.0); 7.8248 (0.7); 7.5667 (0.6); 7.5630 (0.6); 7.5456 (1.0); 7.5422 (1.0); 7.5232 (0.8); 7.5196 (0.8); 7.4361 (0.8); 7.4268 (0.9); 7.4246 (0.8); 7.4153 (1.3); 7.4060 (0.6); 7.4038 (0.6); 7.3945 (0.6); 7.3885 (0.8); 7.3761 (0.8); 7.3685 (0.8); 7.3564 (0.8); 7.2617 (9.5); 5.2775 (1.7); 5.2600 (1.7); 3.7702 (16.0); 2.1270 (0.5); 2.0913 (0.9); 1.7023 (6.6); 1.6848 (6.5); -0.0002 (14.9) |
| I-44: ¹H-NMR(400.6 MHz, CDCl₃): |
| δ= 8.2886 (0.9); 8.2869 (1.0); 8.2850 (1.1); 8.2834 (1.0); 8.2770 (1.0); 8.2753 (1.1); 8.2734 (1.1); 8.2718 (1.0); 8.1543 (1.4); 8.1480 (1.5); 7.8684 (0.7); 7.8622 (0.7); 7.8499 (0.8); 7.8472 (0.9); 7.8437 (0.8); 7.8410 (0.9); 7.8287 (0.8); 7.8224 (0.8); 7.5556 (0.7); 7.5519 (0.8); 7.5348 (1.0); 7.5312 (1.2); 7.5290 (0.9); 7.5119 (1.0); 7.5082 (1.0); 7.4147 (1.0); 7.4055 (1.1); 7.4031 (1.0); 7.3940 (1.7); 7.3848 (0.8); 7.3824 (0.8); 7.3732 (0.7); 7.2618 (17.1); 6.9914 (0.9); 6.9900 (1.0); 6.9840 (1.0); 6.9825 (1.0); 6.9702 (0.9); 6.9687 (1.0); 6.9627 (1.0); 6.9613 (1.0); 4.9522 (11.5); 3.3771 (16.0); 3.3705 (0.8); 2.1725 (3.9); 0.8819 (0.7); 0.0080 (0.6); -0.0002 (21.4); -0.0085 (0.7) |
| I-45: ¹H-NMR(400.6 MHz, CDCl3): |
| δ= 8.2753 (0.8); 8.2734 (0.8); 8.2637 (0.8); 8.2618 (0.8); 8.1311 (1.1); 8.1249 (1.2); 7.8640 (0.5); 7.8578 (0.5); 7.8452 (0.6); 7.8428 (0.7); 7.8390 (0.6); 7.8366 (0.6); 7.8241 (0.6); 7.8179 (0.6); 7.5011 (0.5); 7.4975 (0.5); 7.4804 (0.8); 7.4773 (0.9); 7.4747 (0.6); 7.4575 (0.7); 7.4538 (0.7); 7.3653 (0.7); 7.3562 (0.8); 7.3538 (0.7); 7.3446 (1.2); 7.3355 (0.6); 7.3331 (0.6); 7.2609 (29.4); 6.9670 (0.8); 6.9604 (0.8); 6.9466 (0.7); 6.9392 (0.7); 5.1198 (8.6); 2.0433 (14.2); 1.9725 (15.2); 1.5477 (16.0); 1.2599 (0.7); 0.0081 (1.0); -0.0002 (34.2); -0.0085 (1.2) |
| I-46: ¹H-NMR(400.6 MHz, CDCl3): |
| δ= 8.5752 (1.4); 8.5650 (1.4); 8.5631 (1.4); 8.2657 (1.6); 8.2639 (1.7); 8.2542 (1.7); 8.2523 (1.7); 8.1326 (2.2); 8.1263 (2.4); 7.8629 (1.1); 7.8567 (1.1); 7.8441 (1.3); 7.8418 (1.4); 7.8379 (1.3); 7.8355 (1.3); 7.8230 (1.2); 7.8168 (1.2); 7.6351 (1.0); 7.6306 (1.0); 7.6158 (2.0); 7.6114 (2.0); 7.5966 (1.2); 7.5921 (1.2); 7.5121 (1.0); 7.5084 (1.1); 7.4913 (1.6); 7.4882 (1.8); 7.4854 (1.2); 7.4684 (1.4); 7.4647 (1.4); 7.3682 (1.5); 7.3592 (3.6); 7.3570 (2.7); 7.3475 (2.6); 7.3387 (2.6); 7.3269 (1.1); 7.2609 (39.1); 7.2238 (1.1); 7.2115 (1.1); 7.2049 (1.1); 7.1927 (1.0); 6.9673 (1.6); 6.9599 (1.6); 6.9461 (1.5); 6.9387 (1.6); 5.3574 (11.4); 5.0698 (16.0); 2.1719 (0.9); 1.5601 (2.8); 1.2597 (0.6); 0.8819 (0.9); 0.0078 (1.5); 0.0053 (0.6); -0.0002 (53.1); -0.0085 (1.9) |
| I-47: ¹H-NMR(400.6 MHz, CDCl3): |
| δ= 8.2893 (1.8); 8.2875 (1.8); 8.2778 (1.8); 8.2759 (1.8); 8.1551 (2.4); 8.1488 (2.6); 7.8508 (1.1); 7.8446 (1.1); 7.8321 (1.3); 7.8297 (1.4); 7.8259 (1.4); 7.8235 (1.4); 7.8110 (1.2); 7.8048 (1.2); 7.5321 (1.1); 7.5285 (1.1); 7.5113 (1.6); 7.5085 (1.9); 7.4886 (1.5); 7.4849 (1.5); 7.3914 (1.4); 7.3822 (1.6); 7.3798 (1.5); 7.3707 (2.5); 7.3615 (1.2); 7.3591 (1.2); 7.3500 (1.0); 7.2631 (14.3); 7.1166 (0.8); 6.9713 (1.7); 6.9640 (1.8); 6.9502 (1.7); 6.9428 (1.7); 4.8520 (15.0); 4.2002 (2.2); 4.1824 (6.8); 4.1646 (6.9); 4.1468 (2.3); 3.6798 (1.4); 3.6644 (3.8); 3.6492 (4.0); 3.6337 (1.6); 2.6179 (3.4); 2.6025 (5.4); 2.5874 (3.3); 1.5857 (5.6); 1.2918 (7.8); 1.2740 (16.0); 1.2562 (8.0); 0.0080 (0.6); -0.0002 (20.9); -0.0085 (0.8) |
| I-48: ¹H-NMR(400.6 MHz, CDCl3): |
| δ= 8.2969 (1.4); 8.2953 (1.6); 8.2934 (1.7); 8.2853 (1.6); 8.2837 (1.8); 8.2818 (1.7); 8.1577 (2.2); 8.1514 (2.4); 7.8583 (1.1); 7.8520 (1.1); 7.8396 (1.3); 7.8371 (1.4); 7.8334 (1.3); 7.8308 (1.3); 7.8184 (1.2); 7.8122 (1.2); 7.5328 (1.0); 7.5291 (1.1); 7.5120 (1.6); 7.5086 (1.8); 7.5064 (1.3); 7.4892 (1.5); 7.4855 (1.4); 7.3936 (1.4); 7.3844 (1.6); 7.3820 (1.5); 7.3729 (2.4); 7.3637 (1.1); 7.3613 (1.2); 7.3522 (1.0); 7.2631 (13.4); 7.1160 (0.8); 6.9741 (1.6); 6.9730 (1.6); 6.9667 (1.6); 6.9530 (1.6); 6.9518 (1.5); 6.9455 (1.6); 4.9173 (13.2); 4.2839 (2.0); 4.2661 (6.3); 4.2482 (6.4); 4.2304 (2.1); 4.1605 (6.3); 4.1474 (6.4); 4.1309 (0.9); 4.1131 (0.8); 2.0454 (3.7); 1.5810 (4.7); 1.3269 (7.8); 1.3091 (16.0); 1.2913 (7.9); 1.2774 (1.3); 1.2596 (2.6); 1.2417 (1.1); 0.8819 (1.2); 0.8642 (0.5); 0.0080 (0.5); -0.0002 (19.8); -0.0085 (0.7) |
| I-49: ¹H-NMR(400.6 MHz, CDCl3): |
| δ= 8.3109 (0.7); 8.3091 (0.8); 8.3072 (0.8); 8.3056 (0.7); 8.2992 (0.7); 8.2976 (0.8); 8.2956 (0.8); 8.2941 (0.7); 8.1055 (1.3); 8.1038 (1.3); 8.0994 (1.4); 8.0977 (1.3); 7.5663 (1.3); 7.5602 (1.2); 7.5448 (1.3); 7.5386 (1.3); 7.4810 (0.5); 7.4772 (0.5); 7.4603 (0.8); 7.4581 (0.7); 7.4567 (0.8); 7.4546 (0.6); 7.4376 (0.7); 7.4338 (0.7); 7.3518 (0.7); 7.3427 (0.8); 7.3402 (0.8); 7.3311 (1.2); 7.3220 (0.5); 7.3196 (0.6); 7.2607 (18.0); 6.7239 (1.5); 6.7220 (1.6); 6.7023 (1.5); 6.7005 (1.5); 4.9554 (7.4); 3.9244 (16.0); 3.7958 (13.8); 2.0453 (1.4); 1.5491 (6.9); 1.2597 (1.0); 0.8821 (0.7); 0.0079 (0.8); -0.0002 (27.1); -0.0085 (0.8) |
| I-50: ¹H-NMR(400.6 MHz, d₆-DMSO): |
| δ= 12.9883 (1.4); 8.3151 (0.8); 8.3128 (1.1); 8.3098 (0.9); 8.3035 (0.9); 8.3012 (1.2); 8.2983 (0.9); 8.1041 (1.7); 8.1024 (1.7); 8.0979 (1.8); 8.0962 (1.8); 7.9660 (0.6); 7.9625 (0.7); 7.9451 (0.8); 7.9415 (1.4); 7.9378 (0.8); 7.9205 (0.8); 7.9169 (0.8); 7.6123 (0.8); 7.6028 (1.0); 7.6007 (0.9); 7.5968 (1.7); 7.5909 (2.5); 7.5819 (0.8); 7.5799 (0.8); 7.5752 (1.8); 7.5690 (1.9); 6.8624 (1.9); 6.8606 (1.9); 6.8408 (1.9); 6.8390 (1.9); 5.7571 (0.6); 5.0423 (1.8); 5.0248 (1.9); 3.8484 (16.0); 3.3250 (4.9); 2.5199 (0.6); 2.5112 (8.7); 2.5066 (19.2); 2.5020 (27.1); 2.4975 (19.6); 2.4930 (9.6); 2.4707 (0.6); 2.0859 (1.4); 1.5681 (5.0); 1.5507 (5.0); 0.0080 (0.5); -0.0002 (17.6); -0.0085 (0.7) |
| I-51: ¹H-NMR(400.0 MHz, CDCl3): |
| δ= 8.4512 (3.8); 8.4445 (3.7); 8.2975 (4.0); 8.2458 (2.4); 8.2345 (2.3); 7.5282 (1.8); 7.5234 (2.9); 7.4995 (4.0); 7.4794 (1.5); 7.4759 (1.4); 7.3599 (1.5); 7.3507 (1.9); 7.3393 (2.4); 7.3301 (1.4); 7.3186 (1.0); 7.2616 (13.9); 5.3001 (6.2); 4.9384 (16.0); 4.2929 (2.2); 4.2751 (6.2); 4.2573 (6.2); 4.2394 (2.1); 2.7742 (0.7); 1.5650 (6.9); 1.3050 (7.0); 1.2872 (13.7); 1.2693 (6.7); - 0.0002 (19.9) |
| I-52: ¹H-NMR(400.6 MHz, CDCl3): |
| δ= 8.5510 (1.8); 8.5487 (2.0); 8.5467 (2.0); 8.5444 (1.7); 8.5390 (1.8); 8.5367 (2.1); 8.5345 (2.0); 8.5324 (1.6); 8.2331 (2.3); 8.2313 (2.5); 8.2294 (2.5); 8.2278 (2.1); 8.2214 (2.3); 8.2197 (2.6); 8.2178 (2.5); 8.1223 (3.2); 8.1161 (3.3); 7.8536 (1.6); 7.8473 (1.5); 7.8348 (2.0); 7.8324 (2.0); 7.8286 (1.9); 7.8262 (1.8); 7.8137 (1.6); 7.8074 (1.6); 7.5612 (1.2); 7.5567 (1.2); 7.5419 (2.6); 7.5375 (2.6); 7.5226 (1.7); 7.5185 (1.8); 7.5036 (1.6); 7.4999 (1.6); 7.4829 (2.4); 7.4795 (2.6); 7.4767 (1.8); 7.4598 (2.1); 7.4561 (2.0); 7.3511 (2.0); 7.3421 (2.5); 7.3395 (2.1); 7.3305 (3.8); 7.3216 (3.5); 7.3190 (2.1); 7.3098 (1.8); 7.3045 (2.4); 7.2605 (83.7); 7.1889 (1.5); 7.1756 (1.5); 7.1702 (1.5); 7.1581 (1.4); 6.9607 (2.3); 6.9533 (2.4); 6.9518 (2.1); 6.9395 (2.2); 6.9380 (2.1); 6.9320 (2.2); 6.9305 (1.9); 5.4288 (1.1); 5.4114 (4.3); 5.3940 (4.4); 5.3767 (1.1); 5.3338 (13.9); 1.7655 (16.0); 1.7481 (15.8); 1.5507 (10.8); 0.0079 (3.8); -0.0002 (129.1); -0.0061 (1.9); -0.0085 (3.8) |
| I-53: ¹H-NMR(400.6 MHz, CDCl3): |
| δ= 8.6885 (0.9); 8.2923 (0.8); 8.2907 (0.9); 8.2888 (1.0); 8.2873 (0.9); 8.2808 (0.9); 8.2791 (1.0); 8.2772 (1.0); 8.1466 (1.3); 8.1404 (1.4); 7.8679 (0.6); 7.8616 (0.6); 7.8493 (0.7); 7.8467 (0.8); 7.8431 (0.8); 7.8405 (0.8); 7.8281 (0.7); 7.8218 (0.7); 7.5445 (0.6); 7.5409 (0.6); 7.5237 (1.0); 7.5201 (1.1); 7.5182 (0.8); 7.5010 (0.9); 7.4973 (0.8); 7.4078 (0.8); 7.3987 (1.0); 7.3963 (0.9); 7.3871 (1.4); 7.3779 (0.7); 7.3755 (0.7); 7.3664 (0.6); 7.2613 (15.8); 6.9881 (0.8); 6.9868 (0.9); 6.9806 (0.9); 6.9793 (0.9); 6.9669 (0.8); 6.9655 (0.9); 6.9595 (0.9); 6.9581 (0.9); 4.8859 (9.7); 4.2566 (0.8); 4.2398 (1.2); 4.2230 (0.9); 2.9082 (16.0); 2.0455 (0.6); 1.5543 (8.7); 1.2596 (0.6); 1.1572 (13.6); 1.1404 (13.6); 0.8819 (0.5); 0.0080 (0.7); -0.0002 (24.3); -0.0085 (1.0) |
| I-54: ¹H-NMR(400.0 MHz, CDCl3): |
| δ= 9.1339 (2.0); 8.2860 (2.0); 8.2746 (2.0); 8.1517 (2.7); 8.1460 (2.7); 7.8574 (1.0); 7.8512 (1.0); 7.8362 (1.5); 7.8302 (1.4); 7.8175 (1.1); 7.8112 (1.0); 7.5408 (0.9); 7.5200 (2.3); 7.4967 (1.1); 7.4053 (1.1); 7.3942 (1.6); 7.3846 (2.0); 7.3734 (1.2); 7.3641 (0.8); 7.2612 (74.0); 6.9805 (1.8); 6.9731 (1.8); 6.9592 (1.8); 6.9519 (1.8); 5.3003 (3.1); 4.9221 (13.0); 4.2494 (1.5); 4.2316 (4.7); 4.2138 (4.8); 4.1960 (1.7); 4.1761 (9.3); 4.1311 (0.8); 4.1130 (0.8); 3.0726 (16.0); 2.0451 (3.6); 1.5673 (13.2); 1.3048 (5.6); 1.2869 (11.3); 1.2775 (1.8); 1.2690 (6.4); 1.2596 (4.0); 1.2418 (1.1); 0.8982 (0.6); 0.8818 (1.3); 0.8645 (0.7); 0.0080 (1.2); -0.0002 (39.5); -0.0084 (1.2) |
| I-55: ¹H-NMR(400.0 MHz, CDCl3): |
| δ= 8.2454 (1.1); 8.2356 (1.1); 8.1358 (1.4); 8.1295 (1.5); 7.8564 (0.7); 7.8502 (0.6); 7.8354 (0.9); 7.8314 (0.8); 7.8292 (0.8); 7.8165 (0.7); 7.8102 (0.7); 7.4959 (0.6); 7.4922 (0.6); 7.4752 (0.9); 7.4718 (1.2); 7.4522 (0.9); 7.4484 (0.8); 7.3429 (0.8); 7.3339 (1.0); 7.3314 (0.9); 7.3223 (1.5); 7.3131 (0.7); 7.3106 (0.7); 7.3017 (0.6); 7.2612 (42.3); 6.9602 (1.0); 6.9528 (1.0); 6.9390 (1.0); 6.9315 (0.9); 4.9300 (9.9); 4.2871 (1.2); 4.2692 (3.8); 4.2513 (3.8); 4.2335 (1.2); 1.5513 (16.0); 1.3026 (4.5); 1.2848 (9.2); 1.2669 (4.5); 0.0079 (0.8); -0.0002 (26.3); -0.0086 (0.7) |
| I-56: ¹H-NMR(400.6 MHz, CDCl3): |
| δ= 8.2591 (1.4); 8.2572 (1.4); 8.2475 (1.5); 8.2457 (1.5); 8.1173 (1.9); 8.1113 (2.0); 7.8362 (0.7); 7.8300 (0.7); 7.8156 (1.0); 7.8100 (1.0); 7.7965 (0.7); 7.7904 (0.7); 7.5148 (1.0); 7.5111 (1.0); 7.4940 (1.6); 7.4922 (1.5); 7.4903 (1.7); 7.4887 (1.3); 7.4715 (1.5); 7.4678 (1.4); 7.3878 (1.3); 7.3785 (1.5); 7.3763 (1.4); 7.3670 (2.1); 7.3577 (1.0); 7.3555 (1.0); 7.3462 (0.9); 7.2663 (4.6); 6.9493 (1.5); 6.9478 (1.5); 6.9418 (1.6); 6.9404 (1.5); 6.9281 (1.4); 6.9266 (1.4); 6.9207 (1.4); 6.9192 (1.4); 4.9394 (13.9); 4.2846 (2.0); 4.2667 (6.3); 4.2489 (6.3); 4.2311 (2.1); 3.8782 (0.5); 3.0300 (0.5); 2.9983 (0.5); 1.6151 (4.8); 1.3027 (0.7); 1.2975 (7.8); 1.2850 (1.6); 1.2797 (16.0); 1.2671 (0.8); 1.2619 (7.7); -0.0002 (6.3) |
| I-57: ¹H-NMR(400.6 MHz, CDCl3): |
| δ= 8.2960 (1.2); 8.2940 (1.2); 8.2845 (1.2); 8.2825 (1.2); 8.1270 (1.6); 8.1209 (1.6); 7.9824 (0.7); 7.9762 (0.7); 7.9639 (0.8); 7.9611 (0.9); 7.9577 (0.8); 7.9550 (0.8); 7.9427 (0.7); 7.9365 (0.7); 7.5492 (0.7); 7.5455 (0.7); 7.5281 (1.3); 7.5245 (1.3); 7.5061 (1.0); 7.5025 (1.0); 7.4495 (1.0); 7.4400 (1.2); 7.4381 (1.1); 7.4286 (1.6); 7.4191 (0.8); 7.4172 (0.8); 7.4078 (0.6); 7.2621 (11.3); 7.0078 (1.1); 7.0068 (1.1); 7.0005 (1.1); 6.9865 (1.0); 6.9854 (1.0); 6.9792 (1.1); 5.0231 (10.7); 4.2976 (1.4); 4.2797 (4.2); 4.2619 (4.3); 4.2441 (1.4); 2.0457 (0.9); 1.5588 (16.0); 1.3018 (5.0); 1.2840 (10.2); 1.2777 (0.6); 1.2661 (5.2); 1.2598 (0.9); 0.8819 (0.5); 0.0079 (0.6); -0.0002 (17.1); -0.0085 (0.6) |
| I-16: ¹H-NMR(400.6 MHz, CDCl3): |
| δ= 8.4532 (1.8); 8.4463 (1.9); 8.3017 (1.0); 8.2979 (1.8); 8.2940 (1.0); 8.2515 (0.8); 8.2497 (0.9); 8.2479 (0.9); 8.2461 (0.8); 8.2399 (0.8); 8.2381 (0.9); 8.2362 (0.9); 8.2345 (0.8); 7.5285 (0.8); 7.5261 (0.8); 7.5241 (0.9); 7.5219 (1.2); 7.5172 (0.8); 7.5057 (1.3); 7.5019 (1.9); 7.4994 (1.6); 7.4950 (0.8); 7.4823 (0.9); 7.4786 (0.9); 7.3640 (0.9); 7.3550 (1.0); 7.3524 (0.9); 7.3434 (1.5); 7.3343 (0.7); 7.3317 (0.7); 7.3227 (0.6); 7.2617 (36.6); 4.9588 (8.4); 3.8031 (16.0); 2.9631 (1.0); 2.7750 (0.6); 2.0454 (0.5); 1.5596 (5.9); 0.0080 (0.6); -0.0002 (21.9); -0.0084 (0.7) |
| I-14: ¹H-NMR(400.0 MHz, d₆-DMSO): |
| δ= 13.1318 (0.6); 8.2740 (3.6); 8.2679 (3.6); 8.1682 (4.9); 8.1607 (5.2); 8.0299 (1.4); 8.0237 (1.4); 8.0090 (2.2); 8.0028 (2.1); 7.9891 (1.5); 7.9829 (1.5); 7.9601 (1.7); 7.9526 (1.5); 7.9395 (2.0); 7.9377 (2.3); 7.9301 (2.2); 7.9171 (2.0); 7.9095 (1.8); 7.7365 (2.8); 7.7268 (2.9); 7.7138 (2.4); 7.7042 (2.3); 7.3072 (2.3); 7.3009 (2.3); 7.2859 (2.2); 7.2797 (2.1); 4.9188 (16.0); 3.3231 (1.2); 2.5249 (0.8); 2.5202 (1.0); 2.5114 (16.8); 2.5069 (36.9); 2.5023 (51.5); 2.4978 (36.1); 2.4932 (16.1); 2.1834 (0.6); 1.3559 (4.6); 0.0080 (1.0); -0.0002 (37.2); -0.0085 (1.1) |
| I-13: ¹H-NMR(599.6 MHz, CDCl3): |
| δ= 8.1922 (12.8); 8.1884 (13.0); 7.9613 (0.4); 7.9565 (0.4); 7.9151 (14.6); 7.9102 (14.9); 7.8328 (4.0); 7.8288 (4.0); 7.8189 (6.7); 7.8161 (6.5); 7.8063 (4.3); 7.8022 (4.1); 7.6173 (6.5); 7.6110 (6.7); 7.6022 (7.7); 7.5959 (7.6); 7.5375 (0.4); 7.5334 (0.5); 7.4536 (4.7); 7.4487 (4.7); 7.4410 (5.6); 7.4386 (5.7); 7.4363 (6.0); 7.4339 (5.0); 7.4262 (4.2); 7.4212 (3.8); 7.4115 (0.4); 7.2607 (34.4); 7.0003 (7.3); 6.9959 (7.4); 6.9862 (7.2); 6.9818 (7.2); 6.6449 (0.4); 6.6292 (0.4); 5.3183 (3.0); 5.3067 (9.8); 5.2950 (9.9); 5.2833 (3.1); 5.2697 (0.4); 5.2580 (0.3); 3.9749 (0.7); 2.2711 (1.2); 1.7606 (39.1); 1.7489 (38.9); 1.7322 (1.3); 1.4320 (8.8); 1.2550 (2.8); 0.8797 (0.4); 0.0701 (0.6); 0.0052 (2.2); -0.0001 (50.0); -0.0056 (1.8) |
| I-12: ¹H-NMR(400.0 MHz, CDCl3): |
| δ= 8.1947 (1.3); 8.1885 (1.4); 7.9220 (1.8); 7.9152 (1.8); 7.8470 (0.7); 7.8408 (0.7); 7.8281 (0.8); 7.8259 (0.8); 7.8219 (0.8); 7.8197 (0.8); 7.8070 (0.7); 7.8008 (0.7); 7.6636 (0.8); 7.6540 (0.8); 7.6412 (1.1); 7.6315 (1.1); 7.5082 (0.8); 7.5008 (0.8); 7.4895 (0.9); 7.4857 (0.7); 7.4821 (0.8); 7.4783 (0.7); 7.4670 (0.7); 7.4596 (0.6); 7.2615 (20.4); 7.0048 (0.9); 6.9974 (1.0); 6.9849 (0.9); 6.9836 (0.9); 6.9776 (0.9); 4.9546 (8.7); 3.8287 (16.0); 1.5579 (1.6); 0.8819 (0.6); -0.0002 (14.2) |
| I-11: ¹H-NMR(400.0 MHz, CDCl3): |
| δ= 8.1907 (1.2); 8.1888 (1.0); 8.1865 (1.0); 8.1845 (1.2); 7.8990 (1.7); 7.8916 (1.7); 7.8418 (0.6); 7.8356 (0.6); 7.8229 (0.7); 7.8207 (0.8); 7.8167 (0.7); 7.8145 (0.7); 7.8018 (0.6); 7.7956 (0.6); 7.6469 (0.7); 7.6459 (0.7); 7.6372 (0.8); 7.6362 (0.8); 7.6243 (1.0); 7.6233 (1.0); 7.6147 (1.0); 7.6138 (1.0); 7.4984 (0.8); 7.4910 (0.7); 7.4796 (0.8); 7.4759 (0.6); 7.4722 (0.8); 7.4685 (0.6); 7.4571 (0.6); 7.4497 (0.6); 7.2629 (7.7); 7.0011 (0.8); 6.9998 (0.8); 6.9937 (0.8); 6.9925 (0.8); 6.9800 (0.8); 6.9787 (0.8); 6.9726 (0.8); 6.9712 (0.8); 5.2486 (1.6); 5.2312 (1.6); 3.7972 (16.0); 2.0449 (0.7); 1.7171 (6.3); 1.6996 (6.3); 1.5757 (0.7); 1.2641 (0.8); 1.2594 (0.9); 0.8816 (1.5); 0.8638 (0.6); -0.0002 (5.4) |
| I-01: ¹H-NMR(400.0 MHz, CDCl3): |
| δ= 8.2452 (2.7); 8.2322 (2.8); 8.0794 (1.6); 8.0768 (1.6); 8.0747 (1.5); 8.0691 (1.5); 8.0672 (1.7); 8.0645 (1.6); 8.0626 (1.4); 7.8012 (1.1); 7.7965 (1.0); 7.7826 (1.5); 7.7806 (1.8); 7.7780 (1.5); 7.7759 (1.6); 7.7621 (1.6); 7.7574 (1.5); 7.6457 (2.9); 7.6273 (1.5); 7.6251 (2.2); 7.5212 (1.0); 7.3121 (0.8); 7.2623 (166.4); 7.2124 (0.7); 7.1535 (1.4); 7.1510 (1.4); 7.1413 (1.5); 7.1388 (1.6); 7.1346 (2.5); 7.1325 (2.2); 7.1309 (2.3); 7.1263 (1.7); 7.1209 (2.4); 7.1177 (2.1); 7.1135 (1.4); 6.9983 (1.0); 6.9412 (3.2); 4.9451 (14.8); 4.7010 (0.5); 4.3221 (2.0); 4.3042 (6.2); 4.2864 (6.2); 4.2686 (2.0); 1.5859 (0.5); 1.3326 (8.0); 1.3147 (16.0); 1.2969 (8.6); 1.2533 (4.5); 0.8798 (1.0); 0.8621 (0.6); 0.0079 (2.9); -0.0002 (94.2); -0.0085 (4.0) |
| I-01: ¹H-NMR(599.6 MHz, CDCl3): |
| δ= 8.5362 (1.8); 8.5343 (1.9); 8.5284 (1.9); 8.5262 (1.8); 8.4282 (0.4); 8.2542 (11.2); 8.2456 (11.3); 8.0981 (6.6); 8.0963 (6.6); 8.0901 (6.7); 8.0883 (6.4); 7.8572 (1.2); 7.8543 (1.2); 7.8442 (2.0); 7.8416 (2.0); 7.8312 (1.4); 7.8281 (1.3); 7.7984 (3.7); 7.7954 (3.6); 7.7846 (6.5); 7.7828 (6.3); 7.7723 (4.6); 7.7693 (4.2); 7.7612 (0.3); 7.6572 (0.3); 7.6435 (0.4); 7.6225 (10.6); 7.6088 (9.0); 7.3077 (1.6); 7.2995 (1.7); 7.2954 (1.7); 7.2872 (1.6); 7.2656 (10.5); 7.1614 (5.6); 7.1532 (6.0); 7.1522 (5.9); 7.1475 (8.9); 7.1446 (8.8); 7.1416 (8.7); 7.1394 (7.9); 7.1360 (7.9); 6.9597 (13.5); 5.0092 (0.4); 4.9453 (50.0); 4.9007 (1.0); 4.8887 (15.3); 4.8780 (1.6); 4.3122 (6.6); 4.3003 (19.7); 4.2884 (19.8); 4.2765 (8.7); 4.2646 (6.3); 4.2527 (6.3); 4.2408 (2.1); 1.3248 (21.4); 1.3129 (41.8); 1.3010 (21.0); 1.2948 (7.2); 1.2829 (13.2); 1.2711 (6.6); 1.2556 (0.6); -0.0001 (10.6); -0.0054 (0.4) |
| I-01: ¹H-NMR(400.6 MHz, CDCl3): |
| δ= 8.2433 (1.6); 8.2419 (2.6); 8.2404 (1.5); 8.2306 (1.7); 8.2290 (2.7); 8.0776 (1.2); 8.0755 (1.3); 8.0729 (1.4); 8.0708 (1.3); 8.0654 (1.3); 8.0633 (1.4); 8.0608 (1.4); 8.0586 (1.3); 7.7983 (1.1); 7.7935 (1.1); 7.7798 (1.3); 7.7776 (1.6); 7.7751 (1.3); 7.7729 (1.6); 7.7592 (1.6); 7.7545 (1.6); 7.6490 (1.7); 7.6467 (2.9); 7.6443 (1.7); 7.6284 (1.4); 7.6261 (2.2); 7.6237 (1.2); 7.2629 (24.3); 7.2596 (0.6); 7.1495 (1.5); 7.1470 (1.4); 7.1374 (1.4); 7.1348 (1.6); 7.1327 (1.6); 7.1311 (2.0); 7.1285 (2.9); 7.1249 (1.3); 7.1191 (2.1); 7.1164 (2.8); 7.1119 (1.2); 6.9435 (1.4); 6.9418 (1.7); 6.9401 (2.0); 6.9385 (2.8); 6.9369 (1.9); 6.9353 (1.7); 6.9337 (1.3); 5.3005 (4.9); 4.9434 (14.5); 4.3211 (1.8); 4.3032 (5.8); 4.2854 (5.8); 4.2677 (1.9); 1.5809 (1.2); 1.3313 (7.6); 1.3135 (16.0); 1.2957 (7.4); -0.0002 (15.2) |
| I-01: ¹H-NMR(400.0 MHz, CDCl3): |
| δ= 8.2418 (2.8); 8.2289 (2.9); 8.0755 (1.5); 8.0728 (1.6); 8.0708 (1.4); 8.0633 (1.5); 8.0607 (1.6); 8.0588 (1.4); 7.7974 (1.1); 7.7927 (1.0); 7.7788 (1.4); 7.7768 (1.7); 7.7742 (1.4); 7.7722 (1.6); 7.7583 (1.6); 7.7536 (1.5); 7.6460 (3.1); 7.6253 (2.5); 7.2611 (59.1); 7.1488 (1.4); 7.1463 (1.5); 7.1366 (1.5); 7.1325 (1.9); 7.1280 (3.2); 7.1246 (1.4); 7.1183 (2.2); 7.1156 (3.1); 7.1117 (1.3); 6.9379 (3.1); 4.9718 (0.8); 4.9428 (16.0); 4.3210 (2.0); 4.3031 (6.2); 4.2928 (0.5); 4.2853 (6.3); 4.2674 (2.1); 1.6344 (1.2); 1.3312 (7.4); 1.3133 (15.1); 1.2955 (7.2); 1.2551 (0.6); 0.0080 (0.9); -0.0002 (37.0); -0.0085 (1.2) |
| I-01: ¹H-NMR(400.0 MHz, CDCl3): |
| δ= 8.2428 (2.7); 8.2298 (2.8); 8.0787 (1.3); 8.0766 (1.4); 8.0740 (1.5); 8.0720 (1.4); 8.0665 (1.3); 8.0645 (1.4); 8.0618 (1.4); 8.0598 (1.3); 7.7985 (1.1); 7.7938 (1.1); 7.7799 (1.4); 7.7779 (1.6); 7.7753 (1.4); 7.7732 (1.6); 7.7593 (1.6); 7.7546 (1.5); 7.6478 (1.7); 7.6455 (2.9); 7.6432 (1.7); 7.6272 (1.3); 7.6249 (2.2); 7.6226 (1.3); 7.2629 (20.8); 7.1502 (1.4); 7.1476 (1.5); 7.1380 (1.5); 7.1353 (1.8); 7.1339 (1.7); 7.1293 (3.0); 7.1259 (1.4); 7.1196 (2.0); 7.1169 (2.9); 7.1130 (1.3); 6.9445 (1.5); 6.9430 (1.8); 6.9397 (2.9); 6.9365 (1.7); 6.9350 (1.4); 5.3001 (5.2); 4.9431 (15.4); 4.3209 (2.0); 4.3031 (6.2); 4.2852 (6.2); 4.2674 (2.0); 1.3312 (7.7); 1.3134 (16.0); 1.2955 (7.6); -0.0002 (12.0) |
| I-59: ¹H-NMR(400.6 MHz, CDCl3): |
| δ= 8.2170 (1.3); 8.2109 (1.4); 8.0929 (0.7); 8.0831 (0.7); 7.8722 (0.6); 7.8661 (0.6); 7.8533 (0.7); 7.8512 (0.8); 7.8471 (0.7); 7.8450 (0.7); 7.8324 (0.7); 7.8262 (0.6); 7.8117 (0.5); 7.8071 (0.5); 7.7930 (0.7); 7.7910 (0.9); 7.7886 (0.8); 7.7865 (0.9); 7.7727 (0.8); 7.7681 (0.8); 7.7016 (1.2); 7.6810 (0.8); 7.2675 (2.8); 7.1558 (0.7); 7.1536 (0.7); 7.1437 (0.7); 7.1414 (0.7); 7.1376 (0.7); 7.1353 (0.6); 7.1254 (0.6); 7.1231 (0.6); 7.0153 (0.9); 7.0082 (0.9); 6.9942 (0.8); 6.9871 (0.9); 4.9144 (7.8); 4.7249 (0.9); 4.7068 (1.3); 4.6887 (0.9); 3.7815 (16.0); 2.0093 (0.8); 1.5140 (6.5); 1.4961 (6.5); -0.0002 (3.4) |
| I-59: ¹H-NMR(400.0 MHz, CDCl3): |
| δ= 8.2148 (1.4); 8.2088 (1.4); 8.0885 (0.9); 8.0857 (0.9); 8.0764 (0.9); 8.0737 (0.9); 7.8677 (0.6); 7.8615 (0.6); 7.8466 (0.8); 7.8425 (0.8); 7.8405 (0.8); 7.8277 (0.7); 7.8216 (0.6); 7.8071 (0.6); 7.8024 (0.6); 7.7865 (1.0); 7.7819 (0.9); 7.7681 (0.9); 7.7634 (0.9); 7.7052 (1.8); 7.6845 (1.1); 7.2619 (25.8); 7.1486 (1.1); 7.1462 (1.1); 7.1366 (1.1); 7.1340 (1.1); 7.1305 (1.0); 7.1279 (0.8); 7.1182 (0.8); 7.1157 (0.7); 7.0094 (1.0); 7.0023 (1.0); 6.9881 (0.9); 6.9812 (0.9); 4.9065 (8.6); 4.7245 (0.9); 4.7064 (1.3); 4.6882 (0.9); 4.1310 (1.2); 4.1131 (1.2); 3.7800 (16.0); 2.0452 (5.2); 1.5740 (1.0); 1.5119 (6.7); 1.4940 (6.6); 1.2773 (1.7); 1.2595 (3.4); 1.2416 (1.4); 0.8987 (0.5); 0.8819 (1.7); 0.8642 (0.7); 0.0079 (0.6); -0.0002 (14.9) |
| I-60: ¹H-NMR(400.6 MHz, CDCl3): |
| δ= 8.2130 (2.0); 8.2068 (2.2); 8.1268 (1.3); 8.1248 (1.3); 8.1222 (1.5); 8.1146 (1.4); 8.1100 (1.4); 7.8671 (1.0); 7.8609 (1.1); 7.8483 (1.3); 7.8460 (1.3); 7.8421 (1.3); 7.8398 (1.2); 7.8273 (1.1); 7.8222 (1.5); 7.8178 (1.2); 7.8019 (1.5); 7.7991 (1.4); 7.7972 (1.4); 7.7833 (1.5); 7.7786 (1.4); 7.6286 (2.7); 7.6081 (2.2); 7.5188 (0.9); 7.2603 (167.8); 7.1912 (1.4); 7.1887 (1.3); 7.1790 (1.4); 7.1765 (1.3); 7.1727 (1.3); 7.1702 (1.2); 7.1605 (1.3); 7.1580 (1.2); 7.0254 (1.4); 7.0182 (1.4); 7.0044 (1.5); 6.9968 (2.4); 5.0298 (0.7); 4.9840 (16.0); 4.3501 (1.6); 4.3287 (5.1); 4.3073 (5.3); 4.2859 (1.8); 1.6115 (4.3); 1.2538 (0.6); 0.1457 (0.7); 0.0080 (5.1); -0.0002 (208.2); -0.0085 (7.5); -0.0294 (0.6); -0.1494 (0.7) |
| I-61: ¹H-NMR(400.6 MHz, CDCl3): |
| δ= 8.2552 (1.8); 8.2422 (1.8); 8.0907 (0.9); 8.0881 (1.0); 8.0806 (0.9); 8.0786 (1.0); 8.0760 (1.0); 7.8361 (0.7); 7.8315 (0.6); 7.8176 (0.9); 7.8155 (1.1); 7.8129 (1.0); 7.8109 (1.0); 7.7971 (1.0); 7.7925 (1.0); 7.7156 (1.9); 7.6950 (1.3); 7.2606 (43.7); 7.1807 (0.9); 7.1781 (0.9); 7.1685 (0.9); 7.1660 (0.9); 7.1623 (1.0); 7.1598 (0.8); 7.1501 (0.9); 7.1475 (0.8); 7.1328 (0.8); 7.1293 (1.2); 7.1251 (0.9); 7.1199 (0.9); 7.1163 (1.2); 7.1122 (0.9); 6.9434 (1.9); 5.3003 (2.5); 4.9419 (8.0); 4.1829 (3.7); 4.1696 (3.7); 3.7948 (16.0); 1.5424 (16.0); 1.2556 (1.2); 0.0079 (2.2); -0.0002 (70.3); -0.0085 (2.7) |
| I-62: ¹H-NMR(400.6 MHz, d₆-DMSO): |
| δ= 12.3088 (1.1); 8.2504 (2.8); 8.2443 (2.9); 8.1007 (1.7); 8.0988 (1.9); 8.0962 (2.0); 8.0941 (1.8); 8.0887 (1.8); 8.0867 (2.1); 8.0840 (2.0); 8.0820 (1.8); 8.0231 (1.2); 8.0169 (1.1); 8.0020 (1.8); 7.9958 (1.7); 7.9824 (1.3); 7.9762 (1.2); 7.9594 (1.5); 7.9547 (1.4); 7.9409 (1.8); 7.9388 (2.0); 7.9362 (1.8); 7.9341 (1.8); 7.9203 (1.8); 7.9155 (1.7); 7.6788 (2.0); 7.6766 (3.6); 7.6746 (2.4); 7.6582 (1.8); 7.6561 (3.2); 7.6539 (2.0); 7.3018 (1.9); 7.2960 (2.0); 7.2803 (2.1); 7.2785 (2.5); 7.2757 (3.0); 7.2663 (1.8); 7.2639 (2.0); 7.2599 (1.8); 7.2575 (1.6); 7.2478 (1.7); 7.2453 (1.7); 5.1825 (0.6); 5.1460 (2.1); 5.0933 (2.0); 5.0579 (0.6); 4.2193 (0.6); 4.1869 (0.6); 4.0986 (0.8); 4.0808 (0.8); 4.0557 (1.1); 4.0379 (3.4); 4.0201 (3.4); 4.0024 (1.2); 3.8076 (0.6); 3.7738 (0.6); 3.3220 (30.6); 3.1522 (0.7); 2.7920 (0.7); 2.6705 (0.5); 2.5680 (0.6); 2.5583 (1.1); 2.5484 (0.7); 2.5411 (2.1); 2.5301 (0.8); 2.5243 (1.4); 2.5197 (1.8); 2.5108 (24.5); 2.5063 (54.7); 2.5017 (77.4); 2.4971 (54.8); 2.4926 (25.3); 2.1832 (0.6); 1.9887 (16.0); 1.9090 (1.5); 1.8718 (0.9); 1.8258 (0.7); 1.6402 (0.5); 1.4102 (0.5); 1.3554 (5.2); 1.2351 (0.9); 1.2036 (1.0); 1.1922 (4.7); 1.1858 (2.3); 1.1745 (9.3); 1.1681 (1.1); 1.1567 (4.5); 0.0080 (1.3); -0.0002 (48.1); -0.0085 (1.5) |
| I-63: ¹H-NMR(400.6 MHz, d₆-DMSO): |
| δ= 12.5889 (1.3); 8.2481 (6.1); 8.1032 (4.6); 8.0943 (4.5); 8.0215 (2.1); 8.0155 (2.2); 8.0009 (3.9); 7.9959 (3.8); 7.9808 (3.6); 7.9753 (3.6); 7.9595 (3.1); 7.7000 (3.0); 7.6799 (5.5); 7.6599 (2.8); 7.3025 (4.1); 7.2964 (4.3); 7.2822 (5.5); 7.2748 (7.1); 7.2566 (3.1); 6.8699 (0.6); 5.0144 (10.4); 4.9638 (0.7); 4.0552 (1.3); 4.0378 (3.7); 4.0200 (3.7); 4.0024 (1.3); 3.7947 (1.0); 3.7698 (1.8); 3.7501 (1.5); 3.6783 (1.6); 3.6607 (1.9); 3.6348 (1.6); 3.6097 (1.8); 3.5937 (2.7); 3.5751 (2.6); 3.5638 (4.3); 3.5448 (5.4); 3.5291 (2.7); 3.4985 (1.2); 3.4675 (1.4); 3.4491 (1.4); 3.4370 (1.0); 3.3927 (1.2); 3.3741 (2.3); 3.3206 (26.0); 3.2046 (1.4); 3.1859 (1.8); 3.1672 (1.3); 3.0778 (1.4); 3.0598 (1.9); 3.0420 (1.4); 2.6701 (1.6); 2.5404 (6.6); 2.5014 (226.7); 2.3286 (1.6); 2.2051 (1.2); 2.1850 (2.1); 2.1719 (1.6); 2.1444 (1.6); 2.1259 (1.8); 2.1129 (1.7); 2.0981 (1.7); 2.0809 (1.5); 2.0677 (1.2); 1.9886 (16.0); 1.9701 (1.8); 1.9576 (1.2); 1.9375 (0.9); 1.9087 (0.6); 1.7602 (0.6); 1.3551 (7.0); 1.2352 (3.8); 1.1921 (4.2); 1.1744 (8.0); 1.1565 (4.1); -0.0002 (65.7) |
| I-64: ¹H-NMR(400.6 MHz, CDCl3): |
| δ= 8.2200 (1.3); 8.2178 (1.0); 8.2157 (1.1); 8.2138 (1.3); 8.0980 (0.8); 8.0960 (0.8); 8.0934 (0.9); 8.0914 (0.8); 8.0859 (0.8); 8.0839 (0.9); 8.0813 (0.9); 8.0793 (0.8); 7.8677 (0.7); 7.8615 (0.7); 7.8488 (0.8); 7.8465 (0.8); 7.8426 (0.8); 7.8404 (0.8); 7.8277 (0.7); 7.8215 (0.7); 7.8132 (0.7); 7.8085 (0.6); 7.7948 (0.8); 7.7925 (1.0); 7.7901 (0.8); 7.7879 (1.0); 7.7742 (1.0); 7.7695 (1.0); 7.7110 (1.2); 7.7088 (1.9); 7.7064 (1.1); 7.6904 (0.8); 7.6882 (1.2); 7.6858 (0.7); 7.2623 (25.4); 7.1549 (0.9); 7.1523 (0.9); 7.1428 (0.8); 7.1402 (0.9); 7.1366 (0.9); 7.1340 (0.8); 7.1245 (0.8); 7.1218 (0.8); 7.0115 (1.0); 7.0101 (0.9); 7.0041 (1.0); 7.0026 (0.9); 6.9903 (0.9); 6.9889 (0.8); 6.9829 (0.9); 6.9815 (0.8); 5.3004 (0.9); 4.9476 (7.7); 4.1834 (3.5); 4.1699 (3.5); 3.7905 (16.0); 1.5837 (0.8); -0.0002 (15.3) |
| I-64: ¹H-NMR(400.6 MHz, CDCl3): |
| δ= 8.2218 (0.8); 8.2198 (1.2); 8.2178 (0.9); 8.2157 (0.9); 8.2136 (1.2); 8.2117 (0.8); 8.0975 (0.7); 8.0954 (0.8); 8.0928 (0.8); 8.0907 (0.8); 8.0854 (0.8); 8.0832 (0.8); 8.0807 (0.9); 8.0786 (0.8); 7.8676 (0.7); 7.8613 (0.7); 7.8487 (0.8); 7.8464 (0.8); 7.8426 (0.8); 7.8402 (0.8); 7.8276 (0.7); 7.8214 (0.7); 7.8129 (0.7); 7.8082 (0.6); 7.7946 (0.7); 7.7922 (1.0); 7.7899 (0.8); 7.7875 (1.0); 7.7739 (1.1); 7.7692 (1.0); 7.7117 (1.1); 7.7093 (1.8); 7.7069 (1.1); 7.6910 (0.7); 7.6887 (1.2); 7.6863 (0.7); 7.2624 (8.4); 7.1545 (0.9); 7.1519 (0.9); 7.1424 (0.8); 7.1397 (0.9); 7.1362 (0.9); 7.1335 (0.8); 7.1241 (0.9); 7.1214 (0.8); 7.0113 (0.9); 7.0097 (0.9); 7.0039 (0.9); 7.0023 (0.9); 6.9901 (0.8); 6.9885 (0.9); 6.9827 (0.8); 6.9811 (0.8); 5.3003 (3.8); 4.9474 (7.2); 4.1834 (3.2); 4.1699 (3.2); 3.7905 (16.0); 1.5748 (1.9); -0.0002 (13.5) |
| I-65: ¹H-NMR(400.6 MHz, CDCl3): |
| δ= 8.5021 (1.2); 8.4957 (1.3); 8.3435 (1.5); 8.1153 (1.2); 8.1122 (0.8); 8.1066 (0.8); 8.1034 (1.4); 8.1001 (0.8); 7.8413 (1.4); 7.8377 (2.5); 7.8293 (3.0); 7.8259 (2.5); 7.5117 (0.7); 7.5049 (0.8); 7.5006 (0.7); 7.4894 (0.7); 7.4852 (0.8); 7.4826 (0.8); 7.4783 (0.7); 7.2603 (46.8); 7.2092 (0.9); 7.1972 (1.4); 7.1867 (1.4); 7.1759 (0.8); 4.0858 (4.1); 4.0727 (4.2); 3.8973 (8.2); 3.7025 (16.0); 2.0455 (0.5); 1.5432 (6.4); 0.0081 (1.9); -0.0002 (73.8); -0.0085 (2.8) |
| I-66: ¹H-NMR(599.6 MHz, d₆-DMSO): |
| δ= 13.0936 (0.3); 8.2586 (7.0); 8.2547 (7.0); 8.1131 (4.6); 8.1113 (4.8); 8.1102 (4.3); 8.1061 (4.2); 8.1050 (4.7); 8.1032 (4.6); 8.1022 (4.1); 8.0226 (2.2); 8.0184 (2.3); 8.0088 (3.9); 8.0050 (3.7); 7.9954 (2.4); 7.9913 (2.3); 7.9833 (3.0); 7.9802 (2.8); 7.9707 (4.2); 7.9696 (4.7); 7.9678 (4.4); 7.9667 (4.1); 7.9572 (3.3); 7.9541 (3.2); 7.6807 (7.5); 7.6669 (6.9); 7.2979 (4.2); 7.2938 (4.4); 7.2904 (4.5); 7.2889 (4.3); 7.2824 (6.5); 7.2805 (7.1); 7.2784 (6.3); 7.2699 (3.6); 7.2684 (3.5); 4.9262 (29.8); 4.8965 (0.4); 4.0479 (0.7); 4.0360 (2.2); 4.0242 (2.2); 4.0123 (0.7); 3.3890 (0.5); 3.3773 (0.6); 3.3655 (0.6); 3.3275 (6.4); 2.6153 (0.3); 2.5241 (1.1); 2.5211 (1.4); 2.5179 (1.5); 2.5089 (19.0); 2.5062 (37.4); 2.5032 (50.0); 2.5002 (37.1); 2.4974 (17.9); 1.9890 (9.4); 1.9106 (4.3); 1.3570 (0.7); 1.1869 (2.6); 1.1751 (5.1); 1.1632 (2.5); 1.0908 (0.5); 0.0053 (0.5); -0.0001 (11.6); -0.0056 (0.4) |
| I-66: ¹H-NMR(400.0 MHz, d₆-DMSO): |
| δ= 8.2593 (3.4); 8.2532 (3.5); 8.1151 (2.2); 8.1124 (2.4); 8.1104 (2.2); 8.1029 (2.4); 8.1002 (2.3); 8.0983 (2.2); 8.0305 (1.4); 8.0242 (1.4); 8.0096 (2.2); 8.0033 (2.1); 7.9913 (2.2); 7.9869 (2.0); 7.9835 (1.6); 7.9728 (2.1); 7.9710 (2.4); 7.9682 (2.1); 7.9664 (2.2); 7.9523 (2.1); 7.9476 (1.9); 7.6826 (4.2); 7.6620 (3.8); 7.3034 (2.3); 7.2962 (4.2); 7.2818 (4.2); 7.2753 (4.0); 7.2655 (2.0); 7.2630 (1.9); 4.9246 (16.0); 4.0381 (1.0); 4.0203 (1.0); 3.3238 (8.0); 2.5246 (0.8); 2.5199 (1.0); 2.5111 (16.1); 2.5065 (35.7); 2.5020 (50.4); 2.4974 (35.8); 2.4929 (16.2); 1.9888 (4.3); 1.9093 (1.5); 1.3557 (0.5); 1.1923 (1.2); 1.1746 (2.4); 1.1568 (1.2); 0.0080 (0.6); -0.0002 (23.1); -0.0085 (0.7) |
| I-66: ¹H-NMR(400.6 MHz, CDCl₃): |
| δ= 8.2064 (2.4); 8.2002 (2.6); 8.1164 (1.5); 8.1143 (1.7); 8.1116 (1.8); 8.1096 (1.7); 8.1042 (1.7); 8.1021 (1.8); 8.0994 (1.8); 8.0974 (1.7); 7.8625 (1.4); 7.8563 (1.3); 7.8437 (1.6); 7.8414 (1.7); 7.8375 (1.6); 7.8352 (1.6); 7.8226 (1.4); 7.8164 (1.4); 7.7751 (1.2); 7.7704 (1.2); 7.7565 (1.5); 7.7545 (1.8); 7.7518 (1.5); 7.7498 (1.7); 7.7360 (1.7); 7.7312 (1.7); 7.6097 (3.2); 7.5891 (2.4); 7.5198 (1.8); 7.2891 (1.3); 7.2860 (0.7); 7.2724 (0.6); 7.2693 (1.3); 7.2669 (1.9); 7.2613 (330.6); 7.2547 (3.6); 7.2522 (2.2); 7.2515 (2.1); 7.2499 (1.4); 7.2490 (1.2); 7.2482 (1.2); 7.2283 (0.6); 7.1399 (1.7); 7.1373 (1.7); 7.1277 (1.6); 7.1252 (1.7); 7.1214 (1.8); 7.1189 (1.7); 7.1093 (1.6); 7.1067 (1.6); 7.0058 (1.7); 7.0043 (1.8); 6.9976 (3.3); 6.9847 (1.7); 6.9831 (1.7); 6.9774 (1.9); 5.4649 (0.6); 5.3006 (0.5); 5.0617 (0.6); 5.0243 (16.0); 4.1317 (0.6); 4.1138 (0.6); 2.2718 (0.6); 2.1063 (2.0); 2.0466 (4.4); 2.0037 (2.4); 1.4322 (4.5); 1.3325 (0.9); 1.2843 (1.4); 1.2776 (1.3); 1.2598 (3.5); 1.2540 (4.8); 1.2430 (1.9); 0.8800 (0.8); 0.1459 (0.6); 0.0276 (0.8); 0.0239 (0.5); 0.0080 (6.3); 0.0056 (1.9); -0.0002 (211.9); -0.0085 (6.0); -0.1494 (0.6) |
| I-66: ¹H-NMR(400.0 MHz, d₆-DMSO): |
| δ= 8.2593 (2.9); 8.2532 (3.1); 8.1154 (1.9); 8.1127 (2.0); 8.1108 (2.0); 8.1033 (2.0); 8.1005 (2.0); 8.0987 (1.9); 8.0304 (1.2); 8.0242 (1.1); 8.0096 (1.9); 8.0033 (1.8); 7.9916 (1.8); 7.9899 (1.8); 7.9873 (1.7); 7.9835 (1.3); 7.9714 (2.0); 7.9686 (1.8); 7.9667 (1.9); 7.9527 (1.7); 7.9479 (1.6); 7.6826 (3.6); 7.6620 (3.2); 7.3035 (2.0); 7.2966 (3.6); 7.2822 (3.7); 7.2757 (3.5); 7.2659 (1.6); 7.2636 (1.7); 4.9253 (13.7); 4.0381 (0.6); 4.0203 (0.6); 3.3318 (16.0); 2.5660 (0.5); 2.5250 (0.6); 2.5203 (0.9); 2.5115 (15.3); 2.5070 (34.0); 2.5024 (47.9); 2.4979 (34.2); 2.4934 (15.6); 1.9889 (2.8); 1.9097 (1.6); 1.1924 (0.8); 1.1746 (1.6); 1.1568 (0.8) |
| I-19: ¹H-NMR(400.6 MHz, CDCl3): |
| δ= 8.2086 (1.0); 8.2064 (0.8); 8.2044 (0.8); 8.2024 (1.1); 8.0601 (0.7); 8.0580 (0.7); 8.0554 (0.8); 8.0533 (0.7); 8.0479 (0.7); 8.0459 (0.8); 8.0432 (0.8); 8.0412 (0.7); 7.8631 (0.6); 7.8569 (0.6); 7.8441 (0.7); 7.8419 (0.8); 7.8380 (0.7); 7.8357 (0.7); 7.8230 (0.6); 7.8169 (0.6); 7.7695 (0.6); 7.7648 (0.6); 7.7511 (0.7); 7.7488 (0.8); 7.7463 (0.7); 7.7441 (0.8); 7.7304 (0.9); 7.7257 (0.8); 7.6278 (0.9); 7.6255 (1.6); 7.6232 (0.9); 7.6071 (0.7); 7.6048 (1.2); 7.6025 (0.6); 7.2607 (14.6); 7.1086 (0.8); 7.1060 (0.8); 7.0964 (0.8); 7.0938 (0.8); 7.0901 (0.8); 7.0876 (0.7); 7.0780 (0.7); 7.0754 (0.7); 6.9958 (0.8); 6.9943 (0.8); 6.9884 (0.8); 6.9869 (0.8); 6.9746 (0.7); 6.9731 (0.8); 6.9673 (0.8); 6.9658 (0.7); 5.2765 (1.5); 5.2591 (1.5); 3.8014 (16.0); 1.7213 (6.3); 1.7038 (6.2); 1.5486 (1.1); 0.8819 (0.7); 0.0080 (0.6); -0.0002 (22.4); -0.0085 (0.7) |
| I-19: ¹H-NMR(400.6 MHz, CDCl3): |
| δ= 8.2105 (0.6); 8.2084 (1.0); 8.2064 (0.8); 8.2044 (0.8); 8.2022 (1.0); 8.2004 (0.7); 8.0601 (0.6); 8.0580 (0.7); 8.0554 (0.8); 8.0533 (0.7); 8.0479 (0.7); 8.0459 (0.8); 8.0432 (0.8); 8.0411 (0.7); 7.8632 (0.6); 7.8570 (0.6); 7.8443 (0.7); 7.8421 (0.7); 7.8381 (0.7); 7.8359 (0.7); 7.8231 (0.6); 7.8170 (0.6); 7.7702 (0.6); 7.7655 (0.6); 7.7518 (0.7); 7.7496 (0.9); 7.7471 (0.7); 7.7448 (0.8); 7.7312 (0.9); 7.7264 (0.8); 7.6274 (0.8); 7.6251 (1.6); 7.6228 (0.9); 7.6068 (0.7); 7.6044 (1.2); 7.6021 (0.7); 7.2632 (4.0); 7.1094 (0.8); 7.1068 (0.8); 7.0973 (0.7); 7.0947 (0.8); 7.0910 (0.8); 7.0885 (0.7); 7.0789 (0.7); 7.0763 (0.7); 6.9968 (0.7); 6.9953 (0.8); 6.9894 (0.8); 6.9879 (0.8); 6.9756 (0.7); 6.9740 (0.8); 6.9683 (0.7); 6.9667 (0.7); 5.3000 (9.1); 5.2769 (1.5); 5.2595 (1.5); 3.8016 (16.0); 1.7210 (6.2); 1.7036 (6.1); -0.0002 (5.3) |
| I-67: ¹H-NMR(400.0 MHz, CDCl3): |
| δ= 9.2223 (4.2); 8.7933 (9.7); 8.0386 (1.1); 8.0292 (1.1); 7.7968 (0.6); 7.7920 (0.6); 7.7751 (1.2); 7.7578 (1.2); 7.7530 (1.1); 7.7132 (2.1); 7.6926 (1.2); 7.2614 (56.4); 7.1244 (1.0); 7.1098 (1.1); 7.1066 (1.0); 7.0941 (0.8); 4.9797 (9.5); 4.9559 (0.6); 3.8404 (16.0); 3.8303 (1.3); 1.6019 (1.0); 1.4373 (0.5); 1.2539 (1.8); 0.0073 (2.0); -0.0002 (32.8); -0.0084 (1.5) |
| I-68: ¹H-NMR(599.6 MHz, CDCl3): |
| δ= 9.2245 (0.3); 8.7897 (0.7); 8.6074 (0.4); 7.2712 (3.2); 4.9556 (0.7); 4.3109 (0.3); 4.2995 (0.3); 2.7549 (23.2); 2.6273 (50.0); 2.0446 (0.3); 2.0335 (0.4); 1.8407 (0.6); 1.3738 (0.3); 1.3334 (2.0); 1.3234 (1.1); 1.3110 (1.1); 1.2849 (3.2); 1.2567 (12.3); 0.8911 (0.8); 0.8806 (1.4); 0.8690 (0.8); 0.8422 (0.4); 0.0699 (1.8); -0.0001 (4.0) |
| I-68: ¹H-NMR(400.0 MHz, CDCl3): |
| δ= 8.7895 (0.6); 7.2618 (20.5); 4.9549 (0.6); 2.7741 (16.0); 2.7495 (0.9); 1.3329 (1.3); 1.3232 (0.8); 1.3053 (0.7); 1.2844 (2.0); 1.2552 (6.7); 0.8800 (0.9); 0.0691 (1.8); -0.0002 (12.8) |
| I-69: ¹H-NMR(400.0 MHz, d₆-DMSO): |
| δ= 8.6621 (4.8); 8.6551 (4.8); 8.4160 (2.8); 8.4120 (5.0); 8.4083 (2.6); 8.1422 (2.2); 8.1396 (2.4); 8.1378 (2.2); 8.1301 (2.3); 8.1275 (2.3); 8.1256 (2.1); 8.0476 (1.6); 8.0430 (1.4); 8.0272 (2.4); 8.0243 (2.1); 8.0226 (2.2); 8.0084 (1.9); 8.0038 (1.7); 7.8805 (1.7); 7.8762 (1.8); 7.8736 (1.9); 7.8693 (1.6); 7.8564 (1.7); 7.8521 (1.9); 7.8496 (1.8); 7.8452 (1.6); 7.8140 (4.0); 7.7935 (3.4); 7.3571 (1.9); 7.3548 (2.0); 7.3449 (1.8); 7.3426 (2.1); 7.3385 (2.0); 7.3362 (1.9); 7.3264 (1.7); 7.3240 (1.8); 5.7563 (10.1); 4.0370 (16.0); 2.5250 (1.3); 2.5115 (12.5); 2.5071 (26.1); 2.5026 (35.8); 2.4980 (25.6); 2.4936 (11.9); 1.9891 (0.7); 1.6758 (0.9); 1.3565 (2.4); 1.2341 (1.8); 1.1749 (0.7); 0.0080 (0.9); -0.0002 (25.8); -0.0085 (1.0) |
| I-69: ¹H-NMR(400.0 MHz, CDCl3): |
| δ= 8.5173 (1.6); 8.5102 (1.6); 8.3401 (1.4); 8.1395 (0.7); 8.1276 (0.6); 7.8251 (0.7); 7.8203 (0.6); 7.8063 (0.7); 7.8016 (0.6); 7.7028 (1.2); 7.6823 (0.9); 7.5141 (0.5); 7.5069 (0.6); 7.5027 (0.5); 7.4916 (0.6); 7.4875 (0.6); 7.2606 (54.9); 7.2211 (0.6); 7.2064 (0.6); 7.2024 (0.6); 7.2000 (0.6); 7.1904 (0.5); 6.9782 (0.9); 5.2999 (4.7); 3.9514 (6.4); 2.2715 (1.8); 1.6668 (3.8); 1.5605 (1.1); 1.5150 (8.0); 1.4321 (16.0); 1.2843 (0.5); 1.2550 (1.8); 1.2222 (0.5); 0.0079 (1.2); -0.0002 (43.4); -0.0085 (1.2) |
| I-70: ¹H-NMR(400.6 MHz, CDCl3): |
| δ= 8.2098 (1.4); 8.2076 (1.1); 8.2056 (1.2); 8.2037 (1.4); 8.0585 (0.8); 8.0565 (0.9); 8.0538 (1.0); 8.0518 (0.8); 8.0463 (0.9); 8.0443 (0.9); 8.0417 (0.9); 8.0397 (0.8); 7.8172 (0.7); 7.8110 (0.6); 7.7980 (0.9); 7.7962 (0.9); 7.7918 (0.9); 7.7900 (0.8); 7.7771 (0.7); 7.7709 (0.7); 7.7492 (0.6); 7.7444 (0.6); 7.7309 (0.8); 7.7285 (1.1); 7.7262 (0.8); 7.7238 (1.0); 7.7103 (1.1); 7.7055 (1.0); 7.6507 (1.2); 7.6484 (1.9); 7.6461 (1.1); 7.6300 (0.8); 7.6278 (1.2); 7.6254 (0.6); 7.2617 (41.6); 7.0732 (1.0); 7.0705 (1.0); 7.0610 (1.2); 7.0583 (1.2); 7.0549 (1.2); 7.0522 (1.2); 7.0428 (1.2); 7.0401 (1.1); 6.9728 (1.0); 6.9714 (0.9); 6.9654 (1.0); 6.9518 (0.9); 6.9504 (0.9); 6.9444 (0.9); 5.3003 (2.0); 4.8918 (7.5); 4.1783 (3.4); 4.1656 (3.4); 3.7969 (16.0); 1.5643 (1.7); 1.5504 (0.8); 1.5424 (0.6); 1.5293 (1.2); 1.5161 (0.6); 1.5082 (0.7); 0.8240 (0.6); 0.8132 (1.6); 0.8079 (1.7); 0.8029 (0.8); 0.7975 (1.0); 0.7918 (1.6); 0.7867 (1.4); 0.7765 (0.8); 0.6697 (0.9); 0.6593 (1.7); 0.6560 (1.7); 0.6542 (1.6); 0.6463 (1.5); 0.6422 (1.5); 0.6301 (0.6); 0.0080 (0.7); -0.0002 (26.4); -0.0085 (0.7) |
| I-71: ¹H-NMR(400.6 MHz, CDCl3): |
| δ= 8.2101 (1.1); 8.2041 (1.2); 8.0361 (0.6); 8.0343 (0.7); 8.0315 (0.7); 8.0296 (0.7); 8.0240 (0.7); 8.0221 (0.7); 8.0194 (0.7); 8.0175 (0.6); 7.8217 (0.5); 7.8156 (0.5); 7.8024 (0.7); 7.8009 (0.7); 7.7962 (0.7); 7.7947 (0.7); 7.7815 (0.6); 7.7754 (0.5); 7.7207 (0.5); 7.7160 (0.5); 7.7023 (0.6); 7.7000 (0.8); 7.6977 (0.7); 7.6953 (0.8); 7.6817 (0.8); 7.6770 (0.7); 7.5932 (1.5); 7.5911 (0.9); 7.5726 (1.1); 7.5704 (0.6); 7.2613 (42.9); 7.2574 (0.7); 7.0382 (0.7); 7.0356 (0.7); 7.0260 (0.7); 7.0235 (0.7); 7.0199 (0.7); 7.0173 (0.6); 7.0078 (0.6); 7.0052 (0.6); 6.9609 (0.8); 6.9548 (0.8); 6.9411 (0.7); 6.9399 (0.7); 6.9338 (0.7); 6.9325 (0.7); 5.3003 (0.8); 4.8895 (7.8); 4.5046 (2.0); 4.4889 (4.0); 4.4732 (2.0); 3.6905 (16.0); 2.7120 (1.9); 2.6963 (3.8); 2.6806 (1.8); 1.5684 (0.6); 1.4835 (0.6); 1.4739 (0.5); 1.4703 (0.9); 1.4492 (0.6); 0.7779 (1.2); 0.7728 (1.2); 0.7654 (2.1); 0.7593 (1.2); 0.7518 (1.0); 0.7489 (0.5); 0.7397 (1.3); 0.7322 (1.2); 0.7278 (0.8); 0.7221 (0.6); 0.7191 (1.1); 0.7119 (1.3); 0.7063 (1.0); 0.0080 (0.7); -0.0002 (26.5); -0.0085 (0.8) |
| I-72: ¹H-NMR(400.6 MHz, d₆-DMSO): |
| δ= 12.9557 (0.7); 8.2209 (3.2); 8.2147 (3.3); 8.0566 (2.0); 8.0545 (2.2); 8.0519 (2.6); 8.0498 (2.3); 8.0445 (2.2); 8.0424 (2.5); 8.0397 (2.5); 8.0377 (2.4); 7.9728 (1.5); 7.9665 (1.4); 7.9519 (2.2); 7.9457 (2.1); 7.9319 (1.6); 7.9257 (1.5); 7.9206 (2.0); 7.9158 (1.9); 7.9021 (2.2); 7.8999 (2.4); 7.8973 (2.2); 7.8952 (2.4); 7.8815 (2.4); 7.8767 (2.3); 7.6173 (2.4); 7.6150 (4.5); 7.6128 (2.7); 7.5967 (2.2); 7.5944 (4.1); 7.5921 (2.3); 7.2479 (2.1); 7.2422 (2.1); 7.2268 (2.0); 7.2211 (2.0); 7.2198 (2.0); 7.2013 (2.3); 7.1988 (2.4); 7.1891 (2.1); 7.1867 (2.4); 7.1829 (2.3); 7.1804 (2.1); 7.1707 (2.3); 7.1682 (2.2); 4.8305 (16.0); 4.0555 (1.1); 4.0377 (3.3); 4.0199 (3.4); 4.0022 (1.1); 3.3194 (16.8); 2.6746 (0.7); 2.6700 (1.0); 2.6653 (0.7); 2.5282 (0.8); 2.5238 (3.2); 2.5191 (4.1); 2.5103 (54.2); 2.5058 (121.3); 2.5012 (172.8); 2.4966 (121.6); 2.4920 (54.2); 2.3329 (0.7); 2.3283 (1.0); 2.3236 (0.7); 1.9886 (16.0); 1.9084 (3.2); 1.5421 (0.6); 1.5288 (1.4); 1.5212 (1.4); 1.5160 (0.9); 1.5080 (2.4); 1.5045 (1.2); 1.4945 (1.4); 1.4871 (1.5); 1.4738 (0.7); 1.3551 (1.3); 1.1922 (4.7); 1.1744 (9.9); 1.1566 (4.7); 0.7032 (1.2); 0.6997 (0.6); 0.6907 (2.5); 0.6855 (3.8); 0.6825 (2.0); 0.6778 (2.7); 0.6725 (1.4); 0.6697 (2.7); 0.6640 (3.7); 0.6570 (2.8); 0.6463 (1.1); 0.6409 (2.5); 0.6333 (3.7); 0.6277 (5.6); 0.6205 (3.9); 0.6152 (3.6); 0.6023 (1.2); 0.0080 (3.5); 0.0064 (0.9); 0.0055 (1.0); 0.0047 (1.2); 0.0039 (1.6); 0.0022 (4.6); -0.0002 (119.2); -0.0026 (6.1); -0.0051 (1.7); -0.0059 (1.3); -0.0067 (1.0); -0.0085 (3.4) |
| I-73: ¹H-NMR(400.0 MHz, CDCl3): |
| δ= 8.4859 (2.0); 8.4791 (2.0); 8.3722 (2.1); 8.0363 (1.0); 8.0336 (1.0); 8.0241 (1.0); 8.0216 (1.0); 7.7896 (0.6); 7.7849 (0.6); 7.7689 (1.0); 7.7643 (1.0); 7.7504 (0.9); 7.7458 (0.8); 7.6711 (1.9); 7.6504 (1.3); 7.5296 (0.7); 7.5230 (0.8); 7.5188 (0.8); 7.5072 (0.7); 7.5006 (0.8); 7.4960 (0.6); 7.2611 (40.5); 7.1071 (0.9); 7.0951 (0.9); 7.0913 (0.9); 7.0889 (0.9); 7.0792 (0.7); 7.0766 (0.8); 5.3001 (1.0); 5.2528 (0.5); 5.2356 (1.7); 5.2181 (1.8); 5.2008 (0.5); 4.4914 (1.0); 4.4836 (1.2); 4.4756 (2.2); 4.4681 (2.1); 4.4600 (1.2); 4.4524 (1.1); 3.7263 (0.7); 3.6581 (16.0); 2.6887 (1.8); 2.6729 (3.0); 2.6573 (1.6); 1.7082 (6.5); 1.6908 (6.5); 0.0079 (0.8); -0.0002 (24.2); -0.0084 (0.8) |
| I-74: ¹H-NMR(400.6 MHz, CDCl3): |
| δ= 8.4898 (1.6); 8.4829 (1.7); 8.3764 (0.9); 8.3725 (1.6); 8.3688 (0.9); 8.0743 (0.6); 8.0722 (0.7); 8.0696 (0.8); 8.0675 (0.7); 8.0621 (0.7); 8.0601 (0.8); 8.0575 (0.8); 8.0554 (0.7); 7.7962 (0.5); 7.7915 (0.5); 7.7778 (0.6); 7.7756 (0.8); 7.7731 (0.7); 7.7709 (0.8); 7.7572 (0.8); 7.7525 (0.8); 7.6721 (0.8); 7.6698 (1.6); 7.6675 (1.0); 7.6515 (0.6); 7.6492 (1.1); 7.6468 (0.6); 7.5349 (0.6); 7.5306 (0.6); 7.5280 (0.7); 7.5237 (0.6); 7.5124 (0.6); 7.5081 (0.6); 7.5055 (0.7); 7.5012 (0.6); 7.2611 (42.0); 7.1333 (0.7); 7.1308 (0.7); 7.1212 (0.7); 7.1186 (0.7); 7.1150 (0.8); 7.1124 (0.7); 7.1028 (0.7); 7.1002 (0.7); 4.9554 (7.2); 4.4230 (0.6); 4.4050 (0.6); 4.3959 (1.3); 4.3779 (1.4); 4.3527 (1.3); 4.3386 (1.4); 4.3256 (0.6); 4.3115 (0.6); 3.7263 (2.8); 3.7131 (0.6); 3.6746 (16.0); 2.8456 (0.7); 2.8315 (0.7); 1.2065 (6.6); 1.1981 (1.5); 1.1886 (6.5); 1.1800 (1.5); 0.0080 (0.6); -0.0002 (26.9); -0.0085 (0.8) |
| I-75: ¹H-NMR(400.6 MHz, CDCl3): |
| δ= 8.4904 (1.2); 8.4836 (1.3); 8.3754 (1.4); 8.0741 (0.7); 8.0720 (0.8); 8.0694 (0.9); 8.0674 (0.8); 8.0620 (0.8); 8.0599 (0.8); 8.0573 (0.8); 8.0552 (0.8); 7.8005 (0.6); 7.7958 (0.6); 7.7821 (0.7); 7.7798 (0.9); 7.7774 (0.8); 7.7751 (0.9); 7.7615 (0.9); 7.7567 (0.9); 7.6765 (1.0); 7.6742 (1.7); 7.6719 (1.0); 7.6559 (0.8); 7.6536 (1.2); 7.6513 (0.7); 7.5370 (0.6); 7.5327 (0.7); 7.5302 (0.7); 7.5259 (0.6); 7.5191 (0.6); 7.5145 (0.6); 7.5102 (0.7); 7.5077 (0.7); 7.5033 (0.6); 7.2606 (93.3); 7.1350 (0.8); 7.1324 (0.8); 7.1228 (0.8); 7.1202 (0.8); 7.1166 (0.8); 7.1140 (0.8); 7.1045 (0.8); 7.1019 (0.7); 6.9970 (0.5); 5.3002 (0.6); 4.9523 (8.3); 4.5262 (2.0); 4.5106 (4.1); 4.4949 (2.1); 3.6861 (16.0); 2.7200 (2.0); 2.7043 (3.9); 2.6886 (1.9); 0.0079 (1.6); -0.0002 (59.7); -0.0085 (1.7) |
| I-76: ¹H-NMR(400.6 MHz, CDCl3): |
| δ= 8.4909 (1.2); 8.4841 (1.3); 8.3698 (0.8); 8.3661 (1.3); 8.3626 (0.8); 8.0628 (0.6); 8.0608 (0.7); 8.0581 (0.8); 8.0561 (0.7); 8.0507 (0.7); 8.0486 (0.8); 8.0460 (0.8); 8.0439 (0.7); 7.7910 (0.5); 7.7863 (0.5); 7.7726 (0.6); 7.7703 (0.8); 7.7679 (0.6); 7.7656 (0.8); 7.7520 (0.8); 7.7473 (0.8); 7.6730 (0.8); 7.6707 (1.5); 7.6684 (0.9); 7.6524 (0.6); 7.6501 (1.0); 7.6477 (0.6); 7.5277 (0.6); 7.5234 (0.6); 7.5209 (0.7); 7.5165 (0.6); 7.5053 (0.6); 7.5009 (0.7); 7.4983 (0.6); 7.4940 (0.6); 7.2625 (15.4); 7.1263 (0.7); 7.1237 (0.7); 7.1141 (0.7); 7.1115 (0.7); 7.1079 (0.7); 7.1053 (0.7); 7.0958 (0.7); 7.0932 (0.6); 4.9541 (6.9); 4.4218 (0.6); 4.4039 (0.6); 4.3947 (1.3); 4.3768 (1.3); 4.3508 (1.3); 4.3367 (1.3); 4.3237 (0.6); 4.3096 (0.6); 3.6748 (16.0); 2.8441 (0.6); 2.8300 (0.6); 1.2054 (6.3); 1.1875 (6.2); -0.0002 (9.2) |
| I-77: ¹H-NMR(400.6 MHz, CDCl3): |
| δ= 8.4907 (1.0); 8.4840 (1.0); 8.3677 (1.2); 8.0635 (0.7); 8.0614 (0.8); 8.0588 (0.8); 8.0567 (0.8); 8.0514 (0.7); 8.0493 (0.8); 8.0467 (0.8); 8.0446 (0.7); 7.7950 (0.6); 7.7903 (0.6); 7.7766 (0.7); 7.7744 (0.9); 7.7719 (0.7); 7.7697 (0.9); 7.7560 (0.9); 7.7512 (0.9); 7.6765 (0.9); 7.6742 (1.7); 7.6719 (1.0); 7.6559 (0.7); 7.6536 (1.2); 7.6512 (0.7); 7.5262 (0.6); 7.5219 (0.7); 7.5195 (0.8); 7.5150 (0.6); 7.5038 (0.7); 7.4995 (0.7); 7.4969 (0.7); 7.4926 (0.6); 7.2614 (33.9); 7.1278 (0.8); 7.1252 (0.8); 7.1157 (0.8); 7.1131 (0.8); 7.1095 (0.8); 7.1068 (0.8); 7.0973 (0.8); 7.0947 (0.7); 4.9507 (8.0); 4.5244 (1.9); 4.5087 (4.0); 4.4930 (2.0); 3.6860 (16.0); 2.7187 (1.9); 2.7031 (3.8); 2.6874 (1.8); 0.0080 (0.6); -0.0002 (21.3); -0.0085 (0.6) |
| I-78: ¹H-NMR(400.0 MHz, CDCl3): |
| δ= 8.2065 (2.5); 8.2005 (2.5); 8.0335 (1.5); 8.0305 (1.6); 8.0213 (1.5); 8.0185 (1.5); 7.8191 (1.2); 7.8130 (1.1); 7.7984 (1.6); 7.7923 (1.5); 7.7788 (1.2); 7.7727 (1.1); 7.7079 (1.0); 7.7031 (1.0); 7.6894 (1.3); 7.6871 (1.7); 7.6825 (1.5); 7.6688 (1.5); 7.6641 (1.4); 7.5863 (3.1); 7.5656 (2.1); 7.2612 (36.6); 7.0326 (1.4); 7.0301 (1.4); 7.0205 (1.4); 7.0179 (1.4); 7.0144 (1.4); 7.0119 (1.3); 7.0022 (1.3); 6.9997 (1.2); 6.9599 (1.7); 6.9529 (1.6); 6.9389 (1.6); 6.9316 (1.5); 4.8882 (16.0); 4.3012 (2.1); 4.2834 (6.4); 4.2655 (6.4); 4.2477 (2.1); 1.5788 (0.8); 1.4995 (1.0); 1.4918 (1.1); 1.4787 (1.7); 1.4705 (0.6); 1.4652 (1.1); 1.4575 (1.1); 1.4442 (0.6); 1.3262 (7.4); 1.3084 (14.9); 1.2905 (7.2); 0.8055 (0.8); 0.8022 (0.6); 0.7921 (2.6); 0.7869 (3.0); 0.7792 (3.7); 0.7745 (3.0); 0.7659 (1.7); 0.7560 (0.7); 0.7481 (1.0); 0.7442 (1.9); 0.7356 (2.7); 0.7314 (2.0); 0.7232 (1.9); 0.7152 (2.5); 0.7098 (1.9); 0.7030 (0.7); 0.6969 (0.7); 0.0080 (0.9); -0.0002 (22.0); -0.0085 (0.7) |
| I-79: ¹H-NMR(400.6 MHz, d₆-DMSO): |
| δ= 13.0812 (1.3); 8.6574 (7.1); 8.6503 (7.2); 8.4145 (3.8); 8.4103 (7.1); 8.4061 (3.5); 8.0895 (2.5); 8.0875 (3.0); 8.0849 (3.4); 8.0828 (3.1); 8.0774 (2.8); 8.0754 (3.3); 8.0728 (3.2); 8.0707 (3.0); 8.0018 (2.6); 7.9971 (2.3); 7.9833 (3.1); 7.9812 (3.2); 7.9786 (2.8); 7.9765 (3.0); 7.9627 (3.2); 7.9579 (2.8); 7.8770 (2.4); 7.8727 (2.6); 7.8700 (2.6); 7.8657 (2.3); 7.8529 (2.6); 7.8486 (2.8); 7.8459 (2.4); 7.8417 (2.3); 7.6726 (3.1); 7.6704 (6.0); 7.6682 (3.5); 7.6520 (3.0); 7.6498 (5.4); 7.6475 (3.0); 7.2915 (3.0); 7.2890 (3.2); 7.2794 (2.7); 7.2769 (3.1); 7.2730 (3.0); 7.2705 (2.7); 7.2608 (2.9); 7.2584 (2.8); 5.1724 (1.2); 5.1552 (5.6); 5.1378 (5.6); 5.1203 (1.2); 4.0376 (1.1); 4.0198 (1.1); 3.3519 (0.7); 3.3195 (200.2); 2.6791 (1.0); 2.6743 (2.2); 2.6698 (3.1); 2.6651 (2.3); 2.6605 (1.2); 2.5404 (2.0); 2.5347 (1.6); 2.5317 (1.7); 2.5235 (8.1); 2.5189 (10.6); 2.5101 (161.1); 2.5055 (355.7); 2.5009 (499.5); 2.4964 (345.9); 2.4918 (155.2); 2.3326 (2.0); 2.3280 (2.9); 2.3233 (2.0); 2.3189 (0.9); 1.9884 (5.2); 1.9082 (0.5); 1.6011 (16.0); 1.5837 (15.8); 1.3549 (2.2); 1.2359 (0.9); 1.1921 (1.6); 1.1744 (3.3); 1.1566 (1.6); 0.1456 (1.0); 0.0111 (0.5); 0.0103 (0.6); 0.0080 (8.9); 0.0064 (1.9); 0.0056 (2.0); 0.0048 (2.4); 0.0039 (3.1); -0.0002 (326.8); -0.0050 (5.9); -0.0058 (4.6); -0.0067 (3.9); -0.0085 (10.1); -0.0114 (1.9); -0.0122 (1.4); -0.0131 (1.2); -0.0138 (1.2); -0.0146 (1.2); -0.0154 (1.0); -0.0162 (0.8); - 0.0170 (0.9); -0.0178 (0.7); -0.0186 (0.7); -0.0194 (0.7); -0.0210 (0.8); -0.0281 (1.0); -0.1494 (1.0) |
| I-80: ¹H-NMR(400.6 MHz, d₆-DMSO): |
| δ= 12.6748 (1.4); 8.4653 (2.8); 8.4468 (2.9); 8.2494 (4.0); 8.2451 (3.1); 8.2433 (4.2); 8.0978 (2.6); 8.0958 (2.9); 8.0932 (3.4); 8.0911 (3.0); 8.0857 (2.8); 8.0837 (3.2); 8.0810 (3.2); 8.0789 (3.1); 8.0221 (1.9); 8.0158 (1.8); 8.0024 (2.4); 8.0009 (2.6); 7.9962 (2.4); 7.9947 (2.6); 7.9813 (2.1); 7.9747 (4.1); 7.9699 (2.4); 7.9561 (2.8); 7.9539 (3.2); 7.9514 (2.7); 7.9492 (3.1); 7.9355 (3.2); 7.9307 (3.0); 7.7671 (3.1); 7.7648 (5.8); 7.7626 (3.4); 7.7464 (2.8); 7.7442 (5.0); 7.7419 (2.8); 7.3041 (2.7); 7.2985 (2.7); 7.2972 (2.5); 7.2832 (5.4); 7.2808 (3.9); 7.2773 (2.8); 7.2759 (2.7); 7.2711 (2.8); 7.2686 (3.0); 7.2648 (3.0); 7.2623 (2.7); 7.2527 (3.0); 7.2501 (2.9); 4.8393 (16.0); 4.3306 (2.6); 4.3123 (4.1); 4.2940 (2.6); 4.2758 (0.5); 3.6216 (0.9); 3.6177 (5.2); 3.6155 (2.9); 3.6115 (2.7); 3.6093 (2.1); 3.6074 (3.6); 3.6028 (3.9); 3.6011 (12.0); 3.5989 (4.3); 3.5950 (3.8); 3.5930 (2.1); 3.5908 (2.7); 3.5867 (2.8); 3.5845 (5.4); 3.5806 (1.0); 3.3212 (11.7); 2.6751 (0.6); 2.6705 (0.8); 2.6658 (0.6); 2.5242 (2.1); 2.5196 (2.8); 2.5108 (40.1); 2.5062 (91.1); 2.5016 (129.2); 2.4970 (90.8); 2.4924 (40.8); 2.3333 (0.5); 2.3287 (0.8); 2.3241 (0.5); 2.1829 (0.8); 1.9087 (0.7); 1.7808 (0.8); 1.7761 (5.3); 1.7685 (4.2); 1.7640 (2.6); 1.7595 (15.2); 1.7548 (2.7); 1.7505 (4.2); 1.7429 (5.2); 1.7384 (0.8); 1.3554 (7.3); 1.3262 (15.9); 1.3080 (15.8); 0.1457 (0.6); 0.0079 (6.1); 0.0062 (1.3); 0.0054 (1.5); 0.0045 (1.9); - 0.0002 (240.5); -0.0028 (10.9); -0.0052 (2.8); -0.0061 (2.2); -0.0069 (1.9); -0.0085 (6.9); -0.0108 (0.9); -0.0117 (0.8); -0.0124 (0.8); -0.0132 (0.6); -0.0140 (0.6); -0.1495 (0.7) |
| I-81: ¹H-NMR(400.0 MHz, d₆-DMSO): |
| δ= 13.1448 (1.5); 8.6646 (5.2); 8.6577 (5.2); 8.4348 (2.9); 8.4308 (5.3); 8.4269 (2.7); 8.1063 (2.2); 8.1036 (2.4); 8.1017 (2.2); 8.0941 (2.4); 8.0915 (2.4); 8.0896 (2.1); 8.0078 (1.7); 8.0031 (1.6); 7.9891 (2.2); 7.9872 (2.3); 7.9845 (2.1); 7.9826 (2.1); 7.9686 (2.1); 7.9639 (1.9); 7.8960 (1.8); 7.8918 (1.9); 7.8890 (1.9); 7.8848 (1.7); 7.8719 (1.9); 7.8676 (2.0); 7.8650 (1.8); 7.8607 (1.6); 7.7018 (4.2); 7.6812 (3.7); 7.3075 (2.1); 7.3051 (2.1); 7.2954 (1.9); 7.2929 (2.1); 7.2890 (2.1); 7.2866 (2.0); 7.2768 (2.0); 7.2744 (1.9); 4.9418 (16.0); 4.0380 (0.8); 4.0201 (0.9); 3.3191 (15.8); 2.6705 (0.8); 2.5239 (1.8); 2.5191 (2.7); 2.5104 (43.0); 2.5059 (93.5); 2.5013 (129.9); 2.4967 (92.1); 2.4922 (41.8); 2.3283 (0.7); 1.9885 (3.7); 1.1923 (1.1); 1.1745 (2.1); 1.1567 (1.1); 0.0080 (1.8); -0.0002 (61.1); -0.0086 (1.9) |
| I-82: ¹H-NMR(400.6 MHz, CDCl3): |
| δ= 8.5045 (1.9); 8.4976 (1.9); 8.3531 (1.0); 8.3493 (1.8); 8.3455 (1.0); 8.1167 (0.7); 8.1146 (0.8); 8.1121 (0.8); 8.1100 (0.8); 8.1046 (0.7); 8.1024 (0.8); 8.1001 (0.8); 8.0979 (0.8); 7.8481 (0.5); 7.8455 (1.0); 7.8437 (0.6); 7.8409 (1.0); 7.8276 (1.1); 7.8230 (1.1); 7.8043 (1.0); 7.8015 (1.7); 7.7991 (1.2); 7.7838 (0.6); 7.7813 (0.7); 7.5112 (0.7); 7.5068 (0.8); 7.5043 (0.8); 7.4999 (0.7); 7.4888 (0.7); 7.4845 (0.8); 7.4820 (0.8); 7.4776 (0.7); 7.2645 (5.7); 7.2098 (0.8); 7.2067 (0.8); 7.1977 (0.8); 7.1945 (0.8); 7.1919 (0.8); 7.1888 (0.8); 7.1798 (0.8); 7.1766 (0.7); 5.3006 (3.1); 3.8261 (7.5); 3.5681 (0.9); 3.5552 (16.0); 3.5380 (2.0); 3.5226 (0.9); 2.5160 (1.6); 2.5007 (2.4); 2.4860 (1.6); -0.0002 (8.4) |
| I-83: ¹H-NMR(400.6 MHz, CDCl3): |
| δ= 8.0422 (0.6); 8.0403 (0.6); 8.0377 (0.7); 8.0357 (0.6); 8.0301 (0.6); 8.0282 (0.7); 8.0256 (0.7); 8.0236 (0.6); 7.7573 (0.6); 7.7551 (0.7); 7.7526 (0.6); 7.7504 (0.7); 7.7367 (0.7); 7.7320 (0.6); 7.6417 (0.8); 7.6396 (1.3); 7.6374 (0.8); 7.6211 (0.6); 7.6190 (1.0); 7.6168 (0.6); 7.2612 (30.9); 7.1092 (0.6); 7.1068 (0.6); 7.0971 (0.6); 7.0946 (0.6); 7.0909 (0.6); 7.0884 (0.6); 7.0788 (0.6); 7.0762 (0.6); 5.3001 (0.8); 5.2732 (1.3); 5.2558 (1.3); 3.8017 (16.0); 1.7227 (5.8); 1.7052 (5.7); 0.0080 (0.5); -0.0002 (19.0); - 0.0085 (0.6) |
| I-84: ¹H-NMR(400.0 MHz, d₆-DMSO): |
| δ= 13.1203 (0.9); 8.6592 (2.8); 8.6523 (2.8); 8.4198 (1.5); 8.4157 (2.9); 8.4116 (1.4); 8.0955 (1.1); 8.0927 (1.2); 8.0832 (1.2); 8.0806 (1.2); 8.0010 (0.9); 7.9963 (0.8); 7.9804 (1.2); 7.9759 (1.2); 7.9619 (1.0); 7.9571 (1.0); 7.8808 (0.9); 7.8766 (1.0); 7.8740 (1.0); 7.8697 (0.9); 7.8568 (1.0); 7.8525 (1.1); 7.8499 (1.0); 7.8456 (0.9); 7.6977 (2.2); 7.6771 (2.0); 7.2981 (1.1); 7.2958 (1.1); 7.2860 (1.0); 7.2836 (1.1); 7.2797 (1.1); 7.2773 (1.0); 7.2675 (1.1); 7.2651 (1.0); 4.9299 (8.4); 4.0377 (0.6); 4.0199 (0.6); 3.3182 (16.0); 2.6700 (0.6); 2.5236 (1.2); 2.5189 (1.7); 2.5101 (30.8); 2.5056 (68.0); 2.5010 (95.4); 2.4964 (67.4); 2.4919 (30.4); 2.3278 (0.6); 1.9884 (2.7); 1.1922 (0.7); 1.1744 (1.5); 1.1566 (0.7); 0.0080 (1.4); -0.0002 (51.8); -0.0086 (1.5) |
| I-85: ¹H-NMR(400.6 MHz, CDCl3): |
| δ= 8.4868 (3.2); 8.4799 (3.3); 8.3688 (1.7); 8.3650 (3.2); 8.3610 (1.7); 8.0758 (1.2); 8.0737 (1.3); 8.0711 (1.4); 8.0690 (1.4); 8.0636 (1.3); 8.0616 (1.4); 8.0589 (1.4); 8.0569 (1.4); 7.7869 (1.1); 7.7822 (1.1); 7.7685 (1.2); 7.7663 (1.6); 7.7638 (1.3); 7.7616 (1.6); 7.7478 (1.6); 7.7431 (1.6); 7.6592 (1.6); 7.6568 (3.0); 7.6546 (1.9); 7.6386 (1.2); 7.6362 (2.1); 7.6339 (1.3); 7.5221 (1.3); 7.5178 (1.3); 7.5153 (1.4); 7.5109 (1.3); 7.4996 (1.3); 7.4953 (1.4); 7.4927 (1.3); 7.4884 (1.2); 7.2625 (8.6); 7.1299 (1.4); 7.1273 (1.5); 7.1178 (1.4); 7.1152 (1.4); 7.1115 (1.4); 7.1089 (1.4); 7.0994 (1.4); 7.0968 (1.4); 5.2998 (5.8); 4.9501 (15.3); 4.3264 (1.9); 4.3086 (6.0); 4.2908 (6.1); 4.2730 (2.0); 1.3350 (7.6); 1.3172 (16.0); 1.2994 (7.5); -0.0002 (12.4) |
| I-86: ¹H-NMR(400.6 MHz, CDCl3): |
| δ= 8.4890 (3.0); 8.4821 (3.0); 8.3634 (1.6); 8.3597 (3.0); 8.3560 (1.6); 8.0641 (1.2); 8.0620 (1.3); 8.0594 (1.4); 8.0573 (1.3); 8.0519 (1.3); 8.0499 (1.4); 8.0473 (1.4); 8.0452 (1.3); 7.7815 (1.0); 7.7767 (1.0); 7.7630 (1.3); 7.7608 (1.6); 7.7583 (1.3); 7.7561 (1.5); 7.7424 (1.6); 7.7377 (1.5); 7.6598 (1.7); 7.6574 (2.9); 7.6551 (1.7); 7.6391 (1.3); 7.6368 (2.1); 7.6344 (1.2); 7.5194 (1.3); 7.5151 (1.3); 7.5125 (1.3); 7.5082 (1.2); 7.4969 (1.2); 7.4926 (1.3); 7.4900 (1.3); 7.4857 (1.2); 7.2615 (14.4); 7.1232 (1.4); 7.1206 (1.4); 7.1111 (1.4); 7.1084 (1.4); 7.1048 (1.4); 7.1022 (1.3); 7.0927 (1.3); 7.0901 (1.3); 4.9487 (14.7); 4.3246 (1.8); 4.3068 (5.8); 4.2890 (5.9); 4.2712 (1.9); 1.5849 (0.6); 1.3341 (7.6); 1.3163 (16.0); 1.2985 (7.4); 1.2649 (0.8); 0.8819 (1.5); 0.8643 (0.6); 0.0080 (0.6); -0.0002 (22.2); -0.0085 (0.6) |
| I-87: ¹H-NMR(400.6 MHz, CDCl3): |
| δ= 8.2227 (1.1); 8.2205 (0.9); 8.2184 (1.0); 8.2165 (1.1); 8.0974 (0.7); 8.0954 (0.8); 8.0927 (0.9); 8.0907 (0.7); 8.0853 (0.8); 8.0832 (0.8); 8.0806 (0.8); 8.0786 (0.7); 7.8710 (0.6); 7.8648 (0.6); 7.8521 (0.7); 7.8498 (0.7); 7.8459 (0.7); 7.8436 (0.7); 7.8310 (0.6); 7.8248 (0.6); 7.8107 (0.6); 7.8060 (0.5); 7.7923 (0.7); 7.7901 (0.9); 7.7876 (0.7); 7.7854 (0.8); 7.7718 (0.9); 7.7671 (0.9); 7.7070 (1.1); 7.7046 (1.7); 7.7023 (1.0); 7.6863 (0.8); 7.6840 (1.1); 7.6817 (0.6); 7.2617 (11.1); 7.1530 (0.8); 7.1504 (0.8); 7.1408 (0.9); 7.1381 (1.0); 7.1346 (1.1); 7.1320 (1.0); 7.1225 (1.0); 7.1198 (1.0); 7.0129 (0.8); 7.0114 (0.8); 7.0055 (0.9); 7.0040 (0.8); 6.9917 (0.8); 6.9902 (0.8); 6.9844 (0.8); 6.9829 (0.7); 5.3003 (1.2); 4.9085 (6.8); 4.7300 (0.8); 4.7119 (1.1); 4.6937 (0.8); 3.7782 (16.0); 1.5106 (6.3); 1.4927 (6.3); -0.0002 (16.0) |
| I-88: ¹H-NMR(400.6 MHz, CDCl3): |
| δ= 8.2170 (1.2); 8.2148 (0.9); 8.2127 (1.0); 8.2108 (1.2); 8.0814 (0.7); 8.0795 (0.7); 8.0768 (0.8); 8.0748 (0.7); 8.0693 (0.7); 8.0673 (0.7); 8.0646 (0.8); 8.0627 (0.6); 7.8706 (0.6); 7.8644 (0.6); 7.8517 (0.8); 7.8495 (0.8); 7.8455 (0.8); 7.8433 (0.7); 7.8306 (0.7); 7.8244 (0.6); 7.7887 (0.6); 7.7839 (0.6); 7.7702 (0.7); 7.7680 (0.9); 7.7655 (0.8); 7.7633 (0.9); 7.7496 (0.9); 7.7449 (0.8); 7.6603 (1.0); 7.6581 (1.5); 7.6559 (0.9); 7.6396 (0.7); 7.6375 (1.1); 7.6353 (0.6); 7.2625 (6.7); 7.1273 (0.7); 7.1248 (0.7); 7.1151 (0.7); 7.1126 (0.7); 7.1089 (0.8); 7.1064 (0.7); 7.0968 (0.7); 7.0942 (0.6); 7.0000 (0.8); 6.9987 (0.8); 6.9927 (0.9); 6.9913 (0.8); 6.9789 (0.8); 6.9774 (0.8); 6.9715 (0.8); 6.9701 (0.7); 5.3003 (1.5); 4.9496 (8.3); 4.5246 (2.0); 4.5090 (4.2); 4.4933 (2.1); 3.7260 (1.0); 3.6846 (16.0); 2.7198 (2.0); 2.7041 (4.0); 2.6884 (1.9); 1.5699 (0.6); -0.0002 (9.3) |
| I-02: ¹H-NMR(400.0 MHz, CDCl3): |
| δ= 8.2035 (2.2); 8.1974 (2.2); 8.0728 (1.3); 8.0709 (1.5); 8.0682 (1.5); 8.0662 (1.4); 8.0606 (1.4); 8.0587 (1.6); 8.0560 (1.5); 8.0540 (1.4); 7.8625 (1.1); 7.8563 (1.1); 7.8436 (1.4); 7.8414 (1.4); 7.8374 (1.3); 7.8353 (1.4); 7.8225 (1.2); 7.8163 (1.2); 7.7707 (1.1); 7.7660 (1.0); 7.7522 (1.3); 7.7501 (1.7); 7.7476 (1.3); 7.7454 (1.6); 7.7316 (1.6); 7.7269 (1.5); 7.6448 (1.8); 7.6426 (3.1); 7.6404 (1.9); 7.6242 (1.4); 7.6219 (2.3); 7.6197 (1.3); 7.2628 (17.0); 7.1173 (1.4); 7.1147 (1.4); 7.1051 (1.4); 7.1025 (1.5); 7.0989 (1.4); 7.0963 (1.4); 7.0867 (1.4); 7.0842 (1.3); 6.9960 (1.6); 6.9899 (1.6); 6.9886 (1.6); 6.9748 (1.5); 6.9687 (1.5); 6.9675 (1.5); 5.2996 (1.1); 4.9460 (16.0); 4.3225 (2.0); 4.3047 (6.3); 4.2868 (6.4); 4.2690 (2.1); 1.3331 (7.5); 1.3152 (15.4); 1.2974 (7.3); -0.0002 (10.3) |
| I-02: ¹H-NMR(400.0 MHz, CDCl3): |
| δ= 8.2031 (3.4); 8.0687 (2.3); 8.0591 (2.3); 7.8629 (1.1); 7.8569 (1.1); 7.8435 (2.0); 7.8376 (2.0); 7.8228 (1.2); 7.8169 (1.2); 7.7706 (1.1); 7.7497 (2.3); 7.7313 (1.6); 7.7268 (1.6); 7.6421 (3.7); 7.6215 (2.7); 7.2605 (79.7); 7.1172 (1.8); 7.1044 (2.0); 7.0866 (1.8); 6.9971 (2.4); 6.9900 (2.2); 6.9761 (2.1); 6.9688 (2.0); 4.9720 (0.7); 4.9461 (16.0); 4.3231 (2.0); 4.3052 (6.0); 4.2875 (6.2); 4.2696 (2.1); 4.2455 (0.6); 4.2325 (1.2); 2.8046 (0.5); 1.5504 (2.4); 1.3335 (6.9); 1.3156 (13.8); 1.2978 (7.1); 1.2847 (1.1); 1.2755 (1.0); 1.2551 (0.9); -0.0002 (55.3) |
| I-02: ¹H-NMR(400.0 MHz, CDCl₃): |
| δ= 8.2030 (2.2); 8.2011 (1.7); 8.1968 (2.1); 8.0723 (1.3); 8.0702 (1.4); 8.0676 (1.5); 8.0655 (1.4); 8.0601 (1.4); 8.0581 (1.5); 8.0555 (1.4); 8.0534 (1.3); 7.8619 (1.2); 7.8557 (1.1); 7.8430 (1.4); 7.8408 (1.5); 7.8368 (1.4); 7.8347 (1.4); 7.8219 (1.2); 7.8157 (1.2); 7.7695 (1.1); 7.7648 (1.1); 7.7511 (1.4); 7.7488 (1.7); 7.7464 (1.3); 7.7441 (1.6); 7.7304 (1.7); 7.7257 (1.6); 7.6446 (1.8); 7.6423 (3.2); 7.6400 (1.8); 7.6239 (1.4); 7.6216 (2.3); 7.6193 (1.2); 7.2607 (46.4); 7.1161 (1.5); 7.1135 (1.5); 7.1039 (1.5); 7.1013 (1.5); 7.0977 (1.5); 7.0951 (1.4); 7.0856 (1.4); 7.0830 (1.3); 6.9961 (1.7); 6.9946 (1.6); 6.9886 (1.6); 6.9872 (1.5); 6.9748 (1.4); 6.9734 (1.5); 6.9675 (1.5); 6.9661 (1.4); 4.9706 (0.7); 4.9451 (15.9); 4.3224 (2.0); 4.3046 (6.2); 4.2931 (0.5); 4.2867 (6.2); 4.2689 (2.1); 4.2609 (0.5); 4.2568 (0.6); 4.2541 (0.6); 4.2430 (1.5); 4.2314 (4.0); 4.2252 (1.5); 4.2072 (0.5); 3.9441 (0.6); 2.8027 (1.0); 1.5510 (5.8); 1.3329 (8.0); 1.3284 (0.7); 1.3150 (16.0); 1.3107 (3.1); 1.3055 (0.6); 1.2972 (8.2); 1.2929 (4.6); 1.2837 (1.0); 1.2803 (0.6); 1.2750 (2.1); 1.2656 (0.5); 1.2546 (0.5); 0.0079 (1.0); -0.0002 (31.2); -0.0085 (0.8) |
| I-02: ¹H-NMR(400.0 MHz, CDCl3): |
| δ= 8.2030 (2.3); 8.1969 (2.4); 8.0713 (1.5); 8.0686 (1.6); 8.0666 (1.5); 8.0610 (1.4); 8.0591 (1.6); 8.0564 (1.6); 8.0545 (1.4); 7.8627 (1.1); 7.8565 (1.1); 7.8437 (1.3); 7.8417 (1.5); 7.8376 (1.3); 7.8355 (1.4); 7.8227 (1.2); 7.8165 (1.2); 7.7708 (1.0); 7.7660 (1.0); 7.7522 (1.3); 7.7501 (1.7); 7.7476 (1.4); 7.7454 (1.6); 7.7317 (1.6); 7.7269 (1.5); 7.6421 (3.2); 7.6400 (2.0); 7.6215 (2.3); 7.6193 (1.4); 7.2617 (20.9); 7.1176 (1.4); 7.1151 (1.4); 7.1054 (1.4); 7.1029 (1.5); 7.0992 (1.4); 7.0966 (1.4); 7.0870 (1.3); 7.0845 (1.3); 6.9962 (1.6); 6.9889 (1.6); 6.9750 (1.5); 6.9677 (1.5); 4.9459 (16.0); 4.3228 (2.0); 4.3049 (6.2); 4.2871 (6.3); 4.2693 (2.1); 1.5631 (9.8); 1.3333 (7.4); 1.3155 (15.1); 1.2977 (7.2); 0.0697 (0.7); -0.0002 (17.4); -0.0085 (0.5) |
| I-02: ¹H-NMR(400.0 MHz, CDCl3_5mm): |
| δ= 8.2030 (1.7); 8.1969 (1.7); 8.0734 (1.0); 8.0715 (1.1); 8.0688 (1.2); 8.0670 (1.0); 8.0613 (1.0); 8.0594 (1.1); 8.0567 (1.1); 8.0548 (1.0); 7.8629 (0.8); 7.8567 (0.8); 7.8438 (1.0); 7.8418 (1.0); 7.8377 (1.0); 7.8357 (1.0); 7.8229 (0.8); 7.8167 (0.8); 7.7708 (0.7); 7.7661 (0.7); 7.7522 (1.0); 7.7501 (1.2); 7.7476 (1.0); 7.7455 (1.1); 7.7317 (1.1); 7.7270 (1.0); 7.6419 (2.2); 7.6213 (1.5); 7.2611 (13.8); 7.1177 (1.0); 7.1152 (1.0); 7.1056 (1.0); 7.1030 (1.0); 7.0994 (1.0); 7.0969 (0.9); 7.0872 (0.9); 7.0847 (0.9); 6.9973 (1.2); 6.9901 (1.2); 6.9763 (1.1); 6.9689 (1.1); 4.9460 (10.9); 4.3230 (1.4); 4.3051 (4.2); 4.2873 (4.3); 4.2695 (1.4); 1.5528 (16.0); 1.3335 (5.0); 1.3157 (10.1); 1.2978 (4.8); 0.0079 (0.8); -0.0002 (21.2); -0.0085 (0.6) |
| I-02: ¹H-NMR(400.0 MHz, CDCl3_5mm): |
| δ= 8.2035 (2.5); 8.2019 (2.2); 8.1994 (2.2); 8.1975 (2.6); 8.0730 (1.5); 8.0711 (1.7); 8.0684 (1.8); 8.0665 (1.6); 8.0609 (1.6); 8.0589 (1.8); 8.0563 (1.8); 8.0544 (1.6); 7.8628 (1.2); 7.8566 (1.2); 7.8438 (1.5); 7.8418 (1.6); 7.8377 (1.5); 7.8356 (1.5); 7.8227 (1.2); 7.8166 (1.2); 7.7718 (1.1); 7.7671 (1.1); 7.7533 (1.4); 7.7512 (1.9); 7.7487 (1.6); 7.7465 (1.8); 7.7327 (1.6); 7.7280 (1.6); 7.6445 (2.0); 7.6424 (3.3); 7.6403 (2.2); 7.6239 (1.5); 7.6218 (2.4); 7.6197 (1.5); 7.2660 (4.0); 7.1184 (1.5); 7.1159 (1.6); 7.1062 (1.5); 7.1037 (1.6); 7.1000 (1.6); 7.0975 (1.5); 7.0879 (1.4); 7.0853 (1.4); 6.9970 (1.8); 6.9909 (1.7); 6.9897 (1.7); 6.9770 (1.6); 6.9759 (1.7); 6.9698 (1.6); 6.9685 (1.6); 4.9465 (16.0); 4.3226 (2.0); 4.3048 (6.3); 4.2869 (6.4); 4.2691 (2.1); 1.6396 (0.7); 1.3331 (7.5); 1.3153 (15.1); 1.2975 (7.3); -0.0002 (3.8) |
| I-02: ¹H-NMR(599.6 MHz, CDCl3): |
| δ= 8.2015 (9.8); 8.1979 (9.9); 8.0682 (6.6); 8.0665 (6.7); 8.0602 (6.6); 8.0585 (6.5); 8.0180 (0.5); 7.8543 (3.3); 7.8502 (3.4); 7.8405 (5.7); 7.8366 (5.3); 7.8276 (3.5); 7.8236 (3.3); 7.7621 (3.5); 7.7590 (3.5); 7.7483 (6.7); 7.7468 (6.5); 7.7360 (4.4); 7.7329 (4.4); 7.6389 (11.0); 7.6251 (8.6); 7.2618 (32.2); 7.1099 (5.4); 7.1017 (5.8); 7.0990 (5.5); 7.0977 (5.4); 7.0895 (4.9); 6.9907 (6.1); 6.9860 (6.0); 6.9766 (5.8); 6.9719 (5.6); 4.9453 (50.0); 4.3136 (6.4); 4.3017 (19.2); 4.2898 (19.3); 4.2779 (6.4); 2.9562 (3.2); 2.8836 (3.0); 2.7714 (1.3); 1.5695 (41.5); 1.3271 (21.2); 1.3152 (41.9); 1.3033 (20.7); 1.2924 (0.5); 1.2540 (0.5); 1.2195 (0.4); 1.2077 (0.6); -0.0001 (31.3) |
| I-02: ¹H-NMR(400.6 MHz, CDCl3): |
| δ= 8.2031 (2.1); 8.2010 (1.7); 8.1988 (1.8); 8.1969 (2.2); 8.0736 (1.4); 8.0716 (1.4); 8.0689 (1.6); 8.0670 (1.3); 8.0615 (1.5); 8.0595 (1.5); 8.0568 (1.5); 8.0549 (1.3); 7.8629 (1.2); 7.8568 (1.1); 7.8440 (1.4); 7.8419 (1.4); 7.8378 (1.4); 7.8357 (1.4); 7.8229 (1.2); 7.8168 (1.2); 7.7709 (1.1); 7.7662 (1.1); 7.7524 (1.4); 7.7502 (1.7); 7.7477 (1.4); 7.7455 (1.6); 7.7319 (1.6); 7.7271 (1.6); 7.6441 (1.9); 7.6419 (3.1); 7.6396 (1.8); 7.6235 (1.4); 7.6213 (2.2); 7.6190 (1.2); 7.2612 (21.0); 7.1178 (1.5); 7.1153 (1.4); 7.1057 (1.4); 7.1031 (1.4); 7.0995 (1.5); 7.0969 (1.4); 7.0873 (1.4); 7.0848 (1.3); 6.9977 (1.7); 6.9964 (1.5); 6.9904 (1.6); 6.9890 (1.5); 6.9766 (1.5); 6.9752 (1.4); 6.9692 (1.6); 6.9678 (1.4); 4.9462 (16.0); 4.3230 (2.0); 4.3051 (6.2); 4.2873 (6.3); 4.2695 (2.1); 1.5570 (3.7); 1.3335 (7.6); 1.3157 (15.6); 1.2979 (7.5); 0.0079 (0.8); -0.0002 (28.9); -0.0085 (1.0) |
| I-02: ¹H-NMR(400.0 MHz, CDCl3): |
| δ= 8.2031 (2.3); 8.1969 (2.3); 8.0711 (1.2); 8.0684 (1.3); 8.0588 (1.3); 8.0564 (1.2); 7.8625 (1.2); 7.8564 (1.2); 7.8436 (1.4); 7.8415 (1.6); 7.8374 (1.4); 7.8352 (1.4); 7.8225 (1.2); 7.8163 (1.3); 7.7702 (1.1); 7.7655 (1.1); 7.7518 (1.3); 7.7496 (1.7); 7.7471 (1.4); 7.7448 (1.6); 7.7311 (1.6); 7.7264 (1.6); 7.6423 (2.4); 7.6216 (1.8); 7.2604 (45.3); 7.1170 (1.2); 7.1146 (1.3); 7.1049 (1.2); 7.1024 (1.3); 7.0987 (1.3); 7.0962 (1.2); 7.0866 (1.1); 7.0841 (1.1); 6.9958 (1.7); 6.9896 (1.6); 6.9882 (1.6); 6.9758 (1.5); 6.9743 (1.6); 6.9683 (1.5); 4.9707 (0.7); 4.9457 (15.7); 4.3227 (2.0); 4.3049 (6.3); 4.2870 (6.4); 4.2692 (2.1); 4.2322 (0.5); 1.5533 (0.9); 1.3332 (7.9); 1.3154 (16.0); 1.3058 (0.8); 1.2976 (7.7); 1.2933 (1.5); 1.2844 (0.6); 1.2809 (0.6); 1.2753 (0.6); 1.2540 (0.5); 0.0079 (1.6); -0.0002 (53.8); -0.0085 (1.6) |
| I-02: ¹H-NMR(400.0 MHz, CDCl3): |
| δ= 8.2037 (1.8); 8.1977 (1.8); 8.0702 (0.8); 8.0617 (0.8); 7.8632 (1.1); 7.8570 (1.1); 7.8443 (1.3); 7.8421 (1.4); 7.8381 (1.3); 7.8359 (1.3); 7.8231 (1.2); 7.8169 (1.2); 7.7710 (0.9); 7.7664 (0.9); 7.7525 (1.1); 7.7503 (1.4); 7.7480 (1.2); 7.7457 (1.3); 7.7319 (1.3); 7.7273 (1.2); 7.6426 (1.0); 7.6217 (0.7); 7.2615 (41.8); 7.1182 (0.6); 7.1010 (0.7); 7.0881 (0.5); 6.9972 (1.5); 6.9909 (1.3); 6.9896 (1.3); 6.9771 (1.2); 6.9758 (1.3); 6.9697 (1.3); 6.9684 (1.2); 4.9466 (12.0); 4.8855 (0.9); 4.3229 (2.0); 4.3050 (6.2); 4.2872 (6.4); 4.2693 (2.4); 1.3333 (7.8); 1.3155 (16.0); 1.3025 (0.8); 1.2976 (7.6); 1.2847 (1.3); 1.2668 (0.7); 0.0080 (0.7); - 0.0002 (26.8); -0.0085 (0.8) |
| I-02: ¹H-NMR(599.6 MHz, CDCl3): |
| δ= 8.2023 (10.1); 8.1985 (9.6); 8.0674 (6.6); 8.0657 (6.6); 8.0594 (6.7); 8.0577 (6.3); 7.8542 (3.5); 7.8501 (3.4); 7.8403 (5.6); 7.8373 (5.3); 7.8275 (3.6); 7.8234 (3.3); 7.7620 (3.7); 7.7589 (3.4); 7.7482 (6.8); 7.7467 (6.2); 7.7359 (4.7); 7.7328 (4.2); 7.6398 (10.9); 7.6260 (8.6); 7.2627 (14.9); 7.1107 (5.4); 7.1095 (5.0); 7.1025 (5.6); 7.1012 (5.7); 7.0985 (5.6); 7.0903 (5.0); 7.0891 (4.5); 6.9908 (6.0); 6.9863 (5.9); 6.9767 (5.8); 6.9722 (5.6); 4.9454 (50.0); 4.3135 (6.5); 4.3016 (19.5); 4.2897 (19.6); 4.2778 (6.6); 2.7693 (1.1); 2.0440 (0.4); 1.5813 (11.2); 1.3269 (21.4); 1.3150 (42.4); 1.3031 (21.1); 1.2586 (0.7); 1.2073 (0.4); -0.0001 (23.3); -0.0055 (0.8) |
| I-02: ¹H-NMR(400.0 MHz, CDCl3): |
| δ= 8.2034 (2.4); 8.1970 (2.3); 8.0724 (1.5); 8.0697 (1.5); 8.0603 (1.5); 8.0554 (1.4); 7.8631 (1.1); 7.8570 (1.1); 7.8422 (1.5); 7.8360 (1.5); 7.8233 (1.2); 7.8169 (1.2); 7.7706 (1.1); 7.7657 (1.0); 7.7499 (1.7); 7.7452 (1.6); 7.7315 (1.6); 7.7267 (1.6); 7.6412 (3.2); 7.6206 (2.2); 7.2601 (74.1); 7.1178 (1.4); 7.1153 (1.4); 7.1055 (1.4); 7.1031 (1.6); 7.0996 (1.4); 7.0969 (1.3); 7.0873 (1.2); 7.0847 (1.3); 6.9962 (2.0); 6.9888 (1.7); 6.9751 (1.6); 6.9689 (1.5); 5.0852 (0.8); 4.9459 (16.0); 4.7446 (0.9); 4.3228 (1.9); 4.3049 (6.1); 4.2871 (6.2); 4.2692 (2.0); 1.3333 (7.6); 1.3154 (15.2); 1.2976 (7.5); 1.2809 (0.8); 1.2545 (1.1); 0.0079 (2.9); - 0.0002 (98.9); -0.0085 (2.7) |
| I-89: ¹H-NMR(400.0 MHz, d₆-DMSO): |
| δ= 12.2270 (0.6); 8.2508 (1.4); 8.2446 (1.4); 8.1006 (1.2); 8.0959 (1.1); 8.0837 (1.9); 8.0223 (0.5); 8.0161 (0.5); 8.0016 (0.9); 7.9953 (0.9); 7.9876 (0.7); 7.9821 (1.0); 7.9752 (0.6); 7.9670 (0.9); 7.9642 (0.8); 7.9624 (0.9); 7.9483 (0.8); 7.9437 (0.7); 7.7125 (1.6); 7.6919 (1.4); 7.3045 (0.9); 7.2981 (0.9); 7.2829 (1.6); 7.2764 (0.9); 7.2727 (0.8); 7.2704 (0.9); 7.2663 (0.8); 7.2641 (0.8); 7.2542 (0.8); 7.2519 (0.8); 4.7456 (4.4); 4.1694 (0.6); 3.6177 (1.1); 3.6114 (0.7); 3.6074 (1.0); 3.6011 (2.8); 3.5951 (1.0); 3.5910 (0.7); 3.5846 (1.2); 3.3213 (16.0); 2.5243 (0.5); 2.5195 (0.7); 2.5108 (11.1); 2.5063 (24.1); 2.5017 (33.8); 2.4972 (24.0); 2.4927 (10.8); 2.4756 (1.1); 2.4604 (1.1); 2.3774 (1.1); 2.3589 (1.1); 2.3383 (0.7); 2.3199 (0.7); 1.7761 (1.2); 1.7682 (1.2); 1.7595 (3.5); 1.7508 (1.2); 1.7431 (1.2); 1.3556 (3.0); 1.2348 (0.6); 1.1383 (4.7); 1.1217 (4.6); -0.0002 (17.2); -0.0085 (0.5) |
| I-90: ¹H-NMR(400.6 MHz, CDCl3): |
| δ= 8.2013 (2.4); 8.1993 (1.9); 8.1952 (2.5); 8.0821 (1.5); 8.0801 (1.6); 8.0774 (1.7); 8.0755 (1.5); 8.0700 (1.6); 8.0680 (1.7); 8.0653 (1.8); 8.0634 (1.5); 7.8604 (1.2); 7.8542 (1.2); 7.8414 (1.5); 7.8393 (1.5); 7.8352 (1.5); 7.8331 (1.4); 7.8204 (1.3); 7.8142 (1.3); 7.7761 (1.2); 7.7714 (1.2); 7.7576 (1.6); 7.7555 (1.8); 7.7528 (1.6); 7.7508 (1.7); 7.7371 (1.7); 7.7323 (1.7); 7.6254 (2.1); 7.6234 (3.4); 7.6212 (1.9); 7.6049 (1.6); 7.6028 (2.5); 7.6005 (1.4); 7.2614 (20.9); 7.1265 (1.6); 7.1240 (1.5); 7.1143 (1.6); 7.1119 (1.6); 7.1081 (1.6); 7.1056 (1.5); 7.0959 (1.6); 7.0934 (1.4); 6.9999 (1.7); 6.9985 (1.7); 6.9926 (1.8); 6.9913 (1.6); 6.9788 (1.7); 6.9774 (1.6); 6.9714 (1.7); 5.3002 (2.4); 4.9648 (16.0); 4.2712 (1.4); 4.2552 (1.5); 4.2442 (2.2); 4.2282 (2.2); 4.1520 (2.3); 4.1322 (2.4); 4.1250 (1.6); 4.1052 (1.6); 3.8525 (0.7); 3.8387 (0.8); 3.8317 (1.5); 3.8179 (1.6); 3.8110 (2.9); 3.7974 (1.2); 3.7931 (1.9); 3.7888 (2.1); 3.7710 (2.0); 3.7453 (1.2); 3.7270 (1.8); 3.7086 (1.5); 3.6875 (0.9); 3.5632 (1.9); 3.5495 (2.0); 3.5410 (1.7); 3.5273 (1.7); 2.6265 (0.6); 2.6094 (0.8); 2.5922 (0.7); 2.0324 (0.7); 2.0200 (0.9); 2.0116 (0.5); 2.0070 (0.6); 1.9991 (0.9); 1.9872 (0.7); 1.9803 (0.6); 1.9666 (0.5); 1.6600 (0.5); 1.6426 (0.8); 1.6280 (1.0); 1.6085 (1.0); 1.5937 (0.7); 1.5767 (0.5); 1.5579 (5.7); 1.2556 (0.9); 0.0080 (1.0); -0.0002 (32.8); -0.0057 (0.7); -0.0065 (0.6); -0.0086 (1.1) |
| I-92: ¹H-NMR(400.6 MHz, CDCl3): |
| δ= 8.2153 (1.1); 8.2133 (0.9); 8.2112 (0.9); 8.2091 (1.2); 8.2073 (0.8); 8.0826 (0.6); 8.0805 (0.7); 8.0779 (0.8); 8.0758 (0.7); 8.0704 (0.7); 8.0684 (0.8); 8.0658 (0.8); 8.0637 (0.8); 7.8637 (0.6); 7.8575 (0.6); 7.8449 (0.7); 7.8426 (0.8); 7.8387 (0.8); 7.8364 (0.8); 7.8238 (0.7); 7.8176 (0.7); 7.8057 (0.6); 7.8010 (0.6); 7.7874 (0.7); 7.7851 (1.0); 7.7827 (0.7); 7.7804 (1.0); 7.7668 (1.0); 7.7621 (1.0); 7.7060 (0.9); 7.7036 (1.6); 7.7013 (1.0); 7.6854 (0.6); 7.6830 (1.0); 7.6807 (0.6); 7.2631 (7.7); 7.1452 (0.7); 7.1426 (0.8); 7.1331 (0.7); 7.1304 (0.8); 7.1269 (0.8); 7.1243 (0.8); 7.1148 (0.8); 7.1121 (0.9); 7.0094 (0.8); 7.0078 (0.8); 7.0020 (0.8); 7.0004 (0.9); 6.9882 (0.8); 6.9867 (0.8); 6.9808 (0.8); 6.9793 (0.8); 5.3006 (1.3); 4.8618 (8.2); 3.6806 (16.0); 2.6050 (3.3); 2.5917 (3.2); 1.5888 (1.5); 1.3186 (5.8); 1.3017 (5.8); -0.0002 (11.0) |
| I-93: ¹H-NMR(400.6 MHz, CDCl3): |
| δ= 8.1976 (2.5); 8.1914 (2.6); 8.0650 (1.5); 8.0631 (1.6); 8.0603 (1.8); 8.0584 (1.6); 8.0528 (1.7); 8.0509 (1.8); 8.0482 (1.8); 8.0464 (1.6); 7.8583 (1.3); 7.8522 (1.3); 7.8393 (1.5); 7.8373 (1.6); 7.8331 (1.6); 7.8311 (1.6); 7.8183 (1.4); 7.8121 (1.3); 7.7691 (1.2); 7.7644 (1.2); 7.7507 (1.4); 7.7484 (1.9); 7.7460 (1.5); 7.7438 (1.8); 7.7302 (1.9); 7.7254 (1.9); 7.6649 (2.1); 7.6627 (3.6); 7.6604 (2.1); 7.6442 (1.5); 7.6421 (2.4); 7.6398 (1.4); 7.2609 (37.0); 7.1145 (1.6); 7.1119 (1.6); 7.1024 (1.6); 7.0997 (1.6); 7.0962 (1.7); 7.0936 (1.5); 7.0841 (1.5); 7.0814 (1.5); 6.9975 (2.0); 6.9902 (1.9); 6.9765 (1.8); 6.9691 (1.8); 5.3002 (6.6); 5.0017 (16.0); 4.3332 (0.5); 4.3176 (2.3); 4.2984 (3.3); 4.2919 (0.7); 4.2822 (1.2); 4.1967 (1.3); 4.1810 (3.4); 4.1771 (2.0); 4.1618 (5.8); 4.1541 (1.3); 4.1515 (1.2); 4.1453 (1.1); 3.9016 (0.9); 3.8846 (1.6); 3.8807 (1.6); 3.8681 (1.1); 3.8638 (2.6); 3.8473 (1.3); 3.8124 (1.2); 3.7947 (1.7); 3.7788 (1.4); 3.7743 (1.2); 3.7581 (0.8); 2.0003 (0.7); 1.9877 (0.7); 1.9796 (0.9); 1.9653 (0.8); 1.9499 (0.7); 1.9350 (0.7); 1.9237 (0.7); 1.9196 (1.0); 1.9149 (0.9); 1.9071 (2.0); 1.9026 (1.3); 1.8990 (1.2); 1.8903 (1.9); 1.8863 (1.6); 1.8726 (0.9); 1.8691 (1.3); 1.6458 (0.9); 1.6413 (0.5); 1.6322 (0.7); 1.6241 (0.8); 1.6152 (0.8); 1.5974 (0.7); 1.5487 (8.9); 0.0079 (1.4); - 0.0002 (50.3); -0.0085 (1.9) |
| I-94: ¹H-NMR(400.0 MHz, d₆-DMSO): |
| δ= 8.2765 (1.2); 8.2708 (1.3); 8.1417 (0.8); 8.1390 (0.9); 8.1295 (0.9); 8.1268 (0.9); 8.0737 (0.8); 8.0677 (0.7); 8.0145 (0.6); 8.0098 (0.6); 7.9942 (0.9); 7.9896 (0.9); 7.9753 (0.8); 7.9707 (0.7); 7.6634 (1.6); 7.6429 (1.4); 7.3508 (0.8); 7.3485 (0.8); 7.3386 (0.7); 7.3364 (0.8); 7.3321 (0.8); 7.3298 (0.7); 7.3201 (0.8); 7.3177 (0.7); 7.2963 (0.8); 7.2899 (0.9); 7.2750 (0.8); 7.2684 (0.8); 5.1911 (1.5); 5.1736 (1.5); 3.3189 (16.0); 2.5238 (0.7); 2.5191 (1.0); 2.5103 (18.0); 2.5057 (40.1); 2.5012 (56.5); 2.4966 (40.2); 2.4921 (18.2); 1.9884 (1.1); 1.5950 (4.6); 1.5776 (4.6); 1.3554 (0.8); 1.2356 (0.8); 1.1745 (0.6); 0.0080 (1.3); -0.0002 (50.2); - 0.0085 (1.5) |
| I-95: ¹H-NMR(400.6 MHz, CDCl3): |
| δ= 8.2023 (1.1); 8.1961 (1.2); 8.0763 (0.6); 8.0744 (0.7); 8.0717 (0.7); 8.0698 (0.7); 8.0642 (0.7); 8.0623 (0.7); 8.0595 (0.7); 8.0577 (0.7); 7.8609 (0.6); 7.8547 (0.5); 7.8419 (0.6); 7.8398 (0.7); 7.8357 (0.7); 7.8336 (0.7); 7.8209 (0.6); 7.8147 (0.6); 7.7755 (0.5); 7.7707 (0.5); 7.7570 (0.6); 7.7548 (0.8); 7.7523 (0.7); 7.7501 (0.8); 7.7365 (0.8); 7.7317 (0.8); 7.6538 (0.9); 7.6517 (1.5); 7.6496 (0.9); 7.6332 (0.6); 7.6311 (1.1); 7.6289 (0.6); 7.2612 (14.2); 7.1232 (0.7); 7.1206 (0.7); 7.1110 (0.6); 7.1085 (0.7); 7.1048 (0.7); 7.1023 (0.7); 7.0927 (0.6); 7.0901 (0.6); 6.9979 (0.8); 6.9917 (0.8); 6.9779 (0.7); 6.9706 (0.8); 4.9783 (7.7); 4.4228 (2.0); 4.4059 (4.1); 4.3890 (2.1); 2.7825 (2.2); 2.7657 (4.4); 2.7487 (2.1); 2.1441 (16.0); 1.5776 (0.9); -0.0002 (17.6); -0.0085 (0.6) |
| I-96: ¹H-NMR(400.6 MHz, CDCl3): |
| δ= 8.2002 (1.1); 8.1941 (1.2); 8.0656 (0.6); 8.0629 (0.7); 8.0535 (0.7); 8.0508 (0.7); 7.8599 (0.5); 7.8537 (0.5); 7.8408 (0.6); 7.8388 (0.7); 7.8347 (0.7); 7.8327 (0.6); 7.8199 (0.6); 7.8137 (0.6); 7.7512 (0.6); 7.7490 (0.8); 7.7466 (0.6); 7.7444 (0.7); 7.7307 (0.7); 7.7260 (0.7); 7.6545 (1.4); 7.6339 (1.0); 7.2613 (9.9); 7.1153 (0.6); 7.1128 (0.6); 7.1031 (0.6); 7.1006 (0.6); 7.0970 (0.7); 7.0945 (0.6); 7.0849 (0.6); 7.0823 (0.6); 6.9969 (0.8); 6.9896 (0.8); 6.9757 (0.7); 6.9684 (0.8); 5.0026 (7.3); 4.3973 (1.8); 4.3893 (1.0); 4.3858 (1.9); 4.3819 (1.0); 4.3741 (2.0); 3.6510 (2.1); 3.6431 (1.0); 3.6393 (2.0); 3.6357 (1.1); 3.6277 (2.0); 3.3793 (16.0); -0.0002 (12.0) |
| I-96: ¹H-NMR(400.6 MHz, CDCl3): |
| δ= 8.2008 (0.9); 8.1988 (0.7); 8.1966 (0.7); 8.1946 (0.9); 8.1928 (0.6); 8.0670 (0.6); 8.0649 (0.6); 8.0623 (0.7); 8.0602 (0.6); 8.0549 (0.6); 8.0528 (0.7); 8.0502 (0.7); 8.0481 (0.6); 7.8599 (0.5); 7.8537 (0.5); 7.8410 (0.6); 7.8388 (0.6); 7.8348 (0.6); 7.8326 (0.6); 7.8199 (0.6); 7.8137 (0.5); 7.7698 (0.5); 7.7514 (0.6); 7.7492 (0.8); 7.7467 (0.6); 7.7444 (0.8); 7.7308 (0.8); 7.7261 (0.8); 7.6579 (0.8); 7.6556 (1.4); 7.6533 (0.8); 7.6373 (0.6); 7.6350 (1.0); 7.6326 (0.5); 7.2628 (4.2); 7.1151 (0.7); 7.1125 (0.7); 7.1030 (0.6); 7.1003 (0.7); 7.0968 (0.7); 7.0941 (0.6); 7.0846 (0.6); 7.0820 (0.6); 6.9972 (0.7); 6.9957 (0.7); 6.9898 (0.7); 6.9883 (0.7); 6.9761 (0.6); 6.9746 (0.7); 6.9687 (0.6); 6.9672 (0.6); 5.3002 (2.8); 5.0027 (6.9); 4.3973 (1.7); 4.3893 (0.8); 4.3858 (1.7); 4.3819 (0.8); 4.3740 (1.8); 3.6509 (2.0); 3.6430 (0.9); 3.6392 (1.8); 3.6357 (0.9); 3.6344 (0.7); 3.6276 (1.9); 3.3789 (16.0); 1.5785 (0.9); -0.0002 (6.5) |
| I-97: ¹H-NMR(400.6 MHz, CDCl3): |
| δ= 8.2122 (1.2); 8.2082 (1.0); 8.2060 (1.3); 8.0905 (0.7); 8.0885 (0.8); 8.0859 (0.9); 8.0838 (0.8); 8.0784 (0.8); 8.0763 (0.9); 8.0737 (0.9); 8.0717 (0.8); 7.8644 (0.7); 7.8582 (0.7); 7.8456 (0.8); 7.8433 (0.8); 7.8394 (0.8); 7.8372 (0.8); 7.8245 (0.8); 7.8183 (0.7); 7.8093 (0.6); 7.8046 (0.6); 7.7910 (0.7); 7.7887 (1.0); 7.7863 (0.8); 7.7840 (1.0); 7.7704 (1.0); 7.7657 (1.0); 7.7121 (1.0); 7.7097 (1.9); 7.7075 (1.2); 7.6915 (0.7); 7.6891 (1.2); 7.6868 (0.7); 7.2617 (15.3); 7.1504 (0.9); 7.1477 (0.9); 7.1382 (0.8); 7.1356 (0.9); 7.1321 (0.9); 7.1294 (0.9); 7.1199 (0.9); 7.1173 (0.8); 7.0091 (0.9); 7.0077 (0.9); 7.0017 (0.9); 7.0003 (0.9); 6.9880 (0.9); 6.9866 (0.9); 6.9806 (0.9); 6.9792 (0.9); 5.3003 (6.8); 4.9460 (7.7); 4.1838 (3.5); 4.1704 (3.5); 3.7928 (16.0); 1.5619 (8.1); 1.2557 (0.5); 0.0080 (0.5); -0.0002 (20.8); -0.0085 (0.8) |
| I-98: ¹H-NMR(400.0 MHz, d₆-DMSO): |
| δ= 8.2778 (3.2); 8.2716 (3.4); 8.1260 (1.9); 8.1241 (2.1); 8.1214 (2.4); 8.1195 (2.1); 8.1138 (2.0); 8.1120 (2.3); 8.1093 (2.3); 8.1074 (2.1); 8.0448 (1.4); 8.0386 (1.3); 8.0238 (2.0); 8.0177 (2.0); 8.0040 (1.5); 7.9979 (3.1); 7.9932 (1.6); 7.9792 (2.1); 7.9774 (2.3); 7.9746 (2.0); 7.9727 (2.1); 7.9588 (2.0); 7.9540 (1.9); 7.6868 (4.1); 7.6662 (3.7); 7.3089 (2.2); 7.3020 (4.2); 7.2921 (2.1); 7.2894 (3.4); 7.2862 (3.1); 7.2831 (2.9); 7.2737 (2.0); 7.2713 (1.9); 4.9361 (16.0); 4.0561 (0.5); 4.0382 (1.7); 4.0205 (1.7); 4.0027 (0.6); 3.3224 (3.0); 2.5246 (0.7); 2.5199 (1.0); 2.5112 (17.3); 2.5066 (38.2); 2.5021 (53.5); 2.4975 (37.8); 2.4929 (16.9); 1.9889 (7.4); 1.9094 (3.8); 1.1925 (2.0); 1.1747 (4.0); 1.1569 (2.0); 0.0080 (1.2); -0.0002 (45.3); -0.0085 (1.3) |
| I-99: ¹H-NMR(400.0 MHz, CDCl3): |
| δ= 8.1584 (2.0); 8.1520 (2.2); 8.1026 (1.4); 8.1000 (1.4); 8.0878 (1.4); 7.8516 (0.8); 7.8459 (0.7); 7.8316 (1.2); 7.8118 (0.8); 7.8056 (0.8); 7.7909 (1.1); 7.7862 (1.0); 7.7704 (1.5); 7.7659 (1.4); 7.7517 (1.4); 7.7470 (1.3); 7.6111 (2.7); 7.5906 (2.1); 7.2605 (66.6); 7.1759 (1.3); 7.1637 (1.3); 7.1599 (1.2); 7.1453 (1.2); 6.9948 (1.6); 6.9869 (1.5); 6.9789 (1.2); 6.9730 (1.5); 6.9667 (1.4); 5.0311 (13.8); 5.0208 (0.9); 5.0075 (0.6); 4.9719 (0.6); 4.8594 (0.9); 3.8864 (1.1); 3.1157 (0.5); 2.2716 (1.8); 1.4322 (16.0); 1.2540 (2.8); 1.2224 (0.7); 0.8796 (0.5); 0.0690 (0.9); 0.0080 (2.0); -0.0002 (81.2); -0.0085 (2.6) |
| I-100: ¹H-NMR(400.0 MHz, CDCl3): |
| δ= 8.2105 (2.3); 8.2044 (2.3); 8.0800 (1.3); 8.0773 (1.4); 8.0679 (1.4); 8.0651 (1.4); 7.8667 (1.1); 7.8605 (1.1); 7.8477 (1.4); 7.8456 (1.4); 7.8415 (1.3); 7.8394 (1.4); 7.8266 (1.2); 7.8204 (1.2); 7.7752 (1.1); 7.7704 (1.0); 7.7566 (1.3); 7.7545 (1.7); 7.7520 (1.4); 7.7498 (1.6); 7.7360 (1.6); 7.7313 (1.5); 7.6423 (2.7); 7.6217 (1.9); 7.2617 (11.8); 7.1226 (1.3); 7.1201 (1.3); 7.1104 (1.3); 7.1079 (1.4); 7.1042 (1.3); 7.1017 (1.3); 7.0920 (1.2); 7.0895 (1.2); 6.9979 (1.7); 6.9905 (1.6); 6.9767 (1.6); 6.9694 (1.5); 4.9472 (16.0); 4.3248 (2.0); 4.3069 (6.3); 4.2891 (6.4); 4.2713 (2.1); 1.5627 (5.7); 1.3344 (7.5); 1.3165 (15.4); 1.2987 (7.4); 0.0080 (0.5); -0.0002 (15.4) |
| 1-101: ¹H-NMR(400.0 MHz, CDCl3): |
| δ= 8.1754 (2.3); 8.1693 (2.3); 8.0295 (1.4); 8.0275 (1.5); 8.0249 (1.6); 8.0229 (1.4); 8.0174 (1.4); 8.0154 (1.5); 8.0127 (1.5); 8.0107 (1.4); 7.8344 (1.2); 7.8282 (1.1); 7.8153 (1.4); 7.8134 (1.5); 7.8091 (1.4); 7.8072 (1.4); 7.7943 (1.2); 7.7881 (1.2); 7.7470 (1.1); 7.7422 (1.0); 7.7285 (1.2); 7.7262 (1.7); 7.7239 (1.3); 7.7215 (1.6); 7.7079 (1.7); 7.7031 (1.6); 7.6383 (1.8); 7.6361 (3.3); 7.6339 (2.0); 7.6177 (1.3); 7.6154 (2.2); 7.6131 (1.2); 7.2615 (16.8); 7.0810 (1.4); 7.0785 (1.5); 7.0689 (1.4); 7.0663 (1.5); 7.0628 (1.5); 7.0601 (1.4); 7.0506 (1.4); 7.0480 (1.3); 6.9933 (1.6); 6.9871 (1.6); 6.9721 (1.6); 6.9660 (1.5); 5.2437 (0.9); 5.2262 (3.1); 5.2088 (3.2); 5.1914 (0.9); 4.2901 (1.0); 4.2831 (1.1); 4.2722 (3.1); 4.2654 (3.2); 4.2544 (3.2); 4.2477 (3.1); 4.2366 (1.1); 4.2300 (1.0); 1.7114 (11.9); 1.6939 (11.9); 1.5609 (8.8); 1.3049 (7.9); 1.2871 (16.0); 1.2692 (8.6); 0.8987 (1.0); 0.8818 (3.6); 0.8641 (1.4); 0.0079 (0.5); -0.0002 (19.0); -0.0085 (0.6) |
| 1-102: ¹H-NMR(400.6 MHz, CDCl3): |
| δ= 8.1982 (2.3); 8.1921 (2.4); 8.0465 (1.4); 8.0445 (1.5); 8.0419 (1.6); 8.0399 (1.5); 8.0344 (1.5); 8.0324 (1.6); 8.0298 (1.6); 8.0277 (1.5); 7.8583 (1.2); 7.8521 (1.2); 7.8393 (1.4); 7.8372 (1.5); 7.8332 (1.4); 7.8311 (1.4); 7.8183 (1.3); 7.8121 (1.2); 7.7579 (1.1); 7.7531 (1.1); 7.7395 (1.3); 7.7372 (1.8); 7.7348 (1.4); 7.7325 (1.7); 7.7189 (1.7); 7.7141 (1.7); 7.6375 (1.8); 7.6354 (3.1); 7.6332 (1.9); 7.6169 (1.3); 7.6147 (2.2); 7.6125 (1.2); 7.2626 (23.6); 7.0980 (1.5); 7.0954 (1.5); 7.0859 (1.5); 7.0833 (1.6); 7.0797 (1.6); 7.0771 (1.5); 7.0675 (1.5); 7.0650 (1.4); 6.9934 (1.6); 6.9920 (1.6); 6.9861 (1.6); 6.9847 (1.6); 6.9722 (1.5); 6.9709 (1.6); 6.9650 (1.6); 6.9636 (1.5); 5.2555 (0.9); 5.2381 (3.2); 5.2207 (3.3); 5.2033 (0.9); 4.2935 (1.0); 4.2864 (1.1); 4.2757 (3.1); 4.2687 (3.1); 4.2578 (3.2); 4.2510 (3.1); 4.2401 (1.1); 4.2333 (1.0); 3.5060 (1.9); 3.4885 (6.0); 3.4710 (6.1); 3.4535 (2.0); 1.7152 (12.3); 1.6978 (12.2); 1.5835 (6.5); 1.3055 (7.7); 1.2877 (16.0); 1.2699 (7.7); 1.2263 (6.4); 1.2088 (12.8); 1.1912 (6.2); 0.8820 (0.5); -0.0002 (11.4) |
| 1-103: ¹H-NMR(400.6 MHz, d₆-DMSO): |
| δ= 8.2163 (3.4); 8.2101 (3.6); 8.0964 (2.2); 8.0944 (2.4); 8.0917 (2.7); 8.0897 (2.5); 8.0843 (2.4); 8.0823 (2.6); 8.0796 (2.7); 8.0776 (2.4); 7.9897 (1.6); 7.9834 (1.6); 7.9723 (2.5); 7.9680 (3.6); 7.9637 (2.2); 7.9624 (2.3); 7.9538 (2.6); 7.9518 (2.9); 7.9491 (4.0); 7.9472 (3.0); 7.9427 (1.8); 7.9333 (2.6); 7.9285 (2.4); 7.6686 (2.6); 7.6664 (4.8); 7.6642 (2.8); 7.6480 (2.4); 7.6459 (4.3); 7.6436 (2.4); 7.2933 (2.3); 7.2876 (2.3); 7.2719 (2.4); 7.2693 (3.1); 7.2667 (4.5); 7.2572 (2.3); 7.2547 (2.5); 7.2508 (2.5); 7.2484 (2.2); 7.2387 (2.3); 7.2362 (2.3); 4.8941 (16.0); 4.0382 (0.8); 4.0204 (0.8); 3.3370 (9.0); 2.5256 (0.8); 2.5209 (1.1); 2.5122 (15.4); 2.5076 (34.1); 2.5030 (48.2); 2.4984 (34.1); 2.4939 (15.6); 1.9891 (3.6); 1.9100 (4.0); 1.3559 (1.3); 1.1924 (1.0); 1.1747 (2.2); 1.1569 (1.0) |
| 1-104: ¹H-NMR(400.6 MHz, CDCl3): |
| δ= 8.5475 (2.6); 8.5406 (2.6); 8.3448 (1.5); 8.3411 (2.6); 8.3375 (1.5); 8.1952 (1.2); 8.1930 (1.3); 8.1907 (1.4); 8.1885 (1.3); 8.1831 (1.2); 8.1807 (1.4); 8.1788 (1.4); 8.1764 (1.3); 7.9271 (0.7); 7.9225 (0.7); 7.9096 (0.6); 7.9066 (1.9); 7.9021 (1.9); 7.8892 (2.3); 7.8848 (2.3); 7.8811 (2.1); 7.8788 (2.4); 7.8774 (2.7); 7.8749 (2.4); 7.8606 (0.8); 7.8584 (0.9); 7.8570 (0.6); 7.5385 (1.2); 7.5341 (1.3); 7.5316 (1.3); 7.5272 (1.2); 7.5167 (1.3); 7.5123 (1.4); 7.5098 (1.3); 7.5054 (1.2); 7.3327 (1.4); 7.3289 (1.4); 7.3206 (1.4); 7.3166 (1.5); 7.3153 (1.6); 7.3115 (1.3); 7.3033 (1.4); 7.2993 (1.3); 7.2647 (10.0); 5.3012 (13.1); 4.4380 (15.8); 4.2762 (2.1); 4.2583 (6.8); 4.2405 (6.8); 4.2226 (2.2); 1.6028 (2.1); 1.2834 (7.6); 1.2656 (16.0); 1.2592 (0.6); 1.2568 (0.6); 1.2551 (0.6); 1.2477 (7.4); 0.0705 (3.1); -0.0002 (10.6) |
| 1-105: ¹H-NMR(400.0 MHz, CDCl3): |
| δ= 8.1519 (2.3); 8.1459 (2.3); 8.0663 (1.6); 8.0636 (1.6); 8.0543 (1.6); 8.0514 (1.6); 7.8464 (0.9); 7.8403 (0.8); 7.8259 (1.3); 7.8202 (1.2); 7.8067 (0.9); 7.8006 (0.9); 7.7838 (1.1); 7.7790 (1.2); 7.7633 (1.8); 7.7586 (1.7); 7.7446 (1.6); 7.7399 (1.5); 7.6305 (3.0); 7.6099 (2.3); 7.2608 (31.2); 7.1562 (1.4); 7.1537 (1.4); 7.1440 (1.5); 7.1416 (1.5); 7.1377 (1.5); 7.1351 (1.4); 7.1255 (1.4); 7.1230 (1.3); 6.9861 (1.7); 6.9788 (1.7); 6.9659 (1.7); 6.9579 (1.6); 5.2999 (5.7); 4.9538 (16.0); 4.9476 (1.4); 4.7851 (0.6); 4.3166 (2.1); 4.2987 (6.4); 4.2808 (6.6); 4.2686 (0.7); 4.2630 (2.2); 3.9012 (0.8); 1.5454 (11.2); 1.3279 (7.7); 1.3204 (1.2); 1.3101 (15.6); 1.3025 (1.0); 1.2991 (1.2); 1.2923 (7.6); 1.2812 (0.6); 1.2552 (0.8); 0.0080 (1.3); -0.0002 (42.0); -0.0085 (1.4) |
| 1-106: ¹H-NMR(400.6 MHz, CDCl3): |
| δ= 8.5338 (3.0); 8.5269 (3.1); 8.3349 (1.6); 8.3312 (3.0); 8.3276 (1.6); 8.1816 (1.2); 8.1794 (1.3); 8.1771 (1.4); 8.1749 (1.3); 8.1695 (1.3); 8.1672 (1.4); 8.1651 (1.4); 8.1628 (1.3); 7.9156 (0.8); 7.9110 (0.8); 7.8979 (0.7); 7.8951 (1.9); 7.8906 (1.8); 7.8775 (2.2); 7.8731 (2.2); 7.8668 (2.0); 7.8645 (2.4); 7.8633 (2.7); 7.8609 (2.3); 7.8463 (0.9); 7.8440 (1.0); 7.8428 (0.8); 7.8404 (0.6); 7.5133 (1.3); 7.5089 (1.3); 7.5064 (1.4); 7.5020 (1.2); 7.4913 (1.3); 7.4869 (1.4); 7.4845 (1.3); 7.4801 (1.3); 7.3059 (1.4); 7.3022 (1.4); 7.2937 (1.3); 7.2900 (1.4); 7.2883 (1.6); 7.2846 (1.3); 7.2762 (1.4); 7.2725 (1.4); 7.2626 (22.8); 5.3006 (9.1); 4.4170 (16.0); 4.2811 (1.7); 4.2633 (5.5); 4.2455 (5.8); 4.2277 (1.9); 1.5794 (5.8); 1.2966 (7.4); 1.2788 (15.6); 1.2609 (7.4); 0.0696 (3.7); 0.0080 (0.6); -0.0002 (24.8); -0.0085 (0.7) |
| 1-107: ¹H-NMR(400.0 MHz, CDCl3): |
| δ= 8.1455 (1.9); 8.1395 (1.9); 8.0421 (1.2); 8.0403 (1.3); 8.0376 (1.4); 8.0356 (1.2); 8.0301 (1.3); 8.0281 (1.4); 8.0254 (1.4); 8.0235 (1.3); 7.8393 (0.8); 7.8333 (0.7); 7.8187 (1.1); 7.8131 (1.1); 7.7993 (0.8); 7.7934 (0.8); 7.7702 (1.0); 7.7654 (1.0); 7.7516 (1.2); 7.7497 (1.5); 7.7469 (1.3); 7.7450 (1.4); 7.7311 (1.4); 7.7263 (1.4); 7.6239 (1.6); 7.6217 (2.8); 7.6011 (2.0); 7.2609 (25.4); 7.1369 (1.3); 7.1345 (1.3); 7.1248 (1.3); 7.1223 (1.3); 7.1184 (1.2); 7.1159 (1.1); 7.1063 (1.2); 7.1038 (1.2); 6.9814 (1.4); 6.9741 (1.4); 6.9603 (1.4); 6.9542 (1.3); 5.2716 (0.7); 5.2543 (2.5); 5.2369 (2.6); 5.2195 (0.8); 4.2856 (1.0); 4.2817 (1.0); 4.2678 (3.0); 4.2639 (3.0); 4.2499 (3.1); 4.2462 (2.9); 4.2320 (1.0); 4.2284 (1.0); 1.7113 (0.6); 1.6999 (10.5); 1.6942 (1.0); 1.6825 (10.3); 1.5508 (16.0); 1.3047 (0.6); 1.2986 (6.5); 1.2870 (1.4); 1.2808 (13.5); 1.2693 (1.3); 1.2630 (6.8); 0.8819 (0.6); 0.0080 (0.8); - 0.0002 (28.3); -0.0085 (0.8) |
| 1-108: ¹H-NMR(400.6 MHz, CDCl3): |
| δ= 8.2005 (1.1); 8.1943 (1.2); 8.0860 (0.6); 8.0750 (0.6); 7.8559 (0.6); 7.8496 (0.6); 7.8369 (0.7); 7.8348 (0.8); 7.8308 (0.7); 7.8286 (0.7); 7.8158 (0.6); 7.8097 (0.6); 7.7536 (0.6); 7.2606 (39.7); 6.9925 (0.7); 6.9853 (0.7); 6.9715 (0.7); 6.9642 (0.7); 5.3003 (5.3); 5.1175 (1.3); 5.0434 (0.9); 3.7107 (2.3); 3.6798 (1.6); 3.1571 (0.6); 2.0455 (1.2); 1.5446 (16.0); 1.2596 (0.9); 0.0080 (1.6); 0.0058 (0.5); 0.0050 (0.5); -0.0002 (64.7); -0.0085 (2.0) |
| 1-109: ¹H-NMR(400.6 MHz, CDCl3): |
| δ= 8.2018 (2.0); 8.2000 (2.1); 8.1978 (1.9); 8.1958 (2.2); 8.0848 (1.5); 8.0828 (1.6); 8.0801 (1.7); 8.0781 (1.5); 8.0726 (1.6); 8.0707 (1.6); 8.0680 (1.7); 8.0660 (1.4); 7.8572 (0.6); 7.8540 (0.7); 7.8510 (0.7); 7.8479 (0.7); 7.8381 (0.8); 7.8356 (1.2); 7.8326 (1.3); 7.8296 (1.2); 7.8268 (0.8); 7.8172 (0.7); 7.8140 (0.7); 7.8110 (0.7); 7.8079 (0.7); 7.7792 (0.8); 7.7745 (0.8); 7.7588 (1.5); 7.7574 (1.4); 7.7541 (1.4); 7.7401 (1.2); 7.7353 (1.2); 7.6558 (1.0); 7.6536 (1.6); 7.6513 (1.0); 7.6418 (1.1); 7.6396 (1.7); 7.6373 (1.1); 7.6353 (0.9); 7.6330 (1.2); 7.6307 (0.7); 7.6212 (0.8); 7.6190 (1.2); 7.6167 (0.7); 7.2620 (23.2); 7.1218 (1.6); 7.1192 (1.6); 7.1096 (1.6); 7.1070 (1.6); 7.1034 (1.6); 7.1008 (1.4); 7.0913 (1.5); 7.0887 (1.4); 6.9927 (1.6); 6.9853 (1.7); 6.9715 (1.6); 6.9642 (1.6); 5.3004 (13.5); 4.9833 (5.7); 4.9754 (3.7); 4.9721 (3.3); 3.8745 (1.3); 3.8643 (1.2); 3.8569 (1.3); 3.8449 (1.7); 3.8291 (1.1); 3.8229 (1.3); 3.8130 (1.2); 3.7606 (0.6); 3.7543 (0.9); 3.7428 (15.2); 3.7298 (0.6); 3.7195 (16.0); 3.6631 (0.7); 3.6381 (0.5); 3.5889 (0.7); 3.5588 (0.5); 3.2273 (0.6); 3.2088 (0.9); 3.1904 (0.6); 3.1157 (0.6); 3.0984 (0.8); 3.0796 (0.6); 2.3251 (0.8); 2.3073 (1.0); 2.2893 (0.9); 2.2743 (0.7); 2.2219 (0.6); 2.2033 (1.1); 2.1895 (0.7); 2.1842 (1.3); 2.1646 (1.0); 1.5761 (7.2); 0.0079 (1.0); - 0.0002 (36.5); -0.0085 (1.0) |
| 1-110: ¹H-NMR(400.6 MHz, CDCl3): |
| δ= 8.2024 (1.8); 8.1966 (2.2); 8.0706 (1.5); 8.0588 (1.5); 7.8533 (0.7); 7.8472 (0.8); 7.8324 (1.2); 7.8281 (1.3); 7.8133 (0.8); 7.8072 (0.9); 7.7781 (0.7); 7.7736 (0.7); 7.7573 (1.5); 7.7530 (1.4); 7.7394 (1.3); 7.7349 (1.3); 7.7063 (2.2); 7.6858 (1.1); 7.6621 (0.5); 7.2627 (8.3); 7.1150 (1.0); 7.1124 (1.0); 7.1027 (1.2); 7.1000 (1.3); 7.0973 (1.2); 7.0944 (1.0); 7.0850 (0.9); 7.0822 (0.8); 6.9884 (1.3); 6.9817 (1.3); 6.9675 (1.3); 6.9606 (1.2); 5.3005 (6.3); 5.0258 (8.2); 4.9364 (0.9); 4.9231 (0.8); 4.6059 (0.8); 4.5970 (1.0); 4.5859 (0.9); 4.5759 (0.9); 3.7904 (0.9); 3.7779 (0.8); 3.7717 (0.8); 3.7582 (4.0); 3.7168 (16.0); 3.7011 (1.2); 3.6833 (1.2); 3.6659 (0.6); 3.6588 (0.8); 2.2218 (0.6); 2.2096 (0.7); 2.1958 (0.6); 2.1891 (0.6); 2.1786 (0.7); 2.1666 (0.5); 2.1511 (0.6); 2.1353 (0.6); 2.0640 (0.9); 2.0453 (2.7); 2.0332 (1.1); 2.0200 (0.8); 1.5923 (3.1); 1.2773 (0.6); 1.2594 (1.4); 1.2416 (0.6); -0.0002 (12.8) |
| 1-111: ¹H-NMR(400.6 MHz, d₆-DMSO): |
| δ= 8.2464 (2.3); 8.2421 (2.0); 8.2403 (2.3); 8.1035 (1.2); 8.1015 (1.4); 8.0988 (1.8); 8.0968 (1.8); 8.0943 (0.7); 8.0915 (1.5); 8.0894 (1.5); 8.0867 (1.8); 8.0847 (1.8); 8.0183 (0.9); 8.0122 (0.9); 7.9972 (1.5); 7.9924 (1.2); 7.9910 (1.2); 7.9777 (1.1); 7.9731 (1.5); 7.9716 (1.2); 7.9685 (1.1); 7.9584 (0.5); 7.9546 (1.6); 7.9526 (1.6); 7.9499 (1.3); 7.9479 (1.4); 7.9341 (1.5); 7.9293 (1.3); 7.7515 (1.4); 7.7492 (2.6); 7.7470 (1.6); 7.7309 (1.3); 7.7287 (2.3); 7.7264 (1.3); 7.7059 (0.8); 7.6853 (0.7); 7.3033 (1.6); 7.2975 (1.6); 7.2836 (2.7); 7.2814 (2.8); 7.2764 (1.7); 7.2717 (1.4); 7.2691 (1.6); 7.2654 (1.6); 7.2627 (1.5); 7.2596 (0.5); 7.2532 (1.3); 7.2505 (1.5); 5.0709 (4.1); 5.0654 (3.6); 5.0352 (0.7); 4.8073 (0.6); 4.7709 (0.5); 4.3088 (1.0); 4.2987 (1.2); 4.2870 (1.2); 4.2770 (1.0); 4.0557 (1.1); 4.0379 (3.3); 4.0202 (3.4); 4.0024 (1.1); 3.6395 (0.7); 3.6239 (1.3); 3.6074 (0.9); 3.6012 (1.8); 3.5842 (0.8); 3.5771 (0.6); 3.3227 (5.1); 2.5411 (1.3); 2.5243 (0.8); 2.5197 (1.1); 2.5109 (17.0); 2.5063 (37.8); 2.5017 (53.3); 2.4972 (37.4); 2.4926 (16.9); 2.1946 (0.5); 2.1835 (0.8); 2.1731 (0.8); 2.1634 (0.8); 2.1532 (0.5); 2.1445 (0.7); 1.9888 (16.0); 1.9679 (1.7); 1.9506 (1.2); 1.9334 (0.5); 1.9090 (1.0); 1.9045 (0.6); 1.8858 (0.6); 1.8753 (0.6); 1.8717 (0.5); 1.7593 (0.6); 1.3555 (3.5); 1.2352 (1.2); 1.1922 (4.6); 1.1745 (9.3); 1.1567 (4.4); 0.0080 (1.0); -0.0002 (35.3); -0.0085 (1.0) |
| 1-112: ¹H-NMR(400.6 MHz, CDCl3): |
| δ= 8.2026 (1.9); 8.2004 (1.5); 8.1984 (1.6); 8.1964 (2.0); 8.1944 (1.4); 8.0863 (1.2); 8.0843 (1.4); 8.0816 (1.5); 8.0795 (1.4); 8.0742 (1.4); 8.0721 (1.5); 8.0695 (1.5); 8.0674 (1.4); 7.8561 (1.1); 7.8499 (1.1); 7.8372 (1.3); 7.8350 (1.4); 7.8310 (1.3); 7.8288 (1.3); 7.8161 (1.2); 7.8099 (1.2); 7.7840 (1.1); 7.7792 (1.1); 7.7655 (1.3); 7.7634 (1.6); 7.7608 (1.3); 7.7586 (1.6); 7.7449 (1.7); 7.7402 (1.6); 7.6485 (1.6); 7.6462 (3.0); 7.6439 (1.8); 7.6279 (1.3); 7.6256 (2.2); 7.6232 (1.2); 7.2623 (19.3); 7.1215 (1.4); 7.1189 (1.4); 7.1093 (1.4); 7.1068 (1.5); 7.1031 (1.5); 7.1005 (1.4); 7.0909 (1.4); 7.0883 (1.3); 6.9928 (1.4); 6.9913 (1.4); 6.9855 (1.4); 6.9839 (1.4); 6.9717 (1.4); 6.9701 (1.4); 6.9643 (1.4); 6.9628 (1.4); 5.3003 (14.6); 5.0555 (2.9); 5.0406 (3.0); 4.1890 (2.0); 4.1712 (6.4); 4.1533 (6.5); 4.1355 (2.0); 3.2242 (0.5); 2.9432 (0.5); 2.5978 (0.6); 2.5876 (1.1); 2.5775 (0.6); 2.5612 (0.6); 2.0453 (1.0); 1.9937 (0.8); 1.9686 (0.5); 1.5773 (3.7); 1.2823 (7.4); 1.2774 (0.6); 1.2645 (16.0); 1.2596 (1.3); 1.2467 (7.2); 1.2418 (0.6); 0.0079 (0.8); -0.0002 (30.9); -0.0028 (1.8); -0.0043 (0.9); -0.0052 (0.7); -0.0060 (0.6); -0.0085 (1.0) |
| 1-113: ¹H-NMR(400.6 MHz, CDCl3): |
| δ= 8.3320 (2.4); 8.3303 (2.5); 8.3259 (2.5); 8.3242 (2.4); 8.1160 (1.1); 8.1138 (1.2); 8.1116 (1.4); 8.1093 (1.2); 8.1039 (1.2); 8.1012 (1.4); 8.0999 (1.5); 8.0972 (1.2); 7.8253 (0.6); 7.8208 (0.5); 7.8047 (1.9); 7.8002 (1.8); 7.7879 (2.3); 7.7834 (4.0); 7.7811 (2.7); 7.7785 (2.3); 7.7647 (0.7); 7.7625 (0.7); 7.7028 (2.4); 7.6967 (2.3); 7.6822 (2.7); 7.6761 (2.6); 7.3929 (3.0); 7.3912 (3.0); 7.3723 (2.6); 7.3706 (2.6); 7.2633 (6.3); 7.1806 (1.2); 7.1763 (1.2); 7.1685 (1.2); 7.1639 (2.3); 7.1595 (1.2); 7.1520 (1.2); 7.1474 (1.1); 5.3001 (3.1); 4.2643 (2.0); 4.2465 (6.2); 4.2287 (6.2); 4.2109 (2.0); 3.9398 (16.0); 1.5827 (5.8); 1.3003 (7.2); 1.2824 (14.6); 1.2646 (7.0); 0.0705 (1.7); -0.0002 (8.3) |
| 1-114: ¹H-NMR(400.6 MHz, CDCl3): |
| δ= 8.2039 (1.4); 8.2017 (1.3); 8.1979 (1.8); 8.0764 (1.2); 8.0743 (1.3); 8.0717 (1.4); 8.0697 (1.3); 8.0643 (1.3); 8.0622 (1.4); 8.0596 (1.4); 8.0575 (1.3); 7.8566 (0.7); 7.8503 (0.8); 7.8375 (0.9); 7.8355 (0.9); 7.8312 (1.0); 7.8294 (1.1); 7.8166 (0.8); 7.8103 (0.9); 7.7791 (0.6); 7.7745 (0.6); 7.7610 (1.0); 7.7585 (1.4); 7.7539 (1.1); 7.7405 (1.3); 7.7358 (1.2); 7.7068 (1.2); 7.7045 (2.0); 7.7021 (1.4); 7.6863 (0.8); 7.6840 (1.1); 7.6813 (0.9); 7.6785 (0.6); 7.2619 (16.5); 7.1151 (0.9); 7.1121 (0.9); 7.1029 (1.1); 7.1000 (1.1); 7.0970 (1.0); 7.0941 (0.9); 7.0849 (0.9); 7.0820 (0.8); 6.9900 (1.2); 6.9827 (1.2); 6.9688 (1.2); 6.9615 (1.2); 5.3003 (8.7); 5.1281 (8.8); 5.0156 (1.9); 4.2225 (1.8); 4.1929 (7.5); 3.7930 (3.6); 3.7375 (16.0); 3.1866 (15.4); 3.0605 (3.1); 2.0453 (2.0); 1.5735 (5.7); 1.2773 (0.6); 1.2594 (1.4); 1.2562 (0.6); 1.2416 (0.6); 0.0079 (0.7); -0.0002 (25.4); -0.0085 (0.9) |
| 1-115: ¹H-NMR(400.0 MHz, CDCl3): |
| δ= 9.2263 (4.1); 8.8053 (0.6); 8.7652 (10.3); 8.0695 (0.9); 8.0576 (0.9); 7.8596 (0.6); 7.8423 (1.4); 7.8397 (1.8); 7.8366 (1.4); 7.8280 (1.3); 7.8235 (1.1); 7.8104 (1.1); 7.8059 (1.0); 7.2603 (68.5); 7.1733 (0.8); 7.1697 (0.8); 7.1612 (0.8); 7.1577 (0.9); 7.1523 (0.7); 7.1435 (0.8); 7.1401 (0.7); 4.2757 (1.2); 4.2579 (3.9); 4.2400 (3.8); 4.2222 (1.3); 3.9539 (9.9); 1.7397 (2.8); 1.5435 (16.0); 1.3115 (4.6); 1.2936 (9.2); 1.2758 (4.4); 1.2557 (1.0); 0.0080 (2.6); -0.0002 (93.7); -0.0085 (2.6) |
| 1-115: ¹H-NMR(400.0 MHz, CDCl3): |
| δ= 7.2608 (9.6); 4.2462 (1.7); 4.2283 (5.3); 4.2105 (5.4); 4.1926 (1.8); 3.5824 (16.0); 1.5432 (6.4); 1.3202 (6.1); 1.3023 (12.5); 1.2845 (6.0); -0.0002 (13.5) |
| 1-115: ¹H-NMR(400.0 MHz, CDCl3): |
| δ= 9.2213 (6.2); 8.7667 (16.0); 8.1373 (1.1); 8.1336 (1.5); 8.1228 (1.4); 7.8799 (0.7); 7.8661 (2.4); 7.8620 (4.3); 7.8583 (3.2); 7.8458 (2.0); 7.8413 (2.2); 7.8328 (7.9); 7.8251 (0.6); 7.8207 (0.7); 7.2604 (59.9); 7.2217 (1.3); 7.2169 (1.3); 7.2096 (1.2); 7.2052 (2.2); 7.2006 (1.2); 7.1929 (1.1); 7.1884 (1.0); 5.2999 (0.6); 1.5483 (16.0); 1.2568 (0.6); 0.0080 (2.2); -0.0002 (80.3); - 0.0085 (2.3) |
| 1-116: ¹H-NMR(400.6 MHz, CDCl3): |
| δ= 8.2102 (1.2); 8.2081 (0.9); 8.2041 (1.2); 8.0726 (0.7); 8.0706 (0.8); 8.0679 (0.8); 8.0659 (0.8); 8.0605 (0.8); 8.0585 (0.8); 8.0558 (0.9); 8.0538 (0.8); 7.8670 (0.6); 7.8608 (0.6); 7.8480 (0.7); 7.8459 (0.8); 7.8418 (0.8); 7.8397 (0.8); 7.8270 (0.7); 7.8208 (0.7); 7.7845 (0.6); 7.7797 (0.6); 7.7660 (0.7); 7.7638 (0.9); 7.7613 (0.8); 7.7591 (0.9); 7.7455 (0.9); 7.7407 (0.9); 7.6608 (1.0); 7.6586 (1.7); 7.6563 (1.0); 7.6402 (0.7); 7.6380 (1.2); 7.6357 (0.7); 7.2622 (6.1); 7.1220 (0.8); 7.1194 (0.8); 7.1099 (0.8); 7.1073 (0.8); 7.1036 (0.8); 7.1011 (0.8); 7.0915 (0.8); 7.0889 (0.7); 6.9978 (0.9); 6.9965 (0.8); 6.9904 (0.9); 6.9892 (0.8); 6.9766 (0.8); 6.9754 (0.8); 6.9693 (0.9); 6.9680 (0.8); 4.9481 (8.5); 4.5227 (2.0); 4.5070 (4.3); 4.4913 (2.2); 3.6845 (16.0); 2.7182 (2.0); 2.7026 (4.1); 2.6869 (2.0); 1.5648 (1.0); -0.0002 (9.7) |
| 1-117: ¹H-NMR(400.6 MHz, CDCl3): |
| δ= 8.2124 (1.4); 8.2063 (1.4); 8.0812 (0.9); 8.0784 (1.0); 8.0691 (1.0); 8.0663 (1.0); 7.8607 (0.6); 7.8545 (0.6); 7.8416 (0.8); 7.8396 (0.9); 7.8355 (0.8); 7.8335 (0.8); 7.8207 (0.7); 7.8146 (0.7); 7.8064 (0.6); 7.8017 (0.6); 7.7858 (1.0); 7.7833 (0.9); 7.7812 (1.0); 7.7674 (1.0); 7.7627 (0.9); 7.6988 (1.9); 7.6782 (1.2); 7.2612 (15.7); 7.1464 (0.8); 7.1440 (0.8); 7.1343 (0.8); 7.1318 (0.9); 7.1282 (0.9); 7.1257 (0.8); 7.1160 (0.9); 7.1135 (0.9); 7.0077 (1.0); 7.0005 (1.0); 6.9867 (1.0); 6.9794 (1.0); 4.8751 (8.4); 3.6809 (16.0); 3.6684 (2.4); 3.6532 (2.4); 3.6378 (1.0); 2.6295 (1.9); 2.6142 (3.0); 2.5992 (1.9); 1.5551 (6.5); 1.2556 (0.7); 0.0078 (0.9); -0.0002 (25.1); -0.0085 (1.0) |
| 1-118: ¹H-NMR(400.6 MHz, d₆-DMSO): |
| δ= 12.6501 (1.0); 8.4673 (1.2); 8.4527 (2.6); 8.4379 (1.2); 8.2656 (3.8); 8.2614 (3.0); 8.2594 (3.9); 8.1151 (2.5); 8.1131 (2.8); 8.1105 (3.2); 8.1084 (2.9); 8.1031 (2.7); 8.1010 (3.1); 8.0984 (3.1); 8.0963 (2.8); 8.0331 (1.8); 8.0269 (1.8); 8.0135 (2.3); 8.0119 (2.5); 8.0073 (2.2); 8.0057 (2.4); 7.9924 (2.0); 7.9861 (2.0); 7.9818 (2.4); 7.9770 (2.2); 7.9633 (2.7); 7.9611 (3.0); 7.9586 (2.5); 7.9564 (2.9); 7.9427 (3.1); 7.9379 (2.9); 7.7796 (3.1); 7.7773 (5.7); 7.7750 (3.2); 7.7589 (2.7); 7.7567 (4.8); 7.7544 (2.6); 7.3128 (2.4); 7.3115 (2.6); 7.3059 (2.6); 7.3046 (2.5); 7.2988 (3.0); 7.2963 (3.0); 7.2916 (2.5); 7.2902 (2.8); 7.2866 (3.3); 7.2843 (5.1); 7.2804 (3.1); 7.2779 (2.6); 7.2683 (2.8); 7.2657 (2.7); 4.8733 (16.0); 3.8544 (7.9); 3.8396 (7.9); 3.6217 (0.8); 3.6177 (4.3); 3.6155 (2.4); 3.6115 (2.2); 3.6093 (1.7); 3.6074 (3.0); 3.6029 (3.2); 3.6011 (9.9); 3.5989 (3.4); 3.5950 (3.1); 3.5931 (1.7); 3.5908 (2.1); 3.5867 (2.2); 3.5845 (4.4); 3.5806 (0.7); 3.3225 (12.0); 3.2025 (1.2); 2.6706 (0.6); 2.5244 (1.9); 2.5197 (2.6); 2.5110 (34.2); 2.5064 (74.6); 2.5018 (104.1); 2.4972 (71.1); 2.4926 (30.7); 2.3289 (0.6); 2.1831 (0.8); 1.9950 (1.0); 1.9090 (0.8); 1.7808 (0.7); 1.7761 (4.3); 1.7744 (1.9); 1.7684 (3.4); 1.7641 (2.1); 1.7595 (12.5); 1.7548 (2.2); 1.7505 (3.4); 1.7429 (4.3); 1.7383 (0.6); 1.3555 (7.5); 1.2390 (0.8); 1.0897 (0.5); 1.0744 (0.5); 0.0079 (1.6); 0.0038 (0.7); -0.0002 (63.1); -0.0027 (2.6); -0.0043 (0.9); - 0.0052 (0.6); -0.0060 (0.5); -0.0085 (1.8) |
| 1-118: ¹H-NMR(400.0 MHz, d₆-DMSO): |
| δ= 8.4563 (0.6); 8.4419 (1.2); 8.4273 (0.6); 8.2646 (1.7); 8.2586 (1.7); 8.1125 (1.2); 8.1100 (1.3); 8.1006 (1.2); 8.0978 (1.2); 8.0322 (0.7); 8.0261 (0.7); 8.0116 (1.1); 8.0052 (1.0); 7.9916 (0.7); 7.9853 (0.7); 7.9812 (0.9); 7.9765 (0.7); 7.9606 (1.2); 7.9421 (1.0); 7.9374 (0.9); 7.7760 (2.1); 7.7554 (1.7); 7.3105 (1.1); 7.3038 (1.2); 7.2982 (1.2); 7.2837 (2.0); 7.2653 (0.9); 4.8715 (6.8); 4.0557 (0.6); 4.0378 (1.8); 4.0200 (1.8); 4.0022 (0.6); 3.8496 (3.3); 3.8349 (3.3); 3.3177 (16.0); 2.6701 (0.6); 2.5236 (3.4); 2.5099 (36.6); 2.5055 (75.9); 2.5010 (103.8); 2.4965 (74.1); 2.4920 (34.4); 2.3277 (0.6); 1.9883 (7.7); 1.3551 (1.4); 1.1922 (2.1); 1.1745 (4.1); 1.1566 (2.0); 0.0080 (3.1); -0.0002 (88.2); -0.0085 (3.1) |
| 1-119: ¹H-NMR(400.6 MHz, d₆-DMSO): |
| δ= 8.2715 (1.8); 8.2653 (1.8); 8.1037 (1.1); 8.1017 (1.2); 8.0991 (1.4); 8.0971 (1.2); 8.0916 (1.2); 8.0896 (1.3); 8.0870 (1.3); 8.0849 (1.2); 8.0400 (0.8); 8.0338 (0.8); 8.0203 (1.1); 8.0189 (1.1); 8.0141 (1.0); 8.0127 (1.1); 7.9993 (0.9); 7.9930 (0.8); 7.9721 (1.0); 7.9675 (1.0); 7.9536 (1.2); 7.9515 (1.3); 7.9489 (1.2); 7.9468 (1.2); 7.9330 (1.3); 7.9283 (1.2); 7.6904 (1.3); 7.6883 (2.3); 7.6862 (1.3); 7.6698 (1.2); 7.6677 (2.0); 7.6655 (1.1); 7.3036 (1.2); 7.2980 (1.2); 7.2840 (2.2); 7.2819 (2.3); 7.2767 (1.2); 7.2753 (1.2); 7.2721 (1.2); 7.2696 (1.2); 7.2657 (1.2); 7.2633 (1.1); 7.2536 (1.1); 7.2511 (1.1); 5.7566 (16.0); 4.8364 (3.2); 4.4534 (0.6); 4.4280 (0.6); 4.0379 (1.4); 4.0201 (1.4); 3.3455 (0.5); 2.6703 (0.5); 2.5241 (1.3); 2.5194 (1.7); 2.5107 (26.6); 2.5061 (58.5); 2.5015 (82.1); 2.4969 (56.8); 2.4924 (25.0); 2.3286 (0.5); 1.9886 (6.7); 1.2355 (1.4); 1.1923 (2.0); 1.1745 (3.9); 1.1568 (1.8); 0.0080 (1.3); -0.0002 (50.4); -0.0051 (0.8); -0.0059 (0.6); -0.0067 (0.6); -0.0085 (1.5) |
| 1-119: ¹H-NMR(400.6 MHz, CDCl3): |
| δ= 8.2108 (2.6); 7.8399 (0.7); 7.8342 (0.8); 7.8191 (1.3); 7.8002 (0.8); 7.7943 (0.7); 7.7332 (0.8); 7.2612 (26.6); 7.1863 (1.4); 7.1724 (1.5); 7.1679 (1.5); 7.1539 (1.3); 7.0073 (1.5); 7.0003 (1.6); 6.9861 (1.5); 6.9792 (1.5); 5.3001 (16.0); 4.9022 (1.6); 4.1486 (0.8); 4.1307 (2.1); 4.1129 (2.1); 4.0950 (0.9); 2.0456 (9.1); 1.2837 (0.5); 1.2768 (2.8); 1.2590 (6.5); 1.2412 (2.8); 0.0080 (1.1); -0.0002 (43.6); -0.0085 (1.3) |
| 1-120: ¹H-NMR(400.6 MHz, CDCl3): |
| δ= 9.2075 (1.2); 8.2087 (1.6); 8.2067 (1.3); 8.2045 (1.3); 8.2024 (1.7); 8.0973 (1.0); 8.0953 (1.1); 8.0927 (1.2); 8.0906 (1.0); 8.0852 (1.1); 8.0832 (1.1); 8.0805 (1.1); 8.0785 (1.0); 7.8592 (0.9); 7.8530 (0.9); 7.8404 (1.1); 7.8381 (1.1); 7.8342 (1.0); 7.8318 (1.0); 7.8193 (1.0); 7.8160 (1.0); 7.8130 (1.1); 7.8114 (1.0); 7.7975 (1.0); 7.7954 (1.3); 7.7928 (1.0); 7.7907 (1.2); 7.7769 (1.2); 7.7722 (1.2); 7.6945 (1.3); 7.6923 (2.3); 7.6900 (1.3); 7.6739 (1.0); 7.6717 (1.6); 7.6694 (0.9); 7.2617 (18.1); 7.1651 (1.2); 7.1625 (1.2); 7.1529 (1.1); 7.1504 (1.2); 7.1467 (1.2); 7.1441 (1.1); 7.1345 (1.1); 7.1319 (1.0); 7.0147 (1.2); 7.0132 (1.2); 7.0073 (1.2); 7.0058 (1.1); 6.9936 (1.1); 6.9920 (1.1); 6.9862 (1.2); 6.9847 (1.1); 5.3005 (6.0); 4.9417 (11.9); 4.2445 (1.4); 4.2267 (4.5); 4.2088 (4.6); 4.1910 (1.6); 4.1802 (7.9); 4.1309 (0.6); 4.1131 (0.6); 3.0727 (16.0); 2.0455 (2.8); 1.5653 (1.8); 1.3064 (0.6); 1.3005 (5.8); 1.2827 (12.1); 1.2774 (1.2); 1.2648 (5.8); 1.2595 (2.1); 1.2417 (0.8); 0.0080 (0.8); -0.0002 (29.2); -0.0085 (0.8) |
| 1-22: ¹H-NMR(400.0 MHz, d₆-DMSO): |
| δ= 9.2496 (7.1); 8.8883 (13.9); 8.6788 (2.0); 8.0732 (1.5); 8.0610 (1.5); 8.0130 (1.1); 8.0084 (1.0); 7.9923 (1.8); 7.9738 (1.5); 7.9691 (1.2); 7.7084 (2.9); 7.6877 (2.6); 7.6393 (1.0); 7.6184 (0.9); 7.3226 (0.7); 7.3055 (0.7); 7.2939 (1.8); 7.2793 (1.5); 7.2753 (1.4); 7.2608 (1.4); 5.7568 (5.4); 5.1972 (0.7); 5.1797 (2.3); 5.1622 (2.2); 5.1451 (0.6); 5.1159 (0.6); 5.0986 (0.8); 3.3203 (16.0); 2.6702 (1.0); 2.5239 (2.8); 2.5192 (4.0); 2.5104 (53.4); 2.5059 (115.4); 2.5013 (161.0); 2.4968 (114.4); 2.4923 (52.1); 2.3328 (0.9); 2.3284 (1.2); 2.1168 (0.7); 1.6108 (7.8); 1.5935 (7.6); 1.5741 (2.4); 1.5566 (2.4); 1.5279 (0.6); 1.4686 (0.8); 1.4513 (1.5); 1.2983 (1.6); 1.2583 (2.4); 1.2346 (7.0); 1.1404 (1.9); 1.1068 (1.1); 1.0571 (0.9); 1.0418 (0.9); 1.0135 (2.6); 0.9998 (0.8); 0.8888 (0.5); 0.8720 (1.0); 0.8615 (0.9); 0.8535 (1.4); 0.8363 (0.7); 0.1460 (0.6); 0.0080 (4.0); -0.0002 (142.7); -0.0085 (4.7); -0.1496 (0.6) |
| 1-21: ¹H-NMR(400.6 MHz, d₆-DMSO): |
| δ= 8.6673 (2.8); 8.4204 (0.7); 8.4107 (0.7); 8.4084 (0.7); 8.3131 (5.6); 8.3002 (5.8); 8.1181 (2.9); 8.1161 (3.2); 8.1135 (3.6); 8.1115 (3.2); 8.1060 (3.2); 8.1040 (3.5); 8.1014 (3.5); 8.0994 (3.1); 8.0111 (2.5); 8.0064 (2.3); 7.9925 (3.5); 7.9905 (3.7); 7.9880 (3.5); 7.9859 (3.2); 7.9720 (3.2); 7.9673 (3.2); 7.9496 (0.7); 7.9451 (0.6); 7.6732 (6.0); 7.6548 (3.8); 7.6527 (5.4); 7.6388 (1.4); 7.6181 (1.1); 7.4282 (0.6); 7.4228 (0.7); 7.4119 (0.7); 7.4063 (1.0); 7.3997 (0.8); 7.3889 (0.7); 7.3834 (0.8); 7.3548 (0.8); 7.3479 (0.7); 7.3385 (0.9); 7.3136 (6.3); 7.3012 (6.5); 7.2993 (6.6); 7.2954 (5.3); 7.2830 (3.2); 7.2806 (2.9); 7.2438 (5.5); 6.3099 (1.0); 6.2869 (1.0); 6.1606 (0.5); 6.1577 (0.5); 6.1444 (1.0); 6.1416 (0.9); 5.7569 (12.6); 5.1530 (1.1); 5.1358 (3.8); 5.1184 (4.0); 5.0995 (1.6); 5.0816 (0.9); 4.0557 (0.8); 4.0378 (2.5); 4.0200 (2.5); 4.0022 (0.9); 3.3889 (2.1); 3.3268 (7.8); 2.6754 (0.9); 2.6708 (1.3); 2.6661 (0.9); 2.5412 (1.4); 2.5346 (1.6); 2.5301 (2.4); 2.5246 (4.6); 2.5197 (6.4); 2.5110 (71.1); 2.5065 (148.2); 2.5019 (202.3); 2.4973 (146.2); 2.4928 (72.4); 2.4720 (5.0); 2.4673 (4.4); 2.3335 (1.3); 2.3290 (1.6); 2.3245 (1.3); 2.1172 (1.5); 1.9888 (11.1); 1.9079 (2.2); 1.7542 (0.5); 1.5867 (15.4); 1.5693 (16.0); 1.5641 (6.1); 1.5465 (3.8); 1.5201 (0.7); 1.4509 (0.6); 1.2983 (1.3); 1.2585 (2.1); 1.2355 (6.4); 1.1923 (3.6); 1.1745 (6.7); 1.1567 (3.4); 1.1404 (4.1); 1.1067 (1.6); 1.0573 (1.2); 1.0419 (1.2); 1.0223 (0.8); 1.0133 (0.7); 1.0066 (0.9); 0.9997 (0.6); 0.8884 (0.7); 0.8726 (1.3); 0.8537 (1.5); 0.8363 (0.7); 0.1457 (0.5); 0.0279 (1.3); 0.0080 (5.6); -0.0002 (170.9); -0.0085 (8.4); -0.0344 (1.1); -0.0377 (1.0); -0.1494 (0.7) |
| 1-20: ¹H-NMR(400.0 MHz, d₆-DMSO): |
| δ= 8.6435 (2.7); 8.6360 (0.8); 8.4120 (0.7); 8.3999 (0.8); 8.2493 (3.0); 8.2431 (3.3); 8.0967 (2.5); 8.0844 (2.4); 8.0166 (1.1); 8.0105 (1.2); 7.9964 (1.8); 7.9899 (2.0); 7.9743 (2.5); 7.9695 (2.9); 7.9537 (3.0); 7.9349 (2.2); 7.9302 (2.1); 7.6619 (4.0); 7.6411 (3.8); 7.6169 (0.9); 7.4066 (0.5); 7.3000 (2.5); 7.2935 (2.8); 7.2778 (4.1); 7.2634 (2.2); 7.2591 (2.0); 7.2448 (1.9); 6.3103 (0.5); 5.7568 (16.0); 5.1154 (0.7); 5.0981 (2.5); 5.0807 (2.8); 5.0641 (1.5); 5.0461 (0.8); 4.0379 (1.0); 4.0202 (1.0); 3.3894 (3.8); 3.3388 (6.7); 2.6714 (1.0); 2.5415 (3.2); 2.5247 (2.1); 2.5112 (51.6); 2.5067 (112.5); 2.5021 (157.4); 2.4975 (111.9); 2.4931 (50.8); 2.3332 (0.7); 2.3289 (0.9); 2.1174 (0.6); 1.9888 (4.3); 1.9063 (1.1); 1.5668 (9.4); 1.5494 (9.8); 1.5257 (3.2); 1.3380 (1.5); 1.2977 (0.8); 1.2816 (0.6); 1.2585 (0.9); 1.2348 (5.4); 1.1924 (1.2); 1.1746 (2.3); 1.1567 (1.2); 1.1403 (1.4); 0.8722 (0.6); 0.8537 (1.0); 0.0080 (2.8); -0.0002 (95.8); -0.0085 (3.0) |
| 1-20: ¹H-NMR(400.6 MHz, d₆-DMSO): |
| δ= 8.2714 (1.2); 8.2652 (1.3); 8.1180 (0.8); 8.1160 (0.9); 8.1134 (1.0); 8.1113 (0.9); 8.1059 (0.8); 8.1039 (1.0); 8.1012 (1.0); 8.0992 (0.9); 8.0409 (0.6); 8.0347 (0.6); 8.0213 (0.8); 8.0198 (0.8); 8.0135 (0.8); 7.9984 (1.0); 7.9937 (1.3); 7.9798 (0.9); 7.9777 (1.0); 7.9751 (0.8); 7.9730 (0.9); 7.9592 (1.0); 7.9544 (0.9); 7.6610 (1.0); 7.6587 (1.8); 7.6565 (1.0); 7.6404 (0.9); 7.6381 (1.6); 7.6358 (0.9); 7.3063 (0.8); 7.2992 (0.9); 7.2976 (1.1); 7.2950 (1.0); 7.2853 (1.6); 7.2830 (1.2); 7.2790 (1.7); 7.2767 (1.1); 7.2668 (0.9); 7.2643 (0.9); 5.1628 (1.8); 5.1454 (1.8); 4.0556 (1.1); 4.0378 (3.4); 4.0200 (3.4); 4.0022 (1.1); 3.6011 (0.8); 3.3209 (6.7); 2.5240 (0.9); 2.5193 (1.2); 2.5105 (17.7); 2.5059 (39.0); 2.5014 (54.7); 2.4968 (37.6); 2.4922 (16.5); 1.9886 (16.0); 1.9086 (4.1); 1.7596 (1.0); 1.6032 (4.9); 1.5857 (4.9); 1.1922 (4.8); 1.1744 (9.5); 1.1567 (4.5); 0.0080 (0.8); -0.0002 (35.3); -0.0085 (1.0) |
| 1-123: ¹H-NMR(400.0 MHz, CDCl3): |
| δ= 8.6124 (0.7); 8.6062 (0.6); 8.5847 (3.8); 8.5788 (3.7); 8.3972 (3.8); 8.3926 (3.7); 8.3737 (0.6); 8.3690 (0.6); 8.1031 (1.4); 8.0988 (1.6); 8.0910 (1.4); 8.0877 (2.1); 8.0843 (1.2); 7.8538 (0.6); 7.8333 (0.8); 7.8289 (0.6); 7.8127 (2.2); 7.8083 (2.3); 7.8006 (2.7); 7.7975 (4.1); 7.7952 (3.7); 7.7930 (4.4); 7.7776 (0.9); 7.7719 (0.7); 7.7666 (0.5); 7.7490 (2.7); 7.7438 (3.8); 7.7385 (2.4); 7.2616 (45.4); 7.1818 (1.4); 7.1762 (1.3); 7.1696 (1.4); 7.1663 (1.4); 7.1636 (1.4); 7.1607 (1.4); 7.1547 (1.2); 7.1485 (1.2); 4.2681 (2.0); 4.2502 (6.2); 4.2324 (6.3); 4.2145 (2.1); 3.9438 (16.0); 1.6012 (0.9); 1.5160 (1.3); 1.4928 (0.8); 1.3029 (7.0); 1.2851 (14.1); 1.2672 (6.9); 0.0080 (1.0); -0.0002 (28.8); -0.0085 (1.3) |
| 1-123: ¹H-NMR(400.0 MHz, CDCl3): |
| δ= 8.6236 (3.8); 8.6178 (3.8); 8.3939 (3.8); 8.3892 (3.7); 8.1856 (1.3); 8.1834 (1.4); 8.1813 (1.5); 8.1790 (1.4); 8.1736 (1.3); 8.1710 (1.6); 8.1695 (1.6); 8.1669 (1.2); 7.9170 (0.6); 7.9125 (0.6); 7.8999 (0.6); 7.8964 (2.0); 7.8920 (1.9); 7.8794 (2.4); 7.8753 (4.2); 7.8736 (2.9); 7.8716 (2.8); 7.8690 (2.4); 7.8551 (0.8); 7.8529 (0.8); 7.7589 (2.7); 7.7535 (3.7); 7.7484 (2.6); 7.3065 (1.4); 7.3023 (1.3); 7.2944 (1.3); 7.2897 (2.4); 7.2853 (1.2); 7.2776 (1.4); 7.2731 (1.4); 7.2627 (34.0); 5.3004 (2.1); 4.4141 (16.0); 4.2800 (1.8); 4.2622 (5.9); 4.2443 (6.1); 4.2265 (2.0); 2.0916 (1.6); 1.2962 (7.1); 1.2784 (14.8); 1.2605 (7.1); 0.0079 (0.8); - 0.0002 (21.9); -0.0085 (0.6) |
| 1-124: ¹H-NMR(400.6 MHz, CDCl3): |
| δ= 8.5836 (3.1); 8.5777 (3.2); 8.3964 (3.1); 8.3917 (3.2); 8.1031 (1.1); 8.1007 (1.3); 8.0988 (1.4); 8.0964 (1.3); 8.0911 (1.2); 8.0873 (1.8); 8.0842 (1.2); 7.8333 (0.6); 7.8126 (2.1); 7.8082 (2.2); 7.8007 (2.3); 7.7972 (3.5); 7.7951 (3.2); 7.7931 (3.6); 7.7925 (3.6); 7.7801 (0.6); 7.7777 (0.5); 7.7471 (2.7); 7.7423 (3.1); 7.7413 (3.3); 7.7365 (2.7); 7.2639 (7.0); 7.1816 (1.3); 7.1760 (1.2); 7.1694 (1.3); 7.1660 (1.4); 7.1634 (1.3); 7.1605 (1.3); 7.1544 (1.3); 7.1484 (1.2); 5.2999 (4.0); 4.2678 (2.0); 4.2500 (6.3); 4.2321 (6.4); 4.2143 (2.1); 3.9442 (15.9); 1.6173 (0.9); 1.4921 (0.7); 1.3026 (7.6); 1.2848 (16.0); 1.2670 (7.6); 1.2554 (0.6); -0.0002 (9.4) |
| 1-124: ¹H-NMR(400.6 MHz, CDCl3): |
| δ= 8.5804 (4.5); 8.5752 (4.7); 8.3908 (5.0); 8.3873 (5.1); 8.1760 (2.5); 8.1652 (2.6); 7.8620 (2.5); 7.8574 (2.5); 7.8439 (2.6); 7.8415 (5.0); 7.8394 (3.2); 7.8369 (5.0); 7.8235 (5.4); 7.8188 (5.5); 7.8082 (16.0); 7.7927 (5.3); 7.7725 (2.5); 7.7623 (7.3); 7.7566 (10.1); 7.7517 (7.5); 7.2621 (25.5); 7.2320 (2.9); 7.2291 (2.8); 7.2199 (2.9); 7.2168 (3.2); 7.2140 (3.2); 7.2110 (3.0); 7.2018 (2.6); 7.1989 (2.7); 2.3419 (0.8); 2.3229 (0.6); 1.5277 (3.9); 1.4922 (1.4); 1.4512 (1.3); 1.3336 (0.6); 1.3027 (0.9); 1.2845 (1.6); 1.2542 (10.4); 0.8959 (0.6); 0.8792 (1.9); 0.8616 (0.8); 0.0080 (1.0); -0.0002 (35.0); -0.0084 (1.1) |
| 1-124: ¹H-NMR(400.6 MHz, CDCl3): |
| δ= 8.5818 (0.8); 8.5764 (0.8); 8.4048 (0.8); 8.4001 (0.9); 7.8249 (0.6); 7.8213 (0.7); 7.8191 (0.6); 7.8140 (0.6); 7.8097 (0.5); 7.7924 (0.6); 7.7580 (0.7); 7.7522 (0.8); 7.7475 (0.6); 7.2604 (72.1); 5.3002 (0.8); 2.6590 (1.2); 1.5611 (16.0); 1.5443 (3.2); 1.3137 (0.9); 1.3073 (0.7); 1.2972 (0.5); 1.2843 (0.8); 1.2562 (1.9); 0.0079 (2.5); 0.0063 (0.5); 0.0055 (0.6); 0.0046 (0.7); -0.0002 (94.4); -0.0068 (1.2); -0.0085 (3.0) |
| 1-124: ¹H-NMR(400.6 MHz, CDCl3): |
| δ= 8.5780 (5.7); 8.5722 (5.7); 8.3895 (5.4); 8.3849 (5.6); 8.1785 (1.9); 8.1764 (2.1); 8.1739 (2.3); 8.1718 (2.1); 8.1664 (2.1); 8.1642 (2.2); 8.1618 (2.3); 8.1596 (2.1); 7.8614 (1.7); 7.8567 (1.7); 7.8433 (1.7); 7.8409 (3.2); 7.8388 (1.9); 7.8363 (3.2); 7.8228 (3.5); 7.8182 (3.6); 7.8068 (16.0); 7.7943 (3.4); 7.7917 (5.1); 7.7891 (3.7); 7.7738 (1.7); 7.7713 (2.3); 7.7686 (1.6); 7.7619 (5.0); 7.7571 (5.6); 7.7562 (5.9); 7.7514 (5.0); 7.2634 (11.6); 7.2335 (0.5); 7.2307 (3.1); 7.2277 (3.0); 7.2186 (2.6); 7.2155 (2.7); 7.2126 (2.8); 7.2095 (2.7); 7.2005 (2.4); 7.1974 (2.3); 5.2990 (10.0); 4.8158 (0.6); 4.5075 (0.6); 4.4897 (0.6); 4.4572 (0.6); 4.4393 (0.6); 4.2500 (0.5); 4.2457 (0.6); 4.2319 (0.5); 4.2278 (1.7); 4.2159 (0.5); 4.2100 (1.8); 4.1981 (0.5); 4.1921 (0.6); 3.9441 (0.9); 3.6124 (0.6); 3.6040 (0.6); 3.5829 (3.5); 1.6631 (1.2); 1.5532 (4.0); 1.5514 (4.0); 1.5261 (0.7); 1.4527 (0.8); 1.4511 (1.0); 1.4349 (1.4); 1.4170 (0.7); 1.4059 (0.7); 1.3881 (1.6); 1.3702 (1.0); 1.3423 (0.6); 1.3245 (1.2); 1.3195 (1.6); 1.3174 (0.8); 1.3148 (0.8); 1.3066 (0.8); 1.3017 (3.5); 1.2996 (1.7); 1.2970 (1.4); 1.2872 (0.9); 1.2840 (2.2); 1.2818 (1.0); 1.2792 (0.8); 1.2694 (0.6); 1.2668 (0.8); 1.2540 (1.4); -0.0002 (16.3) |
| 1-124: ¹H-NMR(400.6 MHz, CDCl3): |
| δ= 8.5836 (3.1); 8.5782 (3.4); 8.5777 (3.6); 8.3971 (3.0); 8.3929 (3.2); 8.3924 (3.2); 8.1026 (1.1); 8.1002 (1.2); 8.0983 (1.4); 8.0958 (1.2); 8.0905 (1.2); 8.0869 (1.8); 8.0837 (1.2); 7.8333 (0.5); 7.8127 (1.9); 7.8082 (2.2); 7.8067 (1.1); 7.8010 (2.2); 7.7984 (2.6); 7.7972 (3.2); 7.7954 (3.1); 7.7929 (3.8); 7.7805 (0.5); 7.7779 (0.5); 7.7479 (2.7); 7.7431 (3.1); 7.7421 (3.2); 7.7373 (2.6); 7.2654 (5.0); 7.1813 (1.3); 7.1757 (1.2); 7.1692 (1.2); 7.1659 (1.4); 7.1630 (1.2); 7.1603 (1.3); 7.1543 (1.2); 7.1481 (1.2); 5.2996 (16.0); 4.2677 (1.9); 4.2498 (6.0); 4.2320 (6.1); 4.2142 (2.0); 3.9446 (15.3); 2.0448 (0.7); 1.4915 (0.6); 1.3025 (7.5); 1.2847 (15.4); 1.2668 (7.4); 1.2589 (0.7); 0.8810 (0.6); -0.0002 (7.0) |
| 1-124: ¹H-NMR(400.6 MHz, CDCl3): |
| δ= 8.5819 (0.8); 8.5761 (0.7); 8.4049 (0.7); 8.4002 (0.8); 7.8220 (0.5); 7.8208 (0.5); 7.7579 (0.7); 7.7531 (0.7); 7.7521 (0.8); 7.7473 (0.6); 7.2608 (14.5); 5.3001 (2.4); 1.5610 (16.0); 1.3476 (1.7); 1.2596 (0.8); 0.8819 (1.0); 0.0079 (0.6); 0.0022 (0.7); - 0.0002 (19.1); -0.0084 (0.6) |
| 1-124: ¹H-NMR(400.6 MHz, CDCl3): |
| δ= 7.2613 (7.4); 4.2463 (1.7); 4.2284 (5.3); 4.2106 (5.4); 4.1928 (1.8); 3.5827 (16.0); 1.5457 (3.0); 1.3203 (6.4); 1.3025 (13.1); 1.2846 (6.3); -0.0002 (10.5) |
| 1-125: ¹H-NMR(400.0 MHz, d₆-DMSO): |
| δ= 8.6452 (0.5); 8.6382 (0.5); 8.3818 (0.6); 8.1263 (1.3); 7.8305 (0.6); 4.1539 (0.7); 4.1361 (2.3); 4.1183 (2.3); 4.1005 (0.8); 3.7262 (6.2); 3.3201 (16.0); 2.5105 (3.9); 2.5060 (8.8); 2.5015 (12.5); 2.4969 (9.0); 2.4924 (4.2); 1.2285 (2.5); 1.2107 (5.1); 1.1929 (2.5); -0.0002 (6.5) |
| 1-125: ¹H-NMR(400.0 MHz, d₆-DMSO): |
| δ= 4.1542 (1.9); 4.1364 (5.9); 4.1186 (6.0); 4.1009 (2.0); 3.7246 (16.0); 3.3117 (1.6); 2.5099 (1.1); 2.5056 (2.5); 2.5011 (3.5); 2.4966 (2.5); 2.4922 (1.2); 1.2294 (6.3); 1.2116 (12.6); 1.1938 (6.1); -0.0002 (1.6) |
| 1-126: ¹H-NMR(400.6 MHz, CDCl3): |
| δ= 8.6098 (3.6); 8.6040 (3.7); 8.4036 (3.6); 8.3990 (3.7); 8.1282 (1.4); 8.1255 (1.5); 8.1180 (1.4); 8.1161 (1.5); 8.1134 (1.5); 7.8357 (1.0); 7.8310 (1.0); 7.8170 (1.4); 7.8151 (1.7); 7.8125 (1.4); 7.8104 (1.6); 7.7967 (1.7); 7.7920 (1.7); 7.7548 (2.9); 7.7498 (4.0); 7.7443 (2.8); 7.7239 (3.1); 7.7219 (1.9); 7.7034 (2.1); 7.2613 (65.4); 7.2064 (1.3); 7.2039 (1.3); 7.1943 (1.3); 7.1917 (1.4); 7.1880 (1.4); 7.1855 (1.3); 7.1758 (1.3); 7.1733 (1.2); 5.3003 (1.6); 3.9602 (16.0); 2.1038 (12.7); 1.2595 (0.6); 1.2556 (0.6); 0.0079 (1.2); -0.0002 (48.8); -0.0085 (1.6) |
| II-09: ¹H-NMR(400.0 MHz, CDCl3): |
| δ= 8.5139 (1.5); 8.5030 (1.5); 8.4435 (1.9); 8.1949 (2.9); 8.1891 (2.9); 7.7413 (1.0); 7.7352 (1.0); 7.7205 (1.5); 7.7145 (1.5); 7.7016 (1.1); 7.6955 (1.0); 7.4858 (1.4); 7.4645 (1.6); 7.2926 (1.3); 7.2808 (1.5); 7.2718 (1.6); 7.2608 (82.8); 7.0153 (1.8); 7.0081 (1.9); 6.9942 (1.7); 6.9867 (1.7); 4.9214 (16.0); 4.3124 (1.9); 4.2945 (6.0); 4.2766 (6.0); 4.2589 (2.0); 2.9572 (0.9); 2.8845 (0.7); 1.5848 (2.3); 1.3288 (6.7); 1.3110 (13.5); 1.2932 (6.6); 0.0080 (1.2); -0.0002 (43.2); -0.0084 (1.4) |
| III-08: ¹H-NMR(400.6 MHz, CDCl3): |
| δ= 8.5203 (1.6); 8.5065 (1.6); 8.2626 (1.2); 8.2606 (1.8); 8.2586 (1.5); 8.2565 (1.5); 8.2544 (1.9); 8.2524 (1.4); 7.7684 (1.0); 7.7622 (1.0); 7.7499 (1.2); 7.7473 (1.3); 7.7437 (1.2); 7.7410 (1.3); 7.7288 (1.2); 7.7225 (1.2); 7.2625 (28.3); 7.0724 (1.3); 7.0708 (1.4); 7.0648 (1.6); 7.0632 (1.8); 7.0589 (3.2); 7.0549 (2.3); 7.0513 (1.9); 7.0496 (2.1); 7.0471 (2.4); 7.0434 (4.2); 5.3005 (1.7); 4.9364 (13.7); 4.3247 (1.8); 4.3069 (5.9); 4.2891 (6.0); 4.2713 (2.0); 2.7729 (4.0); 1.3386 (7.7); 1.3208 (16.0); 1.3030 (7.6); 1.2847 (0.5); 0.0079 (0.7); -0.0002 (25.2); -0.0052 (0.5); -0.0085 (0.8) |
| III-09: ¹H-NMR(400.6 MHz, d₆-DMSO): |
| δ= 13.1233 (0.7); 8.5404 (6.3); 8.5364 (4.4); 8.5288 (4.4); 8.5248 (6.5); 8.3405 (3.4); 8.3342 (3.7); 8.0736 (1.5); 8.0674 (1.5); 8.0542 (1.8); 8.0525 (2.0); 8.0480 (1.9); 8.0462 (2.0); 8.0331 (1.6); 8.0268 (1.6); 7.3970 (2.3); 7.3915 (2.3); 7.3757 (2.2); 7.3702 (2.1); 7.1641 (7.8); 7.1602 (5.2); 7.1525 (5.2); 7.1485 (7.8); 5.4145 (0.7); 4.9237 (16.0); 3.6010 (1.0); 3.3361 (9.1); 2.6792 (0.6); 2.6745 (1.4); 2.6698 (1.9); 2.6653 (1.3); 2.6606 (0.6); 2.5643 (7.0); 2.5237 (5.5); 2.5190 (7.1); 2.5102 (102.6); 2.5057 (226.2); 2.5011 (317.8); 2.4965 (217.1); 2.4919 (95.5); 2.3374 (0.6); 2.3327 (1.3); 2.3281 (1.9); 2.3235 (1.3); 2.3189 (0.6); 2.1830 (0.5); 1.9083 (1.3); 1.7596 (1.2); 1.3549 (4.9); 1.2980 (1.0); 1.2584 (1.6); 1.2352 (1.8); 1.1809 (0.5); 0.8568 (0.6); 0.8404 (0.6); 0.0080 (3.3); -0.0002 (131.7); -0.0085 (3.6) |
| 1-122: ¹H-NMR(400.0 MHz, d₆-DMSO): |
| δ= 8.3153 (3.4); 8.3024 (3.5); 8.1155 (1.8); 8.1128 (1.9); 8.1110 (1.8); 8.1033 (1.9); 8.1006 (1.9); 8.0989 (1.8); 8.0130 (1.3); 8.0084 (1.2); 7.9926 (1.9); 7.9881 (1.8); 7.9738 (1.5); 7.9692 (1.4); 7.6986 (3.3); 7.6780 (3.0); 7.3172 (3.0); 7.3149 (3.1); 7.3051 (3.2); 7.3025 (3.1); 7.2985 (1.8); 7.2962 (1.7); 7.2862 (1.5); 7.2840 (1.6); 7.2533 (3.8); 4.9274 (12.7); 3.3292 (16.0); 2.5245 (0.7); 2.5199 (1.0); 2.5110 (17.2); 2.5066 (37.9); 2.5021 (53.1); 2.4975 (38.0); 2.4930 (17.4); 2.0742 (2.6); 1.2344 (0.7) |
| CDCl3_BAES11895-1-1: 1.2 (t, 3H), 1.7 (d, 3H), 4.3 (q, 2H), 6.9 (m, 1H), 7.1 (m, 1H), 7.6 (d, 1H), 7.7 (t, 1H), 7.8 (t, 1H), 8.1 (m, 1H), 8.2 (s, 1H) |
| CDCl3: 1.4 (t, 3H), 4.35 (q, 2H), 5.0 (s, 2H), 6.9 (m, 1H), 7.1 (m, 1H), 7.6 (d, 1H), 7.6-7.7 (m, 2H), 8.1 (d, 1H), 8.2 (s, 1H) |

### B. Formulierungsbeispiele

a) Ein Stäubemittel wird erhalten, indem man 10 Gew. Teile einer Verbindung der Formel (I) und/oder deren Salze und 90 Gew. Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I) und/oder deren Salze, 64 Gew. Teile kaolinhaltigen Quarz als Inertstoff, 10 Gewichtsteile ligninsulfonsaures Kalium und 1 Gew. Teil oleoylmethyltaurinsaures Natrium als Netz und Dispergiermittel mischt und in einer Stiftmühle mahlt.
c) Ein in Wasser leicht dispergierbares Dispersionskonzentrat wird erhalten, indem man 20 Gew. Teile einer Verbindung der Formel (I) und/oder deren Salze mit 6 Gew. Teilen
   Alkylphenolpolyglykolether (^{®}Triton X 207), 3 Gew. Teilen Isotridecanolpolyglykolether (8 EO) und 71 Gew. Teilen paraffinischem Mineralöl (Siedebereich z.B. ca. 255 bis über 277 C) mischt und in einer Reibkugelmühle auf eine Feinheit von unter 5 Mikron vermahlt.
d) Ein emulgierbares Konzentrat wird erhalten aus 15 Gew. Teilen einer Verbindung der Formel (I) und/oder deren Salze, 75 Gew. Teilen Cyclohexanon als Lösungsmittel und 10 Gew. Teilen oxethyliertes Nonylphenol als Emulgator.
e) Ein in Wasser dispergierbares Granulat wird erhalten indem man
   75 Gew. Teile einer Verbindung der Formel (I) und/oder deren Salze,
   10 Gew. Teile ligninsulfonsaures Calcium,
   5 Gew. Teile Natriumlaurylsulfat,
   3 Gew. Teile Polyvinylalkohol und
   7 Gew. Teile Kaolin
   mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man
   25 Gew. Teile einer Verbindung der Formel (I) und/oder deren Salze,
   5 Gew. Teile 2,2' Dinaphthylmethan 6,6' disulfonsaures Natrium,
   2 Gew. Teile oleoylmethyltaurinsaures Natrium,
   1 Gew. Teil Polyvinylalkohol,
   17 Gew. Teile Calciumcarbonat und
   50 Gew. Teile Wasser
   auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Herbizide Wirkung bzw. Kulturpflanzenverträglichkeit im Vorauflauf

Samen von mono- bzw. dikotylen Unkraut und Kulturpflanzen werden in Kunststoff- oder organischen Pflanztöpfen ausgelegt und mit Erde abgedeckt. Die in Form von benetzbaren Pulvern (WP) oder als Emulsionskonzentrate (EC) formulierten erfindungsgemäßen Verbindungen werden dann als wässrige Suspension bzw. Emulsion unter Zusatz von 0,5% Additiv mit einer Wasseraufwandmenge von umgerechnet 600 l/ha auf die Oberfläche der Abdeckerde appliziert. Nach der Behandlung werden die Töpfe im Gewächshaus aufgestellt und unter guten Wachstumsbedingungen für die Testpflanzen gehalten. Nach ca. 3 Wochen wird die Wirkung der Präparate visuell im Vergleich zu unbehandelten Kontrollen in Prozentwerten bonitiert. Beispielsweise bedeutet 100% Wirkung = Pflanzen sind abgestorben, 0% Wirkung = wie Kontrollpflanzen.

In den nachstehenden Tabellen 1 bis 11 sind die Wirkungen ausgewählter Verbindungen der allgemeinen Formel (I) auf verschiedene Schadpflanzen und einer Aufwandmenge entsprechend 320 g/ha, die gemäß zuvor genannter Versuchsvorschrift erhalten wurden, dargestellt.

### Unerwünschte Pflanzen / Weeds:

| | | | |
|---|---|---|---|
| ALOMY: | Alopecurus myosuroides | SETVI: | Setaria viridis |
| AVEFA: | Avena fatua | POLCO: | Fallopia convolvulus |
| AMARE: | Amaranthus retroflexus | ECHCG: | Echinochloa crus-galli |
| LOLRI: | Lolium rigidum | STEME: | Stellaria media |
| VERPE: | Veronica persica | MATIN: | Tripleurospermum inodorum |
| DIGSA: | Digitaria sanguinalis | ABUTH: | Abutylon threophrasti |

### 1. Vorauflaufwirksamkeit

Wie die Ergebnisse aus den Tabellen 1 bis 10 zeigen, weisen erfindungsgemäße Verbindungen eine gute herbizide Vorauflaufwirksamkeit gegen ein breites Spektrum von Ungräsern und Unkräutern auf.

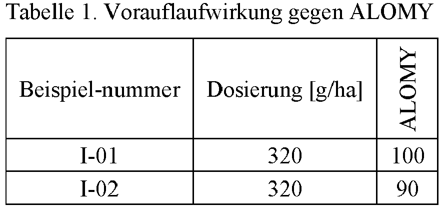

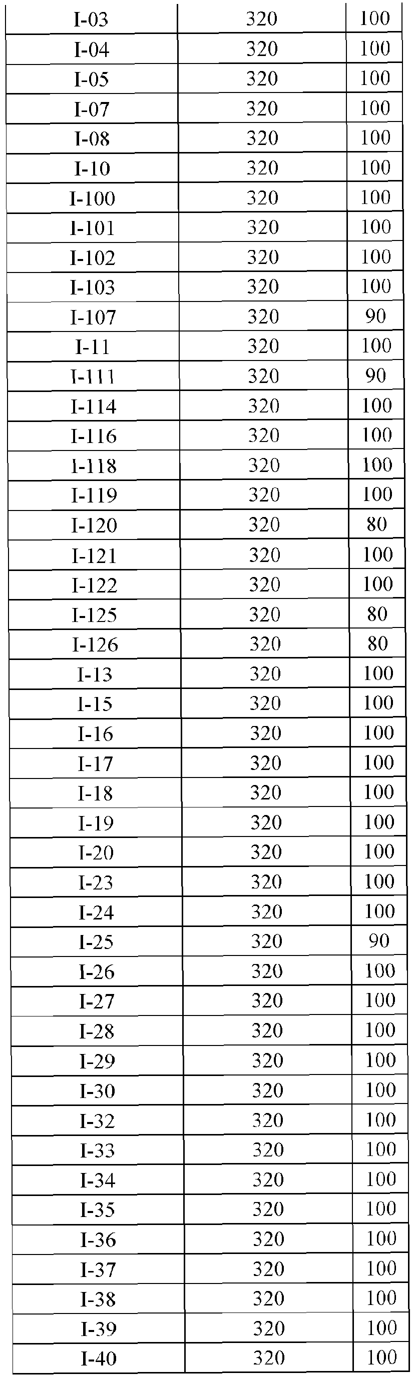

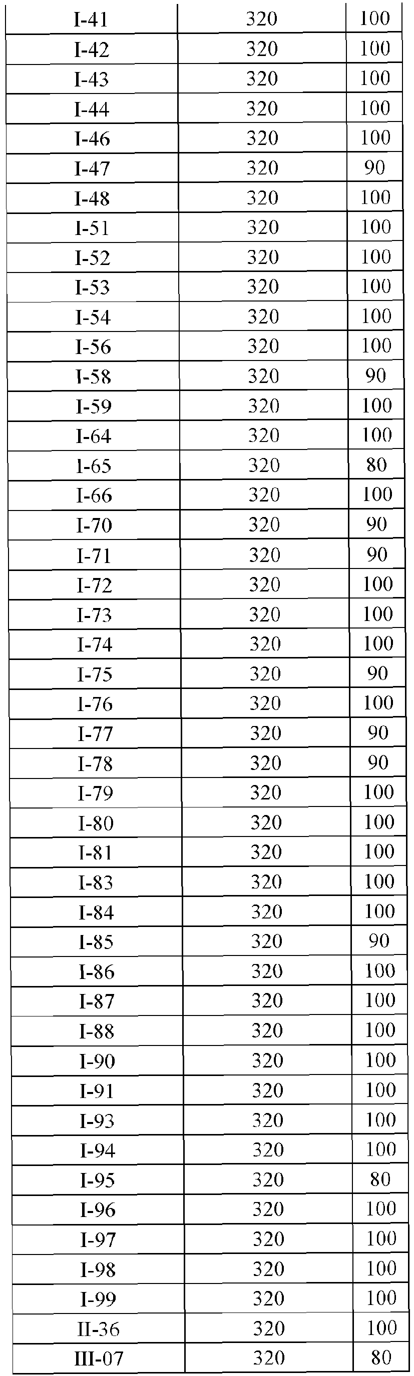

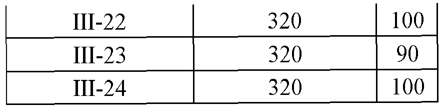

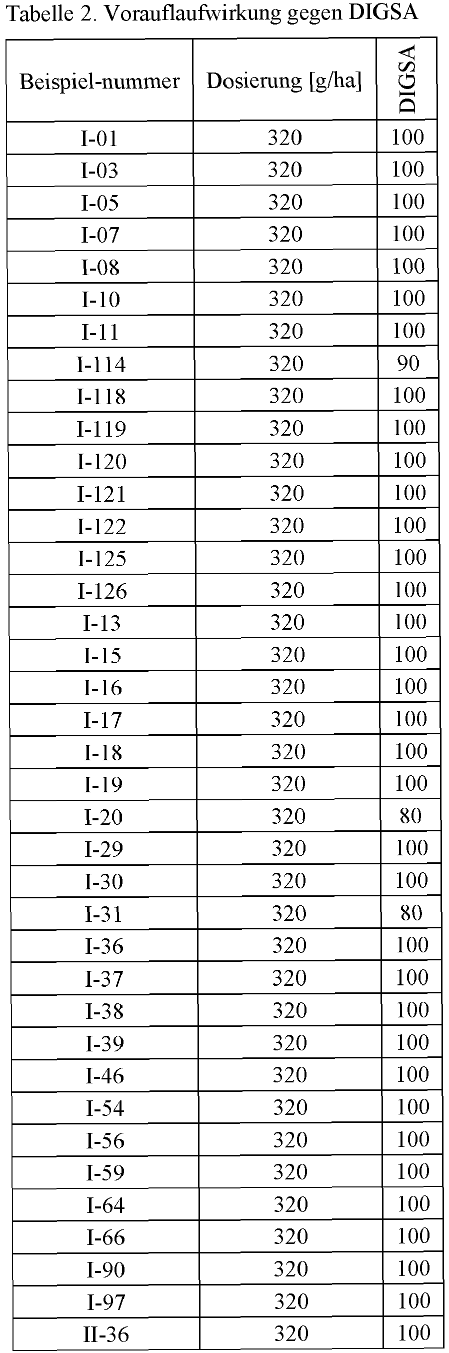

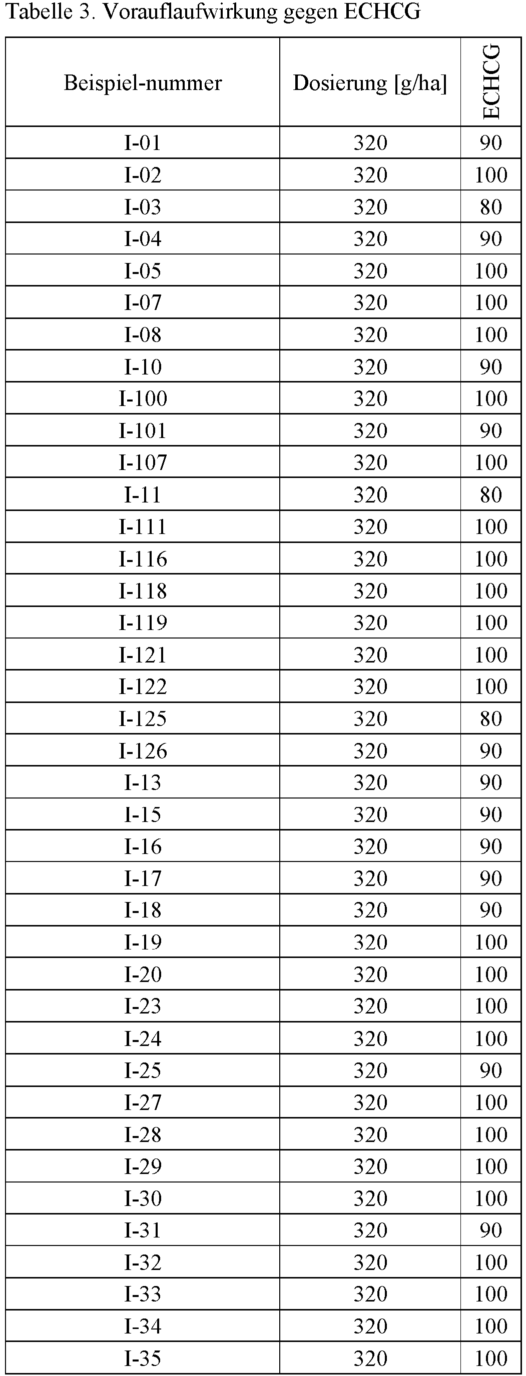

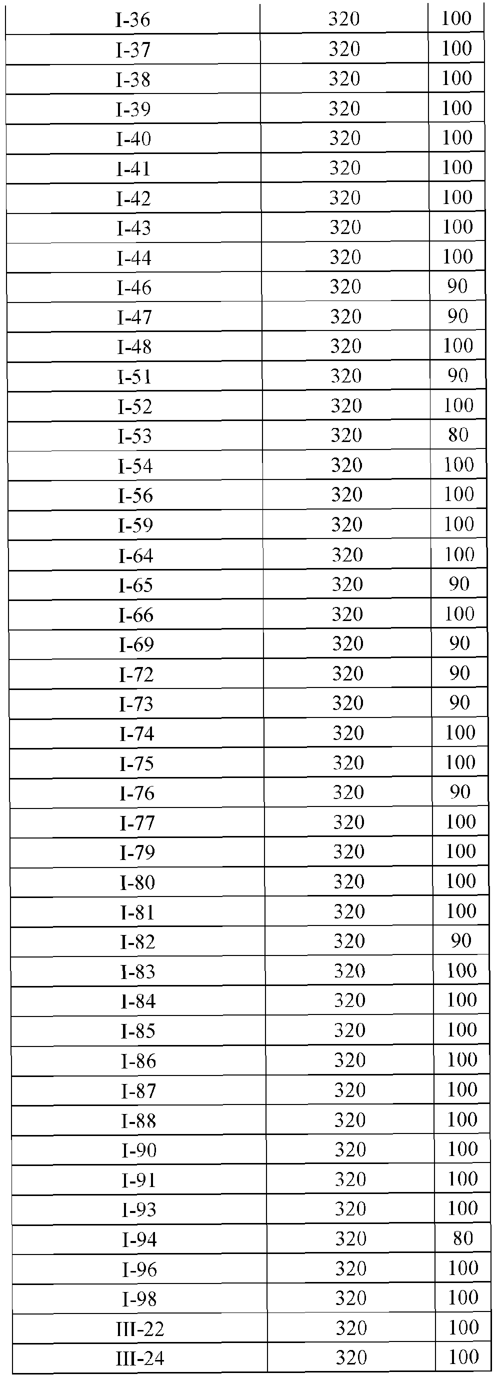

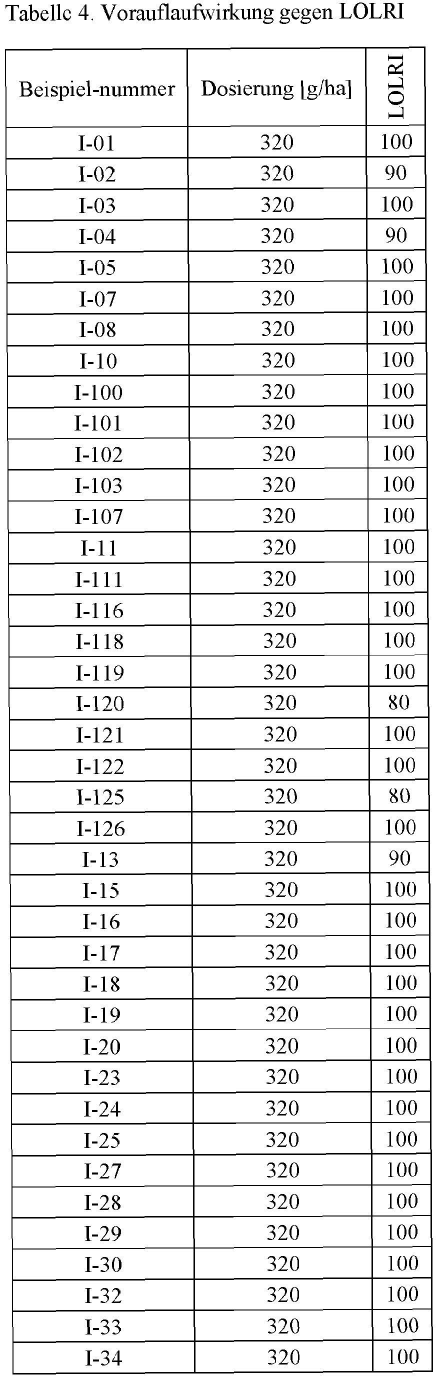

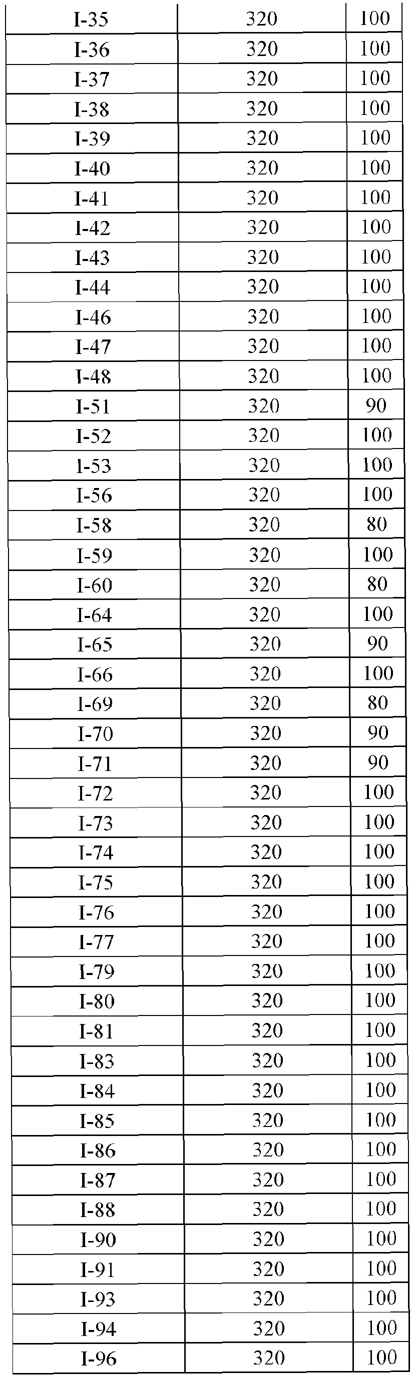

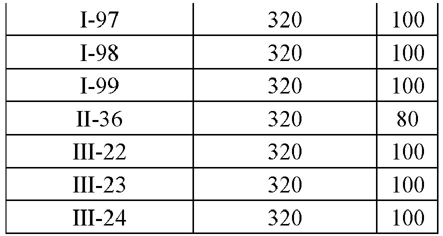

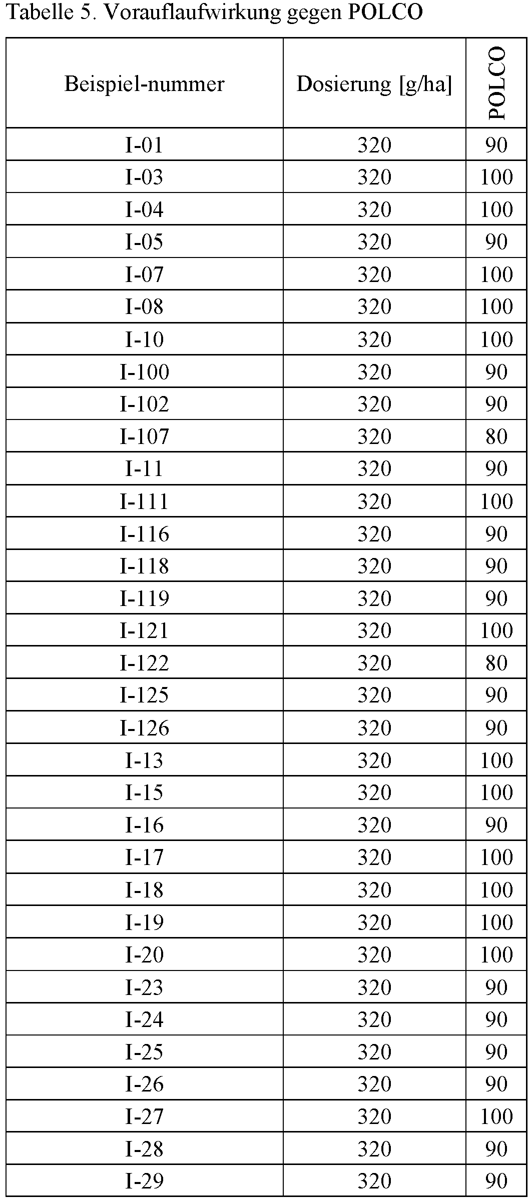

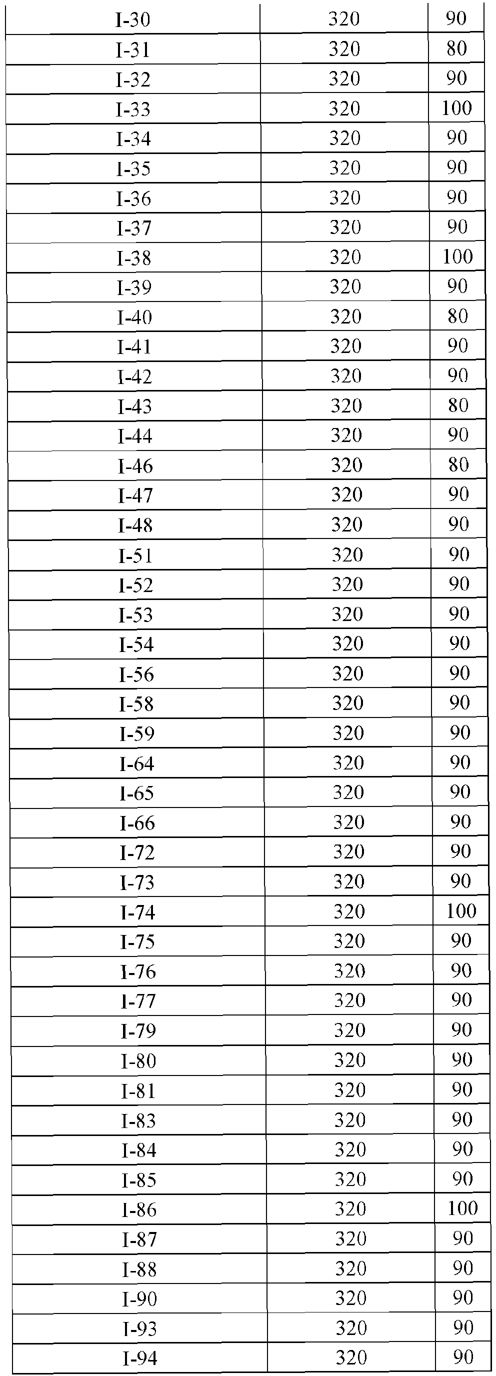

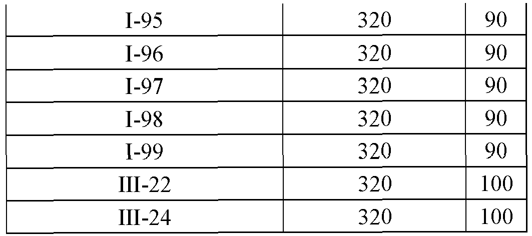

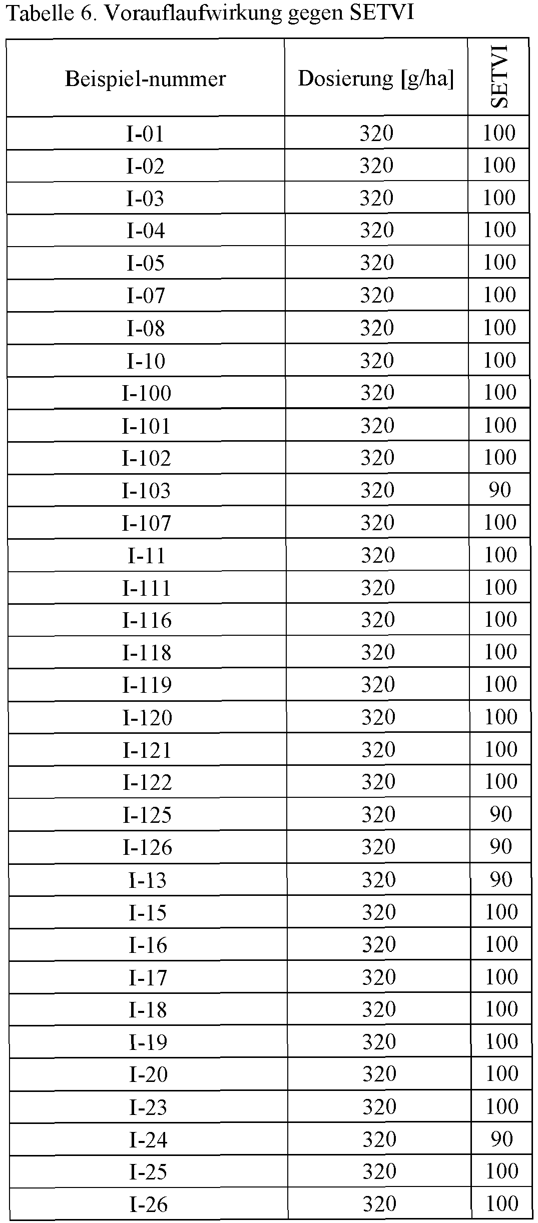

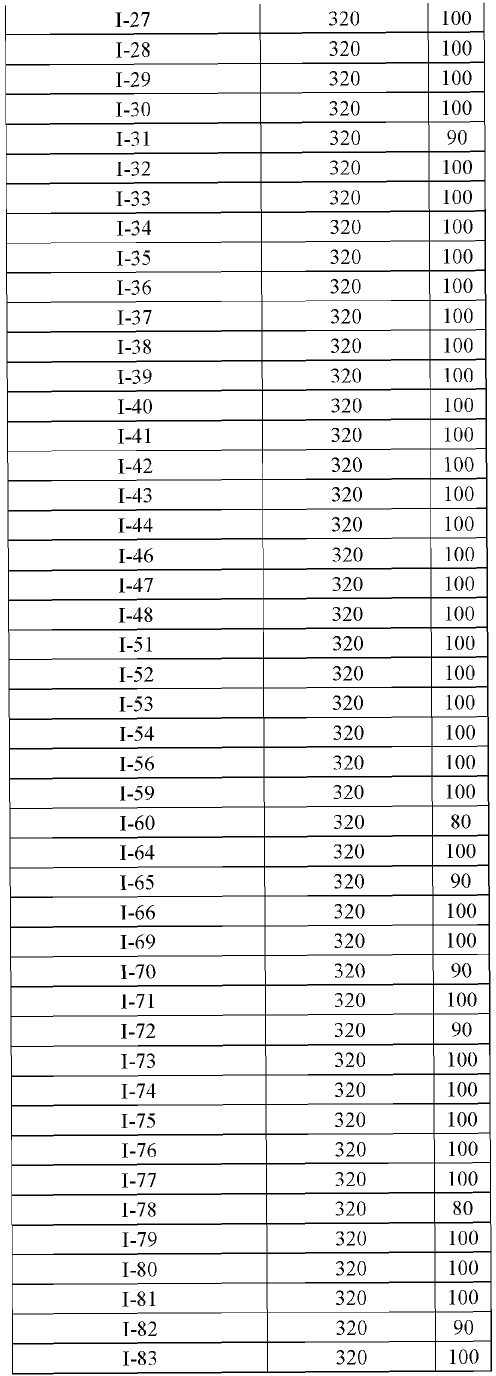

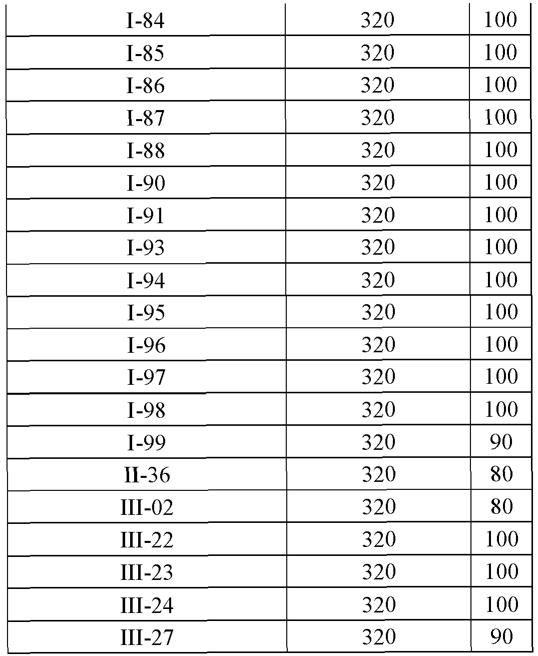

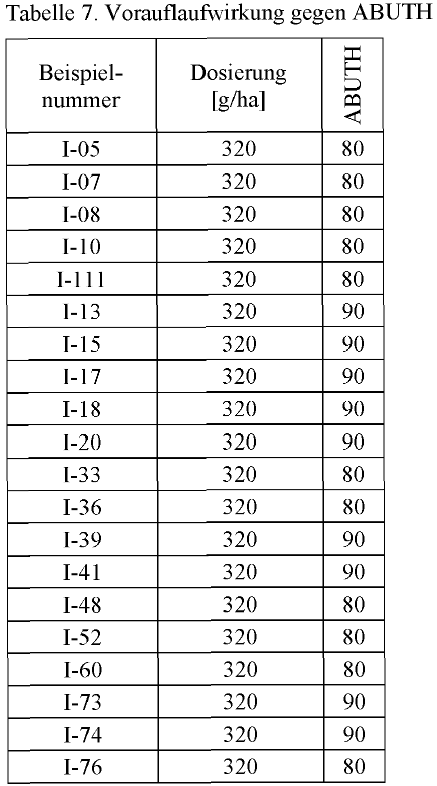

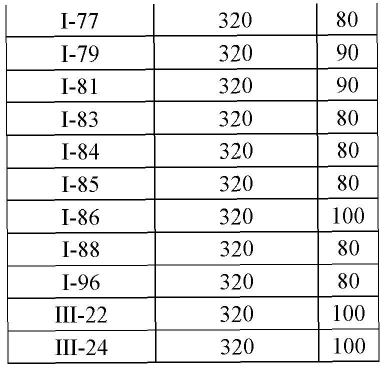

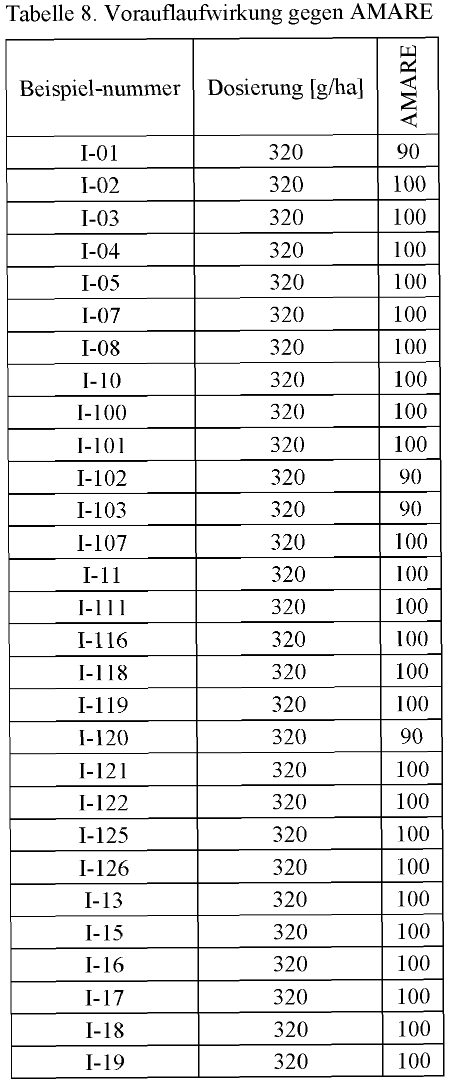

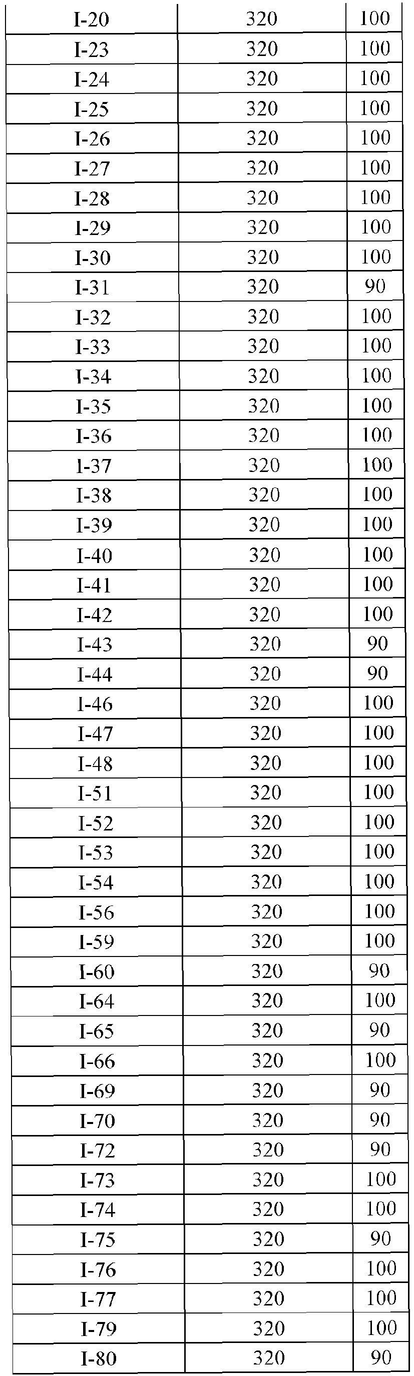

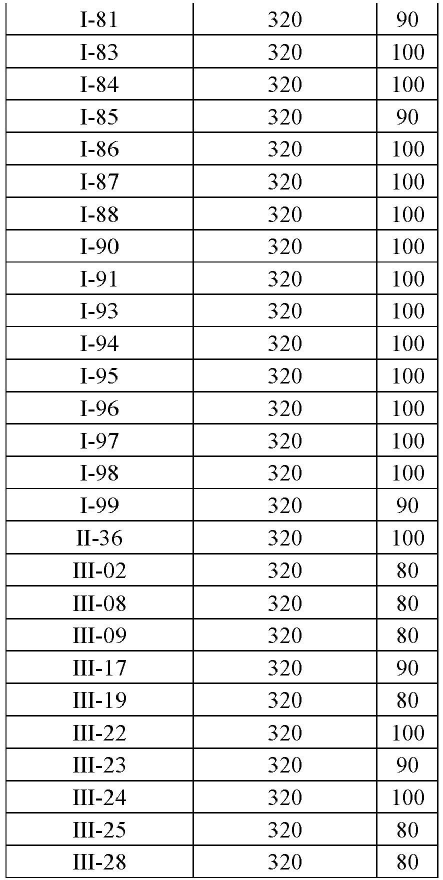

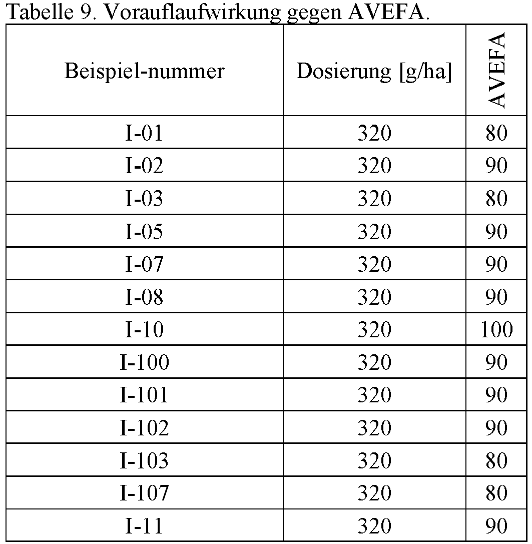

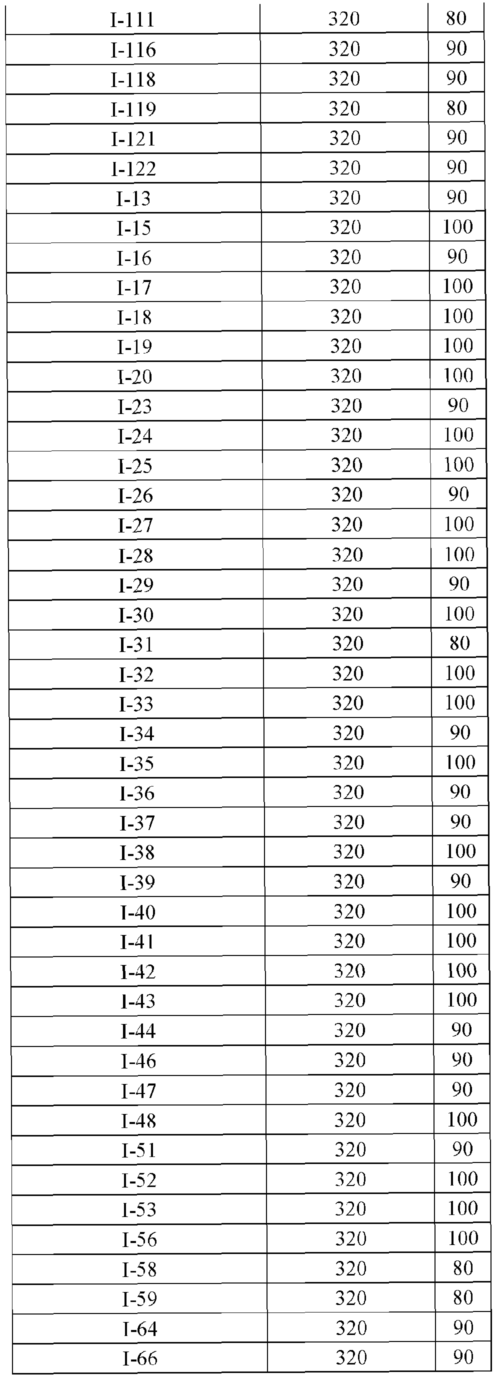

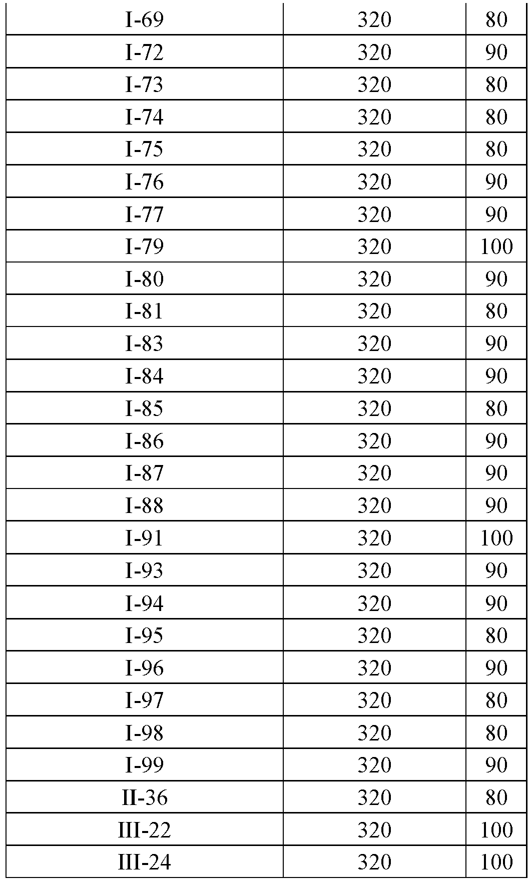

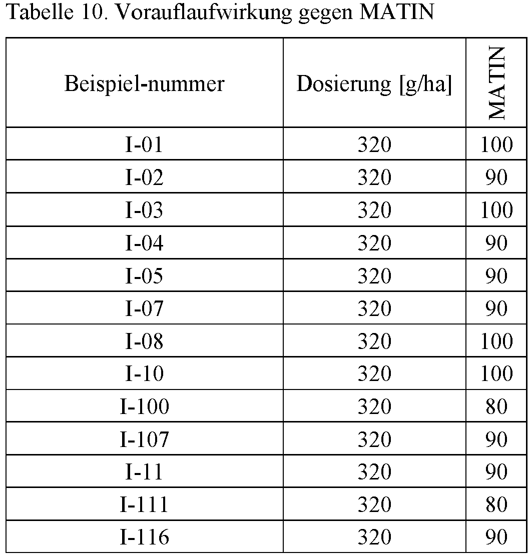

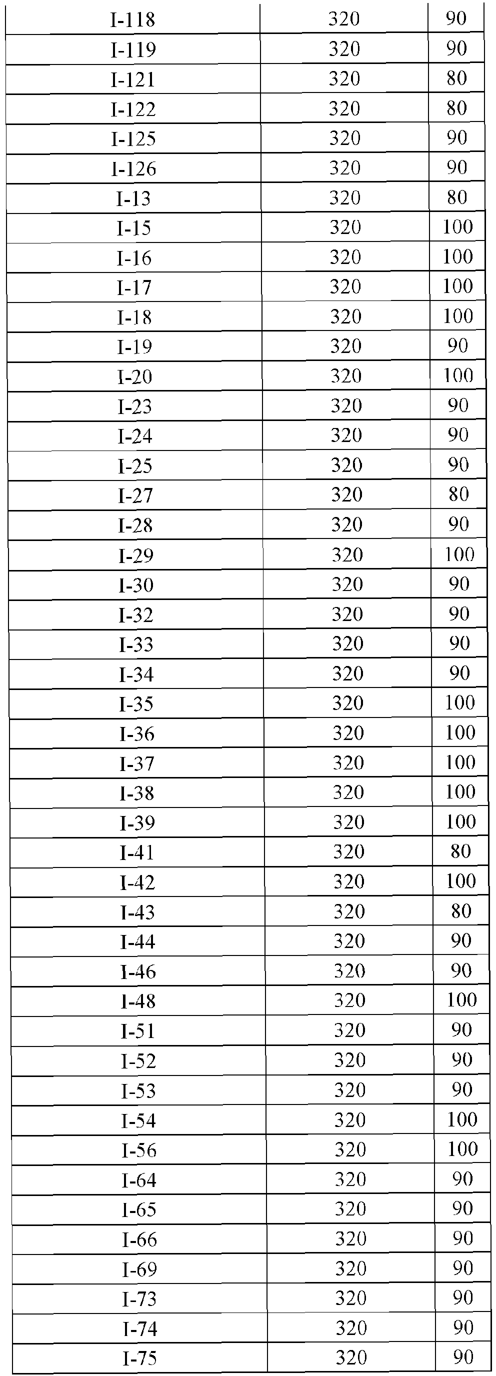

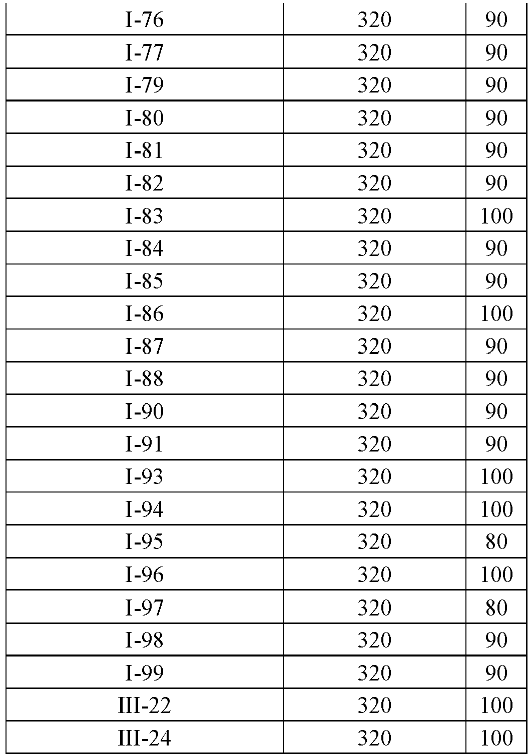

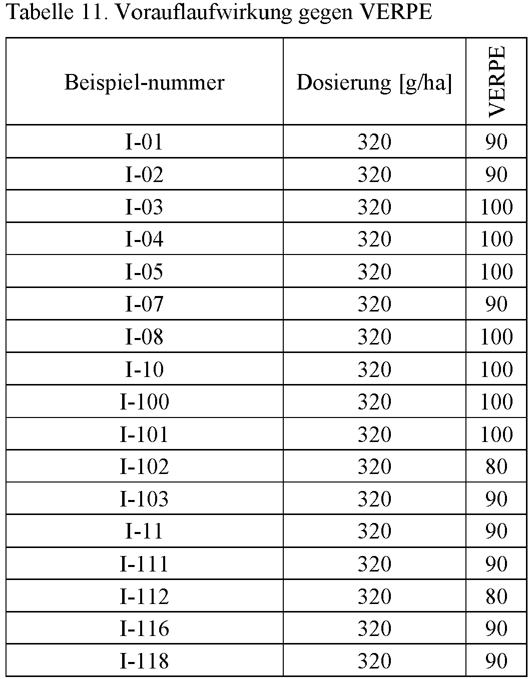

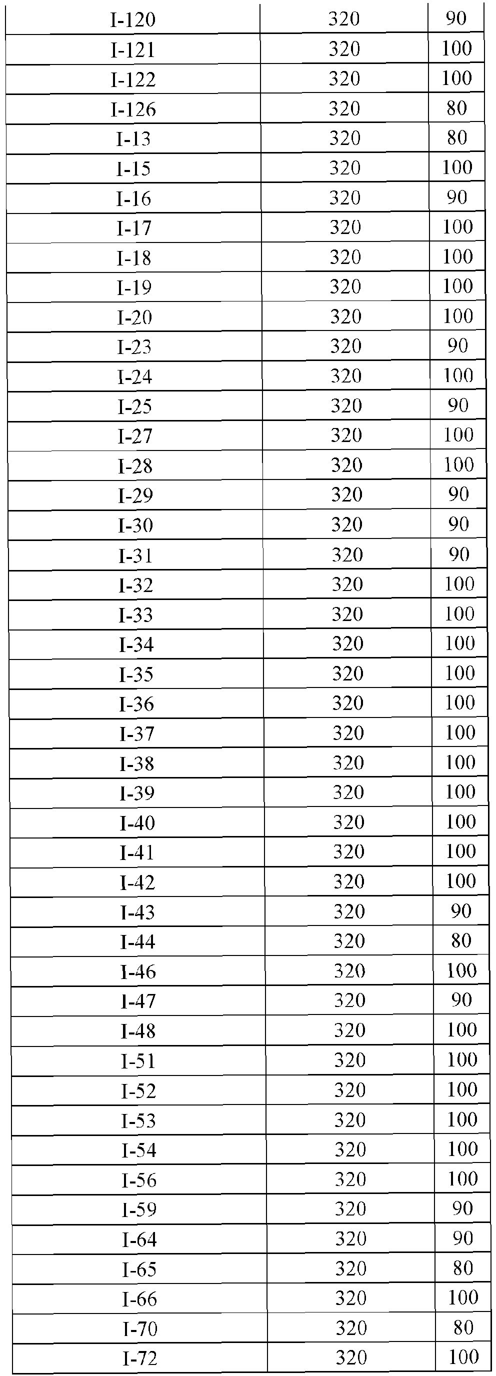

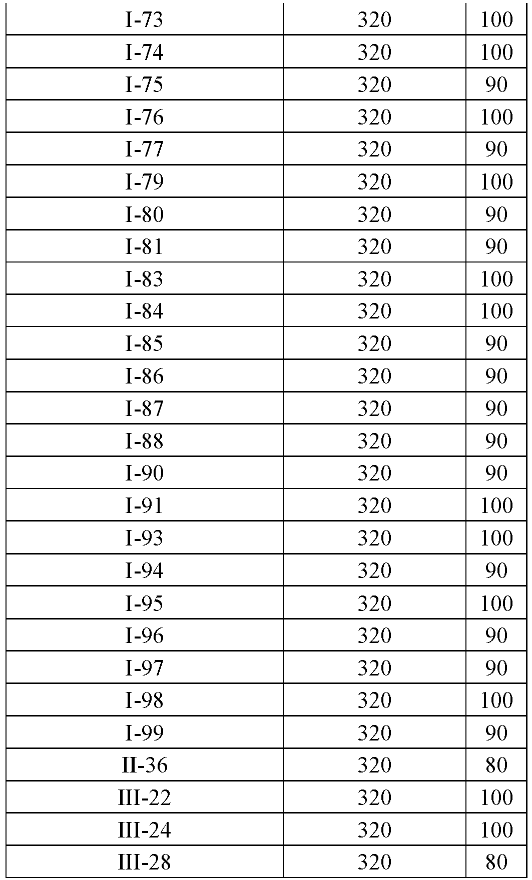

Beispielsweise zeigen die Verbindungen Nr. 1-03, 1-04 und 1-05 in den Tabellen 1 bis 11 bei einer Aufwandmenge von 320 g/ha jeweils eine 90-100%-ige Wirkung gegen *Alopecurus myrosoroides, Digitaria sanguinalis, Lolium rigidu* und *Setaria viridis.*

Die erfindungsgemäßen Verbindungen eignen sich deshalb im Vorauflaufverfahren zur Bekämpfung von unerwünschtem Pflanzenwuchs.

### 2. Herbizide Wirkung bzw. Kulturpflanzenverträglichkeit im Nachauflauf

Samen von mono- bzw. dikotylen Unkraut- bzw. Kulturpflanzen werden in Kunststoff- oder organischen Pflanztöpfen in sandigem Lehmboden ausgelegt, mit Erde abgedeckt und im Gewächshaus unter kontrollierten Wachstumsbedingungen angezogen. 2 bis 3 Wochen nach der Aussaat werden die Versuchspflanzen im Einblattstadium behandelt. Die in Form von benetzbaren Pulvern (WP) oder als Emulsionskonzentrate (EC) formulierten erfindungsgemäßen Verbindungen werden dann als wässrige Suspension bzw. Emulsion unter Zusatz von 0,5% Additiv mit einer

Wasseraufwandmenge von umgerechnet 600 l/ha auf die grünen Pflanzenteile gesprüht. Nach ca. 3 Wochen Standzeit der Versuchspflanzen im Gewächshaus, unter optimalen Wachstumsbedingungen, wird die Wirkung der Präparate visuell im Vergleich zu unbehandelten Kontrollen bonitiert. Beispielsweise bedeutet 100% Wirkung = Pflanzen sind abgestorben, 0% Wirkung = wie Kontrollpflanzen.

In den nachstehenden Tabellen 12 bis 23 sind die Wirkungen ausgewählter Verbindungen der allgemeinen Formel (I) gemäß der Tabellen I bis III auf verschiedene Schadpflanzen und einer Aufwandmenge entsprechend 320 g/ha, die gemäß zuvor genannter Versuchsvorschrift erhalten wurden, dargestellt.

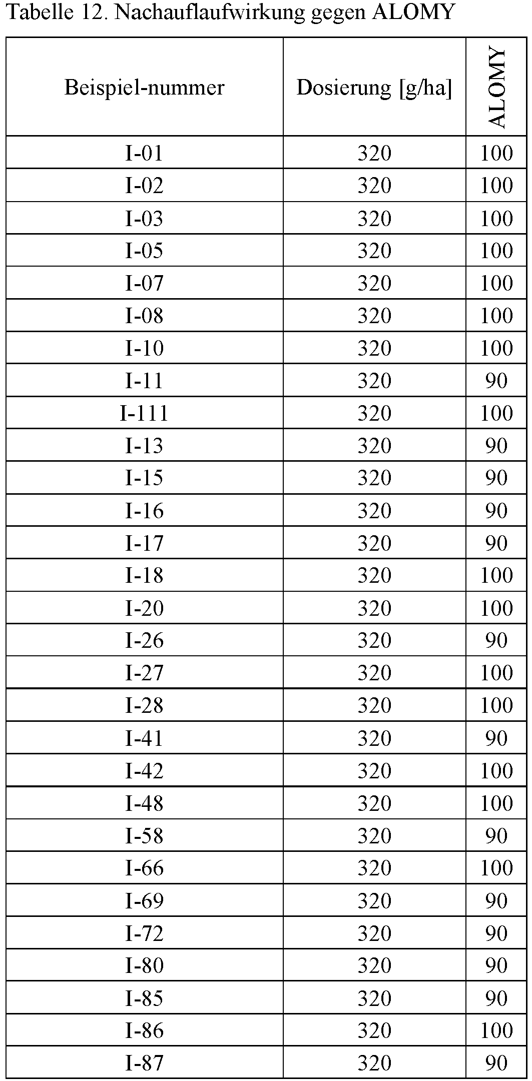

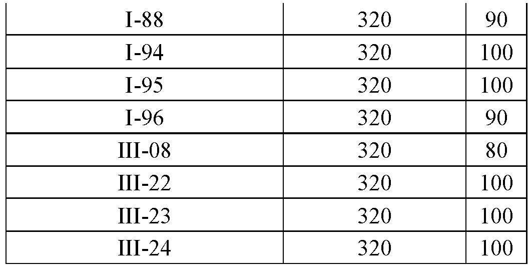

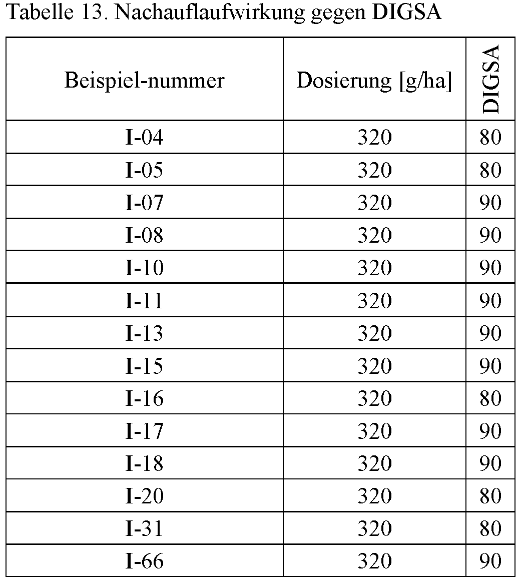

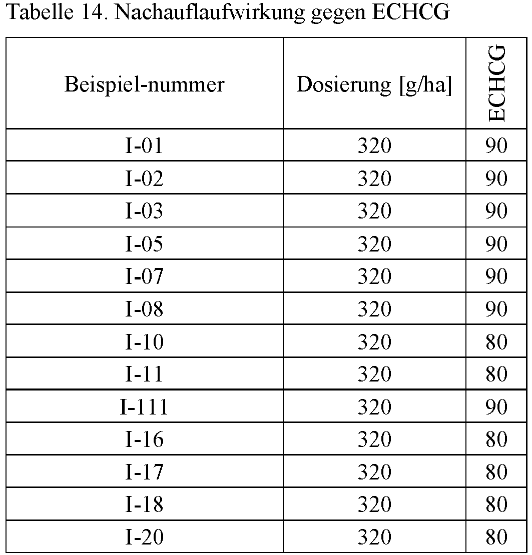

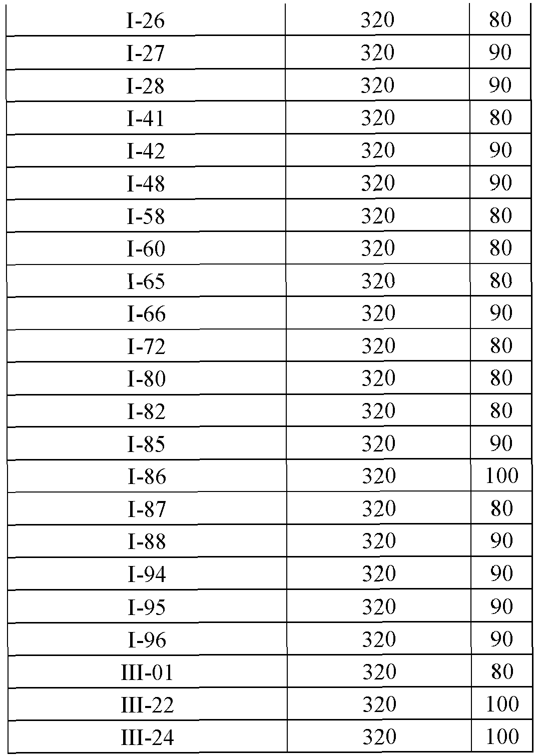

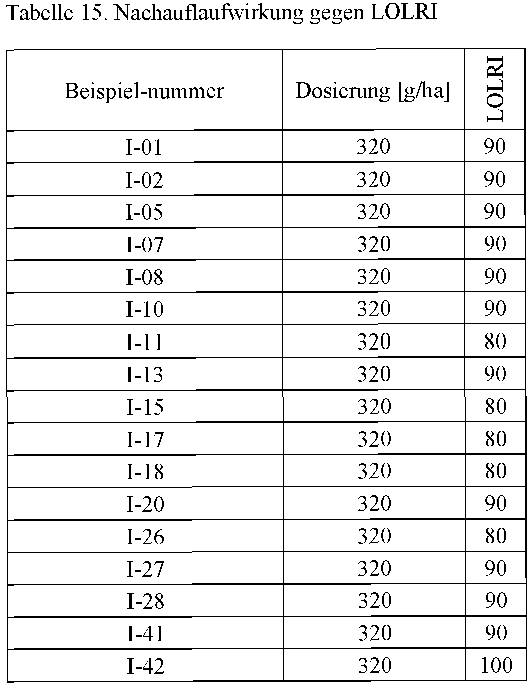

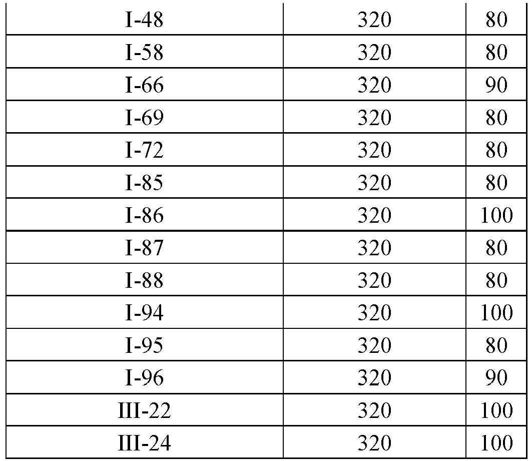

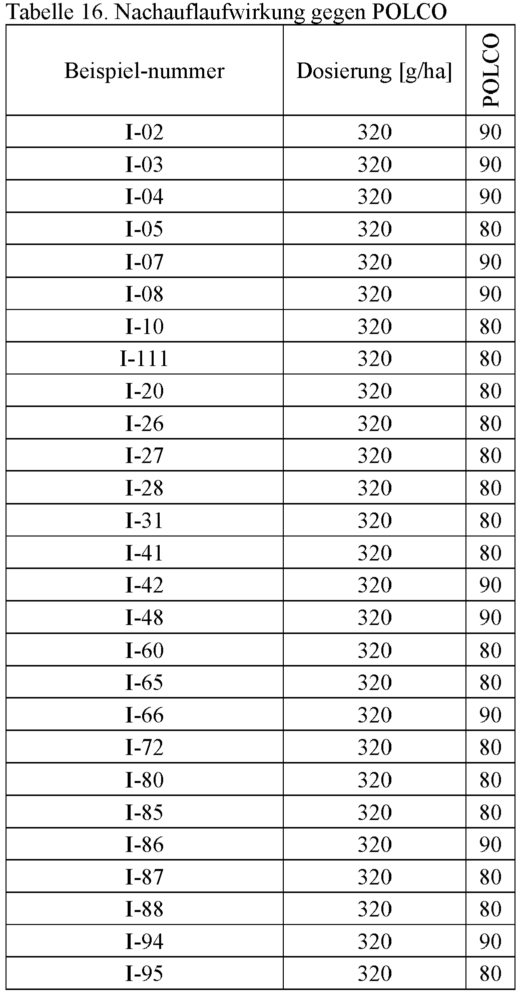

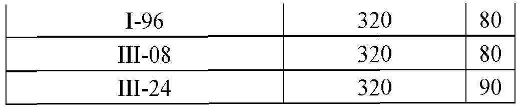

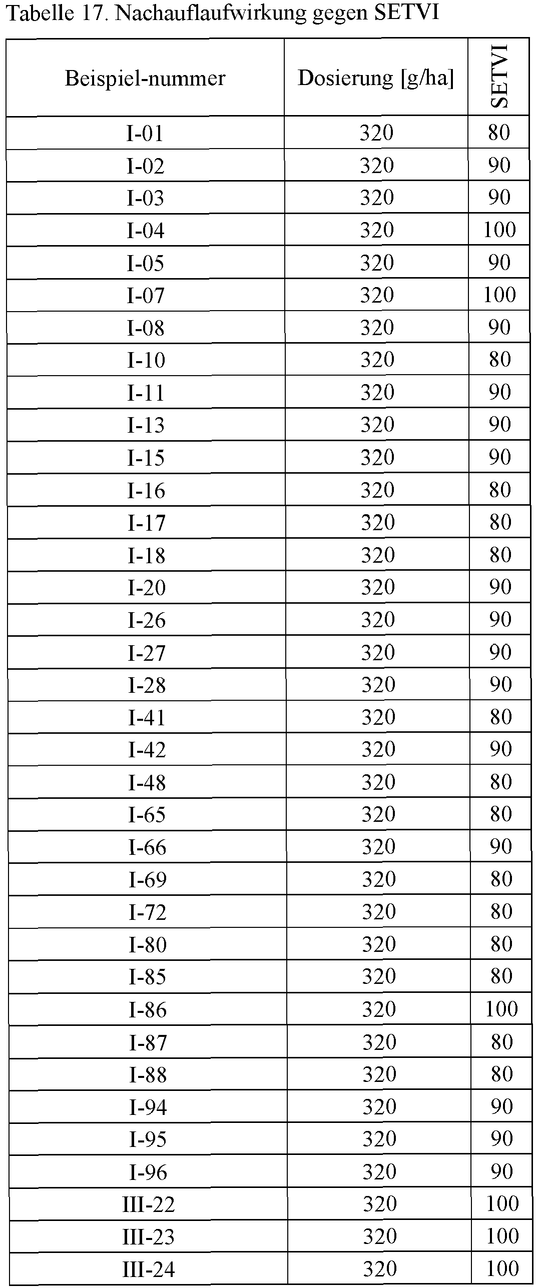

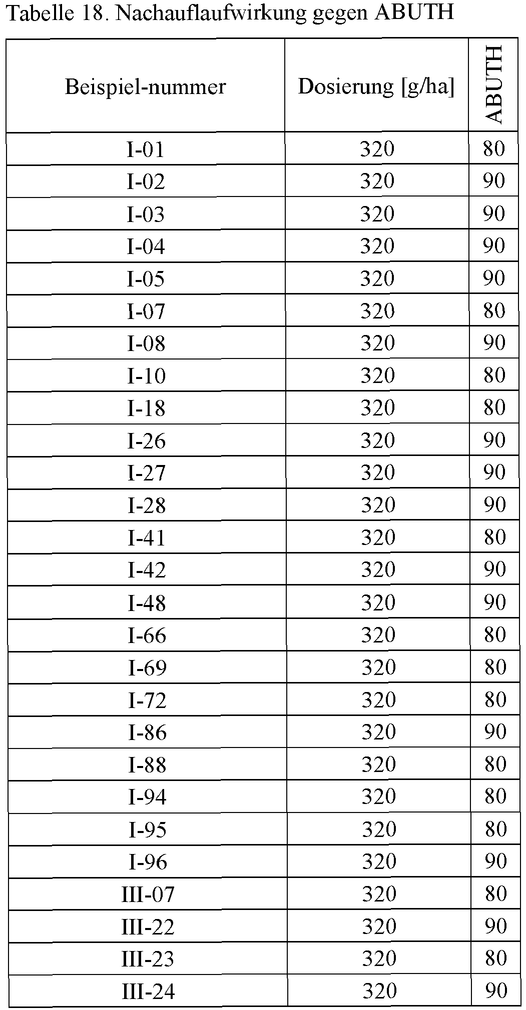

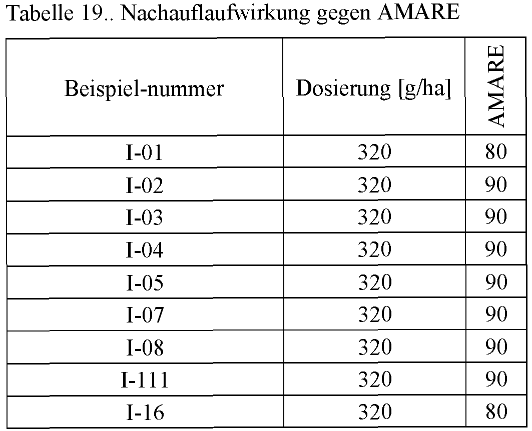

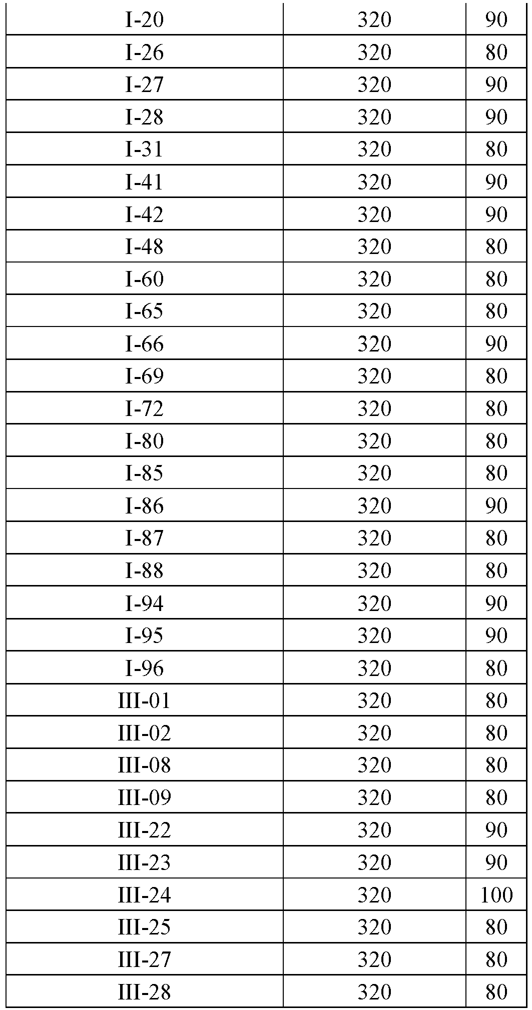

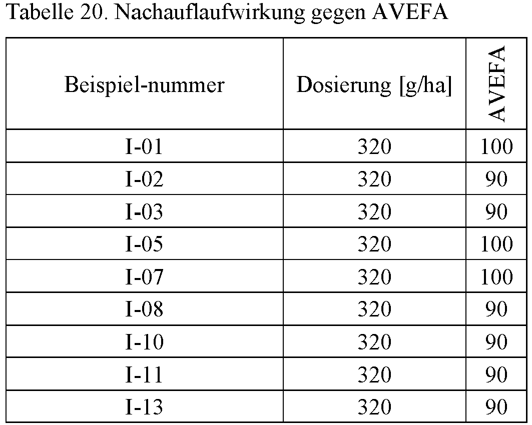

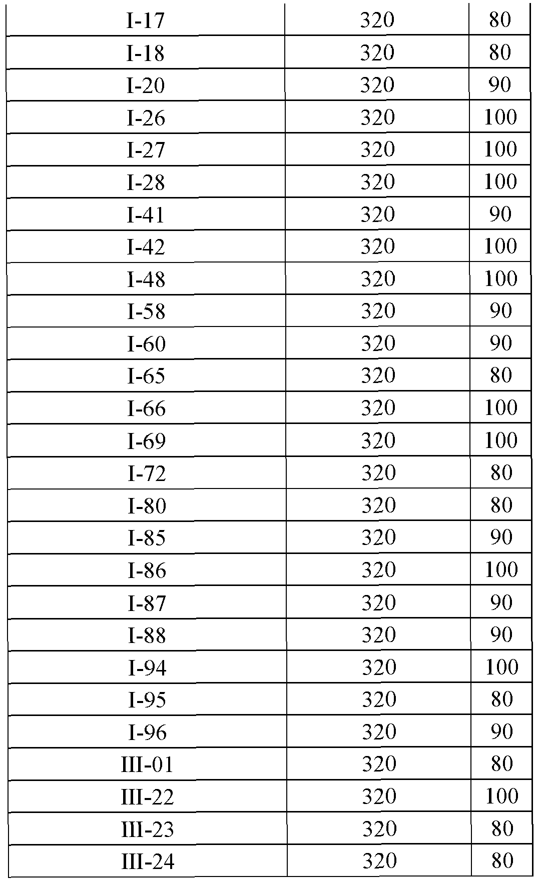

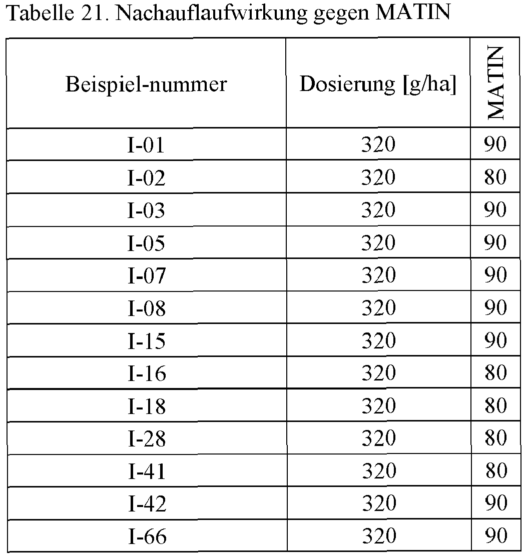

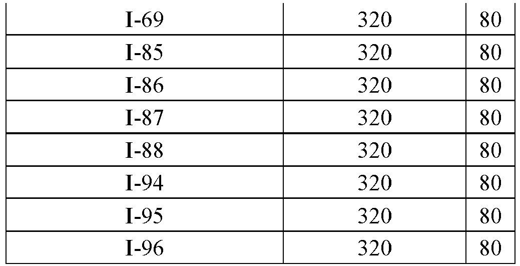

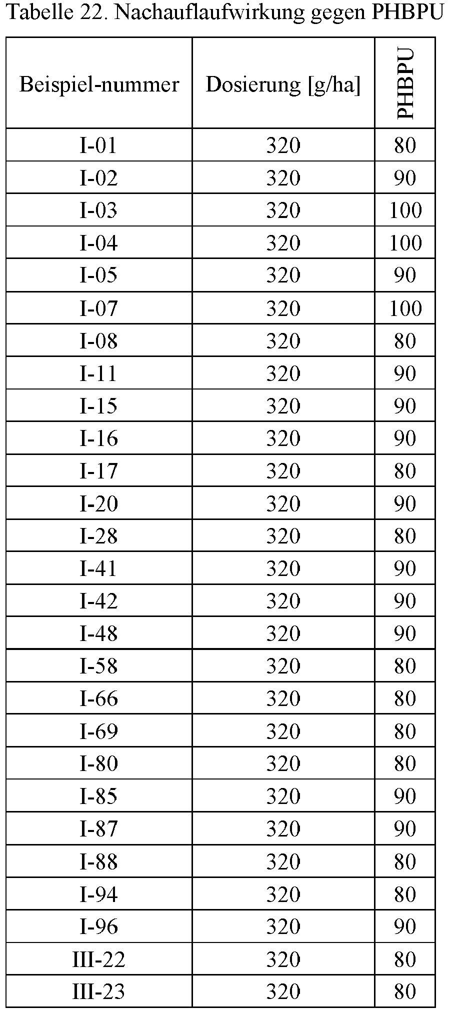

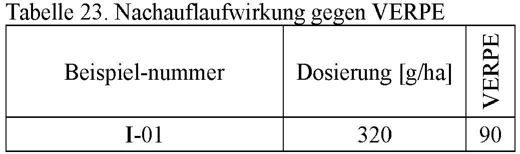

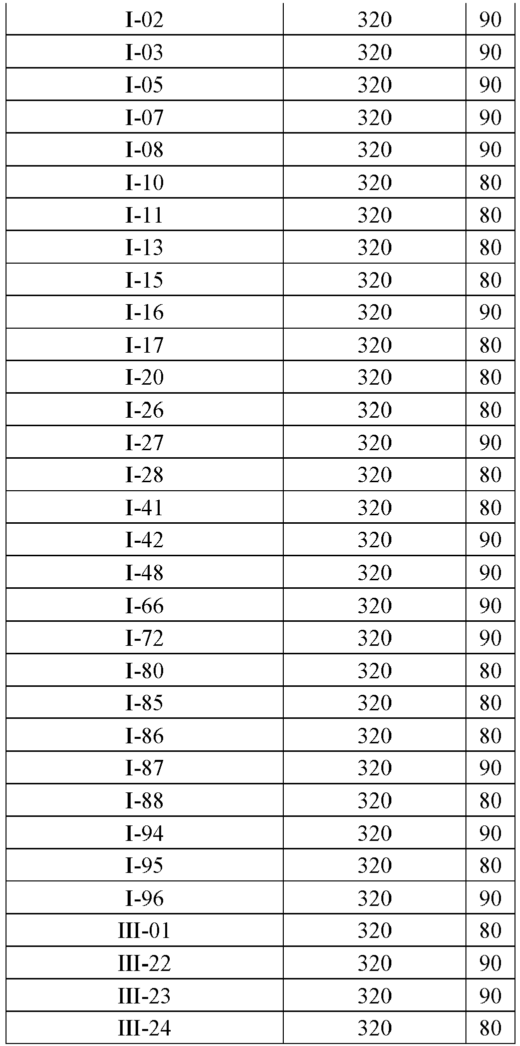

Beispielsweise zeigen alle erfindungsgemäßen Verbindungen 1-08 und 1-04 in den Tabellen 12 und 23 bei einer Aufwandmenge von 320g/ha jeweils eine 90 - 100%-ige Wirkung gegen *Alopecurus myosuroides, Digitaria sanguinalis, Setaria viridis* und *Echinochloa crus-galli* und eignen sich deshalb im Nachauflaufverfahren zur Bekämpfung von unerwünschtem Pflanzenwuchs.

## Patentansprüche

1. 1-Pyridyl-5-azinylpyrazolyl-3-oxyalkylsäuren, 1-Pyridyl-5-azinylpyrazolyl-3-sulfanylalkyl-säuren, 1-Pyridyl-5-azinylpyrazolyl-3-sulfinylalkylsäuren und 1-Pyridyl-5-azinylpyrazolyl-3-sulfonylalkylsäuren sowie deren Derivate der allgemeinen Formel (I) und deren agrochemisch verträgliche Salze, N-oxide, Hydrate und Hydrate der Salze und N-oxide, 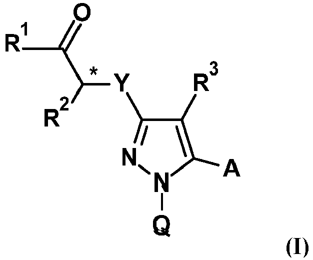 wobei
A ausgewählt ist aus der Gruppe, bestehend aus A1-A15 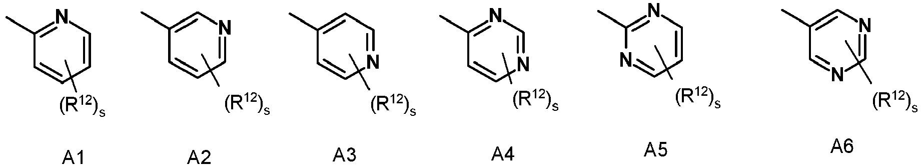 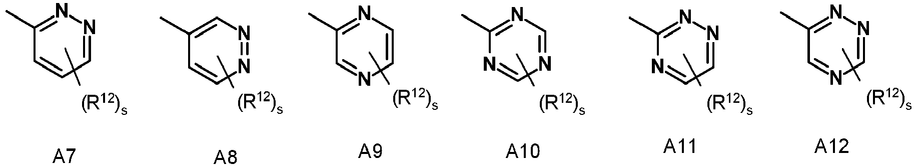 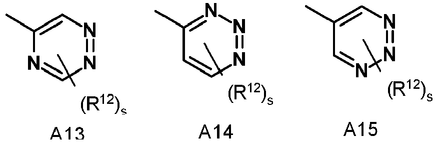
Q ausgewählt ist aus der Gruppe, bestehend aus Q1-Q3 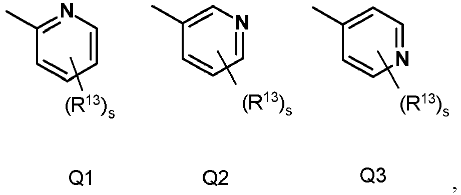
R¹ -OR^{1a}, -NR⁹R¹⁰ oder -O(CH₂)ₓO(CH₂)_{y}O(CH₂)_{z} H bedeutet;
R^{1a} Wasserstoff bedeutet oder (C₁-C₆)-Alkyl, (C₃-C₆)-Cycloalkyl bedeutet, welches jeweils unsubstituiert oder substituiert ist durch einen oder mehrere Substituenten ausgewählt aus der Gruppe bestehend aus Halogen, (C₁-C₆)-Alkyl, (C₁-C₆)-Halogenalkyl, (C₃-C₆)-Cycloalkyl, (C₁-C₆)-Alkoxy, (C₁-C₂)-Halogenalkoxy, (C₁-C₆)-Alkoxycarbonyl, Cyano und Nitro oder (C₂-C₄)-Alkenyl, CH₂C(COOMe)=CH₂, (C₂-C₄)-Alkinyl bedeutet oder (C₁-C₆)-Alkyl-S-(C₁-C₆)-Alkyl-, (C₁-C₆)-Alkyl-SO-(C₁-C₆)-Alkyl-, (C₁-C₆)-Alkyl-SO₂-(C₁-C₆)-Alkyl- bedeutet oder -N=(C₃-C₆)-Cycloalkyl, -N=C(CH₃)₂ bedeutet oder Heterocyclyl, Heteroaryl, Aryl bedeutet oder Heterocyclyl-(C₁-C₄)-Alkyl-, Heteroaryl-(C₁-C₄)-Alkyl-, Aryl-(C₁-C₄)-Alkyl- bedeutet, welches jeweils unsubstituiert oder substituiert ist durch einen oder mehrere Substituenten ausgewählt aus der Gruppe bestehend aus Halogen, (C₁-C₆)-Alkyl, (C₁-C₆)-Halogenalkyl;
R⁹ Wasserstoff, (C₁-C₁₂)-Alkyl bedeutet;
R¹⁰ Wasserstoff, Aryl, Heteroaryl, Heterocyclyl, (C₁-C₁₂)-Alkyl, (C₃-C₈)-Cycloalkyl, (C₃-C₈)-Cycloalkyl-(C₁-C₇)-Alkyl-, (C₂-C₁₂)-Alkenyl, (C₅-C₇)-Cycloalkenyl, (C₂-C₁₂)-Alkinyl, S(O)ₙR⁵, Cyano, Nitro, OR⁵, SO₂NR⁶R⁷, CO₂R⁸, COR⁸, NR⁶R⁸, NR⁶COR⁸, NR⁶CO₂R⁸, NR⁶SO₂R⁸ bedeutet, wobei die oben genannten Alkyl, Cycloalkyl, Alkenyl, Cycloalkenyl und Alkinyl Reste unsubstituiert sind oder jeweils unabhängig voneinander substituiert sind durch "m" Reste ausgewählt aus der Gruppe bestehend aus Wasserstoff, gegebenenfalls einfach oder mehrfach substituiertes Aryl, Halogen, Cyano, Nitro, OR⁵, S(O)ₙR⁵, SO₂NR⁶R⁷, CO₂R⁸, CONR⁶R⁸, COR⁶, NR⁶R⁸, NR⁶COR⁸, NR⁶CONR⁸R⁸, NR⁶CO₂R⁸, NR⁶SO₂R⁸, NR⁶SO₂NR⁶R⁸, C(R⁶)=NOR⁸;
oder
R⁹ und R¹⁰ bilden mit dem Stickstoffatom, an das sie gebunden sind, einen gegebenenfalls ein- bis sechsfach durch folgende Reste aus der Gruppe bestehend aus Halogen, (C₁-C₆)-Alkyl, (C₁-C₆)-Halogenalkyl, OR⁵, S(O)ₙR⁵, CO₂R⁸, CONR⁶R⁸, COR⁶ und C(R⁶)=NOR⁸ substituierten, gesättigten, teilweise oder vollständig ungesättigten fünf-, sechs- oder siebengliedrigen Ring, der neben diesem Stickstoffatom "r" Kohlenstoffatome, "o" Sauerstoffatome, "p" Schwefelatome und "q" Elemente aus der Gruppe bestehend aus NR⁷, CO und NCOR⁷ als Ringatome enthält;
R⁵ (C₁-C₆)-Alkyl, (C₃-C₆)-Cycloalkyl , (C₁-C₆)-Halogenalkyl, Aryl bedeutet;
R⁶ Wasserstoff, (C₁-C₆)-Alkyl, (C₃-C₆)-Cycloalkyl, (C₁-C₆)-Halogenalkyl, Aryl bedeutet;
R⁷ Wasserstoff, (C₁-C₆)-Alkyl, (C₃-C₆)-Cycloalkyl, (C₃-C₄)-Alkenyl, (C₃-C₄)-Alkinyl, CH₂-C(O)O(C₁-C₂)-Alkyl bedeutet;
R⁸ Wasserstoff, (C₁-C₆)-Alkyl, (C₃-C₆)-Cycloalkyl, (C₃-C₄)-Alkenyl, (C₁-C₆)-Alkyl-C(O)O(C₁-C₂)-Alkyl oder (C₃-C₄)-Alkinyl bedeutet;
R² Wasserstoff, Cyano, (C₁-C₆)-Alkyl, (C₁-C₆)-Alkoxy-(C₁-C₆)-Alkyl-, (C₁-C₆)-Halogenalkyl, (C₁-C₆)-Alkoxy, (C₂-C₆)-Alkenyl, (C₂-C₆)-Halogenalkenyl, (C₂-C₆)-Alkinyl, (C₂-C₆)-Halogenalkinyl, (C₃- C₆)-Cycloalkyl bedeutet;
R³ Halogen, Cyano, Isocyano, NO₂, (C₃-C₆)-Cycloalkyl, (C₁-C₆)-Alkyl, (C₃-C₆)-Halogencycloalkyl, (C₁-C₆)-Halogenalkyl, (C₁-C₆)-Alkylcarbonyl-, (C₁-C₆)-Halogenalkylcarbonyl-, (C₁-C₆)-Alkyloxycarbonyl-, (C₂-C₃)-Alkenyl, (C₂-C₃)-Halogenalkenyl, (C₂-C₃)-Alkinyl, (C₂-C₃)-Halogenalkinyl, (C₁-C₆)-Alkyl-S(O)ₙ und (C₁-C₆)-Halogenalkyl-S(O)ₙ, CHO, NH₂ bedeutet;
R¹² Halogen, Cyano, Isocyano, NO₂, (C₁-C₆)-Alkyl, (C₁-C₆)-Halogenalkyl, (C₁-C₆)-Alkylcarbonyl, (C₁-C₆)-Halogenalkylcarbonyl, (C₁-C₆)-Alkyloxycarbonyl, (C₁-C₆)-Alkoxy, (C₁-C₆)-Halogenalkoxy, (C₁-C₆)-Alkyl-, S(O)ₙ, (C₂-C₃)-Alkenyl, (C₂-C₃)-Halogenalkenyl, (C₂-C₃)-Alkinyl, (C₂-C₃)-Halogenalkinyl, NH₂ bedeutet;
R¹³ Halogen, Cyano, Isocyano, NO₂, (C₁-C₆)-Alkyl, (C₁-C₆)-Halogenalkyl, (C₁-C₆)-Alkylcarbonyl, (C₁-C₆)-Halogenalkylcarbonyl, (C₁-C₆)-Alkyloxycarbonyl, (C₁-C₆)-Alkoxy, (C₁-C₆)-Halogenalkoxy, (C₁-C₆)-Alkyl- S(O)ₙ, (C₂-C₃)-Alkenyl, (C₂-C₃)-Halogenalkenyl, (C₂-C₃)-Alkinyl, (C₂-C₃)-Halogenalkinyl, NH₂ bedeutet;
Y Sauerstoff, S(O)ₙ bedeutet;
m 0, 1 oder 2 bedeutet;
n 0, 1 oder 2 bedeutet;
o 0, 1 oder 2 bedeutet;
p 0 oder 1 bedeutet;
q 0 oder 1 bedeutet;
r 3, 4, 5 oder 6 bedeutet;
s 0, 1, 2, 3 oder 4 bedeutet;
x 2, 3 bedeutet:
y 2, 3 bedeutet;
z 1, 2 bedeutet.

2. Verbindungen der Formel (I) gemäß Anspruch 1 und deren agrochemisch verträgliche Salze, N-oxide, Hydrate und Hydrate der Salze und N-oxide, wobei
A ausgewählt ist aus der Gruppe, bestehend aus Al- A4, A6, A8, A9, A12, A15 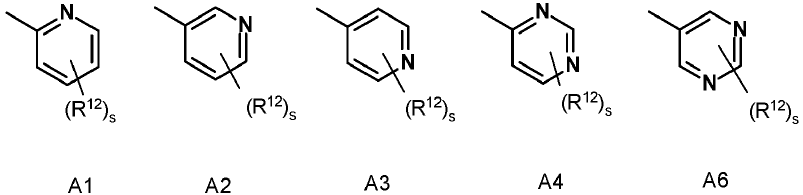 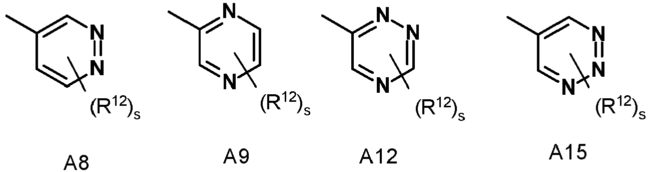
Q ausgewählt ist aus der Gruppe, bestehend aus Q1-Q3 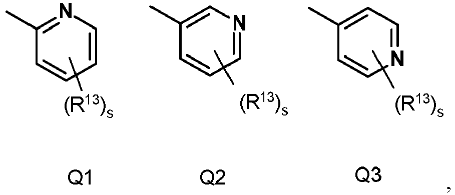
R¹ -OR^{1a}, -NR⁹R¹⁰ oder -O(CH₂)ₓO(CH₂)_{y}O(CH₂)_{z} H bedeutet;
R^{1a} Wasserstoff bedeutet oder (C₁-C₃)-Alkyl, (C₃-C₆)-Cycloalkyl bedeutet, welches jeweils unsubstituiert oder substituiert ist durch einen oder mehrere Substituenten ausgewählt aus der Gruppe bestehend aus Halogen, (C₁-C₄)-Alkyl, (C₁-C₄)-Halogenalkyl, (C₃-C₆)-Cycloalkyl, (C₁-C₄)-Alkoxy, (C₁-C₄)-Alkoxycarbonyl, Cyano oder (C₂-C₄)-Alkenyl, (C₂-C₄)-Alkinyl bedeutet oder (C₂-C₃)-Alkyl-SMe-, (C₂-C₃)-Alkyl-SOMe-, (C₂-C₃)-Alkyl-SO₂Me- bedeutet oder -N=C(CH₃)₂ bedeutet oder Heterocyclyl bedeutet oder Heterocyclyl-(C₁-C₂)-Alkyl-, Heteroaryl-(C₁-C₂)-Alkyl-, Aryl-(C₁-C₂)-Alkyl- bedeutet, welches jeweils unsubstituiert oder substituiert ist durch einen oder mehrere Substituenten ausgewählt aus der Gruppe bestehend aus Halogen, (C₁-C₂)-Alkyl, (C₁-C₂)-Halogenalkyl;
R⁹ Wasserstoff, (C₁-C₄)-Alkyl bedeutet;
R¹⁰ (C₁-C₄)-Alkyl, (C₂-C₄)-Alkenyl, (C₂-C₄)-Alkinyl, S(O)ₙR⁵, SO₂NR⁶R⁷, wobei die oben genannten Alkyl, Alkenyl und Alkinyl Reste unsubstituiert sind oder jeweils unabhängig voneinander substituiert sind durch "m" Reste ausgewählt aus der Gruppe bestehend aus Halogen, Cyano, S(O)ₙR⁵, SO₂NR⁶R⁷, CO₂R⁸, CONR⁶R⁸
oder
R⁹ und R¹⁰ bilden mit dem Stickstoffatom, an das sie gebunden sind, einen gegebenenfalls ein- oder zweifach durch folgende Reste aus der Gruppe bestehend aus (C₁-C₄)-Alkyl, (C₁-C₄)-Halogenalkyl, CO₂R⁸und CONR⁶R⁸ substituierten, gesättigten, teilweise oder vollständig ungesättigten fünf-, sechs- oder siebengliedrigen Ring;
R⁵ (C₁-C₄)-Alkyl, (C₃-C₆)-Cycloalkyl , (C₁-C₄)-Halogenalkyl, Phenyl bedeutet;
R⁶ Wasserstoff, (C₁-C₄)-Alkyl, (C₃-C₆)-Cycloalkyl, (C₁-C₄)-Halogenalkyl, Phenyl bedeutet;
R⁷ Wasserstoff, (C₁-C₄)-Alkyl, (C₃-C₆)-Cycloalkyl, (C₃-C₄)-Alkenyl, (C₃-C₄)-Alkinyl, bedeutet;
R⁸ Wasserstoff, (C₁-C₄)-Alkyl, (C₃-C₆)-Cycloalkyl, (C₃-C₄)-Alkenyl, (C₁-C₄)-Alkyl-C(O)O(C₁-C₂)-Alkyl oder (C₃-C₄)-Alkinyl bedeutet;
R² Wasserstoff, (C₁-C₃)-Alkyl, (C₁-C₃)-Alkoxy, (C₂-C₃)-Alkenyl, (C₂-C₃)-Alkinyl, (C₃-C₆)-Cycloalkyl bedeutet;
R³ Halogen, Cyano, Isocyano, NO₂, (C₃-C₆)-Cycloalkyl, (C₁-C₄)-Alkyl, (C₃-C₆)-Halogencycloalkyl, (C₁-C₄)-Halogenalkyl, (C₁-C₄)-Alkylcarbonyl-, (C₁-C₄)-Halogenalkylcarbonyl-, (C₁-C₄)-Alkyloxycarbonyl-, (C₂-C₃)-Alkenyl, (C₂-C₃)-Alkinyl, (C₂-C₃)-Halogenalkinyl, CHO bedeutet;
R¹² Halogen, Cyano, NO₂, (C₁-C₄)-Alkyl, (C₁-C₄)-Halogenalkyl, (C₁-C₄)-Alkylcarbonyl, (C₁-C₄)-Halogenalkylcarbonyl, (C₁-C₄)-Alkyloxycarbonyl, (C₁-C₄)-Alkoxy, (C₁-C₄)-Halogenalkoxy, (C₂-C₃)-Alkenyl, (C₂-C₃)-Alkinyl, (C₂-C₃)-Halogenalkinyl, NH₂ bedeutet;
R¹³ Halogen, Cyano, NO₂, (C₁-C₄)-Alkyl, (C₁-C₄)-Halogenalkyl, (C₁-C₄)-Alkylcarbonyl, (C₁-C₄)-Halogenalkylcarbonyl, (C₁-C₄)-Alkyloxycarbonyl, (C₁-C₄)-Alkoxy, (C₁-C₄)-Halogenalkoxy, (C₂-C₃)-Alkenyl, (C₂-C₃)-Alkinyl, (C₂-C₃)-Halogenalkinyl, NH₂ bedeutet;
Y Sauerstoff, S(O)ₙ bedeutet;
m 0 oder 1 bedeutet;
n 0, 1 oder 2 bedeutet;
s 0, 1, 2 oder 3 bedeutet;
x 2 bedeutet:
y 2 bedeutet;
z 1 bedeutet.

3. Verbindungen der Formel **(I)** gemäß Anspruch 1 oder 2 und deren agrochemisch verträgliche Salze, N-oxide, Hydrate und Hydrate der Salze und N-oxide, wobei
A ausgewählt ist aus der Gruppe, bestehend aus A2, A3, A6 und A8 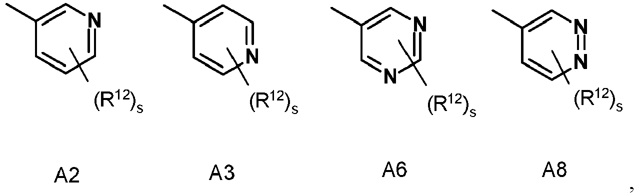
Q ausgewählt ist aus der Gruppe, bestehend aus Q1-Q3 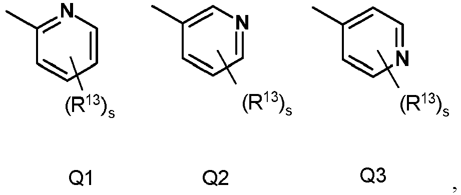
R¹ -OR^{1a}, -NR⁹R¹⁰ oder -O(CH₂)₂O(CH₂)₂OCH₃ bedeutet;
R^{1a} Wasserstoff bedeutet oder (C₁-C₃)-Alkyl bedeutet, welches unsubstituiert oder substituiert ist durch einen Substituenten ausgewählt aus der Gruppe bestehend aus -C(O)OMe, Cyclopropyl, Cyclopentyl, Methoxy, Cyano, Trifluormethyl oder (C₃-C₆)-Cycloalkyl bedeutet oder -N=C(CH₃)₂ bedeutet oder -Prop-2-in-yl oder Oxetan-3-yl-(C₁-C₂)-Alkyl-, Tetrahydrofuran-2-yl-(C₁-C₂)-Alkyl, Tetrahydrofuran-3-yl-(C₁-C₂)-Alkyl, Pyridin-2-yl-(C₁-C₂)-Alkyl-, Pyridin-3-yl-(C₁-C₂)-Alkyl, Pyridin-4-yl-(C₁-C₂)-Alkyl-, Phenyl-(C₁₋C₂)-Alkyl-- bedeutet;
R⁹ Wasserstoff bedeutet;
R¹⁰ (C₁-C₄)-Alkyl bedeutet, wobei der Alkylrest unsubstituiert ist oder einfach substituiert ist durch CO₂R⁸, S(O)₂R⁵oder SO₂NR⁶R⁷;
oder
R⁹ und R¹⁰ bilden mit dem Stickstoffatom, an das sie gebunden sind, einen einfach durch CO₂R⁸ substituierten, gesättigten fünf- oder sechsgliedrigen Ring;
R^{S} Methyl, Ethyl oder CH₂CF₃ bedeutet;
R⁶ Wasserstoff oder Methyl bedeutet;
R⁷ Methyl oder Ethyl bedeutet;
R⁸ Wasserstoff, Methyl oder Ethyl bedeutet;
R² Wasserstoff, Methyl oder Ethyl bedeutet;
R³ Halogen, Cyano, NO₂, (C₃-C₅)-Cycloalkyl, (C₃-C₅)-Halogencycloalkyl, (C₁-C₂)-Alkyl, (C₁-C₂)-Halogenalkyl, bedeutet;
R¹² Fluor oder Chlor bedeutet;
R¹³ Fluor, Chlor, Methyl, Ethyl, Methoxy, Trifluormethyl bedeutet;
Y Sauerstoff bedeutet;
s 0, 1, 2 bedeutet.

4. Verbindungen der Formel **(I)** gemäß einem der Ansprüche 1 bis 3 und deren agrochemisch verträgliche Salze, N-oxide, Hydrate und Hydrate der Salze und N-oxide, wobei
A ausgewählt ist aus der Gruppe, bestehend aus A2, A3 und A6 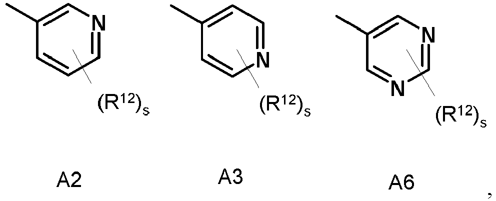
Q ausgewählt ist aus der Gruppe, bestehend aus Q1-Q3 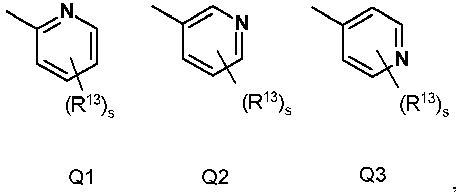
R¹ -OR^{1a} oder -NR⁹R¹⁰ bedeutet,
R^{1a} Wasserstoff bedeutet oder Methyl, Ethyl bedeutet, welches jeweils unsubstituiert oder substituiert ist durch einen Substituenten ausgewählt aus der Gruppe bestehend aus -C(O)OMe, Cyclopropyl, Cyclopentyl, Methoxy, Cyano, Trifluormethyl oder -N=C(CH₃)₂ bedeutet oder -Prop-2-in-yl oder -CH₂-Tetrahydrofuran-2-yl, -CH₂-Tetrahydrofuran-3-yl, -CH₂-Oxetan-3-yl, -CH₂-Pyridyl-2-yl,-CH₂-Pyridin-3-yl, -CH₂-Pyridin-4-yl, -CH₂-Phenyl bedeutet,
R⁹ Wasserstoff bedeutet;
R¹⁰ Methyl oder Ethyl bedeutet, wobei die Reste einfach substituiert sind durch CO₂R⁸
oder
R⁹ und R¹⁰ bilden mit dem Stickstoffatom, an das sie gebunden sind, ein einfach durch CO₂R⁸ substituierten Cyclopentyl- oder Cyclohexylrest;
R⁸ Methyl oder Ethyl bedeutet;
R² Wasserstoff oder Methyl bedeutet;
R³ Halogen, Cyano, NO₂, Cyclopropyl, Cyclobutyl, 2,2-Difluorcyclopropyl, Trifluormethyl bedeutet;
R¹² Fluor bedeutet;
R¹³ Fluor, Chlor, Methyl, Trifluormethyl bedeutet;
Y Sauerstoff bedeutet;
s 0, 1, 2 bedeutet.

5. Verbindungen der Formel **(I)** gemäß einem der Ansprüche 1 bis 4 und deren agrochemisch verträgliche Salze, N-oxide, Hydrate und Hydrate der Salze und N-oxide, wobei
A ausgewählt ist aus der Gruppe, bestehend aus A2, A3 und A6 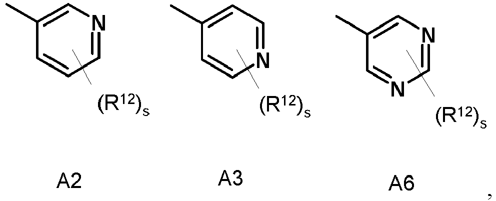
Q ausgewählt ist aus der Gruppe, bestehend aus Q1-Q3 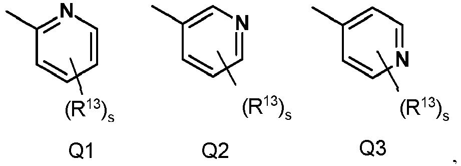
R¹ -OR^{1a} bedeutet;
R^{1a} Wasserstoff, Methyl, Ethyl bedeutet;
R² Wasserstoff oder Methyl bedeutet;
R³ Fluor, Chlor, Brom, Cyano, NO₂, Cyclopropyl, Trifluormethyl bedeutet;
R¹² Fluor bedeutet;
R¹³ Fluor, Chlor, Trifluormethyl bedeutet;
Y Sauerstoff bedeutet;
s 0, 1, 2 bedeutet.

6. Verbindungen der Formel **(Z)** gemäß einem der Ansprüche 1-5 mit Q = Q1, R¹ = -OR^{1a} und Y = O und deren agrochemisch verträgliche Salze, N-oxide, Hydrate und Hydrate der Salze und N-oxide, wobei 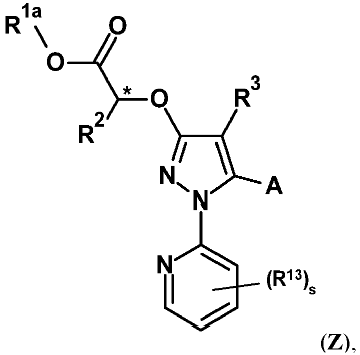 die Substituenten gemäß einem der Ansprüche 1 bis 5 definiert sind.

7. Verbindungen der Formel **(Y)** gemäß einem der Ansprüche 1-5 mit Q = Q1, Y = O und A = A2 und deren agrochemisch verträgliche Salze, N-oxide, Hydrate und Hydrate der Salze und N-oxide, wobei 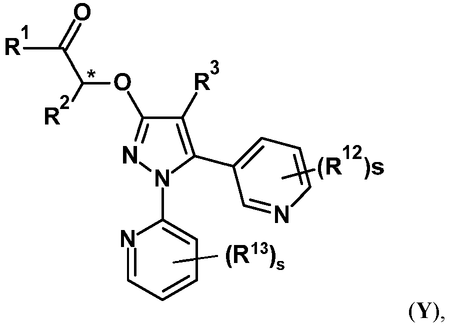 die Substituenten gemäß einem der Ansprüche 1 bis 5 definiert sind.

8. Verfahren zur Herstellung der Verbindungen der Formel **(Ic)** oder ein agrochemisch akzeptables Salz davon gemäß einem der Ansprüche 1 bis 5, indem Verbindungen der allgemeinen Formel **(III)** und **(IV),** 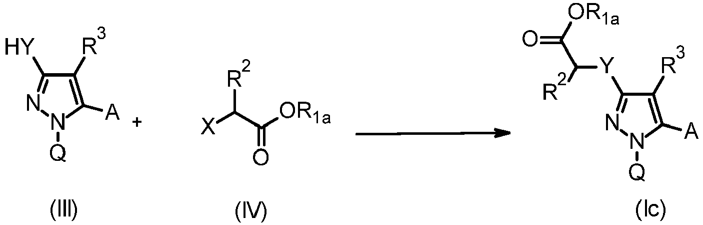 in welcher R², R^{1a}, R³, A, Q und Y die oben angegebene Bedeutung haben und X für Chlor, Brom oder lod steht in Anwesenheit eines Schwefelungsreagenzes wie zum Beispiel Phosphorpentasulfid oder Lawesson-Reagenz umgesetzt werden.

9. Verfahren zur Herstellung der Verbindungen der Formel **(la)** oder ein agrochemisch akzeptables Salz davon gemäß einem der Ansprüche 1 bis 5, indem eine Verbindung der allgemeinen Formel **(Ic)** 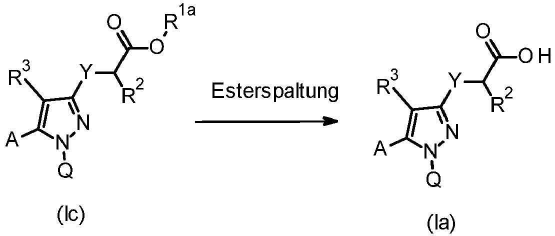 in welcher R², R^{1a}, R³, A, Q und Y die oben angegebene Bedeutungen haben in Anwesenheit einer Base oder einer Lewis-Säure umgesetzt werden.

10. Verfahren zur Herstellung der Verbindungen der Formel **(Ib)** oder ein agrochemisch akzeptables Salz davon gemäß einem der Ansprüche 1 bis 5, indem Verbindungen der allgemeinen Formel **(la)** und **(II)** 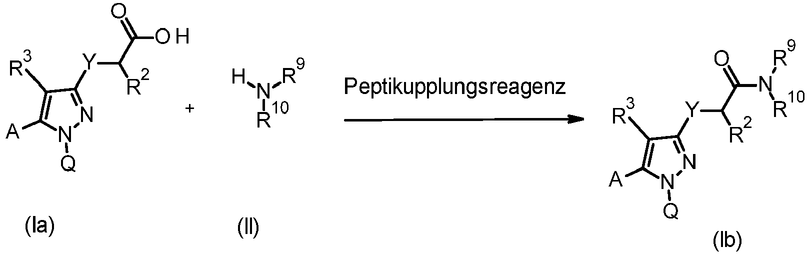 in welcher R⁹, R¹⁰, R², R^{1a}, R³, A, Q und Y die oben angegebene Bedeutungen haben in Gegenwart eines Amidkupplungsreagenzes umgesetzt werden.

11. Agrochemisches Mittel, enthaltend a) mindestens eine Verbindung der Formel **(I)** oder ein agrochemisch akzeptables Salz davon, wie in einem oder mehreren der Ansprüche 1 bis 5 definiert, und b) im Pflanzenschutz übliche Hilfs- und Zusatzstoffe.

12. Agrochemisches Mittel, enthaltend
a) mindestens eine Verbindung der Formel **(I)** oder ein agrochemisch akzeptables Salz davon, wie in einem oder mehreren der Ansprüche 1 bis 5 definiert,
b) einen oder mehrere von Komponente a) verschiedene agrochemische Wirkstoffe, und optional
c) im Pflanzenschutz übliche Hilfs- und Zusatzstoffe.

13. Verfahren zur Bekämpfung von unerwünschten Pflanzen oder zur Wachstumsregulierung von Pflanzen, wobei eine wirksame Menge mindestens einer Verbindung der Formel (I) oder ein agrochemisch akzeptables Salz davon, wie in einem oder mehreren der Ansprüche 1 bis 5 definiert, auf die Pflanzen, das Saatgut oder die Fläche, auf der die Pflanzen wachsen, appliziert wird.

14. Verwendung von Verbindungen der Formel **(I)** oder ein agrochemisch akzeptables Salz davon, wie in einem oder mehreren der Ansprüche 1 bis 5 definiert, als Herbizide oder Pflanzenwachstumsregulatoren.

15. Verwendung nach Anspruch 14, wobei die Verbindungen der Formel **(I)** oder ein agrochemisch akzeptables Salz davon zur Bekämpfung von Schadpflanzen oder zur Wachstumsregulierung in Pflanzenkulturen eingesetzt werden.

16. Verwendung nach Anspruch 15, wobei die Kulturpflanzen transgene oder nicht transgene Kulturpflanzen sind.

## Claims

1. 1-Pyridyl-5-azinylpyrazolyl-3-oxyalkyl acids, 1-pyridyl-5-azinylpyrazolyl-3-sulfanylalkyl acids, 1-pyridyl-5-azinylpyrazolyl-3-sulfinylalkyl acids and 1-pyridyl-5-azinylpyrazolyl-3-sulfonylalkyl acids and derivatives thereof of the general formula (I) and the agrochemically compatible salts, N-oxides, hydrates, and hydrates of the salts and N-oxides thereof, 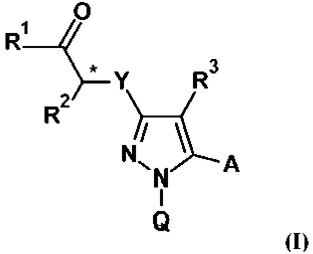 where
A is selected from the group consisting of A1-A15 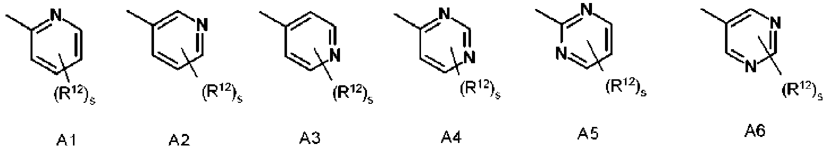 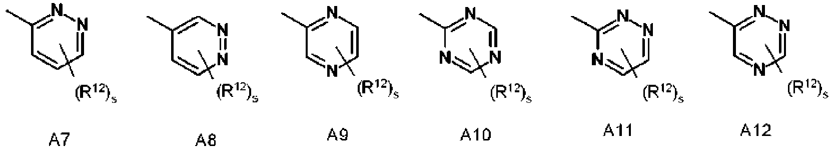 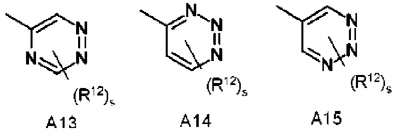
Q is selected from the group consisting of Q1-Q3 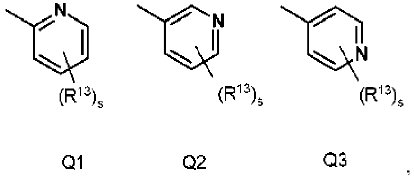
R¹ is -OR^{1a}, -NR⁹R¹⁰ or -O(CH₂)ₓO(CH₂)_{y}O(CH₂)_{z}H;
R^{1a} is hydrogen or
is (C₁-C₆) -alkyl, (C₃-C₆) -cycloalkyl, each of which is unsubstituted or substituted by one or more substituents selected from the group consisting of halogen, (C₁-C₆) - alkyl, (C₁-C₆) -haloalkyl, (C₃-C₆) -cycloalkyl, (C₁-C₆) - alkoxy, (C₁-C₂) -haloalkoxy, (C₁-C₆) -alkoxycarbonyl, cyano and nitro,
or is (C₂-C₄) -alkenyl, CH₂C(COOMe)=CH₂, (C₂-C₄) -alkynyl or is (C₁-C₆) -alkyl-S- (C₁-C₆) -alkyl-, (C₁-C₆) -alkyl-SO- (C₁-C₆) -alkyl-, (C₁-C₆) -alkyl-SO₂- (C₁-C₆) -alkyl- or
is -N= (C₃-C₆) -cycloalkyl, -N=C(CH₃)₂ or
is heterocyclyl, heteroaryl, aryl or
is heterocyclyl-(C₂-C₄)-alkyl-, heteroaryl-(C₂-C₄)-alkyl-, aryl-(C₁-C₄)-alkyl-, each of which is unsubstituted or substituted by one or more substituents selected from the group consisting of halogen, (C₁-C₆) -alkyl, (C₁-C₆)-haloalkyl;
R⁹ is hydrogen, (C₁-C₁₂) -alkyl;
R¹⁰ is hydrogen, aryl, heteroaryl, heterocyclyl, (C₁-C₁₂) -alkyl, (C₃-C₈) -cycloalkyl, (C₃-C₈) -cycloalkyl- (C₁-C₇) -alkyl-, (C₂-C₁₂) -alkenyl, (C₅-C₇) -cycloalkenyl, (C₂-C₁₂)-alkynyl, S(O)ₙR⁵, cyano, nitro, OR⁵, SO₂NR⁶R⁷, CO₂R⁸, COR^{B}, NR⁶R⁸, NR⁶COR⁸, NR⁶CO₂R⁸, NR⁶SO₂R⁸, where the abovementioned alkyl, cycloalkyl, alkenyl, cycloalkenyl and alkynyl radicals are unsubstituted or each substituted independently of one another by "m" radicals selected from the group consisting of hydrogen,
optionally mono- or polysubstituted aryl, halogen, cyano, nitro, OR⁵, S(O)ₙR⁵, SO₂NR⁶R⁷, CO₂R⁸, CONR⁶R⁸, COR⁶, NR⁶R⁸, NR⁶COR⁸, NR⁶CONR⁸R⁸, NR⁶CO₂R⁸, NR⁶SO₂R⁸, NR⁶SO₂NR⁶R⁸, C(R⁶) =NOR⁸;
or
R⁹ and R¹⁰ together with the nitrogen atom to which they are bonded form a saturated or partly or fully unsaturated five-, six- or seven-membered ring which is optionally mono- to hexasubstituted by the following radicals from the group consisting of halogen, (C₁-C₆) - alkyl, (C₁-C₆) -haloalkyl, OR⁵, S(O)ₙR⁵, CO₂R⁸, CONR⁶R⁸, COR⁶ and C (R⁶) =NOR⁸ and which, in addition to this nitrogen atom, contains "r" carbon atoms, "o" oxygen atoms, "p" sulfur atoms and "q" elements from the group consisting of NR⁷, CO and NCOR⁷ as ring atoms;
R⁵ is (C₁-C₆) -alkyl, (C₃-C₆) -cycloalkyl, (C₁-C₆)-haloalkyl, aryl;
R⁶ is hydrogen, (C₁-C₆) -alkyl, (C₃-C₆) -cycloalkyl, (C₁-C₆)-haloalkyl, aryl;
R⁷ is hydrogen, (C₁-C₆) -alkyl, (C₃-C₆) -cycloalkyl, (C₃-C₄) -alkenyl, (C₃-C₄) -alkynyl, CH₂-C(O)O(C₁-C₂)-alkyl;
R⁸ is hydrogen, (C₁-C₆) -alkyl, (C₃-C₆) -cycloalkyl, (C₃-C₄)-alkenyl, (C₁-C₆) -alkyl-C (O) O (C₁-C₂) -alkyl or (C₃-C₄)-alkynyl;
R² is hydrogen, cyano, (C₁-C₆) -alkyl, (C₁-C₆) -alkoxy-(C₁-C₆) -alkyl-, (C₁-C₆)-haloalkyl, (C₁-C₆) -alkoxy, (C₂-C₆) - alkenyl, (C₂-C₆) -haloalkenyl, (C₂-C₆) -alkynyl, (C₂-C₆) - haloalkynyl, (C₃-C₆) -cycloalkyl;
R³ is halogen, cyano, isocyano, NO₂, (C₃-C₆) - cycloalkyl, (C₁-C₆)-alkyl, (C₃-C₆) -halocycloalkyl, (C₁-C₆)-haloalkyl, (C₁-C₆) -alkylcarbonyl-, (C₁-C₆) - haloalkylcarbonyl-, (C₁-C₆) -alkyloxycarbonyl-, (C₂-C₃) - alkenyl, (C₂-C₃) -haloalkenyl, (C₂-C₃) -alkynyl, (C₂-C₃)- haloalkynyl, (C₁-C₆) -alkyl-S(O), and (C₁-C₆) -haloalkylS(O)ₙ, CHO, NH₂;
R¹² is halogen, cyano, isocyano, NO₂, (C₁-C₆) -alkyl, (C₁-C₆)-haloalkyl, (C₁-C₆) -alkylcarbonyl, (C₁-C₆) - haloalkylcarbonyl, (C₁-C₆) -alkyloxycarbonyl, (C₁-C₆) - alkoxy, (C₁-C₆)-haloalkoxy, (C₁-C₆)-alkyl-S(O)ₙ, (C₂-C₃)-alkenyl, (C₂-C₃)-haloalkenyl, (C₂-C₃) -alkynyl, (C₂-C₃)-haloalkynyl, NH₂;
R¹³ is halogen, cyano, isocyano, NO₂, (C₁-C₆) -alkyl, (C₁-C₆) -haloalkyl, (C₁-C₆) -alkylcarbonyl, (C₁-C₆)-haloalkylcarbonyl, (C₁-C₆) -alkyloxycarbonyl, (C₁-C₆)-alkoxy, (C₁-C₆) -haloalkoxy, (C₁-C₆)-alkyl-S(O)ₙ, (C₂-C₃)-alkenyl, (C₂-C₃) -haloalkenyl, (C₂-C₃) -alkynyl, (C₂-C₃)-haloalkynyl, NH₂;
Y is oxygen, S(O)ₙ;
m is 0, 1 or 2;
n is 0, 1 or 2;
o is 0, 1 or 2;
p is 0 or 1;
q is 0 or 1;
r is 3, 4, 5 or 6;
s is 0, 1, 2, 3 or 4;
x is 2 or 3:
y is 2 or 3;
z is 1 or 2.

2. Compounds of the formula (I) according to Claim 1 and the agrochemically compatible salts, N-oxides, hydrates, and hydrates of the salts and N-oxides thereof, where
A is selected from the group consisting of A1-A4, A6, A8, A9, A12, A15 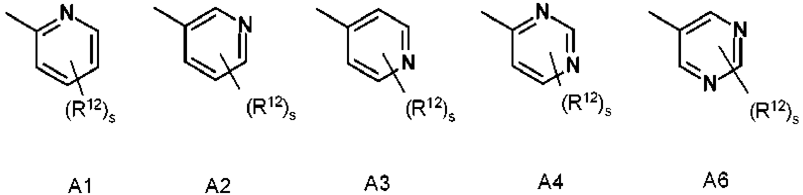 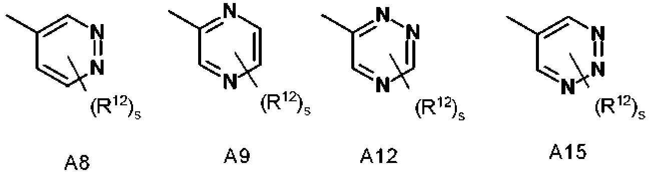
Q is selected from the group consisting of Q1-Q3 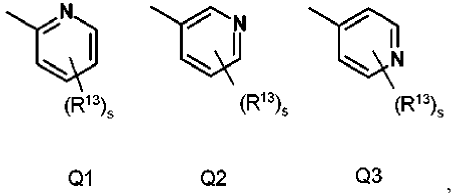
R¹ is -OR^{1a}, -NR⁹R¹⁰ or -O(CH₂)ₓO(CH₂)_{y}O(CH₂)_{z}H;
R^{1a} is hydrogen or
is (C₁-C₃) -alkyl, (C₃-C₆) -cycloalkyl, each of which is unsubstituted or substituted by one or more substituents selected from the group consisting of halogen, (C₁-C₄)-alkyl, (C₁-C₄) -haloalkyl, (C₃-C₆) -cycloalkyl, (C₁-C₄)-alkoxy, (C₁-C₄)-alkoxycarbonyl, cyano,
or is (C₂-C₄) -alkenyl, (C₂-C₄) -alkynyl or
is (C₂-C₃) -alkyl-SMe-, (C₂-C₃) -alkyl-SOMe-, (C₂-C₃) -alkyl-SO₂Me- or
is -N=C(CH₃)₂ or
is heterocyclyl or
is heterocyclyl-(C₂-C₂)-alkyl-, heteroaryl-(C₂-C₂)-alkyl-, aryl-(C₁-C₂)-alkyl-, each of which is unsubstituted or substituted by one or more substituents selected from the group consisting of halogen, (C₁-C₂) -alkyl, (C₁-C₂) - haloalkyl;
R⁹ is hydrogen, (C₁-C₄) -alkyl;
R¹⁰ is (C₁-C₄)-alkyl, (C₂-C₄)-alkenyl, (C₂-C₄)-alkynyl, S(O)ₙR⁵, SO₂NR⁶R⁷, where the abovementioned alkyl, alkenyl and alkynyl radicals are unsubstituted or each substituted independently of one another by "m" radicals selected from the group consisting of halogen, cyano, S(O)ₙR⁵, SO₂NR⁶R⁷, CO₂R⁸, CONR⁶R⁸;
or
R⁹ and R¹⁰ together with the nitrogen atom to which they are bonded form a saturated or partly or fully unsaturated five-, six- or seven-membered ring which is optionally mono- or disubstituted by the following radicals from the group consisting of (C₁-C₄) -alkyl, (C₁-C₄) -haloalkyl, CO₂R⁸ and CONR⁶R⁸;
R⁵ is (C₁-C₄)-alkyl, (C₃-C₆)-cycloalkyl, (C₁-C₄)-haloalkyl, phenyl;
R⁶ is hydrogen, (C₁-C₄)-alkyl, (C₃-C₆)-cycloalkyl, (C₁-C₄)-haloalkyl, phenyl;
R⁷ is hydrogen, (C₁-C₄)-alkyl, (C₃-C₆)-cycloalkyl, (C₃-C₄)-alkenyl, (C₃-C₄)-alkynyl;
R⁸ is hydrogen, (C₁-C₄)-alkyl, (C₃-C₆)-cycloalkyl, (C₃-C₄) -alkenyl, (C₁-C₄)-alkyl-C(O)O(C₁-C₂) -alkyl or (C₃-C₄)-alkynyl;
R² is hydrogen, (C₁-C₃)-alkyl, (C₁-C₃)-alkoxy, (C₂-C₃)-alkenyl, (C₂-C₃)-alkynyl, (C₃-C₆)-cycloalkyl;
R³ is halogen, cyano, isocyano, NO₂, (C₃-C₆)-cycloalkyl, (C₁-C₄)-alkyl, (C₃-C₆)-halocycloalkyl, (C₁-C₄)-haloalkyl, (C₁-C₄)-alkylcarbonyl-, (C₁-C₄)-haloalkylcarbonyl-, (C₁-C₄)-alkyloxycarbonyl-, (C₂-C₃)-alkenyl, (C₂-C₃)-haloalkenyl, (C₂-C₃)-alkynyl, (C₂-C₃)-haloalkynyl, CHO;
R¹² is halogen, cyano, NO₂, (C₁-C₄)-alkyl, (C₁-C₄)-haloalkyl, (C₁-C₄)-alkylcarbonyl, (C₁-C₄)-haloalkylcarbonyl, (C₁-C₄)-alkyloxycarbonyl, (C₁-C₄)-alkoxy, (C₁-C₄)-haloalkoxy, (C₂-C₃)-alkenyl, (C₂-C₃)-haloalkenyl, (C₂-C₃)-alkynyl, (C₂-C₃)-haloalkynyl, NH₂; R¹³ is halogen, cyano, NO₂, (C₁-C₄)-alkyl, (C₁-C₄)-haloalkyl, (C₁-C₄)-alkylcarbonyl, (C₁-C₄)-haloalkylcarbonyl, (C₁-C₄)-alkyloxycarbonyl, (C₁-C₄)-alkoxy, (C₁-C₄)-haloalkoxy, (C₂-C₃)-alkenyl, (C₂-C₃)-haloalkenyl, (C₂-C₃)-alkynyl, (C₂-C₃)-haloalkynyl, NH₂;
Y is oxygen, S(O)ₙ;
m is 0 or 1;
n is 0, 1 or 2;
s is 0, 1, 2 or 3;
x is 2:
y is 2;
z is 1.

3. Compounds of the formula (I) according to Claim 1 or 2 and the agrochemically compatible salts, N-oxides, hydrates, and hydrates of the salts and N-oxides thereof, where
A is selected from the group consisting of A2, A3, A6 and A8 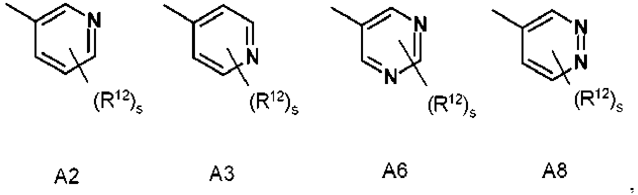
Q is selected from the group consisting of Q1-Q3 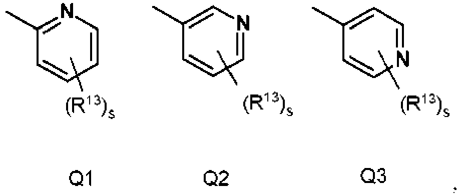
R¹ is -OR^{1a}, -NR⁹R¹⁰ or -O(CH₂)₂O(CH₂)₂OCH₃;
R^{1a} is hydrogen or
is (C₁-C₃)-alkyl which is unsubstituted or substituted by a substituent selected from the group consisting of-C(O)OMe, cyclopropyl, cyclopentyl, methoxy, cyano, trifluoromethyl or
is (C₃-C₆)-cycloalkyl or
is -N=C(CH₃)₂ or
is prop-2-ynyl or
is oxetan-3-yl-(C₁-C₂)-alkyl-, tetrahydrofuran-2-yl-(C₁-C₂)-alkyl-, tetrahydrofuran-3-yl-(C₁-C₂)-alkyl-, pyridin-2-yl- (C₁-C₂)-alkyl-, pyridin-3-yl- (C₁-C₂)-alkyl-, pyridin-4-yl-(C₁-C₂)-alkyl-, phenyl-(C₁-C₂)-alkyl-;
R⁹ is hydrogen;
R¹⁰ is (C₁-C₄)-alkyl where the alkyl radical is unsubstituted or monosubstituted by CO₂R⁸, S(O)₂R⁵ or SO₂NR⁶R⁷ ;
or
R⁹ and R¹⁰ together with the nitrogen atom to which they are bonded form a saturated five-or six-membered ring monosubstituted by CO₂R⁸;
R⁵ is methyl, ethyl or CH₂CF₃;
R⁶ is hydrogen or methyl;
R⁷ is methyl or ethyl;
R⁸ is hydrogen, methyl or ethyl;
R² is hydrogen, methyl or ethyl;
R³ is halogen, cyano, NO₂, (C₃-C₅) -cycloalkyl, (C₃-C₅) - halocycloalkyl, (C₁-C₂) -alkyl, (C₁-C₂)-haloalkyl;
R¹² is fluorine or chlorine;
R¹³ is fluorine, chlorine, methyl, ethyl, methoxy, trifluoromethyl;
Y is oxygen;
s is 0, 1, 2.

4. Compounds of the formula (I) according to any of Claims 1 to 3 and the agrochemically compatible salts, N-oxides, hydrates, and hydrates of the salts and N-oxides thereof, where
A is selected from the group consisting of A2, A3 and A6 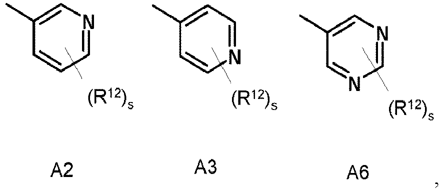
Q is selected from the group consisting of Q1-Q3 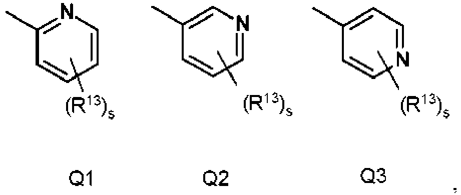
R¹ is -OR^{1a} or -NR⁹R¹⁰;
R^{1a} is hydrogen or
is methyl, ethyl, each of which is unsubstituted or substituted by a substituent selected from the group consisting of -C(O)OMe, cyclopropyl, cyclopentyl, methoxy, cyano, trifluoromethyl or
is -N=C(CH₃)₂ or
is prop-2-ynyl or
is -CH₂-tetrahydrofuran-2-yl, -CH₂-tetrahydrofuran-3-yl, -CH₂-oxetan-3-yl, -CH₂-pyridyl-2-yl, -CH₂-pyridin-3-yl, - CH₂-pyridin-4-yl, -CH₂-phenyl;
R⁹ is hydrogen;
R¹⁰ is methyl or ethyl, where the radicals are monosubstituted by CO₂R⁸;
or
R⁹ and R¹⁰ together with the nitrogen atom to which they are bonded form a cyclopentyl or cyclohexyl radical monosubstituted by CO₂R⁸;
R⁸ is methyl or ethyl;
R² is hydrogen or methyl;
R³ is halogen, cyano, NO₂, cyclopropyl, cyclobutyl, 2,2-difluorocyclopropyl, trifluoromethyl;
R¹² is fluorine;
R¹³ is fluorine, chlorine, methyl, trifluoromethyl;
Y is oxygen;
s is 0, 1, 2.

5. Compounds of the formula (I) according to any of Claims 1 to 4 and the agrochemically compatible salts, N-oxides, hydrates, and hydrates of the salts and N-oxides thereof, where
A is selected from the group consisting of A2, A3 and A6 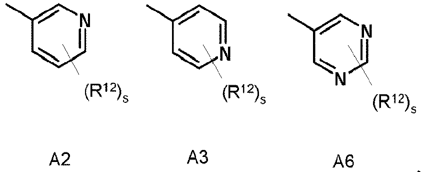
Q is selected from the group consisting of Q1-Q3 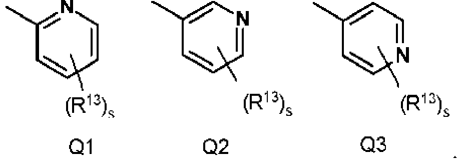
R¹ is -OR^{1a};
R^{1a} is hydrogen, methyl, ethyl;
R² is hydrogen or methyl;
R³ is fluorine, chlorine, bromine, cyano, NO₂, cyclopropyl, trifluoromethyl;
R¹² is fluorine;
R¹³ is fluorine, chlorine, trifluoromethyl;
Y is oxygen;
s is 0, 1, 2.

6. Compounds of the formula (Z) according to any of Claims 1-5 with Q = Q1, R¹ = -OR^{1a} and Y = O and the agrochemically compatible salts, N-oxides, hydrates, and hydrates of the salts and N-oxides thereof, where 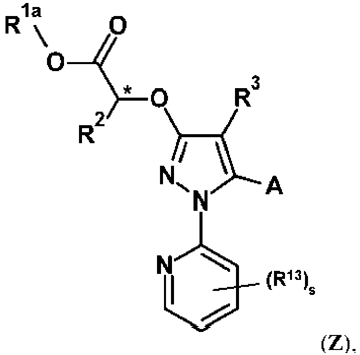 the substituents are defined according to any of Claims 1 to 5.

7. Compounds of the formula (Y) according to any of Claims 1-5 with Q = Q1, Y = O and A = A2 and the agrochemically compatible salts, N-oxides, hydrates, and hydrates of the salts and N-oxides thereof, where 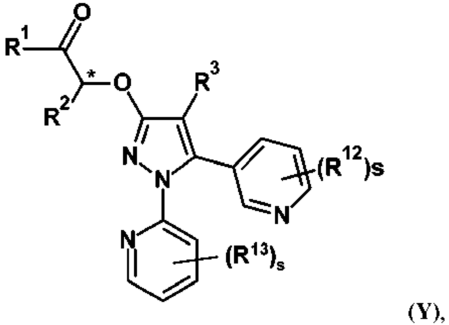 the substituents are defined according to any of Claims 1 to 5.

8. Process for preparing the compounds of the formula (Ic) or an agrochemically acceptable salt thereof according to any of Claims 1 to 5 by converting compounds of the general formulae (III) and (IV) 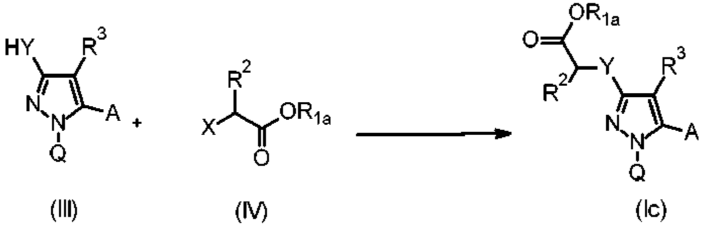 in which R², R^{1a}, R³, A, Q and Y have the definitions given above and X is chlorine, bromine or iodine, in the presence of a thionating reagent, for example phosphorus pentasulfide or Lawesson's reagent.

9. Process for preparing the compounds of the formula (Ia) or an agrochemically acceptable salt thereof according to any of Claims 1 to 5 by converting a compound of the general formula (Ic) 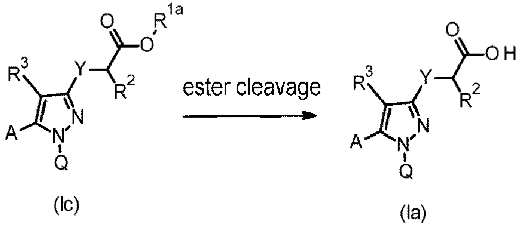 in which R², R^{1a}, R³, A, Q and Y have the definitions given above, in the presence of a base or a Lewis acid.

10. Process for preparing the compounds of the formula (Ib) or an agrochemically acceptable salt thereof according to any of Claims 1 to 5 by converting compounds of the general formulae (Ia) and (II) 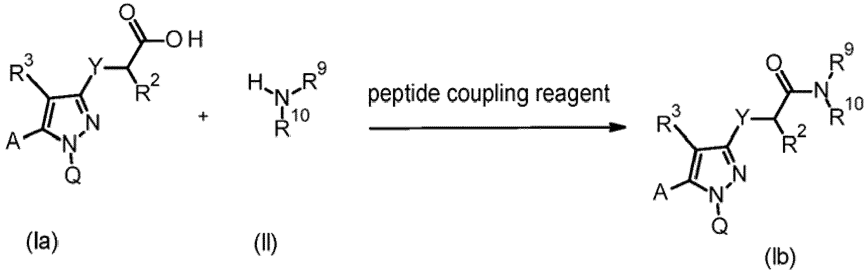 in which R⁹, R¹⁰, R², R^{1a}, R³, A, Q and Y have the definitions given above, in the presence of an amide coupling reagent.

11. Agrochemical composition comprising a) at least one compound of the formula (I) or an agrochemically acceptable salt thereof, as defined in one or more of Claims 1 to 5, and b) auxiliaries and additives customary in crop protection.

12. Agrochemical composition comprising
a) at least one compound of the formula (I) or an agrochemically acceptable salt thereof as defined in one or more of Claims 1 to 5,
b) one or more active agrochemical ingredients other than component a), and optionally
c) auxiliaries and additives customary in crop protection.

13. Method of controlling unwanted plants or of regulating the growth of plants, wherein an effective amount of at least one compound of the formula (I) or an agrochemically acceptable salt thereof, as defined in one or more of Claims 1 to 5, is applied to the plants, the seed or the area in which the plants grow.

14. Use of compounds of the formula (I) or an agrochemically acceptable salt thereof, as defined in one or more of Claims 1 to 5, as herbicides or plant growth regulators.

15. Use according to Claim 14, wherein the compounds of the formula (I) or an agrochemically acceptable salt thereof are used for controlling harmful plants or for regulating growth in plant crops.

16. Use according to Claim 15, wherein the crop plants are transgenic or nontransgenic crop plants.

## Revendications

1. Acides 1-pyridyl-5-azinylpyrazolyl-3-oxyalkyliques, acides 1-pyridyl-5-azinylpyrazolyl-3-sulfanylalkyliques, acides 1-pyridyl-5-azinylpyrazolyl-3-sulfinylalkyliques et acides 1-pyridyl-5-azinylpyrazolyl-3-sulfonylalkyliques ainsi que leurs dérivés de formule générale (I) et leurs sels, N-oxydes, hydrates et hydrates des sels et des N-oxydes, compatibles sur le plan agrochimique, 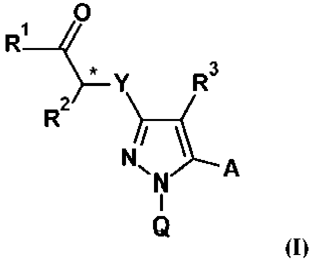 dans laquelle
A est choisi dans le groupe constitué par A1-A15 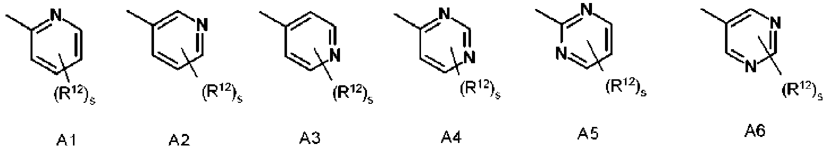 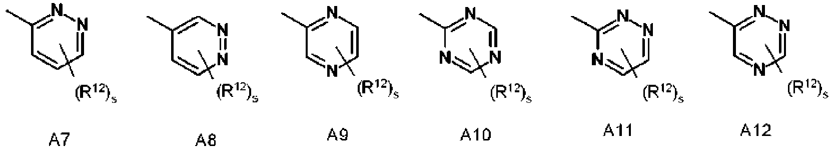 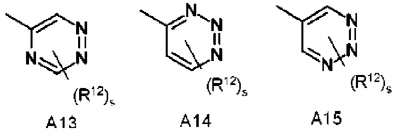
Q est choisi dans le groupe constitué par Q1-Q3 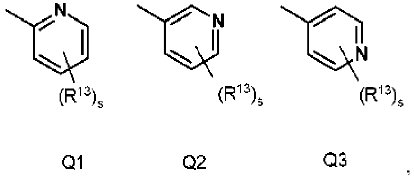
R¹ signifie -OR^{1a}, -NR⁹R¹⁰ ou -O(CH₂)ₓO(CH₂)_{y}O(CH₂)_{z}H ; R^{1a} signifie hydrogène ou
signifie (C₁-C₆) -alkyle, (C₃-C₆) -cycloalkyle, qui est à chaque fois non substitué ou substitué par un ou plusieurs substituants choisis dans le groupe constitué par halogène, (C₁-C₆) -alkyle, (C₁-C₆) -halogénoalkyle, (C₃-C₆) -cycloalkyle, (C₁-C₆) -alcoxy, (C₁-C₂)-halogénoalcoxy, (C₁-C₆)-alcoxycarbonyle, cyano et nitro
ou signifie (C₂-C₄) -alcényle, CH₂C(COOMe)=CH₂, (C₂-C₄)-alcynyle ou signifie (C₁-C₆) -alkyl-S- (C₁-C₆) -alkyle, (C₁-C₆)-alkyl-SO- (C₁-C₆) -alkyle, (C₁-C₆)-alkyl-SO₂-(C₁-C₆) - alkyle ou
signifie -N=(C₃-C₆)-cycloalkyle, -N=C(CH₃)₂ ou
signifie hétérocyclyle, hétéroaryle, aryle ou
signifie hétérocyclyl-(C₂-C₄)-alkyle, hétéroaryl-(C₂-C₄)-alkyle, aryl-(C₁-C₄)-alkyle, qui est à chaque fois non substitué ou substitué par un ou plusieurs substituants choisis dans le groupe constitué par halogène, (C₁-C₆) - alkyle, (C₁-C₆) -halogénoalkyle ;
R⁹ signifie hydrogène, (C₁-C₁₂) -alkyle ;
R¹⁰ signifie hydrogène, aryle, hétéroaryle, hétérocyclyle, (C₁-C₁₂) -alkyle, (C₃-C₈) -cycloalkyle, (C₃-C₈)-cycloalkyl-(C₁-C₇)-alkyle, (C₂-C₁₂) -alcényle, (C₅-C₇)-cycloalcényle, (C₂-C₁₂) -alcynyle, S(O)ₙR⁵, cyano, nitro, OR⁵, SO₂NR⁶R⁷, CO₂R⁸, COR⁸, NR⁶R⁸, NR⁶COR⁸, NR⁶CO₂R⁸, NR⁶SO₂R⁸, les radicaux alkyle, cycloalkyle, alcényle, cycloalcényle et alcynyle susmentionnés étant non substitués ou substitués, à chaque fois indépendamment les uns des autres, par "m" radicaux choisis dans le groupe constitué par hydrogène, aryle le cas échéant monosubstitué ou polysubstitué, halogène, cyano, nitro, OR⁵, S(O)ₙR^{5,} SO₂NR⁶R⁷, CO₂R⁸, CONR⁶R⁸, COR⁶, NR⁶R⁸, NR⁶COR⁸, NR⁶CONR⁸R⁸, NR⁶CO₂R⁸, NR⁶SO₂R⁸, NR⁶SO₂NR⁶R⁸, C(R⁶) =NOR⁸ ;
ou
R⁹ et R¹⁰ forment, avec l'atome d'azote auquel ils sont liés, un cycle le cas échéant monosubstitué à hexasubstitué par les radicaux suivants du groupe constitué par halogène, (C₁-C₆)-alkyle, (C₁₋C₆)-halogénoalkyle, OR⁵, S(O)ₙR⁵, CO₂R⁸, CONR⁶R⁸, COR⁶ et C(R⁶)=NOR⁸, saturé ou partiellement ou totalement insaturé, de cinq, six ou sept chaînons, qui contient, outre cet atome d'azote, "r" atomes de carbone, "o" atomes d'oxygène, "p" atomes de soufre et "q" éléments du groupe constitué par NR⁷, CO et NCOR⁷ en tant qu'atomes de cycle ;
R⁵ signifie (C₁-C₆)-alkyle, (C₃-C₆)-cycloalkyle, (C₁-C₆)-halogénoalkyle, aryle ;
R⁶ signifie hydrogène, (C₁-C₆)-alkyle, (C₃-C₆) - cycloalkyle, (C₁-C₆)-halogénoalkyle, aryle ;
R⁷ signifie hydrogène, (C₁-C₆)-alkyle, (C₃-C₆) - cycloalkyle, (C₃-C₄)-alcényle, (C₃-C₄)-alcynyle, CH₂-C(O)O(C₁-C₂)-alkyle;
R⁸ signifie hydrogène, (C₁-C₆)-alkyle, (C₃-C₆)-cycloalkyle, (C₃-C₄) -alcényle, (C₁-C₆) -alkyl-C (O)O (C₁-C₂) - alkyle ou (C₃-C₄)-alcynyle ;
R² signifie hydrogène, cyano, (C₁-C₆)-alkyle, (C₁-C₆) - alcoxy-(C₁-C₆)-alkyle, (C₁-C₆)-halogénoalkyle, (C₁-C₆)-alcoxy, (C₂-C₆)-alcényle, (C₂-C₆)-halogénoalcényle, (C₂-C₆)-alcynyle, (C₂-C₆)-halogénoalcynyle, (C₃-C₆)-cycloalkyle ;
R³ signifie halogène, cyano, isocyano, NO₂, (C₃-C₆)-cycloalkyle, (C₁-C₆)-alkyle, (C₃-C₆)-halogénocycloalkyle, (C₁-C₆)-halogénoalkyle, (C₁-C₆)-alkylcarbonyle, (C₁-C₆)-halogénoalkylcarbonyle, (C₁-C₆)-alkyloxycarbonyle, (C₂-C₃)-alcényle, (C₂-C₃)-halogénoalcényle, (C₂-C₃)-alcynyle, (C₂-C₃)-halogénoalcynyle, (C₁-C₆)-alkyl-S(O)ₙ et (C₁-C₆)-halogénoalkyl-S(O)ₙ, CHO, NH₂ ;
R¹² signifie halogène, cyano, isocyano, NO₂, (C₁-C₆)-alkyle, (C₁-C₆)-halogénoalkyle, (C₁-C₆)-alkylcarbonyle, (C₁-C₆)-halogénoalkylcarbonyle, (C₁-C₆)-alkyloxycarbonyle, (C₁-C₆)-alcoxy, (C₁-C₆)-halogénoalcoxy, (C₁-C₆)-alkyle, S(O)ₙ, (C₂-C₃)-alcényle, (C₂-C₃)-halogénoalcényle, (C₂-C₃)-alcynyle, (C₂-C₃)-halogénoalcynyle, NH₂ ;
R¹³ signifie halogène, cyano, isocyano, NO₂, (Ci-Ce) - alkyle, (C₁-C₆)-halogénoalkyle, (C₁-C₆)-alkylcarbonyle, (C₁-C₆)-halogénoalkylcarbonyle, (C₁-C₆)-alkyloxycarbonyle, (C₁-C₆)-alcoxy, (C₁-C₆)-halogénoalcoxy, (C₁-C₆)-alkyle, S(O)ₙ, (C₂-C₃)-alcényle, (C₂-C₃)-halogénoalcényle, (C₂-C₃)-alcynyle, (C₂-C₃)-halogénoalcynyle, NH₂ ;
Y signifie oxygène, S(O)ₙ ;
m signifie 0, 1 ou 2 ;
n signifie 0, 1 ou 2 ;
o signifie 0, 1 ou 2 ;
p signifie 0 ou 1 ;
q signifie 0 ou 1 ;
r signifie 3, 4, 5 ou 6 ;
s signifie 0, 1, 2, 3 ou 4 ;
x signifie 2, 3 ;
y signifie 2, 3 ;
z signifie 1, 2.

2. Composés de formule (I) selon la revendication 1 et leurs sels, N-oxydes, hydrates et hydrates des sels et des N-oxydes compatibles sur le plan agrochimique, dans laquelle
A est choisi dans le groupe constitué par A1-A4, A6, A8, A9, A12, A15 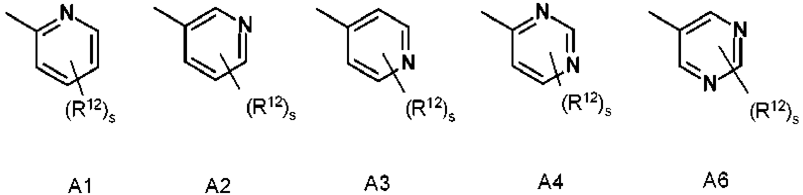 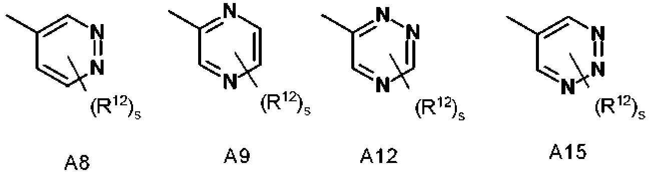
Q est choisi dans le groupe constitué par Q1-Q3 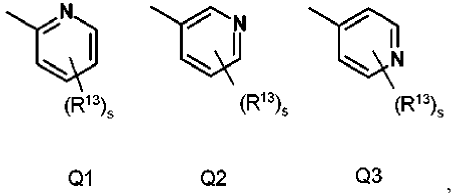
R¹ signifie -OR^{1a}, -NR⁹R¹⁰ ou -O(CH₂)ₓO(CH₂)_{y}O(CH₂)_{z}H ;
R^{1a} signifie hydrogène ou
signifie (C₁-C₃)-alkyle, (C₃-C₆)-cycloalkyle, qui est à chaque fois non substitué ou substitué par un ou plusieurs substituants choisis dans le groupe constitué par halogène, (C₁-C₄)-alkyle, (C₁-C₄)-halogénoalkyle, (C₃-C₆)-cycloalkyle, (C₁-C₄)-alcoxy, (C₁-C₄)-alcoxycarbonyle, cyano
ou (C₂-C₄)-alcényle, (C₂-C₄)-alcynyle ou
signifie (C₂-C₃)-alkyl-SMe-, (C₂-C₃)-alkyl-SOMe-, (C₂-C₃)-alkyl-SO₂Me- ou
signifie -N=C(CH₃)₂ ou
signifie hétérocyclyle ou
signifie hétérocyclyl-(C₁-C₂)-alkyle, hétéroaryl-(C₁-C₂)-alkyle, aryl-(C₁-C₂)-alkyle, qui est à chaque fois non substitué ou substitué par un ou plusieurs substituants choisis dans le groupe constitué par halogène, (C₁-C₂)-alkyle, (C₁-C₂)-halogénoalkyle ;
R⁹ signifie hydrogène, (C₁-C₄)-alkyle ;
R¹⁰ signifie (C₁-C₄)-alkyle, (C₂-C₄)-alcényle, (C₂-C₄)-alcynyle, S(O)ₙR⁵, SO₂NR⁶R⁷, les radicaux alkyle, alcényle et alcynyle susmentionnés étant non substitués ou substitués, à chaque fois indépendamment les uns des autres, par "m" radicaux choisis dans le groupe constitué par halogène, cyano, S(O)ₙR⁵, SO₂NR⁶R⁷, CO₂R⁸, CONR⁶R⁸
ou
R⁹ et R¹⁰ forment, avec l'atome d'azote auquel ils sont liés, un cycle le cas échéant monosubstitué ou disubstitué par les radicaux suivants du groupe constitué par (C₁-C₄)-alkyle, (C₁-C₄)-halogénoalkyle, CO₂R⁸ et CONR⁶R⁸, saturé, partiellement ou complètement insaturé, de cinq, six ou sept chaînons ;
R⁵ signifie (C₁-C₄)-alkyle, (C₃-C₆)-cycloalkyle, (C₁-C₄)-halogénoalkyle, phényle ;
R⁶ signifie hydrogène, (C₁-C₄)-alkyle, (C₃-C₆)-cycloalkyle, (C₁-C₄)-halogénoalkyle, phényle ;
R⁷ signifie hydrogène, (C₁-C₄)-alkyle, (C₃-C₆)-cycloalkyle, (C₃-C₄)-alcényle, (C₃-C₄)-alcynyle ;
R⁸ signifie hydrogène, (C₁-C₄)-alkyle, (C₃-C₆)-cycloalkyle, (C₃-C₄)-alcényle, (C₁-C₄)-alkyl-C(O)O(C₁-C₂)-alkyle ou (C₃-C₄)-alcynyle ;
R² signifie hydrogène, (C₁-C₃)-alkyle, (C₁-C₃)-alcoxy, (C₂-C₃)-alcényle, (C₂-C₃)-alcynyle, (C₃-C₆)-cycloalkyle ; R³ signifie halogène, cyano, isocyano, NO₂, (C₃-C₆)-cycloalkyle, (C₁-C₄)-alkyle, (C₃-C₆)-halogénocycloalkyle, (C₁-C₄)-halogénoalkyle, (C₁-C₄)-alkylcarbonyle, (C₁-C₄)-halogénoalkylcarbonyle, (C₁-C₄)-alkyloxycarbonyle, (C₂-C₃)-alcényle, (C₂-C₃)-halogénoalcényle, (C₂-C₃)-alcynyle, (C₂-C₃)-halogénoalcynyle, CHO ;
R¹² signifie halogène, cyano, NO₂, (C₁-C₄)-alkyle, (C₁-C₄)-halogénoalkyle, (C₁-C₄)-alkylcarbonyle, (C₁-C₄)-halogénoalkylcarbonyle, (C₁-C₄)-alkyloxycarbonyle, (C₁-C₄)-alcoxy, (C₁-C₄)-halogénoalcoxy, (C₂-C₃)-alcényle, (C₂-C₃)-halogénoalcényle, (C₂-C₃)-alcynyle, (C₂-C₃)-halogénoalcynyle, NH₂ ;
R¹³ signifie halogène, cyano, NO₂, (C₁-C₄)-alkyle, (C₁-C₄)-halogénoalkyle, (C₁-C₄)-alkylcarbonyle, (C₁-C₄)-halogénoalkylcarbonyle, (C₁-C₄)-alkyloxycarbonyle, (C₁-C₄)-alcoxy, (C₁-C₄)-halogénoalcoxy, (C₂-C₃)-alcényle, (C₂-C₃)-halogénoalcényle, (C₂-C₃)-alcynyle, (C₂-C₃)-halogénoalcynyle, NH₂ ;
Y signifie oxygène, S(O)ₙ ;
m signifie 0 ou 1 ;
n signifie 0, 1 ou 2 ;
s signifie 0, 1, 2 ou 3 ;
x signifie 2 ;
y signifie 2 ;
z 1.

3. Composés de formule (I) selon la revendication 1 ou 2 et leurs sels, N-oxydes, hydrates et hydrates des sels et des N-oxydes compatibles sur le plan agrochimique, dans laquelle
A est choisi dans le groupe constitué par A2, A3, A6 et A8 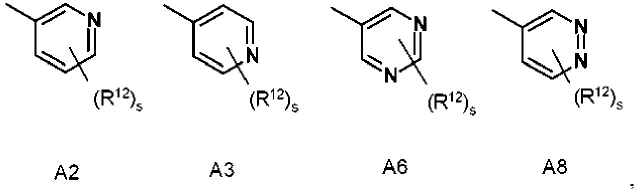
Q est choisi dans le groupe constitué par Q1-Q3 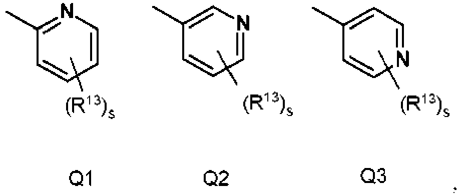
R¹ signifie -OR^{1a}, -NR⁹R¹⁰ ou -O(CH₂)₂O(CH₂)₂OCH₃ ;
R^{1a} signifie hydrogène ou
signifie (C₁-C₃)-alkyle qui est non substitué ou substitué par un substituant choisi dans le groupe constitué par - C(O)OMe, cyclopropyle, cyclopentyle, méthoxy, cyano, trifluorométhyle ou
signifie (C₃-C₆)-cycloalkyle ou
signifie -N=C(CH₃)₂ ou
signifie -prop-2-yn-yle ou
signifie oxétan-3-yl-(C₁-C₂)-alkyle, tétrahydrofurann-2-yl-(C₁-C₂)-alkyle, tétrahydrofurann-3-yl-(C₁-C₂)-alkyle, pyridin-2-yl-(C₁-C₂)-alkyle, pyridin-3-yl-(C₁-C₂)-alkyle, pyridin-4-yl-(C₁-C₂)-alkyle, phényl-(C₁-C₂)-alkyle ;
R⁹ signifie hydrogène ;
R¹⁰ signifie (C₁-C₄)-alkyle, le radical alkyle étant non substitué ou monosubstitué par CO₂R⁸, S(O)₂R⁵ ou SO₂NR⁶R⁷ ; ou
R⁹ et R¹⁰ forment, avec l'atome d'azote auquel ils sont liés, un cycle monosubstitué par CO₂R⁸, saturé, à cinq ou six chaînons ;
R⁵ signifie méthyle, éthyle ou CH₂CF₃ ;
R⁶ signifie hydrogène ou méthyle ;
R⁷ signifie méthyle ou éthyle ;
R⁸ signifie hydrogène, méthyle ou éthyle ;
R² signifie hydrogène, méthyle ou éthyle ;
R³ signifie halogène, cyano, NO₂, (C₃-C₅)-cycloalkyle, (C₃-C₅)-halogénocycloalkyle, (C₁-C₂)-alkyle, (C₁-C₂)-halogénoalkyle ;
R¹² signifie fluor ou chlore ;
R¹³ signifie fluor, chlore, méthyle, éthyle, méthoxy trifluorométhyle ;
Y signifie oxygène ;
s signifie 0, 1, 2.

4. Composés de formule (I) selon l'une des revendications 1 à 3 et leurs sels, N-oxydes, hydrates et hydrates des sels et des N-oxydes compatibles sur le plan agrochimique, dans laquelle
A est choisi dans le groupe constitué par A2, A3 et A6 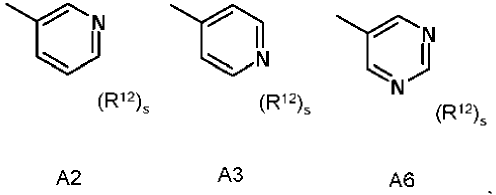
Q est choisi dans le groupe constitué par Q1-Q3 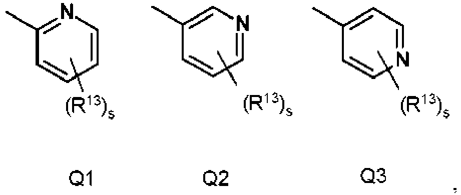
R¹ signifie -OR^{1a} ou -NR⁹R¹⁰,
R^{1a} signifie hydrogène ou
signifie méthyle, éthyle, qui est à chaque fois non substitué ou substitué par un substituant choisi dans le groupe constitué par -C(O)OMe, cyclopropyle, cyclopentyle, méthoxy, cyano, trifluorométhyle ou signifie -N=C(CH₃)₂ ou
signifie -prop-2-yn-yle ou
signifie -CH₂-tétrahydrofurann-2-yle, -CH₂-tétrahydrofurann-3-yle, -CH₂-oxétan-3-yle, -CH₂-pyridyl-2-yle, -CH₂-pyridin-3-yle, -CH₂-pyridin-4-yle, -CH₂-phényle,
R⁹ signifie hydrogène ;
R¹⁰ signifie méthyle ou éthyle, les radicaux étant monosubstitués par CO₂R⁸
ou
R⁹ et R¹⁰ forment, avec l'atome d'azote auquel ils sont liés, un radical cyclopentyle ou cyclohexyle monosubstitué par CO₂R⁸ ;
R⁸ signifie méthyle ou éthyle ;
R² signifie hydrogène ou méthyle ;
R³ signifie halogène, cyano, NO₂, cyclopropyle, cyclobutyle, 2,2-difluorocyclopropyle, trifluorométhyle ;
R¹² signifie fluor ;
R¹³ signifie fluor, chlore, méthyle, trifluorométhyle ;
Y signifie oxygène ;
s signifie 0, 1, 2.

5. Composés de formule (I) selon l'une des revendication 1 à 4 et leurs sels, N-oxydes, hydrates et hydrates des sels et des N-oxydes compatibles sur le plan agrochimique, dans laquelle
A est choisi dans le groupe constitué par A2, A3 et A6 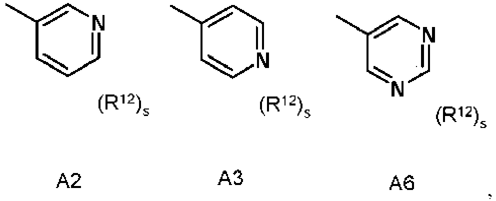
Q est choisi dans le groupe constitué par Q1-Q3 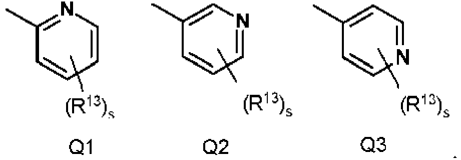
R¹ signifie -OR^{1a}
R^{1a} signifie hydrogène, méthyle ou éthyle ;
R² signifie hydrogène ou méthyle ;
R³ signifie fluor, chlore, brome, cyano, NO₂, cyclopropyle, trifluorométhyle ;
R¹² signifie fluor ;
R¹³ signifie fluor, chlore, trifluorométhyle ;
Y signifie oxygène ;
s signifie 0, 1, 2.

6. Composés de formule (Z) selon l'une des revendications 1-5 avec Q = Q1, R¹ = -OR^{1a} et Y = O, leurs sels, N-oxydes, hydrates et hydrates des sels et des N-oxydes compatibles sur le plan agrochimique, dans laquelle 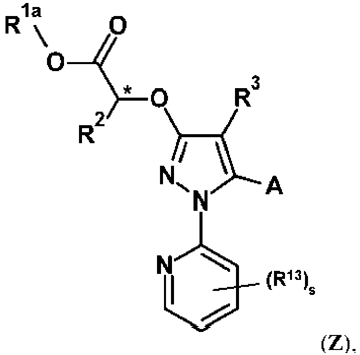 les substituants sont définis selon l'une des revendications 1 à 5.

7. Composés de formule (Y) selon l'une des revendication 1 à 5 avec Q = Q1, Y = O et A = A2 et leurs sels, N-oxydes, hydrates et hydrates des sels et des N-oxydes compatibles sur le plan agrochimique, dans laquelle 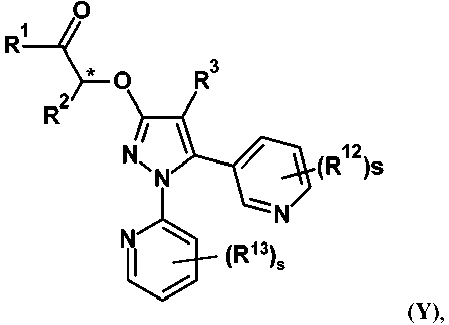 les substituants sont définis selon l'une des revendications 1 à 5.

8. Procédé de préparation des composés de formule (Ic) ou d'un sel acceptable sur le plan agrochimique de ceux-ci selon l'une des revendications 1 à 5, dans lequel des composés de formule générale (III) et (IV), 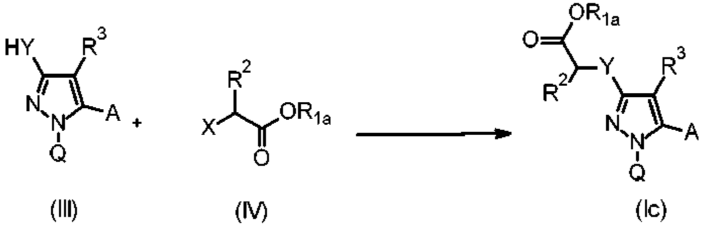 dans lesquelles R², R^{1a}, R³, A, Q et Y présentent la signification indiquée ci-dessus et X représente chlore, brome ou iode, sont transformés en présence d'un réactif de sulfuration, comme par exemple le pentasulfure de phosphore ou le réactif de Lawesson.

9. Procédé de préparation des composés de formule (Ia) ou d'un sel acceptable sur le plan agrochimique de ceux-ci selon l'une des revendications 1 à 5, dans lequel un composé de formule générale (Ic), 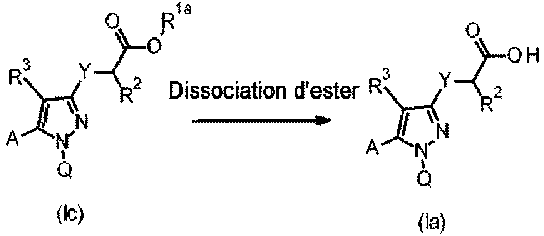 dans laquelle R², R^{1a}, R³, A, Q et Y ont les significations indiquées ci-dessus, est transformé en présence d'une base ou d'un acide de Lewis.

10. Procédé de préparation des composés de formule (Ib) ou d'un sel acceptable sur le plan agrochimique de ceux-ci selon l'une des revendications 1 à 5, dans lequel des composés de formule générale (Ia) et (II) 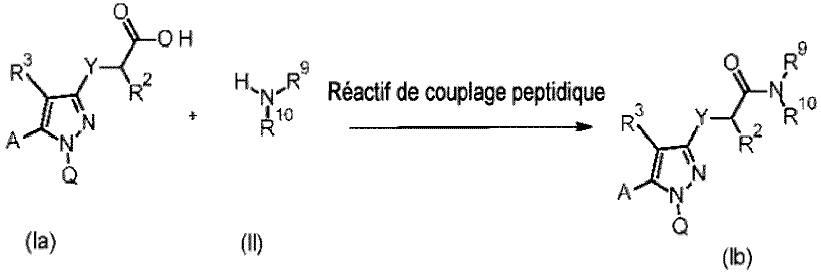 dans lesquelles R⁹, R¹⁰, R², R^{1a}, R³, A, Q et Y présentent les significations indiquées ci-dessus, sont transformés en présence d'un réactif de couplage amide.

11. Agent agrochimique, contenant a) au moins un composé de formule (I) ou un sel acceptable sur le plan agrochimique de celui-ci, tel que défini dans l'une ou plusieurs des revendications 1 à 5, et b) des adjuvants et additifs usuels en phytoprotection.

12. Agent agrochimique, contenant
a) au moins un composé de formule (I) ou un sel acceptable sur le plan agrochimique de celui-ci, tel que défini dans l'une ou plusieurs des revendications 1 à 5,
b) un ou plusieurs principes actifs agrochimiques différents du composant (a) et en option
c) des adjuvants et additifs usuels en phytoprotection.

13. Procédé de lutte contre des plantes indésirables ou de régulation de la croissance de plantes, dans lequel une quantité efficace d'au moins un composé de formule ( I) ou d'un sel acceptable sur le plan agrochimique de celui-ci, tel que défini dans l'une ou plusieurs des revendications 1 à 5, est appliquée sur les plantes, les semences ou la surface sur laquelle poussent les plantes.

14. Utilisation de composés de formule (I) ou d'un sel acceptable sur le plan agrochimique de ceux-ci, tels que définis dans l'une ou plusieurs des revendications 1 à 5, en tant qu'herbicides ou régulateurs de la croissance végétale.

15. Utilisation selon la revendication 14, les composés de formule (I) ou un sel acceptable sur le plan agrochimique de ceux-ci étant utilisés pour la lutte contre des plantes nuisibles ou pour la régulation de la croissance dans des cultures de plantes.

16. Utilisation selon la revendication 15, les plantes cultivées étant des plantes cultivées transgéniques ou non transgéniques.
